# EUROPÄISCHE PATENTSCHRIFT

(11) **EP 2 227 458 B1**
(45) Veröffentlichungstag und Bekanntmachung des Hinweises auf die Patenterteilung: **22.06.2016**
(21) Anmeldenummer: 08854201.4
(22) Anmeldetag: 08.11.2008
(51) Int. Cl.: C07D 277/36, C07D 277/58, C07D 417/12, C07D 417/14, A01N 43/80

(54) **Chirale 2-(Benzylsulfinyl)-Thiazol- und 2-[(1 H-pyrazol-4-ylmethyl)sulfinyl]-Thiazol-Derivate mit S-Konfiguration als herbizide Pflanzenwachstumsregulatoren**
Chiral 2-(Benzylsulfinyl)-thiazol derivatives and 2-[(1H-pyrazol-4-ylmethyl)sulfinyl]-thiazol derivatives with S-configuration as herbicide plant growth regulators
Dérivés chirales de 2-(Benzylsulfinyl)-thiazol et de 2-[(1H-pyrazol-4-ylmethyl)sulfinyl]-thiazol avec configuration S en tant que régulateurs de la croissance de plantes herbicides

(30) Priorität: 30.11.2007 EP 07023198
(43) Veröffentlichungstag der Anmeldung: 15.09.2010
(73) Patentinhaber: Bayer Intellectual Property GmbH, 40789 Monheim am Rhein (DE)
(72) Erfinder: DIETRICH, Hansjörg, 65835 Liederbach (DE); MARTELLETTI, Arianna, 65843 Sulzbach (DE); ROSINGER, Christopher, Hugh, D-65719 Hofheim (DE); DITTGEN, Jan, 60316 Frankfurt (DE); FEUCHT, Dieter, 65760 Eschborn (DE)
(74) Vertreter: BIP Patents
(86) Internationale Anmeldenummer: PCT/EP2008/009437
(87) Internationale Veröffentlichungsnummer: WO 2009/068170

(56) Entgegenhaltungen:
- WO-A-97/20829
- WO-A-2006/123088
- JP-A- 2003 096 059
- VERNIN, G. ET AL.: J. HETEROCYCLIC CHEM., Bd. 15, 1978, Seiten 1361-1366, XP008091148 in der Anmeldung erwähnt
- LEGROS JULIEN ET AL: "Applications of Catalytic Asymmetric Sulfide Oxidations to the Syntheses of Biologically active Sulfoxides" ADVANCED SYNTHESIS AND CATALYSIS, WILEY, WEINHEIM, DE, Bd. 347, 1. Januar 2005 (2005-01-01), Seiten 19-31, XP008091143 ISSN: 1615-4169 in der Anmeldung erwähnt

## Beschreibung

Die vorliegende Erfindung betrifft 2-(Benzylsulfinyl)-Thiazol-Derivate und 2-[(1H-pyrazol-4-ylmethyl)sulfinyl]-Thiazol-Derivate. Weiterer Gegenstand der vorliegenden Erfindung Mischungen der zuvor genannten Thiazol-Derivate mit anderen Herbiziden und/oder Safern. Darüber hinaus berifft die vorliegende Erfindung Verfahren zur Herstellung der zuvor genannten Thiazol-Derivate sowie die Verwendung dieser Verbindungen als Pflanzenwachstumsregulatoren allein und in Mischung mit Safern und/oder in Mischung mit anderen Herbiziden, insbesondere deren Verwendung zur Pflanzenbekämpfung in speziellen Pflanzenkulturen oder als Pflanzenschutzregulatoren.

Aus dem Stand der Technik ist bereits bekannt, dass bestimmte 2-(Benzylsulfinyl)-Thiazol-Derivate und 2-[(1 H-pyrazol-4-ylmethyl)sulfinyl]-Thiazol-Derivate herbizide Eigenschaften besitzen. So sind in der japanischen Patentanmeldungsoffenlegungsschrift JP 2003/096059 herbizid wirksame Thiazol-Derivate beschrieben, welche eine Benzylthio- und Benzylsulfonyl-Gruppe als Substituenten an der 2-Position des Thiazol-Ringes tragen.

WO 2006/123088 beschreibt verschiedene 2-[(Pyrazolylmethyl)thio]-, 2-[(Pyrazolyl-methyl)sulfinyl]- und 2-[(Pyrazolylmethyl)sulfonyl]-Thiazol-Derivate, deren Herstellung sowie deren Verwendung als Herbizide.

Die gemäß den oben benannten Schriften bereits bekannten Wirkstoffe weisen bei ihrer Anwendung jedoch Nachteile auf, sei es,
(a) dass sie keine oder aber eine nur unzureichende herbizide Wirkung gegen Schadpflanzen,
(b) ein zu geringes Spektrum der Schadpflanzen, das mit einem Wirkstoff bekämpft werden kann, oder
(c) eine zu geringe Selektivität in Nutzpflanzenkulturen besitzen.

Insbesondere weisen die aus dem Stand der Technik bekannten herbizid wirkenden Thiazol-Wirkstoffe eine nicht zufriedenstellende herbizide Wirkung gegenüber bestimmten Ungräsern bei einer gleichzeitig nicht zufriedenstellenden Kulturverträglichkeit in in bestimmten Kulturen auf.

Es ist deshalb wünschenswert, alternative chemische Wirkstoffe auf Basis von Thiazol-Derivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Damit ergibt sich im Allgemeinen als Aufgabe der vorliegenden Erfindung, alternative Thiazol-Derivate bereitzustellen, welche als Herbizide oder Pflanzenwachstumsregulatoren, insbesondere mit einer zufriedenstellenden herbiziden Wirkung gegen Schadpflanzen, mit einem breiten Spektrum gegenüber Schadpflanzen und/oder mit einer hohen Selektivität in Nutzpflanzenkulturen, eingesetzt werden können. Diese Thiazol-Derivate sollten dabei vorzugsweise ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen als die aus dem Stand der Technik bekannten Thiazol-Derivate zeigen.

Insbesondere ist es eine Aufgabe der vorliegenden Erfindung, herbizid wirkende Thiazol-Wirkstoffe bereitzustellen, welche eine verbesserte herbizide Wirkung gegen Ungräser im Vergleich zu aus dem Stand der Technik bekannten Thiazol-Derivaten zeigen.

Insbesondere ist es auch eine Aufgabe der vorliegenden Erfindung, herbizid wirkende Thiazol-Wirkstoffe bereitzustellen, welche eine verbesserte Verträglichkeit in spezifischen Kulturen im Vergleich zu aus dem Stand der Technik bekannten Thiazol-Derivaten zeigen.

Insbesondere ist es eine Aufgabe der vorliegenden Erfindung, herbizid wirkende Thiazol-Wirkstoffe bereitzustellen, welche gleichzeitig eine verbesserte herbizide Wirkung gegen bestimmte Ungräser und eine verbesserte Verträglichkeit in spezifischen Kulturen im Vergleich zu aus dem Stand der Technik bekannten Thiazol-Derivaten zeigen.

Erfindungsgemäß wurden nun spezifische, an der Sulfoxid-Funktion optisch aktive 2-(Benzylsulfinyl)-Thiazol-Derivate und 2-[(1 H-pyrazol-4-ylmethyl)sulfinyl]-ThiazolDerivate gefunden, welche Vorteile gegenüber den aus dem Stand der Technik bekannten Verbindungen, beziehungswiese racemischen Mischungen davon, aufweisen.

Erfindungsgemäß wurde gefunden, dass diese erfindungsgemäßen, an der Sulfoxid-Funktion optisch aktiven 2-(Benzylsulfinyl)-Thiazol-Derivate und 2-[(1 H-pyrazol-4-yl-methyl)sulfinyl]-Thiazol-Derivate eine verbesserte herbizide Wirkung gegen bestimmte Ungräser im Vergleich zu aus dem Stand der Technik bekannten Thiazol-Derivaten zeigen.

Erfindungsgemäß wurde ferner gefunden, dass diese eindungsgemäßen, an der Sulfoxid-Funktion optisch aktiven 2-(Benzylsulfinyl)-Thiazol-Derivate und 2-[(1H-pyrazol-4-ylmethyl)sulfinyl]-Thiazol-Derivate eine verbesserte Kulturverträglichkeit in bestimmten Kulturen im Vergleich zu aus dem Stand der Technik bekannten Thiazol-Derivaten zeigen.

Gegenstand der vorliegenden Erfindung sind daher optisch aktive Verbindungen der allgemeinen Formel (I) mit S-Konfiguration , deren agrochemisch verträglichen Salze und deren agrochemisch verträgliche quaternierte Stickstoff-Derivate 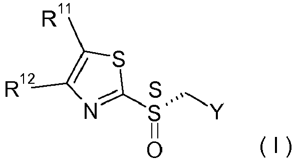 worin
Y entweder 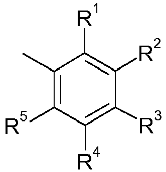 oder 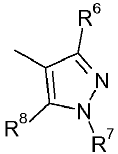 ist und
- die Substituenten R¹ bis R⁸, jeweils unabhängig voneinander, ausgewählt sind aus der Gruppe, bestehend aus
   - Wasserstoff, Halogen, Hydroxy, Cyano, Nitro, Amino, C(O)OH, Formyl,
   - (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl, (C₁-C₆)-Haloalkylcarbonyl-(C₁-C₄)-alkyl, (C₁-C₆)-Alkylcarbonyl-(C₁-C₄)-haloalkyl, (C₁-C₆)-Haloalkylcarbonyl-(C₁-C₄)-haloalkyl,
   - (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl,
   - (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl,
   - (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl,
   - (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy,
   - (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy,
   - (C₆-C₁₄)-Aryl, (C₆-C₁₄)-Aryloxy, (C₆-C₁₄)-Arylcarbonyl, (C₆-C₁₄)-Aryloxycarbonyl,
   - (C₆-C₁₄)-Aryl-(C₁-C₆)-alkyl, (C₆-C₁₄)-Aryl-(C₁-C₆)-alkoxy, (C₆-C₁₄)-Aryloxy-(C₁-C₆)-alkyl, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy,
   - (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy,
   - (C₄-C₁₄)-Arylsulfonyl, (C₆-C₁₄)-Arylthio, (C₆-C₁₄)-Arylsulfinyl,
   - Mono-((C₁-C₆)-alkyl)-amino, Mono-((C₁-C₆)-haloalkyl)-amino, Di-((C₁-C₆)-alkyl)-amino, Di-((C₁-C₆)-haloalkyl)-amino, ((C₁-C₆)-Alkyl-(C₁-C₆)-haloalkyl)-amino, N-((C₁-C₆)-Alkanoyl)-amino, N-((C₁-C₆)-Haloalkanoyl)-amino, Aminocarbonyl-(C₁-C₆)-alkyl, Mono-(C₁-C₆)-Alkylaminocarbonyl-(C₁-C₆)-alkyl, Di-(C₁-C₆)-Alkylaminocarbonyl-(C₁-C₆)-alkyl, Mono-((C₁-C₆)-alkyl)-aminocarbonyl,
   - (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy,
   - (C₃-C₈)-Cycloalkyl, (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy,
   - (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy,
   - (C₃-C₈)-Cycloalkylthio, (C₃-C₈)-Alkenylthio, (C₃-C₈)-Cycloalkenylthio, (C₃-C₆)-Alkinylthio,
   - Hydroxy-(C₁-C₆)-alkyl, Hydroxy-(C₁-C₆)-alkoxy, Cyano-(C₁-C₆)-alkoxy, Cyano-(C₁-C₆)-alkyl,
   - 3-Oxetanyloxy-,
   - C(O)NR⁹R¹⁰ wobei R⁹ und R¹⁰ unabhängig voneinander Wasserstoff, (C₁-C₆)-Alkyl, (C₃-C₆)-Cycloalkyl, (C₁-C₆)-Haloalkyl, oder wo R⁹ und R¹⁰ zusammen eine (C₁-C₆)-Alkylen-Gruppe bilden, die ein Sauerstoff-, oder Schwefel-Atom oder ein oder zwei Amino oder (C₁-C₆)-Alkylamino-Gruppen enthalten kann,
      wobei die genannten Reste gegebenenfalls untereinander zyklisch verknüpft sein können, unter der Voraussetzung, dass sie *ortho-*ständig sind
      und/oder
      zwei ortho-ständige Substituenten zusammen eine (C₁-C₆)-Alkylengruppe bilden, die ein oder mehrere Sauerstoff- und/oder Schwefelatome enthalten können, wobei die (C₁-C₆)-Alkylengruppe durch Halogen einfach oder mehrfach substituiert sein kann und die jeweilgen Halogensubstituenten gleich oder verschieden sein können;
      und
- die Substituenten R¹¹ und R¹², jeweils unabhängig voneinander, ausgewählt sind aus der Gruppe, bestehend aus H, F, Cl, Br, I, Me, CHF₂ und CF₃.

Wenn die Reste, umfassend Cycloalkyl oder Aryl, substituiert sind, so sind die Substituenten vorzugsweise ausgewählt aus der Gruppe, bestehend aus (C₁-C₆)-Alkyl, (C₁-C₆)-Haloalkyl, (C₁-C₆)-Alkoxy, Nitro, Cyano, (C₁-C₃)-Cycloalkyl, (C₁-C₆)-Haloalkoxy, (C₁-C₆)-Alkylthio, (C₁-C₆)-Alkylcarbonyl, (C₁-C₆)-Alkoxycarbonyl oder Halogen, wobei die genannten Reste gegebenenfalls untereinander zyklisch verknüpft sein können, unter der Voraussetzung, dass sie ortho-ständig sind, und

Eine erste Ausführungsform der vorliegenden Erfindung umfasst Verbindungen der allgemeinen Formel (I) mit S-Konfiguration, in denen
- Y: 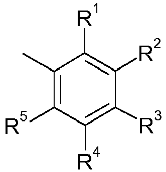 ist und
- R¹ bevorzugt: ausgewählt ist aus der Gruppe, bestehend aus Wasserstoff, Hydroxy, Halogen, Cyano, Nitro, Amino, C(O)OH, (C₁-C₄)-Alkyl, (C₃-C₆)-Cycloalkyl, (C₁-C₄)-Haloalkyl, (C₁-C₄)-Alkoxy, (C₁-C₄)-Haloalkoxy, (C₁-C₄)-Alkoxy-(C₁-C₂)-alkyl, (C₁-C₃)-Alkylcarbonyl, (C₁-C₃)-Alkylcarbonyloxy, (C₁-C₄)-Alkoxycarbonyl, (C₃-C₆)-Cycloalkoxycarbonyl, (C₃-C₆)-Cycloalkyl-(C₁-C₂)-alkoxy, (C₃-C₆)-Cycloalkoxy, (C₁-C₄)-Alkoxycarbonyl-(C₁-C₂)-alkoxy, (C₃-C₄)-Alkenyloxy, (C₃-C₄)-Alkinyloxy, (C₁-C₄)-Alkylthio, (C₁-C₄)-Haloalkylthio, (C₁-C₄)-Alkylsulfinyl, (C₁-C₄)-Haloalkylsulfinyl, (C₁-C₄)-Alkylsulfonyl, (C₁-C₄)-Haloalkylsulfonyl, (C₁-C₄)-Alkylsulfonyloxy, Di-(C₁-C₄)-Alkylamino, C₆-Aryl-(C₁-C₄)-alkyl, (C₃-C₄)-Alkenyloxycarbonyl, (C₂-C₄)-Alkinyloxycarbonyl, C₆-Aryl-(C₁-C₄)-alkoxycarbonyl, C₆-Aryl-(C₁-C₄)-alkoxy, Formyl, (C₂-C₄)-Alkenyl, (C₂-C₄)-Alkinyl, Phenyl, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann;
- R¹ besonders bevorzugt: ausgewählt ist aus der Gruppe, bestehend aus H, F, Cl, Br, I, CN, OH, NH₂, NO₂, Me, Et, Ph, CHF₂, CF₃, OMe, OEt, OPr, OiPr, OBu, OcPen, OcHex, OCHF₂, OCF₃, OCH₂CF₃, C(O)OH, C(O)OMe, C(O)OEt, C(O)OPr, C(O)OiPr, C(O)OBu, C(O)OiBu, C(O)OsBu, C(O)OcPen, C(O)OCH₂CH=CH₂, C(O)OCH₂C≡CH, C(O)OCH₂Ph, CH₂OMe, CH₂OEt, CH₂OBu, OCH₂cPr, OCH₂CH=CH₂, OCH₂C=CH, OCH₂Ph, OCH₂C(O)OMe, OCH₂C(O)OEt, OCH₂CH₂C(O)OMe, OCH₂CH₂C(O)OEt, OC(O)Me, OSO₂Me, S(O)Me, SCF₃, S(O)CF₃ und S(O)₂CF₃;
- R¹ ganz besonders bevorzugt: ausgewählt ist aus der Gruppe, bestehend aus H, F, Cl, Br, Me, CHF₂, CF₃, OMe, OCHF₂, OCF₃, OCH₂CF₃, I und OEt.

Eine zweite Ausführungsform der vorliegenden Erfindung umfasst Verbindungen der allgemeinen Formel (I) mit S-Konfiguration , in denen
- Y: 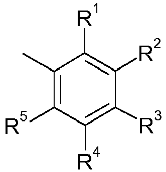 ist und
- R² bevorzugt: ausgewählt ist aus der Gruppe, bestehend aus Wasserstoff, Hydroxy, Halogen, Cyano, Nitro, Amino, C(O)OH, (C₁-C₄)-Alkyl, (C₃-C₆)-Cycloalkyl, (C₁-C₄)-Haloalkyl, (C₁-C₄)-Alkoxy, (C₁-C₄)-Haloalkoxy, (C₁-C₄)-Alkoxy-(C₁-C₂)-alkyl, (C₁-C₃)-Alkylcarbonyl, (C₁-C₃)-Alkylcarbonyloxy, (C₁-C₄)-Alkoxycarbonyl, (C₃-C₆)-Cycloalkoxycarbonyl, (C₃-C₆)-Cycloalkyl-(C₁-C₂)-alkoxy, (C₃-C₆)-Cycloalkoxy, (C₁-C₄)-Alkoxycarbonyl-(C₁-C₂)-alkoxy, (C₃-C₄)-Alkenyloxy, (C₃-C₄)-Alkinyloxy, (C₁-C₄)-Alkylthio, (C₁-C₄)-Haloalkylthio, (C₁-C₄)-Alkylsulfinyl, (C₁-C₄)-Haloalkylsulfinyl, (C₁-C₄)-Alkylsulfonyl, (C₁-C₄)-Haloalkylsulfonyl, (C₁-C₄)-Alkylsulfonyloxy, Di-(C₁-C₄)-Alkylamino, C₆-Aryl-(C₁-C₄)-alkyl, (C₃-C₄)-Alkenyloxycarbonyl, (C₂-C₄)-Alkinyloxycarbonyl, C₆-Aryl-(C₁-C₄)-alkoxycarbonyl, C₆-Aryl-(C₁-C₄)-alkoxy, Formyl, (C₂-C₄)-Alkenyl, (C₂-C₄)-Alkinyl, Phenyl, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann;
- R² besonders bevorzugt: ausgewählt ist aus der Gruppe, bestehend aus H, F, Cl, Br, OH, NO₂, Me, iPr, CHF₂, CF₃, OMe, OEt, OPr, OiPr, OBu, OCHF₂, OCF₃, OCH₂CF₃, C(O)OH und C(O)OMe;
- R² ganz besonders bevorzugt: ausgewählt ist aus der Gruppe, bestehend aus H, F, Cl, Me, CF₃ und OMe.

Eine dritte Ausführungsform der vorliegenden Erfindung umfasst Verbindungen der allgemeinen Formel (I) mit S-Konfiguration , in denen
- Y: 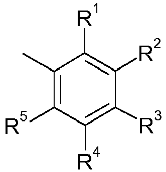 ist und
- R³ bevorzugt: ausgewählt ist aus der Gruppe, bestehend aus Wasserstoff, Hydroxy, Halogen, Cyano, Nitro, Amino, C(O)OH, (C₁-C₄)-Alkyl, (C₃-C₆)-Cycloalkyl, (C₁-C₄)-Haloalkyl, (C₁-C₄)-Alkoxy, (C₁-C₄)-Haloalkoxy, (C₁-C₄)-Alkoxy-(C₁-C₂)-alkyl, (C₁-C₃)-Alkylcarbonyl, (C₁-C₃)-Alkylcarbonyloxy, (C₁-C₄)-Alkoxycarbonyl, (C₃-C₆)-Cycloalkoxycarbonyl, (C₃-C₆)-Cycloalkyl-(C₁-C₂)-alkoxy, (C₃-C₆)-Cycloalkoxy, (C₁-C₄)-Alkoxycarbonyl-(C₁-C₂)-alkoxy, (C₃-C₄)-Alkenyloxy, (C₃-C₄)-Alkinyloxy, (C₁-C₄)-Alkylthio, (C₁-C₄)-Haloalkylthio, (C₁-C₄)-Alkylsulfinyl, (C₁-C₄)-Haloalkylsulfinyl, (C₁-C₄)-Alkylsulfonyl, (C₁-C₄)-Haloalkylsulfonyl, (C₁-C₄)-Alkylsulfonyloxy, Di-(C₁-C₄)-Alkylamino, C₆-Aryl-(C₁-C₄)-alkyl, (C₃-C₄)-Alkenyloxycarbonyl, (C₂-C₄)-Alkinyloxycarbonyl, C₆-Aryl-(C₁-C₄)-alkoxycarbonyl, C₆-Aryl-(C₁-C₄)-alkoxy, Formyl, (C₂-C₄)-Alkenyl, (C₂-C₄)-Alkinyl, Phenyl, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann;
- R³ besonders bevorzugt: ausgewählt ist aus der Gruppe, bestehend aus H, F, Cl, Br, OH, Me, CF₃, OMe, OCHF₂, OCF₃, OCH₂CF₃, C(O)OMe und C(O)OEt; und
- R³ ganz besonders bevorzugt: ausgewählt ist aus der Gruppe, bestehend aus H, F, Cl, Br, CF₃ und Me.

Eine vierte Ausführungsform der vorliegenden Erfindung umfasst Verbindungen der allgemeinen Formel (I) mit S-Konfiguration , in denen
- Y: 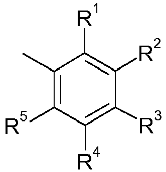 ist und
- R⁴ bevorzugt: ausgewählt ist aus der Gruppe, bestehend aus Wasserstoff, Hydroxy, Halogen, Cyano, Nitro, Amino, C(O)OH, (C₁-C₄)-Alkyl, (C₃-C₆)-Cycloalkyl, (C₁-C₄)-Haloalkyl, (C₁-C₄)-Alkoxy, (C₁-C₄)-Haloalkoxy, (C₁-C₄)-Alkoxy-(C₁-C₂)-alkyl, (C₁-C₃)-Alkylcarbonyl, (C₁-C₃)-Alkylcarbonyloxy, (C₁-C₄)-Alkoxycarbonyl, (C₃-C₆)-Cycloalkoxycarbonyl, (C₃-C₆)-Cycloalkyl-(C₁-C₂)-alkoxy, (C₃-C₆)-Cycloalkoxy, (C₁-C₄)-Alkoxycarbonyl-(C₁-C₂)-alkoxy, (C₃-C₄)-Alkenyloxy, (C₃-C₄)-Alkinyloxy, (C₁-C₄)-Alkylthio, (C₁-C₄)-Haloalkylthio, (C₁-C₄)-Alkylsulfinyl, (C₁-C₄)-Haloalkylsulfinyl, (C₁-C₄)-Alkylsulfonyl, (C₁-C₄)-Haloalkylsulfonyl, (C₁-C₄)-Alkylsulfonyloxy, Di-(C₁-C₄)-Alkylamino, C₆-Aryl-(C₁-C₄)-alkyl, (C₃-C₄)-Alkenyloxycarbonyl, (C₂-C₄)-Alkinyloxycarbonyl, C₆-Aryl-(C₁-C₄)-alkoxycarbonyl, C₆-Aryl-(C₁-C₄)-alkoxy, Formyl, (C₂-C₄)-Alkenyl, (C₂-C₄)-Alkinyl, Phenyl, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann;
- R⁴ besonders bevorzugt: ausgewählt ist aus der Gruppe, bestehend aus H, F, Cl, Br, NO₂, Me, iPr, OMe, OEt, OPr, OiPr, OBu, OCHF₂, CF₃, OCF₃, OCH₂CF₃, OCH₂CH=CH₂ und OCH₂C≡CH; und
- R⁴ ganz besonders bevorzugt: ausgewählt ist aus der Gruppe, bestehend aus H, F, Cl, Me, CF₃ und OMe.

Eine fünfte Ausführungsform der vorliegenden Erfindung umfasst Verbindungen der allgemeinen Formel (I) mit S-Konfiguration , in denen
- Y: 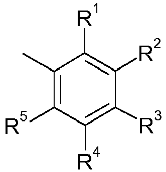 ist und
- R⁵ bevorzugt: ausgewählt ist aus der Gruppe, bestehend aus Wasserstoff, Hydroxy, Halogen, Cyano, Nitro, Amino, C(O)OH, (C₁-C₄)-Alkyl, (C₃-C₆)-Cycloalkyl, (C₁-C₄)-Haloalkyl, (C₁-C₄)-Alkoxy, (C₁-C₄)-Haloalkoxy, (C₁-C₄)-Alkoxy-(C₁-C₂)-alkyl, (C₁-C₃)-Alkylcarbonyl, (C₁-C₃)-Alkylcarbonyloxy, (C₁-C₄)-Alkoxycarbonyl, (C₃-C₆)-Cycloalkoxycarbonyl, (C₃-C₆)-Cycloalkyl-(C₁-C₂)-alkoxy, (C₃-C₆)-Cycloalkoxy, (C₁-C₄)-Alkoxycarbonyl-(C₁-C₂)-alkoxy, (C₃-C₄)-Alkenyloxy, (C₃-C₄)-Alkinyloxy, (C₁-C₄)-Alkylthio, (C₁-C₄)-Haloalkylthio, (C₁-C₄)-Alkylsulfinyl, (C₁-C₄)-Haloalkylsulfinyl, (C₁-C₄)-Alkylsulfonyl, (C₁-C₄)-Haloalkylsulfonyl, (C₁-C₄)-Alkylsulfonyloxy, Di-(C₁-C₄)-Alkylamino, C₆-Aryl-(C₁-C₄)-alkyl, (C₃-C₄)-Alkenyloxycarbonyl, (C₂-C₄)-Alkinyloxycarbonyl, C₆-Aryl-(C₁-C₄)-alkoxycarbonyl, C₆-Aryl-(C₁-C₄)-alkoxy, Formyl, (C₂-C₄)-Alkenyl, (C₂-C₄)-Alkinyl, Phenyl, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann;
- R⁵ besonders bevorzugt: ausgewählt ist aus der Gruppe, bestehend aus H, F, Cl, Br, OH, NO₂, NMe₂, NEt₂, Me, Et, CHF₂, CF₃, OMe, OEt, OPr, OiPr, OBu, OiBu, OCHF₂, OCF₃, OCH₂CF₃, C(O)OH, C(O)OMe, C(O)OEt, C(O)OPr, C(O)OiPr, C(O)OBu, C(O)OiBu, C(O)OsBu, C(O)OCH₂Ph, OCH₂CH=CH₂ und OCH₂C=CH; und
- R⁵ ganz besonders bevorzugt: ausgewählt ist aus der Gruppe, bestehend aus H, F, Cl, Me, CF₃, OCHF₂, OCF₃ und OMe.
Im Rahmen der ersten bis fünften Ausführungsformen der vorliegenden Erfindung ist es möglich, die einzelnen bevorzugten, besonders bevorzugten und ganz besonders bevorzugten Bedeutungen für die Substituenten R¹ bis R⁵ beliebig miteinander zu kombinieren. Das heißt, dass Verbindungen der allgemeinen Formel (I) mit S-Konfiguration mit Y gleich 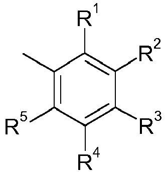 von der vorliegenden Erfindung umfasst sind, in welchen beispielsweise der Substituent R¹ eine bevorzugte Bedeutung aufweist und die Substituenten R² bis R⁵ die allgemeine Bedeutung aufweisen oder aber beispielsweise der Substituent R² eine bevorzugte Bedeutung aufweist, der Substituent R³ eine besonders bevorzugte Bedeutung, der Substituent R⁴ eine ganz besondere Bedeutung und die Substituenten R¹ und R⁵ die allgemeine Bedeutung aufweisen.

Eine sechste Ausführungsform der vorliegenden Erfindung umfasst Verbindungen der allgemeinen Formel (I) mit S-Konfiguration , in denen
- Y: 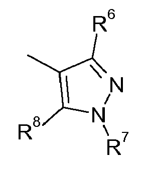 ist und
- R⁶ bevorzugt: ausgewählt ist aus der Gruppe, bestehend aus Wasserstoff, Halogen, Hydroxy, Cyano, Nitro, Amino, (C₁-C₄)-Alkyl, (C₃-C₆)-Cycloalkyl, (C₁-C₄)-Haloalkyl, (C₁-C₄)-Alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-C₄)-Alkylsulfinyl, (C₁-C₄)-Alkylsulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl, Phenyl, Phenyl-(C₁-C₂)-alkyl, Phenyl-(C₁-C₂)-alkoxy, Phenoxy, (C₁-C₄)-Alkylcarbonyloxy, (C₃-C₆)-Cycloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann; und
- R⁶ besonders bevorzugt: ausgewählt ist aus der Gruppe, bestehend aus H, F, Cl, Br, I, CN, Me, Et, Pr, iPr, tBu, CHF₂, CF₃, OMe, OEt, OCHF₂ und OCH₂CF₃; und
- R⁶ ganz besonders bevorzugt: ausgewählt ist aus der Gruppe, bestehend aus F, Cl, Br, CHF₂, CF₃, OCHF₂, OCF₃, OCH₂CF₃, Me, OMe und Et.

Eine siebte Ausführungsform der vorliegenden Erfindung umfasst Verbindungen der allgemeinen Formel (I) mit S-Konfiguration , in denen
- Y: 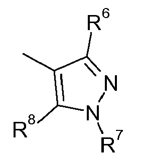 ist und
- R⁷ bevorzugt: ausgewählt ist aus der Gruppe, bestehend aus Wasserstoff, (C₁-C₄)-Alkyl, (C₁-C₄)-Haloalkyl, Phenyl, Phenyl-(C₁-C₂)-alkyl, (C₃-C₆)-Cycloalkyl; (C₃-C₆)-Cycloalkyl-(C₁-C₂)-alkyl, wobei der Cycloalkylrest gegebenenfalls mit (C₁-C₄)-Alkyl substituiert ist; (C₂-C₄)-Alkenyl, (C₂-C₄)-Alkinyl, (C₁-C₄)-Alkoxy-(C₁-C₂)-alkyl, (C₁-C₄)-Alkylthio-(C₁-C₂)-alkyl, (C₁-C₄)-Alkylsulfinyl-(C₁-C₂)-alkyl, Cyano-(C₁-C₂)-alkyl, (C₁-C₄)-Alkylsulfonyl-(C₁-C₂)-alkyl, (C₁-C₄)-Alkoxycarbonyl-(C₁-C₂)-alkyl, Aminocarbonyl-(C₁-C₂)-alkyl, Mono-(C₁-C₄)-Alkylaminocarbonyl-(C₁-C₂)-alkyl, Di-(C₁-C₄)-Alkylaminocarbonyl-(C₁-C₂)-alkyl, Hydroxy-(C₁-C₄)-alkyl, (C₁-C₄)-Alkylcarbonyl-(C₁-C₄)-alkyl, (C₁-C₄)-Alkoxycarbonyl-(C₁-C₂)-alkyl, (C₁-C₄)-Alkylsulfonyl, Phenylsulfonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyl; Phenylcarbonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; und (C₁-C₄)-Alkoxycarbonyl,
- R⁷ besonders bevorzugt: ausgewählt ist aus der Gruppe, bestehend aus H, Me, Et, Pr, cPr, iPr, Bu, iBu, sBu, tBu, cPen, cHex, CHF₂, CH₂CF₃, Ph, Ph(4-Cl), CH₂cPr, CH₂cPr(2-Me), CHMecPr, CH₂cBu, CH₂cPen, CH₂cHex, CH₂Ph, CH₂CH=CH₂, CH₂C≡CH, CHMeC≡CH, CH₂C≡CMe, CH₂OMe, CH₂OEt, CH₂CH₂OH, CH₂CH₂OMe, CH₂CH₂OEt, CH₂CH₂C(O)Me, CH₂SMe, CH₂SO₂Me, CH₂CN, CH₂C(O)OMe, CH₂C(O)OEt, CH₂C(O)NH₂, CH₂C(O)NMe₂, CH₂C(O)Me, SO₂Me, SO₂Ph, C(O)Me, C(O)Ph und C(O)OMe; und
- R⁷ ganz besonders bevorzugt: ausgewählt ist aus der Gruppe, bestehend aus Me, Et und CHF₂.

Eine achte Ausführungsform der vorliegenden Erfindung umfasst Verbindungen der allgemeinen Formel (I) mit S-Konfiguration , in denen
- Y: 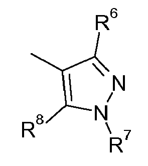 ist und
- R⁸ bevorzugt: ausgewählt ist aus der Gruppe, bestehend aus Wasserstoff, Halogen, Hydroxy, Cyano, Nitro, Amino, (C₁-C₄)-Alkyl, (C₁-C₄)-Haloalkyl, (C₃-C₆)-Cycloalkyl, (C₁-C₄)-Alkoxy, (C₁-C₄)-Alkoxy-(C₁-C₄)-alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-₄)-Alkylsulfinyl, (C₁-C₄)-Alkylsulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Haloalkenyl, Cyano-(C₁-C₄)-alkyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl, Phenyl, Phenyl-(C₁-C₂)-alkyl, Phenyl-(C₁-C₂)-alkoxy, Phenoxy, (C₁-C₄)-Alkylcarbonyloxy, (C₃-C₆)-Cycloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann; und
- R⁸ besonders bevorzugt: ausgewählt ist aus der Gruppe, bestehend aus H, F, Cl, Br, I, CN, Me, Et, CHF₂, CF₃, OCHF₂, OCH₂CF₃, OMe, OEt, OPr, OiPr, OtBu, SO₂Me, SO₂iPr, 3-Oxetanyloxy-, OPh, OCH₂CH=CH₂, OCH₂C≡CH OCH₂CHF₂, SEt, OCH₂CH₂OCH₃, SMe, OCH₂CH₂CH₂F, OCH(CH₂F)₂, OCH₂CF=CH₂, OCH(CH₃)CF₃, OCH₂CN, OCH(CH₃)CH₂F, OCH₂CF₂CHF₂ und OCH(CH₃)₂; und
- R⁸ ganz besonders bevorzugt: ausgewählt ist aus der Gruppe, bestehend aus F, Cl, Br, CHF₂, CF₃, OCHF₂ und OCH₂CF₃.

Im Rahmen dieser sechsten bis achten Ausführungsformen der vorliegenden Erfindung ist es möglich, die einzelnen bevorzugten, besonders bevorzugten und ganz besonders bevorzugten Bedeutungen für die Substituenten R⁶ bis R⁸ miteinander zu kombinieren. Das heißt, dass Verbindungen der allgemeinen Formel (I) mit S-Konfiguration mit Y gleich 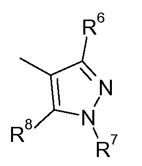 von der vorliegenden Erfindung umfasst sind, in welchen beispielsweise der Substituent R⁶ eine bevorzugte Bedeutung aufweist und die Substituenten R⁷ und R⁸ die allgemeine Bedeutung aufweisen oder aber der Substituent R⁷ eine bevorzugte Bedeutung aufweist, der Substituent R⁸ eine besonders bevorzugte Bedeutung und der Substituent R⁶ eine ganz besondere Bedeutung aufweist.

Die in diesen Ausführungsformen der vorliegenden Erfindung definierten bevorzugten, besonders bevorzugten und ganz besonders bevorzugten Definition für die Reste R¹ bis R⁸ können in beliebiger Kombination mit den im Folgenden als bevorzugt definierten Bedeutungen der Substituenten R¹¹ und R¹² kombiniert werden.

Demgemäß sind bevorzugte, besonders bevorzugte und ganz besonders bevorzugte Bedeutungen der Reste R wie folgt:

Die Bedeutungen der Reste R¹¹ und R¹² können unabhängig voneinander ausgewählt werden aus der Gruppe, bestehend aus H, F, Cl, Br, I, Me, CHF₂, und CF₃, wobei F, Cl, Br und I am bevorzugt sind.

Im Rahmen der vorliegenden Erfindung sind von der Verbindung der allgemeinen Formel (I) mit S-Konfiguration auch Verbindungen umfasst, die durch a) Protonierung, b) Alkylierung oder c) Oxidation an einem Stickstoffatom quaterniert sind.

Die Verbindungen der allgemeinen Formel (I) können gegebenenfalls durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z. B. Amino oder Alkylamino, Salze bilden. Geeignete Substituenten, die in deprotonierter Form, wie z. B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z. B. Sulfonsäuren oder Carbonsäuren, der Wasserstoff durch ein im agrochemischen Bereich geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze mit Kationen der Formel [NRR'R"R"']⁺, worin R bis R'" jeweils unabhängig einen organischen Rest, insbesondere Alkyl, Aryl, Aralkyl oder Alkylaryl darstellen.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, Alkylsulfinyl, Alkylsulfonyl, Haloalkylsulfinyl und Haloalkylsulfonyl, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z. B. Methyl, Ethyl, Propyle wie n- oder i-Propyl, Butyle wie n-, iso- oder tert.-Butyl, Pentyle wie n-Pentyl, iso-Pentyl oder neo-Pentyl, Hexyle wie n-Hexyl, i-Hexyl, 3-Methylpentyl, 2,2-Dimethylbutyl oder 2,3-Dimethylbutyl, Heptyle, wie n-Heptyl, 1-Methylhexy oder 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z. B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z. B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.
Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl. Der Begriff "Halo" wird erfindungsgemäß synonyl zu "Halogen" verwendet.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen (sofern nicht andersweitig definiert), sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder Iod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z. B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z. B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Mit der Definition "mit einem oder mehreren Resten substituiert ist" sind, wenn nicht anders definiert, ein oder mehrere gleiche oder verschiedene Reste gemeint.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bei Resten mit C-Atomen sind solche mit 1 bis 6 C-Atomen, vorzugsweise 1 bis 4 C-Atomen, insbesondere 1 oder 2 C-Atomen, bevorzugt. Bevorzugt sind in der Regel Substituenten aus der Gruppe Halogen, z. B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano.

Wenn ein Arylrest substituiert ist, so kann es sich vorzugsweise um Phenyl, das ein oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)- Halogenalkyl, (C₁-C₄)-Halogenalkoxy, Cyano und Nitro substituiert ist, z. B. o-, m- und p-Tolyl, Dimethylphenyle, 2-, 3- und 4-Chlorphenyl, 2-, 3- und 4-Trifluormethyl und 2-, 3- und 4-Trichlormethyl-phenyl, 2,4-, 3,5-, 2,5- und 2,3-Dichlorphenyl, o-, m- und p-Methoxyphenyl .

Vor allem aus den Gründen der höheren herbiziden Wirkung, besseren Selektivität und/oder besseren Herstellbarkeit sind erfindungsgemäße Verbindungen der allgemeinen Formel (I) oder deren agrochemischen Salze oder quatären N-Derivate von besonderem Interesse, worin einzelne Reste eine der bereits genannten oder im Folgenden genannten bevorzugten Bedeutungen haben, oder insbesondere solche, worin eine oder mehrere der bereits genannten oder im folgenden genannten bevorzugten Bedeutungen kombiniert auftreten.

Die oben angeführten allgemeinen oder in Vorzugsbereichen angeführten Restedefinitionen gelten sowohl für die Endprodukte der allgemeinen Formel (I) als auch entsprechend für die zur Herstellung benötigten Ausgangs- und Zwischenprodukte. Diese Restedefinitionen können untereinander, als auch zwischen den angegebenen bevorzugten Bereichen vertauscht werden.

Die vorliegenden Verbindungen der allgemeinen Formel (I) weisen ein chirales Schwefelatom auf, welches in der oben dargestellten Struktur durch die Kennzeichneung (*) verdeutlicht ist. Gemäß den Regeln nach Cahn, Ingold und Prelog (CIP-Regeln) kann dieses Schwefelatom sowohl eine (R)- als auch eine (S)-Konfiguration aufweisen.

Von der vorliegenden Erfindung werden Verbindungen der allgemeinen Formel (I) mit (S)- Konfiguration erfasst, d.h., dass die vorliegende Erfindung die Verbindungen der allgemeinen Formel (I) erfasst, in welchen das betreffende Schwefelatom eine (S)-Konfiguration
aufweist.

Im Rahmen der vorliegenden Erfindung werden insbesondere Verbindungen der allgemeinen Formel (I) mit (S)-Konfiguration (Verbindungen der allgemeinen Formel (I-S)) im Vergleich zur (R)-Konfiguration (Verbindungen der allgemeinen Formel (I-R)) mit einer Selektivität von 60 bis 100 %, vorzugsweise 80 bis 100 %, insbesondere 90 bis 100 %, ganz besonders bevorzugt 95 bis 100 %, bevorzugt verwendet, wobei die jeweilige (S)-Verbindung mit einer Enantioselektivität von jeweils mehr als 50 % ee, vorzugsweise 60 bis 100 % ee, insbesondere 80 bis 100 % ee, ganz besonders 90 bis 100 % ee, meist bevorzugt 95 bis 100 % ee, bezogen auf den Gesamtgehalt an betreffender (S)-Verbindung vorlliegt, bevorzugt vorliegt.

Daher betrifft die vorliegende Erfindung insbesondere Verbindungen der allgemeinen Formel (I), in welchen die stereochemische Konfiguration an dem mit (*) gekennzeichnet Schwefelatom (S) mit einer stereochemischen Reinheit von 60 bis 100 % (S), vorzugsweise 80 bis 100 % (S), insbesondere 90 bis 100 % (S), ganz besonders 95 bis 100 % (S), vorliegt.

Die Verbindungen der allgemeinen Formel (I) können je nach Art und Verknüpfung der Substituenten weitere Chiralitätszentren als das in Formel (I) mit (*) markierte Schwefelatom enthalten und entsprechend als Stereoisomere vorliegen. Die durch ihre spezifische Raumform definierten möglichen Stereoisomeren, wie Enantiomere, Diastereomere und Z- und E-Isomere, sind im Rahmen der vorliegenden Erfindung vollständig von der Deinition der allgemeinen Formel (I) umfaßt, d.h. dass sowohl die reinen Stereoisomere als auch weniger reine Mischungen davon von der vorliegenden Erfindung erfasst sind. Dabei sind insbesondere Verbindungen bevorzugt, welche an dem mit (*) gekennzeichnet Schwefelatom eine stereochemische Reinheit von 60 bis 100 % (S), vorzugsweise 80 bis 100 % (S), insbesondere 90 bis 100 % (S), ganz besonders 95 bis 100 % (S), aufweisen und an den verbleibenden weiteren Stereozentren in racemischer Form oder in einer mehr oder weniger ausgeprägten stereochemischen Reinheit vorliegen.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Aus-gangs- und/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Weiterer Gegenstand der vorliegenden Erfindung sind Verfahren zur Herstellung entsprechender Verbindungen der allgemeinen Formel (I) und/oder deren Salze und/oder deren agrochemisch verträglichen quaternierten Stickstoff-Derivate:
a) Zur Herstellung von optisch aktiven Sulfoxiden der Formel (I) wird beispielsweise ein Thioether der allgemeinen Formel (II), 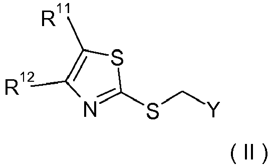 worin Y die gemäß Formel (I) zuvor angegebenen Bedeutungen haben, mit einem Äquivalent eines Oxidationsmittels zu den Sulfoxiden (I) oxidiert werden: 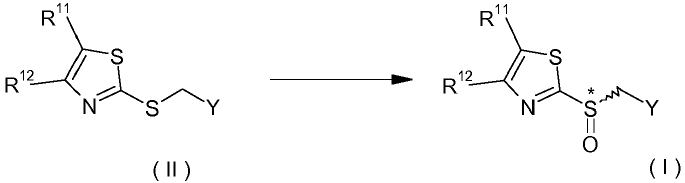
   Die Oxidationsmittel, welche für diese Umsetzung verwendet werrden können, unterliegen keinen besonderen Bestimmungen und es können Oxidationsmittel verwendet werden, welche in der Lage sind, entsprechende Schwefelverbindungen in Sulfoxidverbindungen zu oxidieren. Als Oxidationsmittel zur Herstellung der Sulfoxide sind anorganische Peroxide wie z. B. Wasserstoffperoxid, Natriummetaperjodat, organische Peroxide, wie z. B. tert.-Butylhydroperoxid oder organische Persäuren, wie Peressigsäure oder bevorzugt 3-Chlor-Perbenzoesäure geeignet. Die Reaktion kann in halogenierten Kohlenwasserstoffen, z. B. Dichlormethan, 1,2-Dichlorethan, einem Alkohol, wie z. B. Methanol, oder in Dimethylformamid, Wasser oder Essigsäure oder in einer Mischung der zuvor genannten Lösemittel durchgeführt werden. Die Reaktion kann in einem Temperaturbereich zwischen -80 °C und 120 °C, bevorzugt zwischen -20 °C bis 50 °C durchgeführt. Solche Verfahren sind in der Literatur bekannt und sind z. B. in J. Org. Chem., 58 (1993) 2791, J. Org. Chem., 68 (2003) 3849 und J. Heterocyclic Chem., 15 (1978) 1361 beschrieben, dessen diesbezügliche Offenbarung durch Bezugnahme in die vorliegende Erfindung eingeschlossen wird.
   Die Herstellung der Thioether der allgemeinen Formel (II) ist beispielsweise aus JP 2003/096059 und WO 2006/123088 bekannt.
b) Verbindungen der allgemeinen Formel (I) darüber hinaus können nach Verfahren hergestellt werden, wie sie beispielsweise in JP 2003/096059 und WO 2006/123088, WO 2001/012613, WO 2002/062770, WO 2003/000686, WO 2003/010165, WO 2004/013106, WO 2006/024820, WO 2007/003294, WO 2007/003295 beschrieben sind.
c) Die enantioselektive Synthese von chiralen Sulfoxiden der allgemeinen Formel (I) in optisch angereicherter oder reiner Form kann, ausgehend von Verbindungen der allgemeinen Formel (II), nach Methoden erfolgen, wie sie beispielsweise in Chem. Rev., 103 (2003) 3651-3705, bzw. dort zitierter Literatur, und Adv. Synth. Catal. 347 (2005) 19-31, bzw. dort zitierter Literatur, beschrieben sind. Die absolute Konfiguration des Produkts hängt im Einzelfall von der Struktur des optisch aktiven Katalysators ab.

Die Herstellung entsprechender Salze kann auf dem Fachmann an sich bekannte Weise erfolgen.

Verbindungen der allgemeinen Formel (Ia) 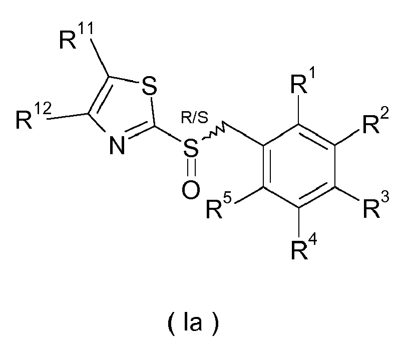 bestehen aus einem Gemisch der jeweiligen, an der Sulfoxid-Funktion chiralen Enantiomeren (Ia-S) und (Ia-R) 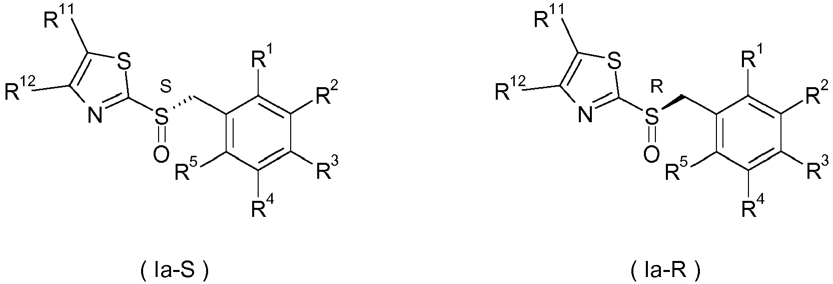 wobei die Reste R¹, R², R³, R⁴, R⁵, R¹¹ und R¹² die gemäß allgemeiner Formel (I) zuvor angegebene Bedeutung haben.

Verbindungen der allgemeinen Formel (Ib) 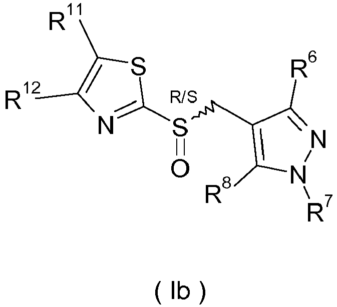 bestehen aus einem Gemisch der jeweiligen, an der Sulfoxid-Funktion chiralen Enantiomeren (Ib-S) und (Ib-R) 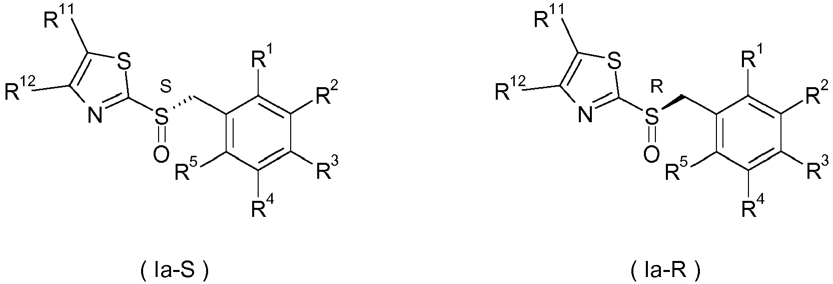 wobei die Reste R⁶, R⁷, R⁸, R¹¹ und R¹² die gemäß allgemeiner Formel (I) zuvor angegebene Bedeutung haben.

Für die Herstellung von Enantiomeren der allgemeinen Formel (I) kommen neben enantioselektiven Synthesen auch übliche Racemattrennungsmethoden in Frage (vgl. Handbücher der Stereochemie).

Racemische Gemische, beispielsweise von optisch aktiven Sulfoxiden der allgemeinen Formel (I), lassen sich nach bekannten Verfahren trennen. Derartige Racemattrennungsmethoden sind in Handbüchern der Stereochemie beschrieben, beispielsweise in "Basic Organic Stereochemistry" (Eds.: Eliel, Ernest L.; Wilen, Samuel H.; Doyle, Michael P.; 2001; John Wiley & Sons) und "Stereochemisty of Organic Compounds (Eds.: Eliel, Ernest L.; Wilen, Samuel H.; Mander, Lewis N.; 1994; John Wiley & Sons), dessen diesbezügliche Offenbarung durch Bezugnahme in die vorliegende Erfindung eingeschlossen wird. In Frage kommen hierbei, z. B. Adduktbildung mit einem optisch aktiven Hilfsreagens, Trennung der diastereomeren Addukte in die entsprechenden Diastereomere, z. B. durch Kristallisation, Chromatographieverfahren, vor allem Säulenchromatographie und Hochdruckflüssigchromatographie, Destillation, gegebenenfalls unter reduziertem Druck, Extraktion und andere Verfahren und anschließende Rückspaltung der Diastereomeren in die Enantiomeren. Für präparative Mengen oder im industriellen Maßstab kommen Verfahren wie die Kristallisation diastereomerer Salze, die aus den Verbindungen (I) mit optisch aktiven Säuren und gegebenenfalls bei vorhandenen sauren Gruppen mit optisch aktiven Basen erhalten werden können, in Frage.

Zur Racemattrennung durch Kristallisation diastereomerer Salze kommen als optisch aktive Säure z. B. Camphersulfonsäure, Camphersäure, Bromcamphersulfonsäure, Chinasäure, Weinsäure, Dibenzoylweinsäure und andere analoge Säuren in Betracht; als optisch aktive Basen kommen z. B. Chinin, Cinchonin, Chinidin, Brucin, 1-Phenylethylamin und andere analoge Basen in Frage.

Die Kristallisationen werden dann meist in wässrigen oder wässrig-organischen Lösemittel durchgeführt, wobei das Diastereomer mit der geringeren Löslichkeit gegebenenfalls nach Animpfen zunächst ausfällt. Das eine Enantiomer der Verbindung der allgemeinen Formel (I) wird danach aus dem ausgefällten Salz oder das andere aus dem Kristallisat durch Ansäuern bzw. mit Base freigesetzt.

Desweiteren können Racemate chromatographisch mit chiralen Stationärphasen getrennt werden. Derartige Enantiomerentrennungen lassen sich vom mg bis in den 100 kg Bereich mit präparativen HPLC Anlagen im Einzel- oder kontinuierlichen Betrieb durchführen.

Mit den in den vorstehenden Verfahrensvarianten bezeichneten "inerten Lösemitteln" sind jeweils Lösemittel gemeint, die unter den jeweiligen Reaktionsbedingungen inert sind, d.h. insbesondere nicht mit den Edukten reagieren, jedoch nicht unter beliebigen Reaktionsbedingungen inert sein müssen.

Bibliotheken aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z. B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen-unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland. Die Reaktionen können beispielsweise auch mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Die erfindungsgemäßen Verbindungen der Formel (I) und deren Salze, im Folgenden synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotylen Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624).

Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259).
- transgene Kulturpflanzen mit modifizierter Fettsäurezusammensetzung (WO 91/013972).
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461)
- gentechnisch veränderte Pflanzen mit reduzierter Photorespiration, die höhere Erträge und höhere Stresstoleranz aufweisen (EP 0305398)
- transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl-oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Als Kombinationspartner für die erfindungsgemäßen Wirkstoffe der allgemeinen Formel (I) in Mischformulierungen oder im Tank-Mix sind beispielsweise bekannte Wirkstoffe, die auf einer Inhibition von beispielsweise

Acetolactat-Synthase, Acetyl-Coenzym-A-Carboxylase, PS I, PS II, HPPDO, Phytoene-Desaturase, Protoporphyrinogen-Oxidase, Glutamine-Synthetase, 5-Enolpyruvylshikimat-3-phosphat-Synthetase oder der Cellulosebiosynthese beruhen, einsetzbar. Solche Verbindungen und auch andere einsetzbare Verbindungen mit teilwiese unbekanntem oder anderem Wirkungsmechanismus sind z. B. in Weed Research 26, 441-445 (1986), oder "The Pesticide Manual", 11. Auflage 1997 (im Folgenden auch kurz "PM") und 12. Auflage 2000, The British Crop Protection Council and the Royal Soc. of Chemistry (Herausgeber), und dort zitierter Literatur beschrieben. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen(-sodium); aclonifen; AKH 7088, d.h. [[[1-[5-[2-Chloro-4-(trifluormethyl)-phenoxy]-2-nitrophenyl]-2-methoxyethylidene]-amino]-oxy]-essigsäure und -essigsäuremethylester; acrolein; alachlor; alloxydim(-sodium); ametryn; amicarbazone, amidochlor, amidosulfuron; aminopyralid, amitrol; AMS, d.h. Ammoniumsulfamat; anilofos; asulam; atraton; atrazin; azafenidin, azimsulfuron (DPX-A8947); aziprotryn; barban; BAS 516 H, d.h. 5-Fluor-2-phenyl-4H-3,1-benz-oxazin-4-on; BCPC; beflubutamid, benazolin(-ethyl); benfluralin; benfuresate; bensulfuron(-methyl); bensulide; bentazone; benzfendizone; benzobicyclon, benzofenap; benzofluor; benzoylprop(-ethyl); benzthiazuron; bifenox; bialaphos; bifenox; bis-pyribac(-sodium), borax; bromacil; bromobutide; bromofenoxim; bromoxynil; bromuron; buminafos; busoxinone; butachlor; butafenacil, butamifos; butenachlor; buthidazole; butralin; butroxydim, butylate; cacodylic acid; calcium chlorate; cafenstrole (CH-900); carbetamide; carfentrazone(-ethyl); caloxydim, CDAA, d.h. 2-Chlor-N,N-di-2-propenylacetamid; CDEC, d.h. Diethyldithiocarbaminsäure-2-chlorallylester; chlorflurenol (-methyl); chlomethoxyfen; clethodim; clomeprop; chloramben; chlorazifop-butyl, chlormesulon; chlorbromuron; chlorbufam; chlorfenac; chlorflurecol-methyl; chloridazon; chlorimuron(-ethyl); chloroacetic acid; chlornitrofen; chlorotoluron; chloroxuron; chlorpropham; chlorsulfuron; chlorthal(-dimethyl); chlorthiamid; chlortoluron, cinidon(-methyl und -ethyl), cinmethylin; cinosulfuron; cis-anilide; clefoxydim, clethodim; clodinafop und dessen Esterderivate (z. B. clodinafop-propargyl); clomazone; clomeprop; cloproxydim; clopyralid; clopyrasulfuron(-methyl); cloransulam(-methyl), cresol; cumyluron (JC 940); cyanamide; cyanazine; cycloate; cyclosulfamuron (AC 104); cycloxydim; cycluron; cyhalofop und dessen Ester-derivate (z. B. Butylester, DEH-112); cyperquat; cyprazine; cyprazole; daimuron; 2,4-D, 2,4-DB, 3,4-DA, 3,4-DB, 2,4-DEB, dalapon; dazomed; desmedipham; desmetryn; di-allate; dicamba; dichlobenil; ortho-dichlorobenzene; para-dichlorobenzene; dichlorprop; dichlorprop-P; diclofop und dessen Ester wie diclofop-methyl; diclosulam, diethatyl(-ethyl); difenoxuron; difenzoquat; difenzoquat-methylsulphate; diflufenican; diflufenzopyr, dimefuron; dimepiperate, dimethachlor; dimethametryn; dimethenamid (SAN-582H); dimethenamid-P; dimethazone, dimexyflam, dimethipin; diemthylarsinic acid; dimetrasulfuron, dinitramine; dinoseb; dinoterb; diphenamid; dipropetryn; diquat; diquat-dibromide; dithiopyr; diuron; DNOC; 3,4-DP; DSMA; EBEP; eglinazine-ethyl; EL77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron(-methyl); ethidimuron; ethiozin; ethofumesate; ethoxyfen und dessen Ester (z. B. Ethylester, HN-252); ethoxysulfuron, etobenzanid (HW 52); F5231, d.h. N-[2-Chlor-4-fluor-5-[4-(3-fluorpropyl)-4,5-dihydro-5-oxo-1H-tetrazol-1-yl]-phenyl]-ethansulfonamid; fenoprop; fenoxan, fenoxaprop und fenoxaprop-P sowie deren Ester, z. B. fenoxaprop-P-ethyl und fenoxaprop-ethyl; fenoxydim; fentrazamide, fenuron; ferrous-sulphate; flamprop(-methyl oder -isopropyl oder - isopropyl-L); flazasulfuron; floazulate, florasulam, fluazifop und fluazifop-P und deren Ester, z. B. fluazifop-butyl und fluazifop-P-butyl; fluazolate; flucarbazone(-sodium), flucetosulfuron; fluchloralin; flufenacet; flufenpyr(-ethyl); flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupropanate, flupyrsulfuron(-methyl oder -sodium), flurenol(-butyl), fluridone; flurochloridone; fluroxypyr(-meptyl); flurprimidol; flurtamone; fluthiacet(-methyl) (KIH-9201); fluthiamide; fomesafen; foramsulfuron; fosamine; furyloxyfen; glufosinate(-ammonium); glyphosate(-isopropylammonium); halosafen; halosulfuron(-methyl) und dessen Ester (z. B. Methylester, NC-319); haloxyfop und dessen Ester; haloxyfop-P (= R-haloxyfop) und dessen Ester; HC-252; hexazinone; imazamethabenz(-methyl); imazapyr; imazaquin und Salze wie das Ammoniumsalz; imazamethapyr, imazamox, imazapic, imazethamethapyr; imazethapyr; imazosulfuron; indanofan, iodomethane; iodosulfuron(methylsodium); ioxynil; isocarbamid; isopropalin; isoproturon; isouron; isoxaben; isoxachlortole, isoxaflutole, isoxapyrifop; karbutilate; lactofen; lenacil; linuron; MAA; MAMA; MCPA; MCPA-2-ethylhexyl; MCPA-thioethyl; MCPB; mecoprop; mecoprop-P; mefenacet; mefluidid; mesosulfuron(-methyl); mesotrione, metamifop; metamitron; metazachlor; methabenzthiazuron; metham; methazole; methoxyphenone; methylarsonic acid; methyldymron; methyl isothiocyanate; metabenzuron, metamifop; methobenzuron; metobromuron; (alpha-)metolachlor; S-metolachlor; metosulam (XRD 511); metoxuron; metribuzin; metsulfuron-methyl; MK-616; MH; molinate; monalide; monocarbamide dihydrogensulfate; monolinuron; monuron; MSMA; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl] -2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; nonanoic acid; norflurazon; oleic acid (fatty acid); orbencarb; orthosulfamuron; oryzalin; oxadiargyl (RP-020630); oxadiazon; oxasulfuron, oxaziclomefone, oxyfluorfen; paraquat; paraquat-dichloride; pebulate; pelargonic acid, pendimethalin; penoxsulam; pentachlorophenol; pentanochlor; pentoxazone, perfluidone; phenisopham; phenmedipham(ethyl); pethoxamid; picloram; picolinafen, pinoxaden, piperophos; piributicarb; pirifenop-butyl; pretilachlor; primisulfuron(-methyl); potassium arsenite; potassium azide; procarbazone-(sodium), procyazine, prodiamine; profluazol; profluralin; profoxydim; proglinazine(-ethyl); prometon; prometryn; propachlor; propanil; propaquizafop und dessen Ester; propazine; propham; propisochlor; propoxycarbazone(-sodium) (BAY MKH 6561); propyzamide; prosulfalin; prosulfocarb; prosulfuron (CGA-152005); prynachlor; pyraclonil; pyraflufen(-ethyl), pyrasulfotole; pyrazolinate; pyrazon; pyrazosulfuron(-ethyl); pyrazoxyfen; pyribambenz-isopropyl; pyribenzoxim, pyributicarb, pyridafol, pyridate; pyriftalid; pyrimidobac(-methyl), pyrimisulfan, pyrithiobac(-sodium) (KIH-2031); pyroxasulfone; pyroxofop und dessen Ester (z. B. Propargylester); pyroxsulam (triflosulam); quinclorac; quinmerac; quinoclamine, quinofop und dessen Esterderivate, quizalofop und quizalofop-P und deren Esterderivate z. B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; SMA; sodium arsenite; sodium azide; sodium chlorate; sulcotrione, sulfentrazon (FMC-97285, F-6285); sulfazuron; sulfometuron(-methyl); sulfosate (ICI-A0224); sulfosulfuron, 2,3,6-TBA; TCA(sodium); tebutam (GCP-5544); tebuthiuron; tefuryltrione, tembotrione, tepraloxydim, terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid_{;} thenylchlor (NSK-850); thiafluamide, thiazafluron; thiazopyr (Mon-13200); thidiazimin (SN-24085); thiencarbazone-methyl, thifensulfuron(-methyl); thiobencarb; tiocarbazil; tralkoxydim; tri-allate; triasulfuron; triaziflam, triazofenamide; tribenuron(-methyl); tricamba; triclopyr; tridiphane; trietazine; trifloxysulfuron(sodium); trifluralin; triflusulfuron und Ester (z. B. Methylester, DPX-66037); trihydroxytriazine; trimeturon; tritosulfuron; tropamezone; tsitodef; vernolate; [3-[2-chloro-4-fluoro-5-(1-methyl-6-trifluormethyl-2,4-dioxo-1,2,3,4-tetrahydropyrimidin-3-yl)phenoxy]-2-pyridyloxy]acetic acid ethyl ester;
WL 110547, d.h. 5-Phenoxy-1-[3-(trifluormethyl)-phenyl]-1 H-tetrazol; UBH-509; D-489; LS 82-556, d.h. [(S)-3-N-(methylbenzyl)carbamoyl-5- propionyl-2,6-lutidine]; KPP-300; NC-324; NC-330; KH-218; DPX-N8189; SC-0774; DOWCO-535; DK-8910; V-53482; PP-600; MBH-001; ET-751, d. h Ethyl-[2-Chlor-5-(4-chlor-5-difluormethoxy-1-methyl-1 H-pyrazol-3-yl)-4-fluor-phenoxy]-acetat; KIH-6127, d.h. Pyriminobac-methyl; KIH-2023, d.h. Bispyribac-Natrium; und SYP-249, d.h Ethyl 2-{2-nitro-5-[(2-chloro-4-trifluoromethyl) phenoxy]-benzoxy}-3-methyl-3-butenoate; SYN-523.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugswiese Getreide. Folgende Gruppen von Verbindungen kommen beispielsweise als Safener für die Verbindungen (I) alleinig oder aber in deren Kombinationen mit wieteren Pestiziden in Frage:
a) Verbindungen der Formeln (S-II) bis (S-IV),
   wobei die Symbole und Indizes folgende Bedeutungen haben: 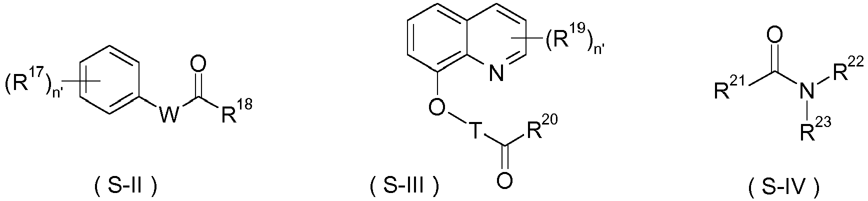
   - n': ist eine natürliche Zahl von 0 bis 5, vorzugsweise 0 bis 3;
   - T: ist eine (C₁ oder C₂)-Alkandiylkette, die unsubstituiert oder mit einem oder zwei (C₁-C₄)-Alkylresten oder mit [(C₁-C₃)-Alkoxy]-carbonyl substituiert ist;
   - W: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W1) bis (W4),

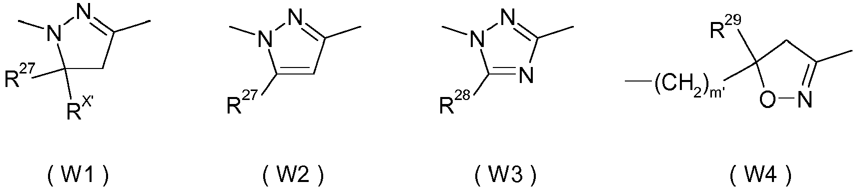

   - m': ist 0 oder 1;
   - R¹⁷, R¹⁹: sind gleich oder verschieden Halogen, (C₁-C₄)-Alkyl, (C₁-C₄)-Alkoxy, Nitro oder (C₁-C₄)-Haloalkyl,
   - R¹⁸, R²⁰: sind gleich oder verschieden OR²⁴, SR²⁴ oder NR²⁴R²⁵ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S-II) bzw. (S-III) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formel OR²⁴, NHR²⁵ oder N(CH₃)₂, insbesondere der Formel OR²⁴;
   - R²⁴: ist Wasserstoff oder ein unsubstituierter oder substituierter aliphatischer Kohlenwasserstoffrest, vorzugsweise mit insgesamt 1 bis 18 C-Atomen;
   - R²⁵: ist Wasserstoff, (C₁-C₆)-Alkyl, (C₁-C₆)-Alkoxy oder substituiertes oder unsubstituiertes Phenyl;
   - R^{X'}: ist H, (C₁-C₈)-Alkyl, (C₁-C₈)-Haloalkyl, (C₁-C₄)-Alkoxy(C₁-C₈)-Alkyl, Cyano oder COOR²⁶, worin R²⁶ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₁₂)-Cycloalkyl oder Tri-(C₁-C₄)-alkyl-silyl ist;
   - R²⁷, R²⁸, R²⁹: sind gleich oder verschieden Wasserstoff, (C₁-C₈)-Alkyl, (C₁-C₈)-Haloalkyl, (C₃-C₁₂)-Cycloalkyl oder substituiertes oder unsubstituiertes Phenyl;
   - R²¹: ist (C₁-C₄)-Alkyl, (C₁-C₄)-Haloalkyl, (C₂-C₄)-Alkenyl, (C₂-C₄)-Haloalkenyl, (C₃-C₇)-Cycloalkyl, vorzugsweise Dichlormethyl;
   - R²², R²³: sind gleich oder verschieden Wasserstoff, (C₁-C₄)-Alkyl, (C₂-C₄)-Alkenyl, (C₂-C₄)-Alkinyl, (C₁-C₄)-Haloalkyl, (C₂-C₄)-Haloalkenyl, (C₁-C₄)-Alkylcarbamoyl-(C₁-C₄)-alkyl, (C₂-C₄)-Alkenylcarbamoyl-(C₁-C₄)-alkyl, (C₁-C₄)-Alkoxy-(C₁-C₄)-alkyl, Dioxolanyl-(C₁-C₄)-alkyl, Thiazolyl, Furyl, Furylalkyl, Thienyl, Piperidyl, substituiertes oder unsubstituiertes Phenyl, oder R²² und R²³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
b) eine oder mehrere Verbindungen aus der Gruppe:
   1,8-Naphthalsäureanhydrid,
   Methyl-diphenylmethoxyacetat,
   1-(2-Chlorbenzyl)-3-(1-methyl-1-phenylethyl)harnstoff (Cumyluron),
   O, O-Diethyl S-2-ethylthioethyl phosphordithioat (Disulfoton),
   4-Chlorphenyl-methylcarbamat (Mephenate),
   O,O-Diethyl-O-phenylphosphorotioat (Dietholate),
   4-Carboxy-3,4-dihydro-2H-1-benzopyran-4-essigsäure (CL-304415, CAS-Regno: 31541-57-8),
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon-O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole),
   2-Dichlormethyl-2-methyl-1,3-dioxolan (MG-191),
   N-(4-Methylphenyl)-N'-(1-methyl-1-phenylethyl)harnstoff (Dymron),
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl 3,6-dichlor-2-methoxybenzoat (Lactidichlor)
   sowie deren Salze und Ester, vorzugsweise (C₁-C₈),
c) N-Acylsulfonamide der Formel (S-V) und ihre Salze, 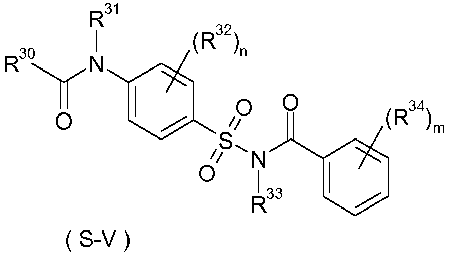 worin
   - R³⁰: Wasserstoff, einen Kohlenwasserstoffrest, einen Kohlenwasserstoffoxyrest, einen Kohlenwasserstoffthiorest oder einen Heterocyclylrest, der vorzugsweise über ein C-Atom gebunden ist, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a'}-R^{a'} substituiert ist, wobei jeder Kohlenwasserstoffteil vorzugsweise 1 bis 20 C-Atome aufweist und ein C-haltiger Rest R³⁰ inklusive Substituenten vorzugsweise 1 bis 30 C-Atome aufweist;
   - R³¹: Wasserstoff oder (C₁-C₄)-Alkyl, vorzugsweise Wasserstoff, oder
   - R³⁰ und R³¹: zusammen mit der Gruppe der Formel -CO-N- den Rest eines 3- bis 8-gliedrigen gesättigten oder ungesättigten Rings;
   - R³²: gleich oder verschieden Halogen, Cyano, Nitro, Amino, Hydroxy, Carboxy, Formyl, CONH₂, SO₂NH₂ oder einen Rest der Formel -Z^{b'}-R^{b'} ;
   - R³³: Wasserstoff oder (C₁-C₄)-Alkyl, vorzugsweise H;
   - R³⁴: gleich oder verschieden Halogen, Cyano, Nitro, Amino, Hydroxy, Carboxy, CHO, CONH₂, SO₂NH₂ oder einen Rest der Formel -Z^{c'}-R^{c'} ;
   - R^{a'}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b'},R^{c'}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)-alkoxy, Mono- und Di-[(C₁-C₄)-alkyl]-amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a'}: eine divalente Gruppe der Formel -O-, -S-, -CO-, -CS-, -CO-O-, -CO-S-, -O-CO-, -S-CO-, -SO-, -SO₂-, -NR*-, -CO-NR*-, -NR*-CO-,-SO₂-NR*- oder -NR*-SO₂-, wobei die rechts angegebene Bindung der jeweiligen divalenten Gruppe die Bindung zum Rest R^{a'} ist und wobei die R* in den letztgenannten 5 Resten unabhängig voneinander jeweils H, (C₁-C₄)-Alkyl oder Halo-(C₁-C₄)-alkyl bedeuten;
   - Z^{b'}, Z^{c'}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR*-, -SO₂-NR*-, -NR*-SO₂-, -CO-NR*-oder -NR*-CO-, wobei die rechts angegebene Bindung der jeweiligen divalenten Gruppe die Bindung zum Rest R^{b'} bzw. R^{c'} ist und wobei die R* in den letztgenannten 5 Resten unabhängig voneinander jeweils H, (C₁-C₄)-Alkyl oder Halo-(C₁-C₄)-alkyl bedeuten;
   - n: eine ganze Zahl von 0 bis 4, vorzugsweise 0, 1 oder 2, insbesondere 0 oder 1,und
   - m: eine ganze Zahl von 0 bis 5, vorzugsweise 0, 1, 2 oder 3, insbesondere 0, 1 oder 2;
   bedeuten;
d) Acylsulfamoylbenzoesäureamide der allgemeinen Formel (S-VI), gegebenenfalls auch in Salzform, 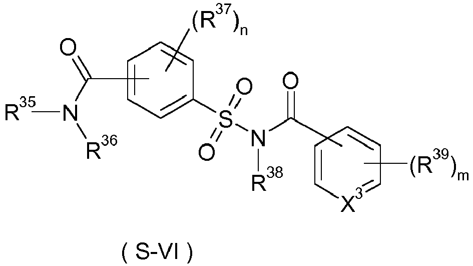 worin
   - X³: CH oder N;
   - R³⁵: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a'}-R^{a'} substituiert sind;
   - R³⁶: Wasserstoff, Hydroxy, (C₁-C₆)-Alkyl, (C₂-C₆)-Alkenyl, (C₂-C₆)-Alkinyl, (C₁-C₆)-Alkoxy, (C₂-C₆)-Alkenyloxy, wobei die fünf letztgenannten Reste gegebenenfalls durch einen oder mehrere, gleiche oder verschiedene Reste aus der Gruppe Halogen, Hydroxy, (C₁-C₄)-Alkyl, (C₁-C₄)-Alkoxy und (C₁-C₄)-Alkylthio substituiert sind, oder
   - R³⁵ und R³⁶: zusammen mit dem sie tragenden Stickstoffatom einen 3- bis 8-gliedrigen gesättigten oder ungesättigten Ring;
   - R³⁷: Halogen, Cyano, Nitro, Amino, Hydroxy, Carboxy, CHO, CONH₂, SO₂NH₂ oder Z^{b'}-R^{b'};
   - R³⁸: Wasserstoff, (C₁-C₄)-Alkyl, (C₂-C₄)-Alkenyl oder (C₂-C₄)-Alkinyl;
   - R³⁹: Halogen, Cyano, Nitro, Amino, Hydroxy, Carboxy, Phosphoryl, CHO, CONH₂, SO₂NH₂ oder Z^{c'}-R^{c'};
   - R^{a'}: einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)-alkyl]-amino substituiert sind;
   - R^{b'},R^{C'}gleich: oder verschieden einen (C₂-C₂₀)-Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)-alkyl]-amino substituiert sind;
   - Z^{a'}: eine divalente Einheit aus der Gruppe O, S, CO, CS, C(O)O, C(O)S, SO, SO₂, NR^{d'}, C(O)NR^{d'} oder SO₂NR^{d'} ;
   - Z^{b'},: Z^{c'} gleich oder verschieden eine direkte Bindung oder eine divalente Einheit aus der Gruppe O, S, CO, CS, C(O)O, C(O)S, SO, SO₂, NR^{d'}, SO₂NR^{d'} oder C(O)NR^{d'};
   - R^{d'}: Wasserstoff, (C₁-C₄)-Alkyl oder (C₁-C₄)-Haloalkyl,
   - N: eine ganze Zahl von 0 bis 4, und
   - m: für den Fall, daß X für CH steht, eine ganze Zahl von 0 bis 5, und für den Fall, daß X für N steht, eine ganze Zahl von 0 bis 4 bedeuten;
e) Verbindungen vom Typ der Acylsulfamoylbenzoesäureamide, z. B. der nachfolgenden Formel (S-VII), die z. B. bekannt sind aus WO 99/16744, 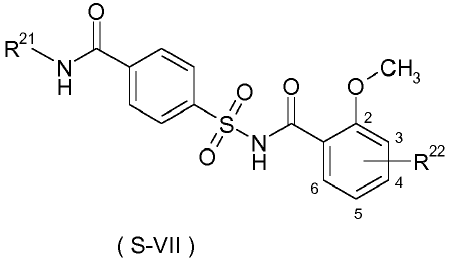 z. B. solche worin
   R²¹ = cyclo-Propyl und R²² = H ist (S-3-1 = 4-Cyclopopylaminocarbonyl-N-(2-methoxybenzoyl)benzolsulfonamid),
   R²¹ = cyclo-Propyl und R²² = 5-CI ist (S-3-2),
   R²¹ = Ethyl und R²² = H ist (S-3-3),
   R²¹ = iso-Propyl und R²² = 5-CI ist (S-3-4) und
   R²¹ = iso-Propyl und R²² = H ist (S-3-5);
f) Verbindungen vom Typ der N-Acylsulfamoylphenylharnstoffe der Formel (S-VIII), die z. B. bekannt sind aus der EP-A-365484, 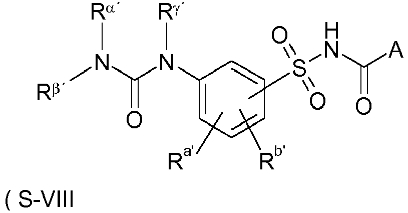 worin
   - A: für einen Rest aus der Gruppe

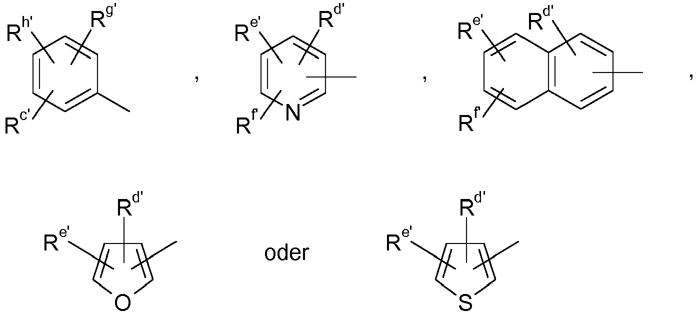

   - R^{α'} und R^{β'}: unabhängig voneinander für Wasserstoff, (C₁-C₈)-Alkyl, (C₃-C₈)-Cycloalkyl, (C₃-C₆)-Alkenyl, (C₃-C₆)-Alkinyl, 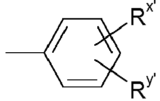 oder durch (C₁-C₄)-Alkoxy oder 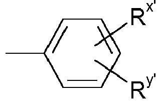 substituiertes (C₁-C₄)-Alkoxy, oder
   - R^{α'} und R^{β'}: gemeinsam für eine (C₄-C₆)-Alkylenbrücke oder eine durch Sauerstoff, Schwefel, SO, SO₂, NH oder -N((C₁-C₄)-Alkyl)- unterbrochene (C₄-C₆)-Alkylenbrücke,
   - R^{y'}: für Wasserstoff oder (C₁-C₄)-Alkyl,
   - R^{a'} und R^{b'}: unabhängig voneinander für Wasserstoff, Halogen, Cyan, Nitro, Trifluormethyl, (C₁-C₄)-Alkyl, (C₁-C₄)-Alkoxy, (C₁-C₄)-Alkylthio, (C₁-C₄)-Alkylsulfinyl, (C₁-C₄)-Alkylsulfonyl, -COOR^{j}, -CONR^{k'}R^{m'}, -COR^{n'},-SO₂NR^{k'}R^{m'} oder -OSO₂-(C₁-C₄)-Alkyl, oder R^{a'} und R^{b'} gemeinsam für eine (C₃-C₄)-Alkylenbrücke, die durch Halogen oder (C₁-C₄)-Alkyl substituiert sein kann, oder eine (C₃-C₄)-Alkenylenbrücke, die durch Halogen oder (C₁-C₄)-Alkyl substituiert sein kann, oder eine C₄-Alkadienylenbrücke, die durch Halogen oder (C₁-C₄)-Alkyl substituiert sein kann, und
   - R^{g'} und R^{h'}: unabhängig voneinander für Wasserstoff, Halogen, (C₁-C₄)-Alkyl, Trifluormethyl, Methoxy, Methylthio oder -COOR^{j'} stehen, wobei
   - R^{c'}: Wasserstoff, Halogen, (C₁-C₄)-Alkyl oder Methoxy,
   - R^{d'}: Wasserstoff, Halogen, Nitro, (C₁-C₄)-Alkyl, (C₁-C₄)-Alkoxy, (C₁-C₄)-Alkylthio, (C₁-C₄)-Alkylsulfinyl, (C₁-C₄)-Alkylsulfonyl, -COOR^{j'} oder-CONR^{k'}R^{m'},
   - R^{e'}: Wasserstoff, Halogen, (C₁-C₄)-Alkyl, -COOR^{j'}, Trifluormethyl oder Methoxy, oder R^{d'} und R^{e'} gemeinsam für eine (C₃-C₄)-Alkylenbrücke,
   - R^{f'}: Wasserstoff, Halogen oder (C₁-C₄)-Alkyl,
   - R^{X'} und R^{Y'}: unabhängig voneinander Wasserstoff, Halogen, (C₁-C₄)-Alkyl, (C₁-C₄)-Alkoxy, (C₁-C₄)-Alkylthio, -COOR^{j'}, Trifluormethyl, Nitro oder Cyan,
   - R^{j'}, R^{k'} und R^{m'}: unabhängig voneinander Wasserstoff oder (C₁-C₄)-Alkyl,
   - R^{k'} und R^{m'}: gemeinsam eine C₄-C₆-Alkylenbrücke oder eine durch Sauerstoff, NH oder -N((C₁-C₄)-Alkyl)- unterbrochene (C₄-C₆)-Alkylenbrücke, und
   - R^{n'}: (C₁-C₄)-Alkyl, Phenyl oder durch Halogen, (C₁-C₄)-Alkyl, Methoxy, Nitro oder Trifluormethyl substituiertes Phenyl bedeuten,
   bevorzugt
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff,
   1-[4-(N-Naphthoylsulfamoyl)phenyl]-3,3-dimethylharnstoff,
g) Verbindungen vom Typ der Acylsulfamoylbenzoesäureamide der Formel (S-IX), bekannt aus EP-A-1019368, gegebenenfalls auch in Salzform, 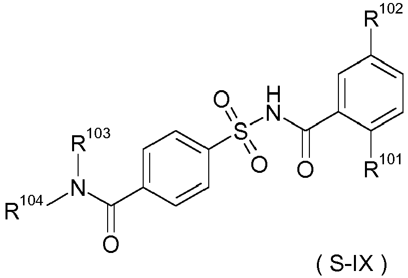 worin
   - R¹⁰¹: bedeutet Methyl, Methoxy oder Trifluormethoxy;
   - R¹⁰²: bedeutet Wasserstoff, Chlor oder Methyl;
   - R¹⁰³: bedeutet Wasserstoff, Ethyl oder Propargyl;
   - R¹⁰⁴: bedeutet Ethyl, Cyclopropyl, iso-Propyl oder Propargyl, oder
   - R¹⁰³ und R¹⁰⁴: bilden gemeinsam die Gruppe (CH₂)₄,
einschließlich der Stereoisomeren und der in der Landwirtschaft gebräuchlichen Salze.

Bevorzugt sind Herbizid-Safener-Kombinationen, enthaltend (A) eine herbizid wirksame Menge an einer oder mehrerer Verbindungen der Formel (I) oder deren Salzen, und (B) eine antidotische wirksame Menge an einem oder mehreren Safenern.

Herbizid wirksame Menge bedeutet im Sinne der Erfindung eine Menge an einem oder mehreren Herbiziden, die geeignet ist, den Pflanzenwuchs negativ zu beeinflussen. Antidotisch wirksame Menge bedeutet im Sinne der Erfindung eine Menge an einem oder mehreren Safenern, die geeignet ist, die phytotoxische Wirkung von Pflanzenschutzmittelwirkstoffen (z. B. von Herbiziden) an Kulturpflanzen zu reduzieren.

Die Verbindungen der Formel (S-II) sind z. B. aus EP-A-0 333 131 (ZA-89/1960), EP-A-0 269 806 (US-A-4,891,057), EP-A-0 346 620 (AU-A-89/34951), EP-A-0 174 562, EP-A-0 346 620 (WO-A-91/08 202), WO-A-91/07 874 oder WO-A 95/07 897 (ZA 94/7120) und der dort zitierten Literatur bekannt oder können nach oder analog den dort beschriebenen Verfahren hergestellt werden. Die Verbindungen der Formel (S-III) sind aus EP-A-0 086 750, EP-A-0 94349 (US-A-4,902,340), EP-A-0 191736 (US-A-4,881,966) und EP-A-0 492 366 und dort zitierter Literatur bekannt oder können nach oder analog den dort beschriebenen Verfahren hergestellt werden. Einige Verbindungen sind ferner in EP-A-0 582 198 und WO 2002/34048 beschrieben.

Die Verbindungen der Formel (S-IV) sind aus zahlreichen Patentanmeldungen bekannt, beispielsweise US-A-4,021,224 und US-A-4,021,229.

Verbindungen der Gruppe B (b) sind weiterhin aus CN-A- 87/102 789, EP-A-365484 sowie aus "The Pesticide Manual", The British Crop Protection Council and the Royal Society of Chemistry, 11th edition, Farnham 1997, bekannt.

Die Verbindungen der Gruppe B (c) sind in der WO-A-97/45016, die der Gruppe B (d) in der WO-A-99/16744, die der Gruppe B (e) in der EP-A-365484 und die der Gruppe B (g) in EP-A-1019368 beschrieben.

Die zitierten Schriften enthalten ausführliche Angaben zu Herstellungsverfahren und Ausgangsmaterialien und nennen bevorzugte Verbindungen. Auf diese Schriften wird ausdrücklich Bezug genommen, sie gelten durch Zitat als Bestandteil dieser Beschreibung.

Bevorzugt sind Herbizid-Safener-Kombinationen, enthaltend Safener der Formel (S-II) und/oder (S-III) bei denen die Symbole und Indizes folgende Bedeutungen haben:
- R²⁴: ist Wasserstoff, (C₁-C₁₈)-Alkyl, (C₃-C₁₂)-Cycloalkyl, (C₂-C₈)-Alkenyl und (C₂-C₁₈)-Alkinyl, wobei die C-haltigen Gruppen durch einen oder mehrere, vorzugsweise bis zu drei, Reste R⁵⁰ substituiert sein können;
- R⁵⁰: ist gleich oder verschieden Halogen, Hydroxy, (C₁-C₈)-Alkoxy, (C₁-C₈)-Alkylthio, (C₂-C₈)-Alkenylthio, (C₂-C₈)-Alkinylthio, (C₂-C₈)-Alkenyloxy, (C₂-C₈)-Alkinyloxy, (C₃-C₇)-Cycloalkyl, (C₃-C₇)-Cycloalkoxy, Cyano, Mono- und Di-((C₁-C₄)-alkyl)-amino, Carboxy, (C₁-C₈)-Alkoxycarbonyl, (C₂-C₈)-Alkenyloxycarbonyl, (C₁-C₈)Alkylthiocarbonyl, (C₂-C₈)-Alkinyloxycarbonyl, (C₁-C₈)-Alkylcarbonyl, (C₂-C₈)-Alkenylcarbonyl, (C₂-C₈)-Alkinylcarbonyl, 1-(Hydroxyimino)-(C₁-C₆)-alkyl, 1-[(C₁-C₄)-Alkylimino]-(C₁-C₄)-alkyl, 1-[(C₁-C₄)-Alkoxyimino]-(C₁-C₆)-alkyl, (C₁-C₈)-Alkylcarbonylamino, (C₂-C₈)-Alkenylcarbonylamino, (C₂-C₈)-Alkinylcarbonylamino, Aminocarbonyl, (C₁-C₈)-Alkylaminocarbonyl, Di-((C₁-C₆)-alkyl)-aminocarbonyl, (C₂-C₆)-Alkenylaminocarbonyl, (C₂-C₆)-Alkinylaminocarbonyl, (C₁-C₈)-Alkoxycarbonylamino, (C₁-C₈)-Alkylaminocarbonylamino, (C₁-C₆)-Alkylcarbonyloxy, das unsubstituiert oder durch R⁵¹ substituiert ist, (C₂-C₆)-Alkenylcarbonyloxy, (C₂-C₆)-Alkinylcarbonyloxy, (C₁-C₈)-Alkylsulfonyl, Phenyl, Phenyl-(C₁-C₆)-alkoxy, Phenyl-(C₁-C₆)-alkoxycarbonyl, Phenoxy, Phenoxy-(C₁-C₆)-alkoxy, Phenoxy-(C₁-C₆)-alkoxycarbonyl, Phenylcarbonyloxy, Phenylcarbonylamino, Phenyl-(C₁-C₆)-alkylcarbonylamino, wobei die letztgenannten 9 Reste im Phenylring unsubstituiert oder ein- oder mehrfach, vorzugsweise bis zu dreifach durch Reste R⁵² substituiert sind; SiR'₃, -O-SiR'₃, R'₃Si-(C₁-C₈)-alkoxy, -CO-O-NR'₂, -O-N=CR'₂, -N=CR'₂, -O-NR'₂, -NR'₂, CH(OR')₂, -O-(CH₂)ₘ -CH(OR')₂, -CR"'(OR')₂, -O-(CH₂)ₘCR'"(OR")₂ oder durch R"O-CHR'"CHCOR"-(C₁-C₆)-alkoxy,
- R⁵¹: ist gleich oder verschieden Halogen, Nitro, (C₁-C₄)-Alkoxy und unsubstituiertes oder mit einem oder mehreren, vorzugsweise bis zu drei Resten R⁵² substituiertes Phenyl;
- R⁵²: ist gleich oder verschieden Halogen, (C₁-C₄)-Alkyl, (C₁-C₄)-Alkoxy, (C₁-C₄)-Haloalkyl, (C₁-C₄)-Haloalkoxy oder Nitro;
- R': ist gleich oder verschieden Wasserstoff, (C₁-C₄)-Alkyl, unsubstituiertes oder durch einen oder mehrere, vorzugsweise bis zu drei, Reste R⁵² substituiertes Phenyl oder zwei Reste R' bilden zusammen eine (C₂-C₆)-Alkandiylkette;
- R": ist gleich oder verschieden (C₁-C₄)-Alkyl oder zwei Reste R" bilden zusammen eine (C₂-C₆)-Alkandiylkette;
- R'": ist Wasserstoff oder (C₁-C₄)-Alkyl,
- m: ist 0, 1, 2, 3, 4, 5 oder 6.

Besonders bevorzugt sind erfindungsgemäße Herbizid-Safener-Kombinationen, enthaltend Safener der Formel (S-II) und/oder (S-III), bei denen die Symbole und Indizes folgende Bedeutungen haben:
- R²⁴: ist Wasserstoff, (C₁-C₈)-Alkyl oder (C₃-C₇)-Cycloalkyl, wobei die vorstehenden C-haltigen Reste unsubstituiert sind oder ein- oder mehrfach durch Halogen oder ein- oder zweifach, vorzugsweise einfach, durch Reste R⁵⁰ substituiert sind,
- R⁵⁰: ist gleich oder verschieden Hydroxy, (C₁-C₄)-Alkoxy, Carboxy, (C₁-C₄)-Alkoxycarbonyl, (C₂-C₆)-Alkenyloxycarbonyl, (C₂-C₆)-Alkinyloxycarbonyl, 1-(Hydroxyimino)-(C₁-C₄)-alkyl, 1-[(C₁-C₄)Alkylimino]-(C₁-C₄)-alkyl und 1-[(C₁-C₄)-Alkoxyimino]-(C₁-C₄)-alkyl, -SiR'₃, -O-N=CR'₂, -N=CR'₂, -NR'₂, und -O-NR'₂, worin R' gleich oder verschieden Wasserstoff, (C₁-C₄)-Alkyl oder paarweise eine (C₄-C₅)-Alkandiylkette bedeutet,
- R²⁷, R²⁸, R²⁹: sind gleich oder verschieden Wasserstoff, (C₁-C₈)-Alkyl, (C₁-C₆)-Haloalkyl, (C₃-C₇)-Cycloalkyl oder Phenyl, das unsubstituiert oder durch einen oder mehrere der Reste aus der Gruppe Halogen, Cyano, Nitro, Amino, Mono- und Di-[(C₁-C₄)-alkyl]-amino, (C₁-C₄)-Alkyl, (C₁-C₄)-Haloalkyl, (C₁-C₄)-Alkoxy, (C₁-C₄)-Haloalkoxy, (C₁-C₄)-Alkylthio und (C₁-C₄)-Alkylsulfonyl substituiert ist;
- R^{x'}: ist Wasserstoff oder COOR²⁴, worin R²⁶ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₇)-Cycloalkyl oder Tri-(C₁-C₄)-alkylsilyl bedeutet,
- R¹⁷, R¹⁹: sind gleich oder verschieden Halogen, Methyl, Ethyl, Methoxy, Ethoxy, (C₁ oder C₂)-Haloalkyl, vorzugsweise Wasserstoff, Halogen oder (C₁ oder C₂)-Haloalkyl.

Ganz besonders bevorzugt sind Safener in welchen die Symbole und Indizes in Formel (S-II) folgende Bedeutungen haben:
- R¹⁷: ist Halogen, Nitro oder (C₁-C₄)-Haloalkyl,
- n': ist 0, 1, 2 oder 3;
- R¹⁸: ist ein Rest der Formel OR²⁴,
- R²⁴: ist Wasserstoff, (C₁-C₈)-Alkyl oder (C₃-C₇)-Cycloalkyl, wobei die vorstehenden C-haltigen Reste unsubstituiert sind oder ein- oder mehrfach, vorzugsweise bis zu dreifach, durch gleiche oder verschiedene Halogen-Reste oder bis zu zweifach, vorzugsweise einfach, durch gleiche oder verschiedene Reste aus der Gruppe Hydroxy, (C₁-C₄)-Alkoxy, (C₁-C₄)-Alkoxycarbonyl, (C₂-C₆)-Alkenyloxycarbonyl, (C₂-C₆)-Alkinyloxycarbonyl, 1-(Hydroxyimino)-(C₁-C₄)-alkyl, 1-[(C₁-C₄)-Alkylimino]-(C₁-C₄)-alkyl, 1-[(C₁-C₄)-Alkoxyimino]-(C₁-C₄)-alkyl und Reste der Formeln -SiR'₃, -O-N=R'₂, -N=CR'₂, -NR'₂ und -O-NR'₂ substituiert sind, wobei die Reste R' in den genannten Formeln gleich oder verschieden Wasserstoff, (C₁-C₄)-Alkyl oder paarweise (C₄ oder C₅)-Alkandiyl bedeuten;
- R²⁷, R²⁸, R²⁹: sind gleich oder verschieden Wasserstoff, (C₁-C₈)-Alkyl, (C₁-C₆)-Haloalkyl, (C₃-C₇)-Cycloalkyl oder Phenyl, das unsubstituiert oder durch einen oder mehrere der Reste aus der Gruppe Halogen, (C₁-C₄)-Alkyl, (C₁-C₄)-Alkoxy, Nitro, (C₁-C₄)-Haloalkyl und (C₁-C₄)-Haloalkoxy substituiert ist, und
- R^{x'}: ist Wasserstoff oder COOR²⁶, worin R²⁶ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₇)-Cycloalkyl oder Tri-(C₁-C₄)-alkylsilyl ist.

Ganz besonders bevorzugt sind auch Safener der Formel (S-III), bei denen die Symbole und Indizes folgende Bedeutungen haben:
- R¹⁹: ist Halogen oder (C₁-C₄)-Haloalkyl,
- n': ist 0, 1, 2 oder 3, wobei (R¹⁹)_{n'} vorzugsweise 5-Cl ist;
- R²⁰: ist ein Rest der Formel OR²⁴;
- T: ist CH₂ oder CH(COO-((C₁-C₃)-Alkyl)) und
- R²⁴: ist Wasserstoff, (C₁-C₈)-Alkyl, (C₁-C₈)-Haloalkyl oder (C₁-C₄)-Alkoxy-(C₁-C₄)-alkyl, vorzugsweise Wasserstoff oder (C₁-C₈)-Alkyl.

Insbesondere bevorzugt sind dabei Safener der Formel (II) bei denen die Symbole und Indizes folgende Bedeutungen haben:
- W: ist (W1);
- R¹⁷: ist Halogen oder (C₁-C₂)-Haloalkyl,
- n': ist 0, 1, 2 oder 3, wobei (R¹⁷)_{n'} vorzugsweise 2,4-Cl₂ ist;
- R¹⁸: ist ein Rest der Formel OR²⁴;
- R²⁴: ist Wasserstoff, (C₁-C₈)-Alkyl, (C₁-C₄)-Haloalkyl, (C₁-C₄)-Hydroxyalkyl, (C₃-C₇)-Cycloalkyl, (C₁-C₄)-Alkoxy-(C₁-C₄)-alkyl oder Tri-(C₁-C₂)-alkylsilyl, vorzugsweise (C₁-C₄)-Alkyl,
- R²⁷: ist Wasserstoff, (C₁-C₈)-Alkyl, (C₁-C₄)-Haloalkyl oder (C₃-C₇)-Cycloalkyl, vorzugsweise Wasserstoff oder (C₁-C₄)-Alkyl, und
- R^{x'}: ist COOR²⁶, worin R²⁶ Wasserstoff, (C₁-C₈)-Alkyl, (C₁-C₄)-Haloalkyl, (C₁-C₄)-Hydroxyalkyl, (C₃-C₇)-Cycloalkyl, (C₁-C₄)-Alkoxy-(C₁-C₄)-alkyl oder Tri-(C₁-C₂)-alkylsilyl, vorzugsweise Wasserstoff oder (C₁-C₄)-Alkyl ist.

Insbesondere bevorzugt sind auch herbizide Mittel, enthaltend einen Safener der Formel (S-II), worin die Symbole und Indizes folgende Bedeutungen haben:
- W: ist (w2);
- R¹⁷: ist Halogen oder (C₁-C₂)-Haloalkyl,
- n': ist 0, 1, 2 oder 3, wobei (R¹⁷)_{n'} vorzugsweise 2,4-Cl₂ ist;
- R¹⁸: ist ein Rest der Formel OR²⁴;
- R²⁴: ist Wasserstoff, (C₁-C₈)-Alkyl, (C₁-C₄)-Haloalkyl, (C₁-C₄)-Hydroxyalkyl, (C₃-C₇)-Cycloalkyl, ((C₁-C₄)-Alkoxy)-(C₁-C₄)-alkyl oder Tri-(C₁-C₂)-alkyl-silyl, vorzugsweise (C₁-C₄)-Alkyl, und
- R²⁷: ist Wasserstoff, (C₁-C₈)-Alkyl, (C₁-C₄)-Haloalkyl, (C₃-C₇)-Cycloalkyl oder unsubstituiertes oder substituiertes Phenyl, vorzugsweise Wasserstoff, (C₁-C₄)-Alkyl oder Phenyl, das unsubstituiert oder durch einen oder mehrere Reste aus der Gruppe Halogen, (C₁-C₄)-Alkyl, (C₁-C₄)-Haloalkyl, Nitro, Cyano oder (C₁-C₄)-Alkoxy substituiert ist.

Insbesondere bevorzugt sind auch Safener der Formel (S-II), worin die Symbole und Indizes folgende Bedeutungen haben:
- W: ist (W3);
- R¹⁷: ist Halogen oder (C₁-C₂)-Haloalkyl,
- n': ist 0, 1, 2 oder 3, wobei (R¹⁷)_{n'} vorzugsweise 2,4-Cl₂ ist;
- R¹⁸: ist ein Rest der Formel OR²⁴;
- R²⁴: ist Wasserstoff, (C₁-C₈)-Alkyl, (C₁-C₄)-Haloalkyl, (C₁-C₄)-Hydroxyalkyl, (C₃-C₇)-Cycloalkyl, (C₁-C₄)-Alkoxy-(C₁-C₄)-alkyl oder Tri-(C₁-C₂)-alkylsilyl, vorzugsweise (C₁-C₄)-Alkyl, und
- R²⁸: ist (C₁-C₈)-Alkyl oder (C₁-C₄)-Haloalkyl, vorzugsweise C₁-Haloalkyl.

Insbesondere bevorzugt sind auch Safener der Formel (S-II), worin die Symbole und Indizes folgende Bedeutung haben:
- W: ist (w4);
- R¹⁷: ist Halogen, Nitro, (C₁-C₄)-Alkyl, (C₁-C₂)-Haloalkyl, vorzugsweise CF₃, oder (C₁-C₄)-Alkoxy;
- n': ist 0, 1, 2 oder 3;
- m': ist 0 oder 1;
- R¹⁸: ist ein Rest der Formel OR²⁴;
- R²⁴: ist Wasserstoff, (C₁-C₄)-Alkyl, Carboxy-(C₁-C₄)-alkyl, (C₁-C₄)-Alkoxycarbonyl-(C₁-C₄)-alkyl, vorzugsweise (C₁-C₄)-Alkoxy-CO-CH₂-, (C₁-C₄)-Alkoxy-CO-C(CH₃)H-, HO-CO-CH₂- oder HO-CO-C(CH₃)H-, und
- R²⁹: ist Wasserstoff, (C₁-C₄)-Alkyl, (C₁-C₄)-Haloalkyl, (C₃-C₇)-Cycloalkyl oder Phenyl, das unsubstituiert oder durch einen oder mehrere der Reste aus der Gruppe Halogen, (C₁-C₄)-Alkyl, (C₁-C₄)-Haloalkyl, Nitro, Cyano und (C₁-C₄)-Alkoxy substituiert ist.

Folgende Gruppen von Verbindungen sind insbesondere als Safener für die herbiziden Wirkstoffe der Formel (I) geeignet:
b) Verbindungen vom Typ der Dichlorphenylpyrazolin-3-carbonsäure (d.h. der Formel (S-II), worin W = (W1) und (R¹⁷)_{n'} = 2,4-Cl₂), vorzugsweise Verbindungen wie 1-(2,4-Dichlorphenyl)-5-(ethoxycarbonyl)-5-methyl-2-pyrazolin-3-carbonsäureethylester (S-II-1, Mefenpyr-diethyl), Mefenpyrdimethyl und Mefenpyr (S-II-0), und verwandte Verbindungen, wie sie in der WO-A 91/07874 beschrieben sind;
c) Derivate der Dichlorphenylpyrazolcarbonsäure (d.h. der Formel (S-II), worin W = (W2) und (R¹⁷)_{n'} = 2,4-Cl₂ ist), vorzugsweise Verbindungen wie 1-(2,4-Dichlorphenyl)-5-methyl-pyrazol-3-carbonsäureethylester (S-II-2), 1-(2,4-Dichlorphenyl)-5-isopropyl-pyrazol-3-carbonsäureethylester (S-II-3), 1-(2,4-Dichlorphenyl)-5-(1,1-dimethyl-ethyl)pyrazol-3-carbonsäureethylester (S-II-4), 1-(2,4-Dichlorphenyl)-5-phenyl-pyrazol-3-carbonsäureethylester (S-II-5) und verwandte Verbindungen, wie sie in EP-A-0 333 131 und EP-A-0 269 806 beschrieben sind;
d) Verbindungen vom Typ der Triazolcarbonsäuren (d.h. der Formel (S-II), worin W = (W3) und (R¹⁷)_{n'} = 2,4-Cl₂ ist), vorzugsweise Verbindungen wie Fenchlorazol-ethyl, d.h. 1-(2,4-Dichlorphenyl)-5-trichlormethyl-(1H)-1,2,4-triazol-3-carbonsäureethylester (S-II-6), und verwandte Verbindungen (siehe EP-A-0 174 562 und EP-A-0 346 620);
e) Verbindungen vom Typ der 5-Benzyl- oder 5-Phenyl-2-isoxazolin-3-carbonsäure oder der 5,5-Diphenyl-2-isoxazolin-3-carbonsäure wie Isoxadifen (S-II-12), (worin W = (W4) ist), vorzugsweise Verbindungen wie 5-(2,4-Dichlorbenzyl)-2-isoxazolin-3-carbonsäureethylester (S-II-7) oder 5-Phenyl-2-isoxazolin-3-carbonsäureethylester (S-II-8) und verwandte Verbindungen, wie sie in WO-A- 91/08202 beschrieben sind, oder der 5,5-Diphenyl-2-isoxazolin-carbonsäureethylester (S-II-9, Isoxadifen-ethyl) oder -n-propylester (S-II-10) oder der 5-(4-Fluorphenyl)-5-phenyl-2-isoxazolin-3-carbonsäureethylester (S-II-11), wie sie in der WO-A-95/07897 beschrieben sind.
e) Verbindungen vom Typ der 8-Chinolinoxyessigsäure, z. B. solche der Formel (S-III), worin (R¹⁹)_{n'} = 5-Cl, R²⁰ = OR²⁴ und T = CH₂ ist, vorzugsweise die Verbindungen
   (5-Chlor-8-chinolinoxy)essigsäure-(1-methylhexyl)-ester (S-III-1, Cloquintocetmexyl),
   (5-Chlor-8-chinolinoxy)essigsäure-(1,3-dimethyl-but-1-yl)-ester (S-III-2),
   (5-Chlor-8-chinolinoxy)essigsäure-4-allyl-oxy-butylester (S-III-3),
   (5-Chlor-8-chinolinoxy)essigsäure-1-allyloxy-prop-2-ylester (S-III-4),
   (5-Chlor-8-chinolinoxy)essigsäureethylester (S-III-5),
   (5-Chlor-8-chinolinoxy)essigsäuremethylester (S-III-6),
   (5-Chlor-8-chinolinoxy)essigsäureallylester (S-III-7),
   (5-Chlor-8-chinolinoxy)essigsäure-2-(2-propyliden-iminoxy)-1-ethylester (S-III-8),
   (5-Chlor-8-chinolinoxy)essigsäure-2-oxo-prop-1-ylester (S-III-9),
   (5-Chlor-8-chinolinoxy)essigsäure (S-III-10) und dessen Salze wie sie z. B. in der WO-A-2002/34048 beschrieben sind,
   und verwandte Verbindungen, wie sie in EP-A-0 860 750, EP-A-0 094 349 und EP-A-0 191 736 oder EP-A-0 492 366 beschrieben sind.
g) Verbindungen vom Typ der (5-Chlor-8-chinolinoxy)-malonsäure, d.h. der Formel (S-III), worin (R¹⁹)_{n'} = 5-Cl, R²⁰= OR²⁴, T = -CH(COO-Alkyl)- ist, vorzugsweise die Verbindungen (5-Chlor-8-chinolinoxy)-malonsäure-diethylester (S-III-11), (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.
h) Verbindungen vom Typ der Dichloracetamide, d.h. der Formel (S-IV), vorzugsweise:
   N,N-Diallyl-2,2-dichloracetamid (Dichlormid (S-IV-1), aus US-A 4,137,070),
   4-Dichloracetyl-3,4-dihydro-3-methyl-2H-1,4-benzoxazin (IV-2, Benoxacor, aus EP 0 149 974),
   N1,N2-Diallyl-N2-dichloracetylglycinamid (DKA-24 (IV-3), aus HU 2143821),
   4-Dichloracetyl-1-oxa-4-aza-spiro[4,5]decan (AD-67),
   2,2-Dichlor-N-(1,3-dioxolan-2-ylmethyl)-N-(2-propenyl)acetamid (PPG-1292),
   3-Dichloracetyl-2,2,5-trimethyloxazolidin (R-29148, S-IV-4),
   3-Dichloracetyl-2,2-dimethyl-5-phenyloxazolidin,
   3-Dichloracetyl-2,2-dimethyl-5-(2-thienyl)oxazolidin,
   3-Dichloracetyl-5-(2-furanyl)-2,2-dimethyloxazolidin (Furilazole (S-IV-5), MON 13900),
   1-Dichloracetyl-hexahydro-3,3,8a-trimethylpyrrolo[1,2-a]pyrimidin-6(2H)-on (Dicyclonon, BAS 145138),
i) Verbindungen der Gruppe B(b), vorzugsweise
   1,8-Naphthalsäureanhydrid (S-b-1),
   Methyl-diphenylmethoxyacetat (S-b-2),
   Cyanomethoxyimino(phenyl)acetonitril (Cyometrinil) (S-b-3),
   1-(2-Chlorbenzyl)-3-(1-methyl-1-phenylethyl)harnstoff (Cumyluron) (S-b-4),
   O, O-Diethyl S-2-ethylthioethyl phosphordithioat (Disulfoton) (S-b-5),
   4-Chlorphenyl-methylcarbamat (Mephenate) (S-b-6),
   O,O-Diethyl-O-phenylphosphorotioat (Dietholate) (S-b-7),
   4-Carboxy-3,4-dihydro-2H-1-benzopyran-4-essigsäure (CL-304415, CAS-Regno: 31541-57-8) (S-b-8),
   1,3-Dioxolan-2-ylmethoxyimino(phenyl)acetonitril (Oxabetrinil) (S-b-9),
   4'-Chlor-2,2,2-trifluoracetophenon O-1,3-dioxolan-2-ylmethyloxim (Fluxofenim) (S-b-10),
   4,6-Dichlor-2-phenylpyrimidin (Fenclorim) (S-b-11),
   Benzyl-2-chlor-4-trifluormethyl-1,3-thiazol-5-carboxylat (Flurazole) (S-b-12),
   2-Dichlormethyl-2-methyl-1,3-dioxolan (MG-191) (S-b-13),
   N-(4-Methylphenyl)-N'-(1-methyl-1-phenylethyl)harnstoff (Dymron) (S-b-14),
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA), 4-(4-Chlor-o-tolyloxy)buttersäure, 4-(4-Chlorphenoxy)buttersäure, 3,6-Dichlor-2-methoxybenzoesäure (Dicamba), 1-(Ethoxycarbonyl)ethyl 3,6-dichlor-2-methoxybenzoat (Lactidichlor) sowie deren Salze und Ester, vorzugsweise (C₁-C₈).

Bevorzugt sind als Safener weiterhin Verbindungen der Formel (S-V) oder deren Salze, worin
- R³⁰: Wasserstoff, (C₁-C₆)-Alkyl, (C₃-C₆)-Cycloalkyl, Furanyl oder Thienyl, wobei jeder der letztgenannten 4 Reste unsubstituiert oder durch einen oder mehrere Substituenten aus der Gruppe Halogen, (C₁-C₄)-Alkoxy, Halogen-(C₁-C₆)-alkoxy und (C₁-C₄)-Alkylthio und im Falle cyclischer Reste auch (C₁-C₄)-Alkyl und (C₁-C₄)-Haloalkyl substituiert ist,
- R³¹: Wasserstoff,
- R³²: Halogen, Halogen-(C₁-C₄)-alkyl, Halogen-(C₁-C₄)-alkoxy, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl, vorzugsweise Halogen, (C₁-C₄)-Halogenalkyl, wie Trifluormethyl, (C₁-C₄)-Alkoxy, Halogen-(C₁-C₄)-alkoxy, (C₁-C₄)-Alkoxycarbonyl oder (C₁-C₄)-Alkylsulfonyl,
- R³³: Wasserstoff,
- R³⁴: Halogen, (C₁-C₄)-Alkyl, Halogen-(C₁-C₄)-alkyl, Halogen-(C₁-C₄)-alkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl, vorzugsweise Halogen, (C₁-C₄)-Alkyl, (C₁-C₄)-Halogenalkyl, wie Trifluormethyl, Halogen-(C₁-C₄)-alkoxy, (C₁-C₄)-Alkoxy oder (C₁-C₄)-Alkylthio,
- n: 0, 1 oder 2 und
- m: 1 oder 2 bedeuten.

Besonders bevorzugt sind Verbindungen der Formel (S-V), worin
R³⁰ = H₃C-O-CH₂-, R³¹ = R³³ = H, R³⁴ = 2-OMe ist (S-V-1),
R³⁰ = H₃C-O-CH₂-, R³¹ = R³³ = H, R³⁴ = 2-OMe-5-Cl ist (S-V-2),
R³⁰ = Cyclopropyl, R³¹ = R³³ = H, R³⁴ = 2-OMe ist (S-V-3),
R³⁰ = Cyclopropyl, R³¹ = R³³ = H, R³⁴ = 2-OMe-5-Cl ist (S-V-4),
R³⁰ = Cyclopropyl, R³¹ = R³³ = H, R³⁴ = 2-Me ist (S-V-5),
R³⁰ = tert. Butyl, R³¹ = R33 = H, R³⁴ = 2-OMe ist (S-V-6).

Weiterhin bevorzugt sind Safener der Formel (S-VI), in der
- X³: CH;
- R³⁵: Wasserstoff, (C₁-C₆)-Alkyl, (C₃-C₆)-Cycloalkyl, (C₂-C₆)-Alkenyl, (C₅-C₆)-Cycloalkenyl, Phenyl oder 3- bis 6-gliedriges Heterocyclyl mit bis zu drei Heteroatomen aus der Gruppe Stickstoff, Sauerstoff und Schwefel, wobei die sechs letztgenannten Reste gegebenenfalls durch einen oder mehrere, gleiche oder verschiedene Substituenten aus der Gruppe Halogen, (C₁-C₆)-Alkoxy, (C₁-C₆)-Haloalkoxy, (C₁-C₂)-Alkylsulfinyl, (C₁-C₂)-Alkylsulfonyl, (C₃-C₆)-Cycloalkyl, (C₁-C₄)-Alkoxycarbonyl, (C₁-C₄)-Alkylcarbonyl und Phenyl und im Falle cyclischer Reste auch (C₁-C₄)-Alkyl und (C₁-C₄)-Haloalkyl substituiert sind;
- R³⁶: Wasserstoff, (C₁-C₆)-Alkyl, (C₂-C₆)-Alkenyl, (C₂-C₆)-Alkinyl, wobei die drei letztgenannten Reste gegebenenfalls durch einen oder mehrere, gleiche oder verschiedene Substituenten aus der Gruppe Halogen, Hydroxy, (C₁-C₄)-Alkyl, (C₁-C₄)-Alkoxy und (C₁-C₄)-Alkylthio substituiert sind;
- R³⁷: Halogen, (C₁-C₄)-Haloalkyl, (C₁-C₄)-Haloalkoxy, Nitro, (C₁-C₄)-Alkyl, (C₁-C₄)-Alkoxy, (C₁-C₄)-Alkylsulfonyl, (C₁-C₄)-Alkoxycarbonyl oder (C₁-C₄)-Alkylcarbonyl;
- R³⁸: Wasserstoff;
- R³⁹: Halogen, Nitro, (C₁-C₄)-Alkyl, (C₁-C₄)-Haloalkyl, (C₁-C₄)-Haloalkoxy, (C₃-C₆)-Cycloalkyl, Phenyl, (C₁-C₄)-Alkoxy, Cyano, (C₁-C₄)-Alkylthio, (C₁-C₄)-Alkylsulfinyl, (C₁-C₄)-Alkylsulfonyl, (C₁-C₄)-Alkoxycarbonyl oder (C₁-C₄)-Alkylcarbonyl;
- n: 0, 1 oder 2 und
- m: 1 oder 2 bedeuten.

Bevorzugte Safener der Formel (S-VII) sind (S-3-1), (S-3-2), (S-3-3), (S-3-4) und (S-3-5).

Bevorzugte Safener der Formel (VIII) sind
1-[4-(N-2-Methoxybenzoylsulfamoyl)phenyl]-3-methylharnstoff (S-VIII-1),
1-[4-(N-2-Methoxybenzoylsulfamoyl)phenyl]-3,3-dimethylharnstoff (S-VIII-2),
1-[4-(N-4,5-Dimethylbenzoylsulfamoyl)phenyl]-3-methylharnstoff (S-VIII-3) und
1-[4-(N-Naphthoylsulfamoyl)phenyl]-3,3-dimethylharnstoff (S-VIII-4).

Bevorzugte Safener der Formel S-IX sind sind Verbindungen der Formeln S-IX-A1 bis S-IX-A4, 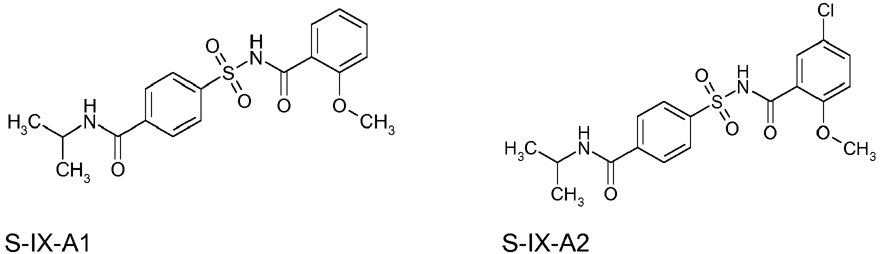 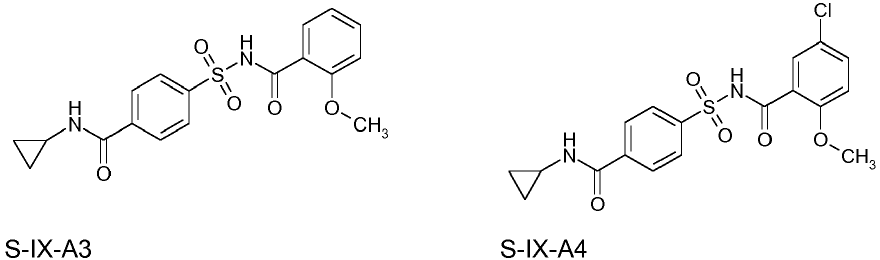 von denen wiederum die Verbindung S-IX-A3 als Safener ganz besonders bevorzugt ist.

Besonders bevorzugte Kombinationen von Herbiziden Wirkstoffen der allgemeinen Formel (I) wie in einer der Tabellen 1 bis 4 benannt und Safenern (B) sind solche, bei denen der Safener (B) ausgewählt ist aus der Gruppe von Safenern bestehend aus den Verbindungen der Formeln S-II-1 (Mefenpyr-diethyl), S-II-9 (Isoxadifen-ethyl), S-III-1 (Chloquintocet-mexyl), S-b-11 (Fenclorim), S-b-14 (Dymron), S-IX-A3 (4-Cyclopropylaminocarbonyl-N-(2-methoxybenzoyl)benzolsulfonamid N-({4-[(cyclopropylamino)carbonyl]phenyl}sulfonyl)-2-methoxybenzamide, ganz besonders bevorzugt als Safener (B) sind Verbindungen S-II-1 und S-IX-A3).
Für die Anwendung in Reis besonders bevorzugt ist Isoxadifen-ethyl. Für die Anwendung in Getreide besonders bevorzugt sind Mefenpyr-diethyl, Cloquintocetmexyl und 4-Cyclopropylaminocarbonyl-N-(2-methoxybenzoyl) benzolsulfonamid N-({4-[(cyclopropylamino)carbonyl] phenyl}sulfonyl)-2-methoxybenzamide, in Mais insbesondere Isoxadifen-ethyl und 4-Cyclopropylaminocarbonyl-N-(2-methoxy-benzoyl)benzolsulfonamid N-({4-[(cyclopropylamino)carbonyl]phenyl} sulfonyl)-2-methoxy-benzamide. Für die Anwendung in Zuckerrohr bevorzugt ist Isoxadifen-ethyl. Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser.

Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird ahand der nachfolgenden Beispiele näher erläutert, welche die Erfindung jedoch keinesfalls beschränken.

### A. Synthesebeispiele

Nachfolgend sind einige Synthesebeispiele von Verbindungen der allgemeinen Formel (I) oder deren Salze beispielhaft beschrieben.

5-Brom-2-[(S)-{[5-(difluormethoxy)-1-methyl-3-(trifluormethyl)-1H-pyrazol-4-yl]methyl}sulfinyl]-1,3-thiazol (Bsp. 2637) und 5-Brom-2-[(R)-{[5-(difluormethoxy)-1-methyl-3-(trifluormethyl)-1 H-pyrazol-4-yl]methyl}sulfinyl]-1,3-thiazol (Bsp. 3725)

Das Edukt [5-(Difluormethoxy)-1-methyl-3-(trifluormethyl)-1H-pyrazol-4-yl]methyl imidothiocarbamathydrobromid erhält man durch Mischen von äquimolaren Mengen von 4-(Brommethyl)-5-(difluormethoxy)-1-methyl-3-(trifluormethyl)-1 H-pyrazol und Thioharnstoff in Ethanol und 8 stündigem Refluxieren, Einengen der resultierenden Mischung und Umkristallisieren aus Tetrahydrofuran.

Zu einer heftig gerührten Mischung, bestehend aus 100 ml Toluol und 50 %iger wäßriger Natronlauge (50 g), wird [5-(Difluormethoxy)-1-methyl-3-(trifluormethyl)-1 H-pyrazol-4-yl]methyl imidothiocarbamathydrobromid (7.928 g, 21 mmol) gegeben und weitere 1,5 Stunden heftig gerührt. Danach werden Tetra-n-Butylammoniumbromid (1.858 g, 6 mmol) und 2,5-Dibromo-1,3-thiazol (5.0 g, 21 mmol) zugegeben und weitere 5 Stunden bei 25 °C stark gerührt und über Nacht stehengelassen. Zur Aufarbeitung wird die Reaktionslösung auf Wasser gegeben und mit Toluol extrahiert. Die vereinigten organischen Phasen werden getrocknet und eingeengt. Zur Reinigung wird an Kieselgel chromatographiert (Heptan/Ethylacetat, Gradient). Man erhält 5.62 g Produkt (61.1 % d. Th.).
NMR (CDCl₃, 400 MHz): 3.8 (s, 3H, CH₃); 4.31 (s, 2H, SCH₂); 6.69 (t, 1 H, OCHF₂); 7.58 (s, 1 H, Thiazolyl-H).

Das so erhaltene 5-Brom-2-({[5-(difluormethoxy)-1-methyl-3-(trifluormethyl)-1H-pyrazol-4-yl]methyl}sulfanyl)-1,3-thiazol (4.0 g, 9.429 mmol) wird in 100 ml Toluol vorgelegt. Dann wird unter Rühren portionsweise 3-Chlor-perbenzoesäure (1.976 g, 8.015 mmol, 77 %ig) zugegeben und weitere 4 Stunden bei Raumtemperatur gerührt. Zur Aufarbeitung wird das Reaktionsgemisch nacheinander mit Wasser, wässriger NaHSO₃-Lösung, wässriger NaHCO₃-Lösung und schließlich mit NaCl-Lösung gewaschen. Die organische Phase wird über Magnesiumsulfat getrocknet, abfiltriert und eingeengt. Der Rückstand wird mit n-Heptan verrührt, abfiltriert und getrocknet. Das erhaltene racemische 5-Bromo-2-({[5-(difluormethoxy)-1-methyl-3-(trifluormethyl)-1H-pyrazol-4-yl]methyl}sulfinyl)-1,3-thiazol (3.75 g) wird durch präparative chirale HPLC (Säule: Chiralcel^{®} OD, Eluent: n-Hexan/2-Propanol 90:10; Flow: 0.6 ml/min; Säulentemperatur: 25 °C) gereinigt und in die Enantiomeren getrennt. Man erhält so 1.505 g (36.2 % d. Th.) 5-Brom-2-[(S)-{[5-(difluormethoxy)-1-methyl-3-(trifluormethyl)-1H-pyrazol-4-yl]methyl}sulfinyl]-1,3-thiazol (Rt = 10.258 min) und 1.305 g (31.4 % d. Th.) 5-Bromo-2-[(R)-{[5-(difluormethoxy)-1-methyl-3-(trifluormethyl)-1H-pyrazol-4-yl]methyl}sulfinyl]-1,3-thiazol (Rt = 12.557 min).

Die absolute Konfiguration von 5-Brom-2-[(S)-{[5-(difluormethoxy)-1-methyl-3-(trifluormethyl)-1H-pyrazol-4-yl]methyl}sulfinyl]-1,3-thiazol wurde mittels Röntgenstrukturanalyse bestätigt.

### 5-Brom-2-[(S)-{[5-chlor-1-methyl-3-(trifluormethyl)-1H-pyrazol-4-yl]methyl}sulfinyl]-1,3-thiazol (Bsp. 2632) und 5-Brom-2-[(R)-{[5-chlor-1-methyl-3-(trifluormethyl)-1H-pyrazol-4-yl]methyl}sulfinyl]-1,3-thiazol (Bsp. 3720)

Molsieb 4A wird (0.610 g) in Dichlormethan (5 ml) vorgelegt. Der Katalysator Titan(IV)isopropylat (0.18 ml, 0.62 mmol) und der Ligand (*R*)-(-)-Mandelsäure (0.142 g, 0.93 mmol) werden zugesetzt. Man rührt weitere 30 Minuten bei Raumtemperatur. Nun wird 5-Brom-2-({[5-chlor-1-methyl-3-(trifluormethyl)-1H-pyrazol-4-yl]methyl}sul-fanyl)-1,3-thiazol, welches gemäß WO 2006/123088 erhalten werden kann, zugegeben (0.610 g, 1.55 mmol). Es wird eine weitere Stunde bei Raumtemperatur weitergerührt, währendessen der Katalysator-Ligand-Komplex sich bildet. Nun wird Cumylhydroperoxid zugetropft (0.29 ml, 80 %, 1.55 mmol). Dann wird sieben Stunden bei Raumtemperatur weitergerührt und eine Woche im Kühlschrank gelagert (-5 °C). Zur Aufarbeitung wird das Reaktionsgemisch nacheinander mit Wasser, zweimal mit wässriger 2M NaOH-Lösung, und schließlich mit NaCl-Lösung gewaschen. Die organische Phase wird über Magnesiumsulfat getrocknet, abfiltriert und eingeengt. Das erhaltene Rohprodukt enthält 5-Brom-2-[{[5-chlor-1-methyl-3-(trifluormethyl)-1H-pyrazol-4-yl]methyl}sulfinyl]-1,3-thiazol und das entschprechende Sulfon (0.3 g, ca. 1:1) und wird mittels präparativer HPLC gereinigt. Man erhält 0.116 g (18.2 % d. Th.) 5-Brom-2-[{[5-chlor-1-methyl-3-(trifluormethyl)-1H-pyrazol-4-yl]methyl}sulfinyl]-1,3-thiazol mit 11 % ee (S), welches weiterhin durch präparative chirale HPLC in die Enantiomeren getrennt wird. Man erhält 0.063 g (10 % d. Th.) 5-Brom-2-[(S)-{[5-chlor-1-methyl-3-(trifluormethyl)-1H-pyrazol-4-yl]methyl}sulfinyl]-1,3-thiazol (Rt = 18.600 min) und 0.042 g (7 % d. Th.) 5-Brom-2-[(R)-{[5-chlor-1-methyl-3-(trifluormethyl)-1H-pyrazol-4-yl]methyl}sulfinyl]-1,3-thiazol (Rt = 22.652 min).

### 5-Brom-2-[(S)-(2,6-difluorbenzyl)sulfinyl]-1,3-thiazol (Bsp. 259) und 5-Brom-2-[(R)-(2,6-difluorbenzyl)sulfinyl]-1,3-thiazol (Bsp. 1449)

### a) Herstellung von 5-Brom-2-[(2,6-difluorbenzyl)sulfanyl]-1,3-thiazol

Das Edukt (2,6-Difluorbenzyl)imidothiocarbamathydrochlorid erhält man durch Mischen von äquimolaren Mengen von 2,6-Difluorbenzylchlorid und Thioharnstoff in Ethanol, 8stündigem Refluxieren, Einengen und Umkristallisieren aus Tetrahydrofuran.

Zu einer heftig gerührten Mischung, bestehend aus 50 ml Toluol und 50 %iger wäßriger Natronlauge (28 g), wird (2,6-Difluorbenzyl)imidothiocarbamathydrochlorid (1.972 g, 8 mmol) gegeben und weitere 1,5 Stunden heftig gerührt. Danach werden Tetra-n-Butylammoniumbromid (0.746 g, 2 mmol) und 5-Bromo-2-(methylsulfonyl)-1,3-thiazol (2.0 g, 8 mmol) zugegeben und weitere 6 Stunden bei 25 °C stark gerührt und über Nacht stehengelassen. Zur Aufarbeitung wird die Reaktionslösung auf Wasser gegeben und mit Toluol extrahiert. Die vereinigten organischen Phasen werden getrocknet und eingeengt. Zur Reinigung wird an Kieselgel chromatographiert (Heptan/Ethylacetat, Gradient). Man erhält 1.7 g Produkt (60.6 % d. Th.).
NMR (CDCl₃, 400 MHz): 4.42 (s, 2H, SCH₂); 6.89 (m, 2H, Ar); 7.24 (m, 1 H, Ar); 7.61 (s, 1 H, Thiazolyl-H).

### b) Asymmetrische Sulfoxidierung

Der Ligand (R, R)-1,2-Diphenylethan-1,2-diol [(R, R)-(+)-Hydrobenzoin, 0.033 g, 0.15 mmol] wird in CCl₄ (10 ml) vorgelegt. Der Katalysator Titan(IV)isopropylat (0.02 ml, 0.077 mmol) wird zugegeben und anschließend wird Wasser (0.028 ml, 1.55 mmol) zugetropft. Man rührt das Gemisch 15 Minuten bei Raumtemperatur. Anschließend wird 5-Brom-2-[(2,6-difluorbenzyl)sulfanyl]-1,3-thiazol (0.5 g, 1.55 mmol) zugegeben und weitere 15 Minuten bei Raumtemperatur gerührt. Unter Eisbadkühlung wird dann tert-Butylhydroperoxid (TBHP, 70 %ig in Wasser, 0.44 ml, 3 mmol) zugetropft. Man rührt weiter zwei Stunden und lässt über Nacht auftauchen. Zur Aufarbeitung wird das Reaktionsgemisch mit Dichlormethan verdünnt, nacheinander mit Wasser, zweimal mit 5 %ger Na₂S₂O₅-Lösung und schließlich mit NaCl-Lösung gewaschen. Die organische Phase wird über Magnesiumsulfat getrocknet, abfiltriert und eingeengt. Der Rückstand wird über Silicagel chromatographiert (Heptan/Essigester 10:0 bis bis 7:3). Das erhaltene Gemisch (0.120 g) enthält 5-Brom-2-[(S)-(2,6-difluorbenzyl)sulfinyl]-1,3-thiazol mit 23 % ee (S) und das entsprechende Sulfon und wird direkt durch präparative chirale HPLC in die Enantiomeren getrennt. Man erhält 0.032 g (6 % d. Th.) 5-Brom-2-[(S)-(2,6-difluorbenzyl)sulfinyl]-1,3-thiazol (Rt = 17.053 min) und 0.032 g (6 % d. Th.) 5-Brom-2-[(R)-(2,6-difluorbenzyl)sulfinyl]-1,3-thiazol (Rt = 19.430 min).

Retentionszeiten (Rₜ, in Minuten) und Enantiomerenüberschuss (ee) von chiralen Verbindungen werden mittels analytischer chiralen HPLC ermittelt [Chiralcel^{®} OD Säule (250 x 4.6 mm, Korngröße 5 µm), Temperatur 25 °C, Fluss 0.6 ml/min, Hexan / 2-Propanol 90:10 v/v].

Die Racemate oder enantiomeren Gemische werden mittels präparative chiralen HPLC in den jeweiligen Enantiomeren getrennt [Chiralcel^{®} OD Säule (250 x 5 mm, Korngröße 10 µm), Temperatur 25 °C, Fluss 0.6 ml/min, Hexan / 2-Propanol 90:10 v/v].

Die in den nachfolgenden Tabellen 1 - 4 beschriebenen Verbindungen erhält man gemäß oder analog zu den oben beschriebenen Synthesebeispielen.

In den Tabellen bedeuten:
- Me: = Methyl
- Et: = Ethyl
- Ph: = Phenyl
- Pr: = n-Propyl
- cPr: = Cyclopropyl
- iPr: = Isopropyl
- Bu: = n-Butyl
- cBu: = Cyclobutyl
- iBu: = Isobutyl
- sBu: = sek.-Butyl
- tBu: = tert.-Butyl
- cPen: = Cyclopentyl
- cHex: = Cyclohexyl

**Tabelle 1: Verbindungen der Formel la-S**

| | | | | | | | |
|---|---|---|---|---|---|---|---|
| 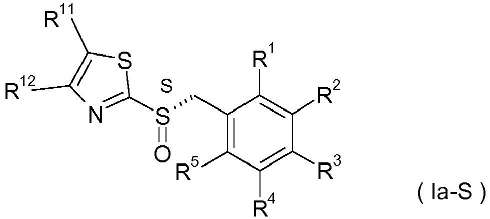 | | | | | | | |

| Bsp. Nr. | R¹¹ | R¹² | R¹ | R² | R³ | R⁴ | R⁵ |
|---|---|---|---|---|---|---|---|
| 1. | H | H | H | H | H | H | H |
| 2. | H | H | F | H | H | H | H |
| 3. | H | H | F | H | H | F | H |
| 4. | H | H | F | H | H | H | F |
| 5. | H | H | F | Me | H | H | F |
| 6. | H | H | F | H | H | H | Cl |
| 7. | H | H | CF₃ | H | H | H | H |
| 8. | H | H | Me | H | H | H | H |
| 9. | H | H | F | H | H | H | CF₃ |
| 10. | H | H | F | CF₃ | H | H | F |
| 11. | H | H | Br | H | H | H | H |
| 12. | H | H | l | H | H | H | H |
| 13. | H | H | Cl | H | H | H | H |
| 14. | H | H | Cl | H | Cl | H | H |
| 15. | H | H | Cl | H | H | H | Cl |
| 16. | H | H | H | Cl | Cl | H | H |
| 17. | H | H | Cl | Cl | Cl | H | H |
| 18. | H | H | Cl | Cl | H | Cl | H |
| 19. | H | H | Cl | Cl | Cl | H | Cl |
| 20. | H | H | Cl | Cl | Cl | Cl | H |
| 21. | H | H | Cl | Cl | Cl | Cl | Cl |
| 22. | H | H | Cl | Cl | H | H | Cl |
| 23. | H | H | Cl | H | Cl | Cl | H |
| 24. | H | H | Cl | H | H | Cl | Cl |
| 25. | H | H | H | Cl | Cl | Cl | H |
| 26. | H | H | NO₂ | H | H | H | H |
| 27. | H | H | H | Cl | H | H | H |
| 28. | H | H | H | H | Cl | H | H |
| 29. | H | H | Cl | H | Cl | H | Cl |
| 30. | H | H | Cl | Cl | H | H | H |
| 31. | H | H | Cl | H | H | Cl | H |
| 32. | H | H | H | Cl | H | Cl | H |
| 33. | H | H | H | OMe | H | H | H |
| 34. | H | H | C(O)OMe | H | H | H | H |
| 35. | H | H | F | Cl | H | H | H |
| 36. | H | H | F | Me | H | H | H |
| 37. | H | H | H | Me | H | H | H |
| 38. | H | H | OMe | H | H | H | H |
| 39. | H | H | F | F | F | H | H |
| 40. | H | H | F | F | H | F | H |
| 41. | H | H | H | F | F | F | H |
| 42. | H | H | F | H | F | F | H |
| 43. | H | H | Me | H | Me | H | H |
| 44. | H | H | Me | H | H | Me | H |
| 45. | H | H | F | H | H | CF₃ | H |
| 46. | H | H | F | H | Br | H | H |
| 47. | H | H | Me | Me | H | H | H |
| 48. | H | H | F | F | F | F | F |
| 49. | H | H | F | H | H | H | OMe |
| 50. | H | H | Cl | H | F | H | H |
| 51. | H | H | NO₂ | H | Cl | H | H |
| 52. | H | H | NO₂ | H | H | Me | H |
| 53. | H | H | F | H | H | H | l |
| 54. | H | H | F | H | H | H | Br |
| 55. | H | H | Br | H | H | H | Br |
| 56. | H | H | Cl | H | H | H | Me |
| 57. | H | H | Cl | H | H | H | OCHF₂ |
| 58. | H | H | Cl | H | H | H | OMe |
| 59. | H | H | Me | H | H | H | OMe |
| 60. | H | H | OEt | H | H | H | CF₃ |
| 61. | H | H | OC(O)Me | H | H | H | H |
| 62. | H | H | OEt | H | H | H | Me |
| 63. | H | H | Me | Me | H | H | Me |
| 64. | H | H | Cl | H | H | H | C(O)OMe |
| 65. | H | H | Cl | H | H | OMe | H |
| 66. | H | H | F | F | H | F | F |
| 67. | H | H | Cl | H | H | F | H |
| 68. | H | H | F | H | H | F | Cl |
| 69. | H | H | F | H | H | Cl | H |
| 70. | H | H | Cl | H | H | CF₃ | H |
| 71. | H | H | Cl | Me | H | H | H |
| 72. | H | H | OCHF₂ | H | H | H | H |
| 73. | H | H | OCH₂CF₃ | H | H | H | H |
| 74. | H | H | CF₃ | H | H | H | OCHF₂ |
| 75. | H | H | CF₃ | H | H | H | OCH₂CF₃ |
| 76. | H | H | Me | H | H | H | Me |
| 77. | H | H | Cl | H | H | H | F |
| 78. | H | H | F | H | F | H | H |
| 79. | H | H | F | Me | H | H | Cl |
| 80. | H | H | F | H | H | OMe | H |
| 81. | H | H | Cl | H | OCH₂O | | H |
| 82. | H | H | Me | H | H | F | H |
| 83. | H | H | OCF₃ | H | H | H | H |
| 84. | H | H | F | F | H | H | H |
| 85. | H | H | OMe | H | H | Cl | H |
| 86. | F | H | H | H | H | H | H |
| 87. | F | H | F | H | H | H | H |
| 88. | F | H | F | H | H | F | H |
| 89. | F | H | F | H | H | H | F |
| 90. | F | H | F | Me | H | H | F |
| 91. | F | H | F | H | H | H | Cl |
| 92. | F | H | CF₃ | H | H | H | H |
| 93. | F | H | Me | H | H | H | H |
| 94. | F | H | F | H | H | H | CF₃ |
| 95. | F | H | F | CF₃ | H | H | F |
| 96. | F | H | Br | H | H | H | H |
| 97. | F | H | l | H | H | H | H |
| 98. | F | H | Cl | H | H | H | H |
| 99. | F | H | Cl | H | Cl | H | H |
| 100. | F | H | Cl | H | H | H | Cl |
| 101. | F | H | H | Cl | Cl | H | H |
| 102. | F | H | Cl | Cl | Cl | H | H |
| 103. | F | H | Cl | Cl | H | Cl | H |
| 104. | F | H | Cl | Cl | Cl | H | Cl |
| 105. | F | H | Cl | Cl | Cl | Cl | H |
| 106. | F | H | Cl | Cl | Cl | Cl | Cl |
| 107. | F | H | Cl | Cl | H | H | Cl |
| 108. | F | H | Cl | H | Cl | Cl | H |
| 109. | F | H | Cl | H | H | Cl | Cl |
| 110. | F | H | H | Cl | Cl | Cl | H |
| 111. | F | H | NO₂ | H | H | H | H |
| 112. | F | H | H | Cl | H | H | H |
| 113. | F | H | H | H | Cl | H | H |
| 114. | F | H | Cl | H | Cl | H | Cl |
| 115. | F | H | Cl | Cl | H | H | H |
| 116. | F | H | Cl | H | H | Cl | H |
| 117. | F | H | H | Cl | H | Cl | H |
| 118. | F | H | H | OMe | H | H | H |
| 119. | F | H | C(O)OMe | H | H | H | H |
| 120. | F | H | F | Cl | H | H | H |
| 121. | F | H | F | Me | H | H | H |
| 122. | F | H | H | Me | H | H | H |
| 123. | F | H | OMe | H | H | H | H |
| 124. | F | H | F | F | F | H | H |
| 125. | F | H | F | F | H | F | H |
| 126. | F | H | H | F | F | F | H |
| 127. | F | H | F | H | F | F | H |
| 128. | F | H | Me | H | Me | H | H |
| 129. | F | H | Me | H | H | Me | H |
| 130. | F | H | F | H | H | CF₃ | H |
| 131. | F | H | F | H | Br | H | H |
| 132. | F | H | Me | Me | H | H | H |
| 133. | F | H | F | F | F | F | F |
| 134. | F | H | F | H | H | H | OMe |
| 135. | F | H | Cl | H | F | H | H |
| 136. | F | H | NO₂ | H | Cl | H | H |
| 137. | F | H | NO₂ | H | H | Me | H |
| 138. | F | H | F | H | H | H | l |
| 139. | F | H | F | H | H | H | Br |
| 140. | F | H | Br | H | H | H | Br |
| 141. | F | H | Cl | H | H | H | Me |
| 142. | F | H | Cl | H | H | H | OCHF₂ |
| 143. | F | H | Cl | H | H | H | OMe |
| 144. | F | H | Me | H | H | H | OMe |
| 145. | F | H | OEt | H | H | H | CF₃ |
| 146. | F | H | OC(O)Me | H | H | H | H |
| 147. | F | H | OEt | H | H | H | Me |
| 148. | F | H | Me | Me | H | H | Me |
| 149. | F | H | Cl | H | H | H | C(O)OMe |
| 150. | F | H | Cl | H | H | OMe | H |
| 151. | F | H | F | F | H | F | F |
| 152. | F | H | Cl | H | H | F | H |
| 153. | F | H | F | H | H | F | Cl |
| 154. | F | H | F | H | H | Cl | H |
| 155. | F | H | Cl | H | H | CF₃ | H |
| 156. | F | H | Cl | Me | H | H | H |
| 157. | F | H | OCHF₂ | H | H | H | H |
| 158. | F | H | OCH₂CF₃ | H | H | H | H |
| 159. | F | H | CF₃ | H | H | H | OCHF₂ |
| 160. | F | H | CF₃ | H | H | H | OCH₂CF₃ |
| 161. | F | H | Me | H | H | H | Me |
| 162. | F | H | Cl | H | H | H | F |
| 163. | F | H | F | H | F | H | H |
| 164. | F | H | F | Me | H | H | Cl |
| 165. | F | H | F | H | H | OMe | H |
| 166. | F | H | Cl | H | OCH₂O | | H |
| 167. | F | H | Me | H | H | F | H |
| 168. | F | H | OCF₃ | H | H | H | H |
| 169. | F | H | F | F | H | H | H |
| 170. | F | H | OMe | H | H | Cl | H |
| 171. | Cl | H | H | H | H | H | H |
| 172. | Cl | H | F | H | H | H | H |
| 173. | Cl | H | F | H | H | F | H |
| 174. | Cl | H | F | H | H | H | F |
| 175. | Cl | H | F | Me | H | H | F |
| 176. | Cl | H | F | H | H | H | Cl |
| 177. | Cl | H | CF₃ | H | H | H | H |
| 178. | Cl | H | Me | H | H | H | H |
| 179. | Cl | H | F | H | H | H | CF₃ |
| 180. | Cl | H | F | CF₃ | H | H | F |
| 181. | Cl | H | Br | H | H | H | H |
| 182. | Cl | H | l | H | H | H | H |
| 183. | Cl | H | Cl | H | H | H | H |
| 184. | Cl | H | Cl | H | Cl | H | H |
| 185. | Cl | H | Cl | H | H | H | Cl |
| 186. | Cl | H | H | Cl | Cl | H | H |
| 187. | Cl | H | Cl | Cl | Cl | H | H |
| 188. | Cl | H | Cl | Cl | H | Cl | H |
| 189. | Cl | H | Cl | Cl | Cl | H | Cl |
| 190. | Cl | H | Cl | Cl | Cl | Cl | H |
| 191. | Cl | H | Cl | Cl | Cl | Cl | Cl |
| 192. | Cl | H | Cl | Cl | H | H | Cl |
| 193. | Cl | H | Cl | H | Cl | Cl | H |
| 194. | Cl | H | Cl | H | H | Cl | Cl |
| 195. | Cl | H | H | Cl | Cl | Cl | H |
| 196. | Cl | H | NO₂ | H | H | H | H |
| 197. | Cl | H | H | Cl | H | H | H |
| 198. | Cl | H | H | H | Cl | H | H |
| 199. | Cl | H | Cl | H | Cl | H | Cl |
| 200. | Cl | H | Cl | Cl | H | H | H |
| 201. | Cl | H | Cl | H | H | Cl | H |
| 202. | Cl | H | H | Cl | H | Cl | H |
| 203. | Cl | H | H | OMe | H | H | H |
| 204. | Cl | H | C(O)OMe | H | H | H | H |
| 205. | Cl | H | F | Cl | H | H | H |
| 206. | Cl | H | F | Me | H | H | H |
| 207. | Cl | H | H | Me | H | H | H |
| 208. | Cl | H | OMe | H | H | H | H |
| 209. | Cl | H | F | F | F | H | H |
| 210. | Cl | H | F | F | H | F | H |
| 211. | Cl | H | H | F | F | F | H |
| 212. | Cl | H | F | H | F | F | H |
| 213. | Cl | H | Me | H | Me | H | H |
| 214. | Cl | H | Me | H | H | Me | H |
| 215. | Cl | H | F | H | H | CF₃ | H |
| 216. | Cl | H | F | H | Br | H | H |
| 217. | Cl | H | Me | Me | H | H | H |
| 218. | Cl | H | F | F | F | F | F |
| 219. | Cl | H | F | H | H | H | OMe |
| 220. | Cl | H | Cl | H | F | H | H |
| 221. | Cl | H | NO₂ | H | Cl | H | H |
| 222. | Cl | H | NO₂ | H | H | Me | H |
| 223. | Cl | H | F | H | H | H | I |
| 224. | Cl | H | F | H | H | H | Br |
| 225. | Cl | H | Br | H | H | H | Br |
| 226. | Cl | H | Cl | H | H | H | Me |
| 227. | Cl | H | Cl | H | H | H | OCHF₂ |
| 228. | Cl | H | Cl | H | H | H | OMe |
| 229. | Cl | H | Me | H | H | H | OMe |
| 230. | Cl | H | OEt | H | H | H | CF₃ |
| 231. | Cl | H | OC(O)Me | H | H | H | H |
| 232. | Cl | H | OEt | H | H | H | Me |
| 233. | Cl | H | Me | Me | H | H | Me |
| 234. | Cl | H | Cl | H | H | H | C(O)OMe |
| 235. | Cl | H | Cl | H | H | OMe | H |
| 236. | Cl | H | F | F | H | F | F |
| 237. | Cl | H | Cl | H | H | F | H |
| 238. | Cl | H | F | H | H | F | Cl |
| 239. | Cl | H | F | H | H | Cl | H |
| 240. | Cl | H | Cl | H | H | CF₃ | H |
| 241. | Cl | H | Cl | Me | H | H | H |
| 242. | Cl | H | OCHF₂ | H | H | H | H |
| 243. | Cl | H | OCH₂CF₃ | H | H | H | H |
| 244. | Cl | H | CF₃ | H | H | H | OCHF₂ |
| 245. | Cl | H | CF₃ | H | H | H | OCH₂CF₃ |
| 246. | Cl | H | Me | H | H | H | Me |
| 247. | Cl | H | Cl | H | H | H | F |
| 248. | Cl | H | F | H | F | H | H |
| 249. | Cl | H | F | Me | H | H | Cl |
| 250. | Cl | H | F | H | H | OMe | H |
| 251. | Cl | H | Cl | H | OCH₂O | | H |
| 252. | Cl | H | Me | H | H | F | H |
| 253. | Cl | H | OCF₃ | H | H | H | H |
| 254. | Cl | H | F | F | H | H | H |
| 255. | Cl | H | OMe | H | H | Cl | H |
| 256. | Br | H | H | H | H | H | H |
| 257. | Br | H | F | H | H | H | H |
| 258. | Br | H | F | H | H | F | H |
| 259. | Br | H | F | H | H | H | F |
| 260. | Br | H | F | Me | H | H | F |
| 261. | Br | H | F | H | H | H | Cl |
| 262. | Br | H | CF₃ | H | H | H | H |
| 263. | Br | H | Me | H | H | H | H |
| 264. | Br | H | F | H | H | H | CF₃ |
| 265. | Br | H | F | CF₃ | H | H | F |
| 266. | Br | H | Br | H | H | H | H |
| 267. | Br | H | I | H | H | H | H |
| 268. | Br | H | Cl | H | H | H | H |
| 269. | Br | H | Cl | H | Cl | H | H |
| 270. | Br | H | Cl | H | H | H | Cl |
| 271. | Br | H | H | Cl | Cl | H | H |
| 272. | Br | H | Cl | Cl | Cl | H | H |
| 273. | Br | H | Cl | Cl | H | Cl | H |
| 274. | Br | H | Cl | Cl | Cl | H | Cl |
| 275. | Br | H | Cl | Cl | Cl | Cl | H |
| 276. | Br | H | Cl | Cl | Cl | Cl | Cl |
| 277. | Br | H | Cl | Cl | H | H | Cl |
| 278. | Br | H | Cl | H | Cl | Cl | H |
| 279. | Br | H | Cl | H | H | Cl | Cl |
| 280. | Br | H | H | Cl | Cl | Cl | H |
| 281. | Br | H | NO₂ | H | H | H | H |
| 282. | Br | H | H | Cl | H | H | H |
| 283. | Br | H | H | H | Cl | H | H |
| 284. | Br | H | Cl | H | Cl | H | Cl |
| 285. | Br | H | Cl | Cl | H | H | H |
| 286. | Br | H | Cl | H | H | Cl | H |
| 287. | Br | H | H | Cl | H | Cl | H |
| 288. | Br | H | H | OMe | H | H | H |
| 289. | Br | H | C(O)OMe | H | H | H | H |
| 290. | Br | H | F | Cl | H | H | H |
| 291. | Br | H | F | Me | H | H | H |
| 292. | Br | H | H | Me | H | H | H |
| 293. | Br | H | OMe | H | H | H | H |
| 294. | Br | H | F | F | F | H | H |
| 295. | Br | H | F | F | H | F | H |
| 296. | Br | H | H | F | F | F | H |
| 297. | Br | H | F | H | F | F | H |
| 298. | Br | H | Me | H | Me | H | H |
| 299. | Br | H | Me | H | H | Me | H |
| 300. | Br | H | F | H | H | CF₃ | H |
| 301. | Br | H | F | H | Br | H | H |
| 302. | Br | H | Me | Me | H | H | H |
| 303. | Br | H | F | F | F | F | F |
| 304. | Br | H | F | H | H | H | OMe |
| 305. | Br | H | Cl | H | F | H | H |
| 306. | Br | H | NO₂ | H | Cl | H | H |
| 307. | Br | H | NO₂ | H | H | Me | H |
| 308. | Br | H | F | H | H | H | I |
| 309. | Br | H | F | H | H | H | Br |
| 310. | Br | H | Br | H | H | H | Br |
| 311. | Br | H | Cl | H | H | H | Me |
| 312. | Br | H | Cl | H | H | H | OCHF₂ |
| 313. | Br | H | Cl | H | H | H | OMe |
| 314. | Br | H | Me | H | H | H | OMe |
| 315. | Br | H | OEt | H | H | H | CF₃ |
| 316. | Br | H | OC(O)Me | H | H | H | H |
| 317. | Br | H | OEt | H | H | H | Me |
| 318. | Br | H | Me | Me | H | H | Me |
| 319. | Br | H | Cl | H | H | H | C(O)OMe |
| 320. | Br | H | Cl | H | H | OMe | H |
| 321. | Br | H | F | F | H | F | F |
| 322. | Br | H | Cl | H | H | F | H |
| 323. | Br | H | F | H | H | F | Cl |
| 324. | Br | H | F | H | H | Cl | H |
| 325. | Br | H | Cl | H | H | CF₃ | H |
| 326. | Br | H | Cl | Me | H | H | H |
| 327. | Br | H | OCHF₂ | H | H | H | H |
| 328. | Br | H | OCH₂CF₃ | H | H | H | H |
| 329. | Br | H | CF₃ | H | H | H | OCHF₂ |
| 330. | Br | H | CF₃ | H | H | H | OCH₂CF₃ |
| 331. | Br | H | Me | H | H | H | Me |
| 332. | Br | H | Cl | H | H | H | F |
| 333. | Br | H | F | H | F | H | H |
| 334. | Br | H | F | Me | H | H | Cl |
| 335. | Br | H | F | H | H | OMe | H |
| 336. | Br | H | Cl | H | OCH₂O | | H |
| 337. | Br | H | Me | H | H | F | H |
| 338. | Br | H | OCF₃ | H | H | H | H |
| 339. | Br | H | F | F | H | H | H |
| 340. | Br | H | OMe | H | H | Cl | H |
| 341. | I | H | H | H | H | H | H |
| 342. | I | H | F | H | H | H | H |
| 343. | I | H | F | H | H | F | H |
| 344. | I | H | F | H | H | H | F |
| 345. | I | H | F | Me | H | H | F |
| 346. | I | H | F | H | H | H | Cl |
| 347. | I | H | CF₃ | H | H | H | H |
| 348. | I | H | Me | H | H | H | H |
| 349. | I | H | F | H | H | H | CF₃ |
| 350. | I | H | F | CF₃ | H | H | F |
| 351. | I | H | Br | H | H | H | H |
| 352. | I | H | I | H | H | H | H |
| 353. | I | H | Cl | H | H | H | H |
| 354. | I | H | Cl | H | Cl | H | H |
| 355. | I | H | Cl | H | H | H | Cl |
| 356. | I | H | H | Cl | Cl | H | H |
| 357. | I | H | Cl | Cl | Cl | H | H |
| 358. | I | H | Cl | Cl | H | Cl | H |
| 359. | I | H | Cl | Cl | Cl | H | Cl |
| 360. | I | H | Cl | Cl | Cl | Cl | H |
| 361. | I | H | Cl | Cl | Cl | Cl | Cl |
| 362. | I | H | Cl | Cl | H | H | Cl |
| 363. | I | H | Cl | H | Cl | Cl | H |
| 364. | I | H | Cl | H | H | Cl | Cl |
| 365. | I | H | H | Cl | Cl | Cl | H |
| 366. | I | H | NO₂ | H | H | H | H |
| 367. | I | H | H | Cl | H | H | H |
| 368. | I | H | H | H | Cl | H | H |
| 369. | I | H | Cl | H | Cl | H | Cl |
| 370. | I | H | Cl | Cl | H | H | H |
| 371. | I | H | Cl | H | H | Cl | H |
| 372. | I | H | H | Cl | H | Cl | H |
| 373. | I | H | H | OMe | H | H | H |
| 374. | I | H | C(O)OMe | H | H | H | H |
| 375. | I | H | F | Cl | H | H | H |
| 376. | I | H | F | Me | H | H | H |
| 377. | I | H | H | Me | H | H | H |
| 378. | I | H | OMe | H | H | H | H |
| 379. | I | H | F | F | F | H | H |
| 380. | I | H | F | F | H | F | H |
| 381. | I | H | H | F | F | F | H |
| 382. | I | H | F | H | F | F | H |
| 383. | I | H | Me | H | Me | H | H |
| 384. | I | H | Me | H | H | Me | H |
| 385. | I | H | F | H | H | CF₃ | H |
| 386. | I | H | F | H | Br | H | H |
| 387. | I | H | Me | Me | H | H | H |
| 388. | I | H | F | F | F | F | F |
| 389. | I | H | F | H | H | H | OMe |
| 390. | I | H | Cl | H | F | H | H |
| 391. | I | H | NO₂ | H | Cl | H | H |
| 392. | I | H | NO2 | H | H | Me | H |
| 393. | I | H | F | H | H | H | I |
| 394. | I | H | F | H | H | H | Br |
| 395. | I | H | Br | H | H | H | Br |
| 396. | I | H | Cl | H | H | H | Me |
| 397. | I | H | Cl | H | H | H | OCHF₂ |
| 398. | I | H | Cl | H | H | H | OMe |
| 399. | I | H | Me | H | H | H | OMe |
| 400. | I | H | OEt | H | H | H | CF₃ |
| 401. | I | H | OC(O)Me | H | H | H | H |
| 402. | I | H | OEt | H | H | H | Me |
| 403. | I | H | Me | Me | H | H | Me |
| 404. | I | H | Cl | H | H | H | C(O)OMe |
| 405. | I | H | Cl | H | H | OMe | H |
| 406. | I | H | F | F | H | F | F |
| 407. | I | H | Cl | H | H | F | H |
| 408. | I | H | F | H | H | F | Cl |
| 409. | I | H | F | H | H | Cl | H |
| 410. | I | H | Cl | H | H | CF₃ | H |
| 411. | I | H | Cl | Me | H | H | H |
| 412. | I | H | OCHF₂ | H | H | H | H |
| 413. | I | H | OCH₂CF₃ | H | H | H | H |
| 414. | I | H | CF₃ | H | H | H | OCHF₂ |
| 415. | I | H | CF₃ | H | H | H | OCH₂CF₃ |
| 416. | I | H | Me | H | H | H | Me |
| 417. | I | H | Cl | H | H | H | F |
| 418. | I | H | F | H | F | H | H |
| 419. | I | H | F | Me | H | H | Cl |
| 420. | I | H | F | H | H | OMe | H |
| 421. | I | H | Cl | H | OCH₂O | | H |
| 422. | I | H | Me | H | H | F | H |
| 423. | I | H | OCF₃ | H | H | H | H |
| 424. | I | H | F | F | H | H | H |
| 425. | I | H | OMe | H | H | Cl | H |
| 426. | H | Cl | H | H | H | H | H |
| 427. | H | Cl | F | H | H | H | H |
| 428. | H | Cl | F | H | H | F | H |
| 429. | H | Cl | F | H | H | H | F |
| 430. | H | Cl | F | Me | H | H | F |
| 431. | H | Cl | F | H | H | H | Cl |
| 432. | H | Cl | CF₃ | H | H | H | H |
| 433. | H | Cl | Me | H | H | H | H |
| 434. | H | Cl | F | H | H | H | CF₃ |
| 435. | H | Cl | F | CF₃ | H | H | F |
| 436. | H | Cl | Br | H | H | H | H |
| 437. | H | Cl | I | H | H | H | H |
| 438. | H | Cl | Cl | H | H | H | H |
| 439. | H | Cl | Cl | H | Cl | H | H |
| 440. | H | Cl | Cl | H | H | H | Cl |
| 441. | H | Cl | H | Cl | Cl | H | H |
| 442. | H | Cl | Cl | Cl | Cl | H | H |
| 443. | H | Cl | Cl | Cl | H | Cl | H |
| 444. | H | Cl | Cl | Cl | Cl | H | Cl |
| 445. | H | Cl | Cl | Cl | Cl | Cl | H |
| 446. | H | Cl | Cl | Cl | Cl | Cl | Cl |
| 447. | H | Cl | Cl | Cl | H | H | Cl |
| 448. | H | Cl | Cl | H | Cl | Cl | H |
| 449. | H | Cl | Cl | H | H | Cl | Cl |
| 450. | H | Cl | H | Cl | Cl | Cl | H |
| 451. | H | Cl | NO₂ | H | H | H | H |
| 452. | H | Cl | H | Cl | H | H | H |
| 453. | H | Cl | H | H | Cl | H | H |
| 454. | H | Cl | Cl | H | Cl | H | Cl |
| 455. | H | Cl | Cl | Cl | H | H | H |
| 456. | H | Cl | Cl | H | H | Cl | H |
| 457. | H | Cl | H | Cl | H | Cl | H |
| 458. | H | Cl | H | OMe | H | H | H |
| 459. | H | Cl | C(O)OMe | H | H | H | H |
| 460. | H | Cl | F | Cl | H | H | H |
| 461. | H | Cl | F | Me | H | H | H |
| 462. | H | Cl | H | Me | H | H | H |
| 463. | H | Cl | OMe | H | H | H | H |
| 464. | H | Cl | F | F | F | H | H |
| 465. | H | Cl | F | F | H | F | H |
| 466. | H | Cl | H | F | F | F | H |
| 467. | H | Cl | F | H | F | F | H |
| 468. | H | Cl | Me | H | Me | H | H |
| 469. | H | Cl | Me | H | H | Me | H |
| 470. | H | Cl | F | H | H | CF₃ | H |
| 471. | H | Cl | F | H | Br | H | H |
| 472. | H | Cl | Me | Me | H | H | H |
| 473. | H | Cl | F | F | F | F | F |
| 474. | H | Cl | F | H | H | H | OMe |
| 475. | H | Cl | Cl | H | F | H | H |
| 476. | H | Cl | NO₂ | H | Cl | H | H |
| 477. | H | Cl | NO₂ | H | H | Me | H |
| 478. | H | Cl | F | H | H | H | I |
| 479. | H | Cl | F | H | H | H | Br |
| 480. | H | Cl | Br | H | H | H | Br |
| 481. | H | Cl | Cl | H | H | H | Me |
| 482. | H | Cl | Cl | H | H | H | OCHF₂ |
| 483. | H | Cl | Cl | H | H | H | OMe |
| 484. | H | Cl | Me | H | H | H | OMe |
| 485. | H | Cl | OEt | H | H | H | CF₃ |
| 486. | H | Cl | OC(O)Me | H | H | H | H |
| 487. | H | Cl | OEt | H | H | H | Me |
| 488. | H | Cl | Me | Me | H | H | Me |
| 489. | H | Cl | Cl | H | H | H | C(O)OMe |
| 490. | H | Cl | Cl | H | H | OMe | H |
| 491. | H | Cl | F | F | H | F | F |
| 492. | H | Cl | Cl | H | H | F | H |
| 493. | H | Cl | F | H | H | F | Cl |
| 494. | H | Cl | F | H | H | Cl | H |
| 495. | H | Cl | Cl | H | H | CF₃ | H |
| 496. | H | Cl | Cl | Me | H | H | H |
| 497. | H | Cl | OCHF₂ | H | H | H | H |
| 498. | H | Cl | OCH₂CF₃ | H | H | H | H |
| 499. | H | Cl | CF₃ | H | H | H | OCHF₂ |
| 500. | H | Cl | CF₃ | H | H | H | OCH₂CF₃ |
| 501. | H | Cl | Me | H | H | H | Me |
| 502. | H | Cl | Cl | H | H | H | F |
| 503. | H | Cl | F | H | F | H | H |
| 504. | H | Cl | F | Me | H | H | Cl |
| 505. | H | Cl | F | H | H | OMe | H |
| 506. | H | Cl | Cl | H | OCH₂O | | H |
| 507. | H | Cl | Me | H | H | F | H |
| 508. | H | Cl | OCF₃ | H | H | H | H |
| 509. | H | Cl | F | F | H | H | H |
| 510. | H | Cl | OMe | H | H | Cl | H |
| 511. | H | Br | H | H | H | H | H |
| 512. | H | Br | F | H | H | H | H |
| 513. | H | Br | F | H | H | F | H |
| 514. | H | Br | F | H | H | H | F |
| 515. | H | Br | F | Me | H | H | F |
| 516. | H | Br | F | H | H | H | Cl |
| 517. | H | Br | CF₃ | H | H | H | H |
| 518. | H | Br | Me | H | H | H | H |
| 519. | H | Br | F | H | H | H | CF₃ |
| 520. | H | Br | F | CF₃ | H | H | F |
| 521. | H | Br | Br | H | H | H | H |
| 522. | H | Br | I | H | H | H | H |
| 523. | H | Br | Cl | H | H | H | H |
| 524. | H | Br | Cl | H | Cl | H | H |
| 525. | H | Br | Cl | H | H | H | Cl |
| 526. | H | Br | H | Cl | Cl | H | H |
| 527. | H | Br | Cl | Cl | Cl | H | H |
| 528. | H | Br | Cl | Cl | H | Cl | H |
| 529. | H | Br | Cl | Cl | Cl | H | Cl |
| 530. | H | Br | Cl | Cl | Cl | Cl | H |
| 531. | H | Br | Cl | Cl | Cl | Cl | Cl |
| 532. | H | Br | Cl | Cl | H | H | Cl |
| 533. | H | Br | Cl | H | Cl | Cl | H |
| 534. | H | Br | Cl | H | H | Cl | Cl |
| 535. | H | Br | H | Cl | Cl | Cl | H |
| 536. | H | Br | NO₂ | H | H | H | H |
| 537. | H | Br | H | Cl | H | H | H |
| 538. | H | Br | H | H | Cl | H | H |
| 539. | H | Br | Cl | H | Cl | H | Cl |
| 540. | H | Br | Cl | Cl | H | H | H |
| 541. | H | Br | Cl | H | H | Cl | H |
| 542. | H | Br | H | Cl | H | Cl | H |
| 543. | H | Br | H | OMe | H | H | H |
| 544. | H | Br | C(O)OMe | H | H | H | H |
| 545. | H | Br | F | Cl | H | H | H |
| 546. | H | Br | F | Me | H | H | H |
| 547. | H | Br | H | Me | H | H | H |
| 548. | H | Br | OMe | H | H | H | H |
| 549. | H | Br | F | F | F | H | H |
| 550. | H | Br | F | F | H | F | H |
| 551. | H | Br | H | F | F | F | H |
| 552. | H | Br | F | H | F | F | H |
| 553. | H | Br | Me | H | Me | H | H |
| 554. | H | Br | Me | H | H | Me | H |
| 555. | H | Br | F | H | H | CF₃ | H |
| 556. | H | Br | F | H | Br | H | H |
| 557. | H | Br | Me | Me | H | H | H |
| 558. | H | Br | F | F | F | F | F |
| 559. | H | Br | F | H | H | H | OMe |
| 560. | H | Br | Cl | H | F | H | H |
| 561. | H | Br | NO₂ | H | Cl | H | H |
| 562. | H | Br | NO₂ | H | H | Me | H |
| 563. | H | Br | F | H | H | H | I |
| 564. | H | Br | F | H | H | H | Br |
| 565. | H | Br | Br | H | H | H | Br |
| 566. | H | Br | Cl | H | H | H | Me |
| 567. | H | Br | Cl | H | H | H | OCHF₂ |
| 568. | H | Br | Cl | H | H | H | OMe |
| 569. | H | Br | Me | H | H | H | OMe |
| 570. | H | Br | OEt | H | H | H | CF₃ |
| 571. | H | Br | OC(O)Me | H | H | H | H |
| 572. | H | Br | OEt | H | H | H | Me |
| 573. | H | Br | Me | Me | H | H | Me |
| 574. | H | Br | Cl | H | H | H | C(O)OMe |
| 575. | H | Br | Cl | H | H | OMe | H |
| 576. | H | Br | F | F | H | F | F |
| 577. | H | Br | Cl | H | H | F | H |
| 578. | H | Br | F | H | H | F | Cl |
| 579. | H | Br | F | H | H | Cl | H |
| 580. | H | Br | Cl | H | H | CF₃ | H |
| 581. | H | Br | Cl | Me | H | H | H |
| 582. | H | Br | OCHF₂ | H | H | H | H |
| 583. | H | Br | OCH₂CF₃ | H | H | H | H |
| 584. | H | Br | CF₃ | H | H | H | OCHF₂ |
| 585. | H | Br | CF₃ | H | H | H | OCH₂CF₃ |
| 586. | H | Br | Me | H | H | H | Me |
| 587. | H | Br | Cl | H | H | H | F |
| 588. | H | Br | F | H | F | H | H |
| 589. | H | Br | F | Me | H | H | Cl |
| 590. | H | Br | F | H | H | OMe | H |
| 591. | H | Br | Cl | H | OCH₂O | | H |
| 592. | H | Br | Me | H | H | F | H |
| 593. | H | Br | OCF₃ | H | H | H | H |
| 594. | H | Br | F | F | H | H | H |
| 595. | H | Br | OMe | H | H | Cl | H |
| 596. | Me | H | H | H | H | H | H |
| 597. | Me | H | F | H | H | H | H |
| 598. | Me | H | F | H | H | F | H |
| 599. | Me | H | F | H | H | H | F |
| 600. | Me | H | F | Me | H | H | F |
| 601. | Me | H | F | H | H | H | Cl |
| 602. | Me | H | CF₃ | H | H | H | H |
| 603. | Me | H | Me | H | H | H | H |
| 604. | Me | H | F | H | H | H | CF₃ |
| 605. | Me | H | F | CF₃ | H | H | F |
| 606. | Me | H | Br | H | H | H | H |
| 607. | Me | H | I | H | H | H | H |
| 608. | Me | H | Cl | H | H | H | H |
| 609. | Me | H | Cl | H | Cl | H | H |
| 610. | Me | H | Cl | H | H | H | Cl |
| 611. | Me | H | H | Cl | Cl | H | H |
| 612. | Me | H | Cl | Cl | Cl | H | H |
| 613. | Me | H | Cl | Cl | H | Cl | H |
| 614. | Me | H | Cl | Cl | Cl | H | Cl |
| 615. | Me | H | Cl | Cl | Cl | Cl | H |
| 616. | Me | H | Cl | Cl | Cl | Cl | Cl |
| 617. | Me | H | Cl | Cl | H | H | Cl |
| 618. | Me | H | Cl | H | Cl | Cl | H |
| 619. | Me | H | Cl | H | H | Cl | Cl |
| 620. | Me | H | H | Cl | Cl | Cl | H |
| 621. | Me | H | NO₂ | H | H | H | H |
| 622. | Me | H | H | Cl | H | H | H |
| 623. | Me | H | H | H | Cl | H | H |
| 624. | Me | H | Cl | H | Cl | H | Cl |
| 625. | Me | H | Cl | Cl | H | H | H |
| 626. | Me | H | Cl | H | H | Cl | H |
| 627. | Me | H | H | Cl | H | Cl | H |
| 628. | Me | H | H | OMe | H | H | H |
| 629. | Me | H | C(O)OMe | H | H | H | H |
| 630. | Me | H | F | Cl | H | H | H |
| 631. | Me | H | F | Me | H | H | H |
| 632. | Me | H | H | Me | H | H | H |
| 633. | Me | H | OMe | H | H | H | H |
| 634. | Me | H | F | F | F | H | H |
| 635. | Me | H | F | F | H | F | H |
| 636. | Me | H | H | F | F | F | H |
| 637. | Me | H | F | H | F | F | H |
| 638. | Me | H | Me | H | Me | H | H |
| 639. | Me | H | Me | H | H | Me | H |
| 640. | Me | H | F | H | H | CF₃ | H |
| 641. | Me | H | F | H | Br | H | H |
| 642. | Me | H | Me | Me | H | H | H |
| 643. | Me | H | F | F | F | F | F |
| 644. | Me | H | F | H | H | H | OMe |
| 645. | Me | H | Cl | H | F | H | H |
| 646. | Me | H | NO₂ | H | Cl | H | H |
| 647. | Me | H | NO₂ | H | H | Me | H |
| 648. | Me | H | F | H | H | H | I |
| 649. | Me | H | F | H | H | H | Br |
| 650. | Me | H | Br | H | H | H | Br |
| 651. | Me | H | Cl | H | H | H | Me |
| 652. | Me | H | Cl | H | H | H | OCHF₂ |
| 653. | Me | H | Cl | H | H | H | OMe |
| 654. | Me | H | Me | H | H | H | OMe |
| 655. | Me | H | OEt | H | H | H | CF₃ |
| 656. | Me | H | OC(O)Me | H | H | H | H |
| 657. | Me | H | OEt | H | H | H | Me |
| 658. | Me | H | Me | Me | H | H | Me |
| 659. | Me | H | Cl | H | H | H | C(O)OMe |
| 660. | Me | H | Cl | H | H | OMe | H |
| 661. | Me | H | F | F | H | F | F |
| 662. | Me | H | Cl | H | H | F | H |
| 663. | Me | H | F | H | H | F | Cl |
| 664. | Me | H | F | H | H | Cl | H |
| 665. | Me | H | Cl | H | H | CF₃ | H |
| 666. | Me | H | Cl | Me | H | H | H |
| 667. | Me | H | OCHF₂ | H | H | H | H |
| 668. | Me | H | OCH₂CF₃ | H | H | H | H |
| 669. | Me | H | CF₃ | H | H | H | OCHF₂ |
| 670. | Me | H | CF₃ | H | H | H | OCH₂CF₃ |
| 671. | Me | H | Me | H | H | H | Me |
| 672. | Me | H | Cl | H | H | H | F |
| 673. | Me | H | F | H | F | H | H |
| 674. | Me | H | F | Me | H | H | Cl |
| 675. | Me | H | F | H | H | OMe | H |
| 676. | Me | H | Cl | H | OCH₂O | | H |
| 677. | Me | H | Me | H | H | F | H |
| 678. | Me | H | OCF₃ | H | H | H | H |
| 679. | Me | H | F | F | H | H | H |
| 680. | Me | H | OMe | H | H | Cl | H |
| 681. | H | Me | H | H | H | H | H |
| 682. | H | Me | F | H | H | H | H |
| 683. | H | Me | F | H | H | F | H |
| 684. | H | Me | F | H | H | H | F |
| 685. | H | Me | F | Me | H | H | F |
| 686. | H | Me | F | H | H | H | Cl |
| 687. | H | Me | CF₃ | H | H | H | H |
| 688. | H | Me | Me | H | H | H | H |
| 689. | H | Me | F | H | H | H | CF₃ |
| 690. | H | Me | F | CF₃ | H | H | F |
| 691. | H | Me | Br | H | H | H | H |
| 692. | H | Me | I | H | H | H | H |
| 693. | H | Me | Cl | H | H | H | H |
| 694. | H | Me | Cl | H | Cl | H | H |
| 695. | H | Me | Cl | H | H | H | Cl |
| 696. | H | Me | H | Cl | Cl | H | H |
| 697. | H | Me | Cl | Cl | Cl | H | H |
| 698. | H | Me | Cl | Cl | H | Cl | H |
| 699. | H | Me | Cl | Cl | Cl | H | Cl |
| 700. | H | Me | Cl | Cl | Cl | Cl | H |
| 701. | H | Me | Cl | Cl | Cl | Cl | Cl |
| 702. | H | Me | Cl | Cl | H | H | Cl |
| 703. | H | Me | Cl | H | Cl | Cl | H |
| 704. | H | Me | Cl | H | H | Cl | Cl |
| 705. | H | Me | H | Cl | Cl | Cl | H |
| 706. | H | Me | NO₂ | H | H | H | H |
| 707. | H | Me | H | Cl | H | H | H |
| 708. | H | Me | H | H | Cl | H | H |
| 709. | H | Me | Cl | H | Cl | H | Cl |
| 710. | H | Me | Cl | Cl | H | H | H |
| 711. | H | Me | Cl | H | H | Cl | H |
| 712. | H | Me | H | Cl | H | Cl | H |
| 713. | H | Me | H | OMe | H | H | H |
| 714. | H | Me | C(O)OMe | H | H | H | H |
| 715. | H | Me | F | Cl | H | H | H |
| 716. | H | Me | F | Me | H | H | H |
| 717. | H | Me | H | Me | H | H | H |
| 718. | H | Me | OMe | H | H | H | H |
| 719. | H | Me | F | F | F | H | H |
| 720. | H | Me | F | F | H | F | H |
| 721. | H | Me | H | F | F | F | H |
| 722. | H | Me | F | H | F | F | H |
| 723. | H | Me | Me | H | Me | H | H |
| 724. | H | Me | Me | H | H | Me | H |
| 725. | H | Me | F | H | H | CF₃ | H |
| 726. | H | Me | F | H | Br | H | H |
| 727. | H | Me | Me | Me | H | H | H |
| 728. | H | Me | F | F | F | F | F |
| 729. | H | Me | F | H | H | H | OMe |
| 730. | H | Me | Cl | H | F | H | H |
| 731. | H | Me | NO₂ | H | Cl | H | H |
| 732. | H | Me | NO₂ | H | H | Me | H |
| 733. | H | Me | F | H | H | H | I |
| 734. | H | Me | F | H | H | H | Br |
| 735. | H | Me | Br | H | H | H | Br |
| 736. | H | Me | Cl | H | H | H | Me |
| 737. | H | Me | Cl | H | H | H | OCHF₂ |
| 738. | H | Me | Cl | H | H | H | OMe |
| 739. | H | Me | Me | H | H | H | OMe |
| 740. | H | Me | OEt | H | H | H | CF₃ |
| 741. | H | Me | OC(O)Me | H | H | H | H |
| 742. | H | Me | OEt | H | H | H | Me |
| 743. | H | Me | Me | Me | H | H | Me |
| 744. | H | Me | Cl | H | H | H | C(O)OMe |
| 745. | H | Me | Cl | H | H | OMe | H |
| 746. | H | Me | F | F | H | F | F |
| 747. | H | Me | Cl | H | H | F | H |
| 748. | H | Me | F | H | H | F | Cl |
| 749. | H | Me | F | H | H | Cl | H |
| 750. | H | Me | Cl | H | H | CF₃ | H |
| 751. | H | Me | Cl | Me | H | H | H |
| 752. | H | Me | OCHF₂ | H | H | H | H |
| 753. | H | Me | OCH₂CF₃ | H | H | H | H |
| 754. | H | Me | CF₃ | H | H | H | OCHF₂ |
| 755. | H | Me | CF₃ | H | H | H | OCH₂CF₃ |
| 756. | H | Me | Me | H | H | H | Me |
| 757. | H | Me | Cl | H | H | H | F |
| 758. | H | Me | F | H | F | H | H |
| 759. | H | Me | F | Me | H | H | Cl |
| 760. | H | Me | F | H | H | OMe | H |
| 761. | H | Me | Cl | H | OCH₂O | | H |
| 762. | H | Me | Me | H | H | F | H |
| 763. | H | Me | OCF₃ | H | H | H | H |
| 764. | H | Me | F | F | H | H | H |
| 765. | H | Me | OMe | H | H | Cl | H |
| 851. | CHF₂ | H | H | H | H | H | H |
| 852. | CHF₂ | H | F | H | H | H | H |
| 853. | CHF₂ | H | F | H | H | F | H |
| 854. | CHF₂ | H | F | H | H | H | F |
| 855. | CHF₂ | H | F | Me | H | H | F |
| 856. | CHF₂ | H | F | H | H | H | Cl |
| 857. | CHF₂ | H | CF₃ | H | H | H | H |
| 858. | CHF₂ | H | Me | H | H | H | H |
| 859. | CHF₂ | H | F | H | H | H | CF₃ |
| 860. | CHF₂ | H | F | CF₃ | H | H | F |
| 861. | CHF₂ | H | Br | H | H | H | H |
| 862. | CHF₂ | H | I | H | H | H | H |
| 863. | CHF₂ | H | Cl | H | H | H | H |
| 864. | CHF₂ | H | Cl | H | Cl | H | H |
| 865. | CHF₂ | H | Cl | H | H | H | Cl |
| 866. | CHF₂ | H | H | Cl | Cl | H | H |
| 867. | CHF₂ | H | Cl | Cl | Cl | H | H |
| 868. | CHF₂ | H | Cl | Cl | H | Cl | H |
| 869. | CHF₂ | H | Cl | Cl | Cl | H | Cl |
| 870. | CHF₂ | H | Cl | Cl | Cl | Cl | H |
| 871. | CHF₂ | H | Cl | Cl | Cl | Cl | Cl |
| 872. | CHF₂ | H | Cl | Cl | H | H | Cl |
| 873. | CHF₂ | H | Cl | H | Cl | Cl | H |
| 874. | CHF₂ | H | Cl | H | H | Cl | Cl |
| 875. | CHF₂ | H | H | Cl | Cl | Cl | H |
| 876. | CHF₂ | H | NO₂ | H | H | H | H |
| 877. | CHF₂ | H | H | Cl | H | H | H |
| 878. | CHF₂ | H | H | H | Cl | H | H |
| 879. | CHF₂ | H | Cl | H | Cl | H | Cl |
| 880. | CHF₂ | H | Cl | Cl | H | H | H |
| 881. | CHF₂ | H | Cl | H | H | Cl | H |
| 882. | CHF₂ | H | H | Cl | H | Cl | H |
| 883. | CHF₂ | H | H | OMe | H | H | H |
| 884. | CHF₂ | H | C(O)OMe | H | H | H | H |
| 885. | CHF₂ | H | F | Cl | H | H | H |
| 886. | CHF₂ | H | F | Me | H | H | H |
| 887. | CHF₂ | H | H | Me | H | H | H |
| 888. | CHF₂ | H | OMe | H | H | H | H |
| 889. | CHF₂ | H | F | F | F | H | H |
| 890. | CHF₂ | H | F | F | H | F | H |
| 891. | CHF₂ | H | H | F | F | F | H |
| 892. | CHF₂ | H | F | H | F | F | H |
| 893. | CHF₂ | H | Me | H | Me | H | H |
| 894. | CHF₂ | H | Me | H | H | Me | H |
| 895. | CHF₂ | H | F | H | H | CF₃ | H |
| 896. | CHF₂ | H | F | H | Br | H | H |
| 897. | CHF₂ | H | Me | Me | H | H | H |
| 898. | CHF₂ | H | F | F | F | F | F |
| 899. | CHF₂ | H | F | H | H | H | OMe |
| 900. | CHF₂ | H | Cl | H | F | H | H |
| 901. | CHF₂ | H | NO₂ | H | Cl | H | H |
| 902. | CHF₂ | H | NO₂ | H | H | Me | H |
| 903. | CHF₂ | H | F | H | H | H | I |
| 904. | CHF₂ | H | F | H | H | H | Br |
| 905. | CHF₂ | H | Br | H | H | H | Br |
| 906. | CHF₂ | H | Cl | H | H | H | Me |
| 907. | CHF₂ | H | Cl | H | H | H | OCHF₂ |
| 908. | CHF₂ | H | Cl | H | H | H | OMe |
| 909. | CHF₂ | H | Me | H | H | H | OMe |
| 910. | CHF₂ | H | OEt | H | H | H | CF₃ |
| 911. | CHF₂ | H | OC(O)Me | H | H | H | H |
| 912. | CHF₂ | H | OEt | H | H | H | Me |
| 913. | CHF₂ | H | Me | Me | H | H | Me |
| 914. | CHF₂ | H | Cl | H | H | H | C(O)OMe |
| 915. | CHF₂ | H | Cl | H | H | OMe | H |
| 916. | CHF₂ | H | F | F | H | F | F |
| 917. | CHF₂ | H | Cl | H | H | F | H |
| 918. | CHF₂ | H | F | H | H | F | Cl |
| 919. | CHF₂ | H | F | H | H | Cl | H |
| 920. | CHF₂ | H | Cl | H | H | CF₃ | H |
| 921. | CHF₂ | H | Cl | Me | H | H | H |
| 922. | CHF₂ | H | OCHF₂ | H | H | H | H |
| 923. | CHF₂ | H | OCH₂CF₃ | H | H | H | H |
| 924. | CHF₂ | H | CF₃ | H | H | H | OCHF₂ |
| 925. | CHF₂ | H | CF₃ | H | H | H | OCH₂CF₃ |
| 926. | CHF₂ | H | Me | H | H | H | Me |
| 927. | CHF₂ | H | Cl | H | H | H | F |
| 928. | CHF₂ | H | F | H | F | H | H |
| 929. | CHF₂ | H | F | Me | H | H | Cl |
| 930. | CHF₂ | H | F | H | H | OMe | H |
| 931. | CHF₂ | H | Cl | H | OCH₂O | | H |
| 932. | CHF₂ | H | Me | H | H | F | H |
| 933. | CHF₂ | H | OCF₃ | H | H | H | H |
| 934. | CHF₂ | H | F | F | H | H | H |
| 935. | CHF₂ | H | OMe | H | H | Cl | H |
| 936. | Cl | Cl | H | H | H | H | H |
| 937. | Cl | Cl | F | H | H | H | H |
| 938. | Cl | Cl | F | H | H | F | H |
| 939. | Cl | Cl | F | H | H | H | F |
| 940. | Cl | Cl | F | Me | H | H | F |
| 941. | Cl | Cl | F | H | H | H | Cl |
| 942. | Cl | Cl | CF₃ | H | H | H | H |
| 943. | Cl | Cl | Me | H | H | H | H |
| 944. | Cl | Cl | F | H | H | H | CF₃ |
| 945. | Cl | Cl | F | CF₃ | H | H | F |
| 946. | Cl | Cl | Br | H | H | H | H |
| 947. | Cl | Cl | I | H | H | H | H |
| 948. | Cl | Cl | Cl | H | H | H | H |
| 949. | Cl | Cl | Cl | H | Cl | H | H |
| 950. | Cl | Cl | Cl | H | H | H | Cl |
| 951. | Cl | Cl | H | Cl | Cl | H | H |
| 952. | Cl | Cl | Cl | Cl | Cl | H | H |
| 953. | Cl | Cl | Cl | Cl | H | Cl | H |
| 954. | Cl | Cl | Cl | Cl | Cl | H | Cl |
| 955. | Cl | Cl | Cl | Cl | Cl | Cl | H |
| 956. | Cl | Cl | Cl | Cl | Cl | Cl | Cl |
| 957. | Cl | Cl | Cl | Cl | H | H | Cl |
| 958. | Cl | Cl | Cl | H | Cl | Cl | H |
| 959. | Cl | Cl | Cl | H | H | Cl | Cl |
| 960. | Cl | Cl | H | Cl | Cl | Cl | H |
| 961. | Cl | Cl | NO₂ | H | H | H | H |
| 962. | Cl | Cl | H | Cl | H | H | H |
| 963. | Cl | Cl | H | H | Cl | H | H |
| 964. | Cl | Cl | Cl | H | Cl | H | Cl |
| 965. | Cl | Cl | Cl | Cl | H | H | H |
| 966. | Cl | Cl | Cl | H | H | Cl | H |
| 967. | Cl | Cl | H | Cl | H | Cl | H |
| 968. | Cl | Cl | H | OMe | H | H | H |
| 969. | Cl | Cl | C(O)OMe | H | H | H | H |
| 970. | Cl | Cl | F | Cl | H | H | H |
| 971. | Cl | Cl | F | Me | H | H | H |
| 972. | Cl | Cl | H | Me | H | H | H |
| 973. | Cl | Cl | OMe | H | H | H | H |
| 974. | Cl | Cl | F | F | F | H | H |
| 975. | Cl | Cl | F | F | H | F | H |
| 976. | Cl | Cl | H | F | F | F | H |
| 977. | Cl | Cl | F | H | F | F | H |
| 978. | Cl | Cl | Me | H | Me | H | H |
| 979. | Cl | Cl | Me | H | H | Me | H |
| 980. | Cl | Cl | F | H | H | CF₃ | H |
| 981. | Cl | Cl | F | H | Br | H | H |
| 982. | Cl | Cl | Me | Me | H | H | H |
| 983. | Cl | Cl | F | F | F | F | F |
| 984. | Cl | Cl | F | H | H | H | OMe |
| 985. | Cl | Cl | Cl | H | F | H | H |
| 986. | Cl | Cl | NO₂ | H | Cl | H | H |
| 987. | Cl | Cl | NO₂ | H | H | Me | H |
| 988. | Cl | Cl | F | H | H | H | I |
| 989. | Cl | Cl | F | H | H | H | Br |
| 990. | Cl | Cl | Br | H | H | H | Br |
| 991. | Cl | Cl | Cl | H | H | H | Me |
| 992. | Cl | Cl | Cl | H | H | H | OCHF₂ |
| 993. | Cl | Cl | Cl | H | H | H | OMe |
| 994. | Cl | Cl | Me | H | H | H | OMe |
| 995. | Cl | Cl | OEt | H | H | H | CF₃ |
| 996. | Cl | Cl | OC(O)Me | H | H | H | H |
| 997. | Cl | Cl | OEt | H | H | H | Me |
| 998. | Cl | Cl | Me | Me | H | H | Me |
| 999. | Cl | Cl | Cl | H | H | H | C(O)OMe |
| 1000. | Cl | Cl | Cl | H | H | OMe | H |
| 1001. | Cl | Cl | F | F | H | F | F |
| 1002. | Cl | Cl | Cl | H | H | F | H |
| 1003. | Cl | Cl | F | H | H | F | Cl |
| 1004. | Cl | Cl | F | H | H | Cl | H |
| 1005. | Cl | Cl | Cl | H | H | CF₃ | H |
| 1006. | Cl | Cl | Cl | Me | H | H | H |
| 1007. | Cl | Cl | OCHF₂ | H | H | H | H |
| 1008. | Cl | Cl | OCH₂CF₃ | H | H | H | H |
| 1009. | Cl | Cl | CF₃ | H | H | H | OCHF₂ |
| 1010. | Cl | Cl | CF₃ | H | H | H | OCH₂CF₃ |
| 1011. | Cl | Cl | Me | H | H | H | Me |
| 1012. | Cl | Cl | Cl | H | H | H | F |
| 1013. | Cl | Cl | F | H | F | H | H |
| 1014. | Cl | Cl | F | Me | H | H | Cl |
| 1015. | Cl | Cl | F | H | H | OMe | H |
| 1016. | Cl | Cl | Cl | H | OCH₂O | | H |
| 1017. | Cl | Cl | Me | H | H | F | H |
| 1018. | Cl | Cl | OCF₃ | H | H | H | H |
| 1019. | Cl | Cl | F | F | H | H | H |
| 1020. | Cl | Cl | OMe | H | H | Cl | H |
| 1021. | Me | Cl | H | H | H | H | H |
| 1022. | Me | Cl | F | H | H | H | H |
| 1023. | Me | Cl | F | H | H | F | H |
| 1024. | Me | Cl | F | H | H | H | F |
| 1025. | Me | Cl | F | Me | H | H | F |
| 1026. | Me | Cl | F | H | H | H | Cl |
| 1027. | Me | Cl | CF₃ | H | H | H | H |
| 1028. | Me | Cl | Me | H | H | H | H |
| 1029. | Me | Cl | F | H | H | H | CF₃ |
| 1030. | Me | Cl | F | CF₃ | H | H | F |
| 1031. | Me | Cl | Br | H | H | H | H |
| 1032. | Me | Cl | I | H | H | H | H |
| 1033. | Me | Cl | Cl | H | H | H | H |
| 1034. | Me | Cl | Cl | H | Cl | H | H |
| 1035. | Me | Cl | Cl | H | H | H | Cl |
| 1036. | Me | Cl | H | Cl | Cl | H | H |
| 1037. | Me | Cl | Cl | Cl | Cl | H | H |
| 1038. | Me | Cl | Cl | Cl | H | Cl | H |
| 1039. | Me | Cl | Cl | Cl | Cl | H | Cl |
| 1040. | Me | Cl | Cl | Cl | Cl | Cl | H |
| 1041. | Me | Cl | Cl | Cl | Cl | Cl | Cl |
| 1042. | Me | Cl | Cl | Cl | H | H | Cl |
| 1043. | Me | Cl | Cl | H | Cl | Cl | H |
| 1044. | Me | Cl | Cl | H | H | Cl | Cl |
| 1045. | Me | Cl | H | Cl | Cl | Cl | H |
| 1046. | Me | Cl | NO₂ | H | H | H | H |
| 1047. | Me | Cl | H | Cl | H | H | H |
| 1048. | Me | Cl | H | H | Cl | H | H |
| 1049. | Me | Cl | Cl | H | Cl | H | Cl |
| 1050. | Me | Cl | Cl | Cl | H | H | H |
| 1051. | Me | Cl | Cl | H | H | Cl | H |
| 1052. | Me | Cl | H | Cl | H | Cl | H |
| 1053. | Me | Cl | H | OMe | H | H | H |
| 1054. | Me | Cl | C(O)OMe | H | H | H | H |
| 1055. | Me | Cl | F | Cl | H | H | H |
| 1056. | Me | Cl | F | Me | H | H | H |
| 1057. | Me | Cl | H | Me | H | H | H |
| 1058. | Me | Cl | OMe | H | H | H | H |
| 1059. | Me | Cl | F | F | F | H | H |
| 1060. | Me | Cl | F | F | H | F | H |
| 1061. | Me | Cl | H | F | F | F | H |
| 1062. | Me | Cl | F | H | F | F | H |
| 1063. | Me | Cl | Me | H | Me | H | H |
| 1064. | Me | Cl | Me | H | H | Me | H |
| 1065. | Me | Cl | F | H | H | CF₃ | H |
| 1066. | Me | Cl | F | H | Br | H | H |
| 1067. | Me | Cl | Me | Me | H | H | H |
| 1068. | Me | Cl | F | F | F | F | F |
| 1069. | Me | Cl | F | H | H | H | OMe |
| 1070. | Me | Cl | Cl | H | F | H | H |
| 1071. | Me | Cl | NO₂ | H | Cl | H | H |
| 1072. | Me | Cl | NO₂ | H | H | Me | H |
| 1073. | Me | Cl | F | H | H | H | I |
| 1074. | Me | Cl | F | H | H | H | Br |
| 1075. | Me | Cl | Br | H | H | H | Br |
| 1076. | Me | Cl | Cl | H | H | H | Me |
| 1077. | Me | Cl | Cl | H | H | H | OCHF₂ |
| 1078. | Me | Cl | Cl | H | H | H | OMe |
| 1079. | Me | Cl | Me | H | H | H | OMe |
| 1080. | Me | Cl | OEt | H | H | H | CF₃ |
| 1081. | Me | Cl | OC(O)Me | H | H | H | H |
| 1082. | Me | Cl | OEt | H | H | H | Me |
| 1083. | Me | Cl | Me | Me | H | H | Me |
| 1084. | Me | Cl | Cl | H | H | H | C(O)OMe |
| 1085. | Me | Cl | Cl | H | H | OMe | H |
| 1086. | Me | Cl | F | F | H | F | F |
| 1087. | Me | Cl | Cl | H | H | F | H |
| 1088. | Me | Cl | F | H | H | F | Cl |
| 1089. | Me | Cl | F | H | H | Cl | H |
| 1090. | Me | Cl | Cl | H | H | CF₃ | H |
| 1091. | Me | Cl | Cl | Me | H | H | H |
| 1092. | Me | Cl | OCHF₂ | H | H | H | H |
| 1093. | Me | Cl | OCH₂CF₃ | H | H | H | H |
| 1094. | Me | Cl | CF₃ | H | H | H | OCHF₂ |
| 1095. | Me | Cl | CF₃ | H | H | H | OCH₂CF₃ |
| 1096. | Me | Cl | Me | H | H | H | Me |
| 1097. | Me | Cl | Cl | H | H | H | F |
| 1098. | Me | Cl | F | H | F | H | H |
| 1099. | Me | Cl | F | Me | H | H | Cl |
| 1100. | Me | Cl | F | H | H | OMe | H |
| 1101. | Me | Cl | Cl | H | OCH₂O | | H |
| 1102. | Me | Cl | Me | H | H | F | H |
| 1103. | Me | Cl | OCF₃ | H | H | H | H |
| 1104. | Me | Cl | F | F | H | H | H |
| 1105. | Me | Cl | OMe | H | H | Cl | H |
| 1106. | Cl | Me | H | H | H | H | H |
| 1107. | Cl | Me | F | H | H | H | H |
| 1108. | Cl | Me | F | H | H | F | H |
| 1109. | Cl | Me | F | H | H | H | F |
| 1110. | Cl | Me | F | Me | H | H | F |
| 1111. | Cl | Me | F | H | H | H | Cl |
| 1112. | Cl | Me | CF₃ | H | H | H | H |
| 1113. | Cl | Me | Me | H | H | H | H |
| 1114. | Cl | Me | F | H | H | H | CF₃ |
| 1115. | Cl | Me | F | CF₃ | H | H | F |
| 1116. | Cl | Me | Br | H | H | H | H |
| 1117. | Cl | Me | I | H | H | H | H |
| 1118. | Cl | Me | Cl | H | H | H | H |
| 1119. | Cl | Me | Cl | H | Cl | H | H |
| 1120. | Cl | Me | Cl | H | H | H | Cl |
| 1121. | Cl | Me | H | Cl | Cl | H | H |
| 1122. | Cl | Me | Cl | Cl | Cl | H | H |
| 1123. | Cl | Me | Cl | Cl | H | Cl | H |
| 1124. | Cl | Me | Cl | Cl | Cl | H | Cl |
| 1125. | Cl | Me | Cl | Cl | Cl | Cl | H |
| 1126. | Cl | Me | Cl | Cl | Cl | Cl | Cl |
| 1127. | Cl | Me | Cl | Cl | H | H | Cl |
| 1128. | Cl | Me | Cl | H | Cl | Cl | H |
| 1129. | Cl | Me | Cl | H | H | Cl | Cl |
| 1130. | Cl | Me | H | Cl | Cl | Cl | H |
| 1131. | Cl | Me | NO₂ | H | H | H | H |
| 1132. | Cl | Me | H | Cl | H | H | H |
| 1133. | Cl | Me | H | H | Cl | H | H |
| 1134. | Cl | Me | Cl | H | Cl | H | Cl |
| 1135. | Cl | Me | Cl | Cl | H | H | H |
| 1136. | Cl | Me | Cl | H | H | Cl | H |
| 1137. | Cl | Me | H | Cl | H | Cl | H |
| 1138. | Cl | Me | H | OMe | H | H | H |
| 1139. | Cl | Me | C(O)OMe | H | H | H | H |
| 1140. | Cl | Me | F | Cl | H | H | H |
| 1141. | Cl | Me | F | Me | H | H | H |
| 1142. | Cl | Me | H | Me | H | H | H |
| 1143. | Cl | Me | OMe | H | H | H | H |
| 1144. | Cl | Me | F | F | F | H | H |
| 1145. | Cl | Me | F | F | H | F | H |
| 1146. | Cl | Me | H | F | F | F | H |
| 1147. | Cl | Me | F | H | F | F | H |
| 1148. | Cl | Me | Me | H | Me | H | H |
| 1149. | Cl | Me | Me | H | H | Me | H |
| 1150. | Cl | Me | F | H | H | CF₃ | H |
| 1151. | Cl | Me | F | H | Br | H | H |
| 1152. | Cl | Me | Me | Me | H | H | H |
| 1153. | Cl | Me | F | F | F | F | F |
| 1154. | Cl | Me | F | H | H | H | OMe |
| 1155. | Cl | Me | Cl | H | F | H | H |
| 1156. | Cl | Me | NO₂ | H | Cl | H | H |
| 1157. | Cl | Me | NO₂ | H | H | Me | H |
| 1158. | Cl | Me | F | H | H | H | I |
| 1159. | Cl | Me | F | H | H | H | Br |
| 1160. | Cl | Me | Br | H | H | H | Br |
| 1161. | Cl | Me | Cl | H | H | H | Me |
| 1162. | Cl | Me | Cl | H | H | H | OCHF₂ |
| 1163. | Cl | Me | Cl | H | H | H | OMe |
| 1164. | Cl | Me | Me | H | H | H | OMe |
| 1165. | Cl | Me | OEt | H | H | H | CF₃ |
| 1166. | Cl | Me | OC(O)Me | H | H | H | H |
| 1167. | Cl | Me | OEt | H | H | H | Me |
| 1168. | Cl | Me | Me | Me | H | H | Me |
| 1169. | Cl | Me | Cl | H | H | H | C(O)OMe |
| 1170. | Cl | Me | Cl | H | H | OMe | H |
| 1171. | Cl | Me | F | F | H | F | F |
| 1172. | Cl | Me | Cl | H | H | F | H |
| 1173. | Cl | Me | F | H | H | F | Cl |
| 1174. | Cl | Me | F | H | H | Cl | H |
| 1175. | Cl | Me | Cl | H | H | CF₃ | H |
| 1176. | Cl | Me | Cl | Me | H | H | H |
| 1177. | Cl | Me | OCHF₂ | H | H | H | H |
| 1178. | Cl | Me | OCH₂CF₃ | H | H | H | H |
| 1179. | Cl | Me | CF₃ | H | H | H | OCHF₂ |
| 1180. | Cl | Me | CF₃ | H | H | H | OCH₂CF₃ |
| 1181. | Cl | Me | Me | H | H | H | Me |
| 1182. | Cl | Me | Cl | H | H | H | F |
| 1183. | Cl | Me | F | H | F | H | H |
| 1184. | Cl | Me | F | Me | H | H | Cl |
| 1185. | Cl | Me | F | H | H | OMe | H |
| 1186. | Cl | Me | Cl | H | OCH₂O | | H |
| 1187. | Cl | Me | Me | H | H | F | H |
| 1188. | Cl | Me | OCF₃ | H | H | H | H |
| 1189. | Cl | Me | F | F | H | H | H |
| 1190. | Cl | Me | OMe | H | H | Cl | H |

**Tabelle 2: Verbindungen der Formel la-R**

| | | | | | | | |
|---|---|---|---|---|---|---|---|
| 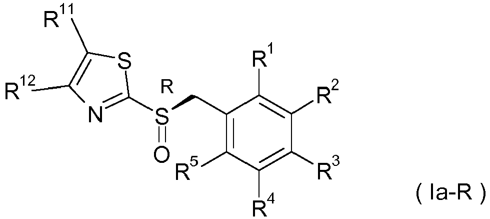 | | | | | | | |

| Die in der nachfolgenden Tabelle 2 zusammengefassten Verbindungen der Formel la-R sind lediglich Referenzbeispiele im Hinblick auf die biologischen Beispiele der vorliegenden Erfindung. | | | | | | | |
|---|---|---|---|---|---|---|---|
| Bsp. Nr. | R¹¹ | R¹² | R¹ | R² | R³ | R⁴ | R⁵ |
| 1191. | H | H | H | H | H | H | H |
| 1192. | H | H | F | H | H | H | H |
| 1193. | H | H | F | H | H | F | H |
| 1194. | H | H | F | H | H | H | F |
| 1195. | H | H | F | Me | H | H | F |
| 1196. | H | H | F | H | H | H | Cl |
| 1197. | H | H | CF₃ | H | H | H | H |
| 1198. | H | H | Me | H | H | H | H |
| 1199. | H | H | F | H | H | H | CF₃ |
| 1200. | H | H | F | CF₃ | H | H | F |
| 1201. | H | H | Br | H | H | H | H |
| 1202. | H | H | I | H | H | H | H |
| 1203. | H | H | Cl | H | H | H | H |
| 1204. | H | H | Cl | H | Cl | H | H |
| 1205. | H | H | Cl | H | H | H | Cl |

| Bsp. Nr. | R¹¹ | R¹² | R¹ | R² | R³ | R⁴ | R⁵ |
|---|---|---|---|---|---|---|---|
| 1206. | H | H | H | Cl | Cl | H | H |
| 1207. | H | H | Cl | Cl | Cl | H | H |
| 1208. | H | H | Cl | Cl | H | Cl | H |
| 1209. | H | H | Cl | Cl | Cl | H | Cl |
| 1210. | H | H | Cl | Cl | Cl | Cl | H |
| 1211. | H | H | Cl | Cl | Cl | Cl | Cl |
| 1212. | H | H | Cl | Cl | H | H | Cl |
| 1213. | H | H | Cl | H | Cl | Cl | H |
| 1214. | H | H | Cl | H | H | Cl | Cl |
| 1215. | H | H | H | Cl | Cl | Cl | H |
| 1216. | H | H | NO₂ | H | H | H | H |
| 1217. | H | H | H | Cl | H | H | H |
| 1218. | H | H | H | H | Cl | H | H |
| 1219. | H | H | Cl | H | Cl | H | Cl |
| 1220. | H | H | Cl | Cl | H | H | H |
| 1221. | H | H | Cl | H | H | Cl | H |
| 1222. | H | H | H | Cl | H | Cl | H |
| 1223. | H | H | H | OMe | H | H | H |
| 1224. | H | H | C(O)OMe | H | H | H | H |
| 1225. | H | H | F | Cl | H | H | H |
| 1226. | H | H | F | Me | H | H | H |
| 1227. | H | H | H | Me | H | H | H |
| 1228. | H | H | OMe | H | H | H | H |
| 1229. | H | H | F | F | F | H | H |
| 1230. | H | H | F | F | H | F | H |
| 1231. | H | H | H | F | F | F | H |
| 1232. | H | H | F | H | F | F | H |
| 1233. | H | H | Me | H | Me | H | H |
| 1234. | H | H | Me | H | H | Me | H |
| 1235. | H | H | F | H | H | CF₃ | H |
| 1236. | H | H | F | H | Br | H | H |
| 1237. | H | H | Me | Me | H | H | H |
| 1238. | H | H | F | F | F | F | F |
| 1239. | H | H | F | H | H | H | OMe |
| 1240. | H | H | Cl | H | F | H | H |
| 1241. | H | H | NO₂ | H | Cl | H | H |
| 1242. | H | H | NO₂ | H | H | Me | H |
| 1243. | H | H | F | H | H | H | I |
| 1244. | H | H | F | H | H | H | Br |
| 1245. | H | H | Br | H | H | H | Br |
| 1246. | H | H | Cl | H | H | H | Me |
| 1247. | H | H | Cl | H | H | H | OCHF₂ |
| 1248. | H | H | Cl | H | H | H | OMe |
| 1249. | H | H | Me | H | H | H | OMe |
| 1250. | H | H | OEt | H | H | H | CF₃ |
| 1251. | H | H | OC(O)Me | H | H | H | H |
| 1252. | H | H | OEt | H | H | H | Me |
| 1253. | H | H | Me | Me | H | H | Me |
| 1254. | H | H | Cl | H | H | H | C(O)OMe |
| 1255. | H | H | Cl | H | H | OMe | H |
| 1256. | H | H | F | F | H | F | F |
| 1257. | H | H | Cl | H | H | F | H |
| 1258. | H | H | F | H | H | F | Cl |
| 1259. | H | H | F | H | H | Cl | H |
| 1260. | H | H | Cl | H | H | CF₃ | H |
| 1261. | H | H | Cl | Me | H | H | H |
| 1262. | H | H | OCHF₂ | H | H | H | H |
| 1263. | H | H | OCH₂CF₃ | H | H | H | H |
| 1264. | H | H | CF₃ | H | H | H | OCHF₂ |
| 1265. | H | H | CF₃ | H | H | H | OCH₂CF₃ |
| 1266. | H | H | Me | H | H | H | Me |
| 1267. | H | H | Cl | H | H | H | F |
| 1268. | H | H | F | H | F | H | H |
| 1269. | H | H | F | Me | H | H | Cl |
| 1270. | H | H | F | H | H | OMe | H |
| 1271. | H | H | Cl | H | OCH₂O | | H |
| 1272. | H | H | Me | H | H | F | H |
| 1273. | H | H | OCF₃ | H | H | H | H |
| 1274. | H | H | F | F | H | H | H |
| 1275. | H | H | OMe | H | H | Cl | H |
| 1276. | F | H | H | H | H | H | H |
| 1277. | F | H | F | H | H | H | H |
| 1278. | F | H | F | H | H | F | H |
| 1279. | F | H | F | H | H | H | F |
| 1280. | F | H | F | Me | H | H | F |
| 1281. | F | H | F | H | H | H | Cl |
| 1282. | F | H | CF₃ | H | H | H | H |
| 1283. | F | H | Me | H | H | H | H |
| 1284. | F | H | F | H | H | H | CF₃ |
| 1285. | F | H | F | CF₃ | H | H | F |
| 1286. | F | H | Br | H | H | H | H |
| 1287. | F | H | I | H | H | H | H |
| 1288. | F | H | Cl | H | H | H | H |
| 1289. | F | H | Cl | H | Cl | H | H |
| 1290. | F | H | Cl | H | H | H | Cl |
| 1291. | F | H | H | Cl | Cl | H | H |
| 1292. | F | H | Cl | Cl | Cl | H | H |
| 1293. | F | H | Cl | Cl | H | Cl | H |
| 1294. | F | H | Cl | Cl | Cl | H | Cl |
| 1295. | F | H | Cl | Cl | Cl | Cl | H |
| 1296. | F | H | Cl | Cl | Cl | Cl | Cl |
| 1297. | F | H | Cl | Cl | H | H | Cl |
| 1298. | F | H | Cl | H | Cl | Cl | H |
| 1299. | F | H | Cl | H | H | Cl | Cl |
| 1300. | F | H | H | Cl | Cl | Cl | H |
| 1301. | F | H | NO₂ | H | H | H | H |
| 1302. | F | H | H | Cl | H | H | H |
| 1303. | F | H | H | H | Cl | H | H |
| 1304. | F | H | Cl | H | Cl | H | Cl |
| 1305. | F | H | Cl | Cl | H | H | H |
| 1306. | F | H | Cl | H | H | Cl | H |
| 1307. | F | H | H | Cl | H | Cl | H |
| 1308. | F | H | H | OMe | H | H | H |
| 1309. | F | H | C(O)OMe | H | H | H | H |
| 1310. | F | H | F | Cl | H | H | H |
| 1311. | F | H | F | Me | H | H | H |
| 1312. | F | H | H | Me | H | H | H |
| 1313. | F | H | OMe | H | H | H | H |
| 1314. | F | H | F | F | F | H | H |
| 1315. | F | H | F | F | H | F | H |
| 1316. | F | H | H | F | F | F | H |
| 1317. | F | H | F | H | F | F | H |
| 1318. | F | H | Me | H | Me | H | H |
| 1319. | F | H | Me | H | H | Me | H |
| 1320. | F | H | F | H | H | CF₃ | H |
| 1321. | F | H | F | H | Br | H | H |
| 1322. | F | H | Me | Me | H | H | H |
| 1323. | F | H | F | F | F | F | F |
| 1324. | F | H | F | H | H | H | OMe |
| 1325. | F | H | Cl | H | F | H | H |
| 1326. | F | H | NO₂ | H | Cl | H | H |
| 1327. | F | H | NO₂ | H | H | Me | H |
| 1328. | F | H | F | H | H | H | I |
| 1329. | F | H | F | H | H | H | Br |
| 1330. | F | H | Br | H | H | H | Br |
| 1331. | F | H | Cl | H | H | H | Me |
| 1332. | F | H | Cl | H | H | H | OCHF₂ |
| 1333. | F | H | Cl | H | H | H | OMe |
| 1334. | F | H | Me | H | H | H | OMe |
| 1335. | F | H | OEt | H | H | H | CF₃ |
| 1336. | F | H | OC(O)Me | H | H | H | H |
| 1337. | F | H | OEt | H | H | H | Me |
| 1338. | F | H | Me | Me | H | H | Me |
| 1339. | F | H | Cl | H | H | H | C(O)OMe |
| 1340. | F | H | Cl | H | H | OMe | H |
| 1341. | F | H | F | F | H | F | F |
| 1342. | F | H | Cl | H | H | F | H |
| 1343. | F | H | F | H | H | F | Cl |
| 1344. | F | H | F | H | H | Cl | H |
| 1345. | F | H | Cl | H | H | CF₃ | H |
| 1346. | F | H | Cl | Me | H | H | H |
| 1347. | F | H | OCHF₂ | H | H | H | H |
| 1348. | F | H | OCH₂CF₃ | H | H | H | H |
| 1349. | F | H | CF₃ | H | H | H | OCHF₂ |
| 1350. | F | H | CF₃ | H | H | H | OCH₂CF₃ |
| 1351. | F | H | Me | H | H | H | Me |
| 1352. | F | H | Cl | H | H | H | F |
| 1353. | F | H | F | H | F | H | H |
| 1354. | F | H | F | Me | H | H | Cl |
| 1355. | F | H | F | H | H | OMe | H |
| 1356. | F | H | Cl | H | OCH₂O | | H |
| 1357. | F | H | Me | H | H | F | H |
| 1358. | F | H | OCF₃ | H | H | H | H |
| 1359. | F | H | F | F | H | H | H |
| 1360. | F | H | OMe | H | H | Cl | H |
| 1361. | Cl | H | H | H | H | H | H |
| 1362. | Cl | H | F | H | H | H | H |
| 1363. | Cl | H | F | H | H | F | H |
| 1364. | Cl | H | F | H | H | H | F |
| 1365. | Cl | H | F | Me | H | H | F |
| 1366. | Cl | H | F | H | H | H | Cl |
| 1367. | Cl | H | CF₃ | H | H | H | H |
| 1368. | Cl | H | Me | H | H | H | H |
| 1369. | Cl | H | F | H | H | H | CF₃ |
| 1370. | Cl | H | F | CF₃ | H | H | F |
| 1371. | Cl | H | Br | H | H | H | H |
| 1372. | Cl | H | I | H | H | H | H |
| 1373. | Cl | H | Cl | H | H | H | H |
| 1374. | Cl | H | Cl | H | Cl | H | H |
| 1375. | Cl | H | Cl | H | H | H | Cl |
| 1376. | Cl | H | H | Cl | Cl | H | H |
| 1377. | Cl | H | Cl | Cl | Cl | H | H |
| 1378. | Cl | H | Cl | Cl | H | Cl | H |
| 1379. | Cl | H | Cl | Cl | Cl | H | Cl |
| 1380. | Cl | H | Cl | Cl | Cl | Cl | H |
| 1381. | Cl | H | Cl | Cl | Cl | Cl | Cl |
| 1382. | Cl | H | Cl | Cl | H | H | Cl |
| 1383. | Cl | H | Cl | H | Cl | Cl | H |
| 1384. | Cl | H | Cl | H | H | Cl | Cl |
| 1385. | Cl | H | H | Cl | Cl | Cl | H |
| 1386. | Cl | H | NO₂ | H | H | H | H |
| 1387. | Cl | H | H | Cl | H | H | H |
| 1388. | Cl | H | H | H | Cl | H | H |
| 1389. | Cl | H | Cl | H | Cl | H | Cl |
| 1390. | Cl | H | Cl | Cl | H | H | H |
| 1391. | Cl | H | Cl | H | H | Cl | H |
| 1392. | Cl | H | H | Cl | H | Cl | H |
| 1393. | Cl | H | H | OMe | H | H | H |
| 1394. | Cl | H | C(O)OMe | H | H | H | H |
| 1395. | Cl | H | F | Cl | H | H | H |
| 1396. | Cl | H | F | Me | H | H | H |
| 1397. | Cl | H | H | Me | H | H | H |
| 1398. | Cl | H | OMe | H | H | H | H |
| 1399. | Cl | H | F | F | F | H | H |
| 1400. | Cl | H | F | F | H | F | H |
| 1401. | Cl | H | H | F | F | F | H |
| 1402. | Cl | H | F | H | F | F | H |
| 1403. | Cl | H | Me | H | Me | H | H |
| 1404. | Cl | H | Me | H | H | Me | H |
| 1405. | Cl | H | F | H | H | CF₃ | H |
| 1406. | Cl | H | F | H | Br | H | H |
| 1407. | Cl | H | Me | Me | H | H | H |
| 1408. | Cl | H | F | F | F | F | F |
| 1409. | Cl | H | F | H | H | H | OMe |
| 1410. | Cl | H | Cl | H | F | H | H |
| 1411. | Cl | H | NO₂ | H | Cl | H | H |
| 1412. | Cl | H | NO₂ | H | H | Me | H |
| 1413. | Cl | H | F | H | H | H | I |
| 1414. | Cl | H | F | H | H | H | Br |
| 1415. | Cl | H | Br | H | H | H | Br |
| 1416. | Cl | H | Cl | H | H | H | Me |
| 1417. | Cl | H | Cl | H | H | H | OCHF₂ |
| 1418. | Cl | H | Cl | H | H | H | OMe |
| 1419. | Cl | H | Me | H | H | H | OMe |
| 1420. | Cl | H | OEt | H | H | H | CF₃ |
| 1421. | Cl | H | OC(O)Me | H | H | H | H |
| 1422. | Cl | H | OEt | H | H | H | Me |
| 1423. | Cl | H | Me | Me | H | H | Me |
| 1424. | Cl | H | Cl | H | H | H | C(O)OMe |
| 1425. | Cl | H | Cl | H | H | OMe | H |
| 1426. | Cl | H | F | F | H | F | F |
| 1427. | Cl | H | Cl | H | H | F | H |
| 1428. | Cl | H | F | H | H | F | Cl |
| 1429. | Cl | H | F | H | H | Cl | H |
| 1430. | Cl | H | Cl | H | H | CF₃ | H |
| 1431. | Cl | H | Cl | Me | H | H | H |
| 1432. | Cl | H | OCHF₂ | H | H | H | H |
| 1433. | Cl | H | OCH₂CF₃ | H | H | H | H |
| 1434. | Cl | H | CF₃ | H | H | H | OCHF₂ |
| 1435. | Cl | H | CF₃ | H | H | H | OCH₂CF₃ |
| 1436. | Cl | H | Me | H | H | H | Me |
| 1437. | Cl | H | Cl | H | H | H | F |
| 1438. | Cl | H | F | H | F | H | H |
| 1439. | Cl | H | F | Me | H | H | Cl |
| 1440. | Cl | H | F | H | H | OMe | H |
| 1441. | Cl | H | Cl | H | OCH₂O | | H |
| 1442. | Cl | H | Me | H | H | F | H |
| 1443. | Cl | H | OCF₃ | H | H | H | H |
| 1444. | Cl | H | F | F | H | H | H |
| 1445. | Cl | H | OMe | H | H | Cl | H |
| 1446. | Br | H | H | H | H | H | H |
| 1447. | Br | H | F | H | H | H | H |
| 1448. | Br | H | F | H | H | F | H |
| 1449. | Br | H | F | H | H | H | F |
| 1450. | Br | H | F | Me | H | H | F |
| 1451. | Br | H | F | H | H | H | Cl |
| 1452. | Br | H | CF₃ | H | H | H | H |
| 1453. | Br | H | Me | H | H | H | H |
| 1454. | Br | H | F | H | H | H | CF₃ |
| 1455. | Br | H | F | CF₃ | H | H | F |
| 1456. | Br | H | Br | H | H | H | H |
| 1457. | Br | H | I | H | H | H | H |
| 1458. | Br | H | Cl | H | H | H | H |
| 1459. | Br | H | Cl | H | Cl | H | H |
| 1460. | Br | H | Cl | H | H | H | Cl |
| 1461. | Br | H | H | Cl | Cl | H | H |
| 1462. | Br | H | Cl | Cl | Cl | H | H |
| 1463. | Br | H | Cl | Cl | H | Cl | H |
| 1464. | Br | H | Cl | Cl | Cl | H | Cl |
| 1465. | Br | H | Cl | Cl | Cl | Cl | H |
| 1466. | Br | H | Cl | Cl | Cl | Cl | Cl |
| 1467. | Br | H | Cl | Cl | H | H | Cl |
| 1468. | Br | H | Cl | H | Cl | Cl | H |
| 1469. | Br | H | Cl | H | H | Cl | Cl |
| 1470. | Br | H | H | Cl | Cl | Cl | H |
| 1471. | Br | H | NO₂ | H | H | H | H |
| 1472. | Br | H | H | Cl | H | H | H |
| 1473. | Br | H | H | H | Cl | H | H |
| 1474. | Br | H | Cl | H | Cl | H | Cl |
| 1475. | Br | H | Cl | Cl | H | H | H |
| 1476. | Br | H | Cl | H | H | Cl | H |
| 1477. | Br | H | H | Cl | H | Cl | H |
| 1478. | Br | H | H | OMe | H | H | H |
| 1479. | Br | H | C(O)OMe | H | H | H | H |
| 1480. | Br | H | F | Cl | H | H | H |
| 1481. | Br | H | F | Me | H | H | H |
| 1482. | Br | H | H | Me | H | H | H |
| 1483. | Br | H | OMe | H | H | H | H |
| 1484. | Br | H | F | F | F | H | H |
| 1485. | Br | H | F | F | H | F | H |
| 1486. | Br | H | H | F | F | F | H |
| 1487. | Br | H | F | H | F | F | H |
| 1488. | Br | H | Me | H | Me | H | H |
| 1489. | Br | H | Me | H | H | Me | H |
| 1490. | Br | H | F | H | H | CF₃ | H |
| 1491. | Br | H | F | H | Br | H | H |
| 1492. | Br | H | Me | Me | H | H | H |
| 1493. | Br | H | F | F | F | F | F |
| 1494. | Br | H | F | H | H | H | OMe |
| 1495. | Br | H | Cl | H | F | H | H |
| 1496. | Br | H | NO₂ | H | Cl | H | H |
| 1497. | Br | H | NO₂ | H | H | Me | H |
| 1498. | Br | H | F | H | H | H | I |
| 1499. | Br | H | F | H | H | H | Br |
| 1500. | Br | H | Br | H | H | H | Br |
| 1501. | Br | H | Cl | H | H | H | Me |
| 1502. | Br | H | Cl | H | H | H | OCHF₂ |
| 1503. | Br | H | Cl | H | H | H | OMe |
| 1504. | Br | H | Me | H | H | H | OMe |
| 1505. | Br | H | OEt | H | H | H | CF₃ |
| 1506. | Br | H | OC(O)Me | H | H | H | H |
| 1507. | Br | H | OEt | H | H | H | Me |
| 1508. | Br | H | Me | Me | H | H | Me |
| 1509. | Br | H | Cl | H | H | H | C(O)OMe |
| 1510. | Br | H | Cl | H | H | OMe | H |
| 1511. | Br | H | F | F | H | F | F |
| 1512. | Br | H | Cl | H | H | F | H |
| 1513. | Br | H | F | H | H | F | Cl |
| 1514. | Br | H | F | H | H | Cl | H |
| 1515. | Br | H | Cl | H | H | CF₃ | H |
| 1516. | Br | H | Cl | Me | H | H | H |
| 1517. | Br | H | OCHF₂ | H | H | H | H |
| 1518. | Br | H | OCH₂CF₃ | H | H | H | H |
| 1519. | Br | H | CF₃ | H | H | H | OCHF₂ |
| 1520. | Br | H | CF₃ | H | H | H | OCH₂CF₃ |
| 1521. | Br | H | Me | H | H | H | Me |
| 1522. | Br | H | Cl | H | H | H | F |
| 1523. | Br | H | F | H | F | H | H |
| 1524. | Br | H | F | Me | H | H | Cl |
| 1525. | Br | H | F | H | H | OMe | H |
| 1526. | Br | H | Cl | H | OCH₂O | | H |
| 1527. | Br | H | Me | H | H | F | H |
| 1528. | Br | H | OCF₃ | H | H | H | H |
| 1529. | Br | H | F | F | H | H | H |
| 1530. | Br | H | OMe | H | H | Cl | H |
| 1531. | I | H | H | H | H | H | H |
| 1532. | I | H | F | H | H | H | H |
| 1533. | I | H | F | H | H | F | H |
| 1534. | I | H | F | H | H | H | F |
| 1535. | I | H | F | Me | H | H | F |
| 1536. | I | H | F | H | H | H | Cl |
| 1537. | I | H | CF₃ | H | H | H | H |
| 1538. | I | H | Me | H | H | H | H |
| 1539. | I | H | F | H | H | H | CF₃ |
| 1540. | I | H | F | CF₃ | H | H | F |
| 1541. | I | H | Br | H | H | H | H |
| 1542. | I | H | I | H | H | H | H |
| 1543. | I | H | Cl | H | H | H | H |
| 1544. | I | H | Cl | H | Cl | H | H |
| 1545. | I | H | Cl | H | H | H | Cl |
| 1546. | I | H | H | Cl | Cl | H | H |
| 1547. | I | H | Cl | Cl | Cl | H | H |
| 1548. | I | H | Cl | Cl | H | Cl | H |
| 1549. | I | H | Cl | Cl | Cl | H | Cl |
| 1550. | I | H | Cl | Cl | Cl | Cl | H |
| 1551. | I | H | Cl | Cl | Cl | Cl | Cl |
| 1552. | I | H | Cl | Cl | H | H | Cl |
| 1553. | I | H | Cl | H | Cl | Cl | H |
| 1554. | I | H | Cl | H | H | Cl | Cl |
| 1555. | I | H | H | Cl | Cl | Cl | H |
| 1556. | I | H | NO₂ | H | H | H | H |
| 1557. | I | H | H | Cl | H | H | H |
| 1558. | I | H | H | H | Cl | H | H |
| 1559. | I | H | Cl | H | Cl | H | Cl |
| 1560. | I | H | Cl | Cl | H | H | H |
| 1561. | I | H | Cl | H | H | Cl | H |
| 1562. | I | H | H | Cl | H | Cl | H |
| 1563. | I | H | H | OMe | H | H | H |
| 1564. | I | H | C(O)OMe | H | H | H | H |
| 1565. | I | H | F | Cl | H | H | H |
| 1566. | I | H | F | Me | H | H | H |
| 1567. | I | H | H | Me | H | H | H |
| 1568. | I | H | OMe | H | H | H | H |
| 1569. | I | H | F | F | F | H | H |
| 1570. | I | H | F | F | H | F | H |
| 1571. | I | H | H | F | F | F | H |
| 1572. | I | H | F | H | F | F | H |
| 1573. | I | H | Me | H | Me | H | H |
| 1574. | I | H | Me | H | H | Me | H |
| 1575. | I | H | F | H | H | CF₃ | H |
| 1576. | I | H | F | H | Br | H | H |
| 1577. | I | H | Me | Me | H | H | H |
| 1578. | I | H | F | F | F | F | F |
| 1579. | I | H | F | H | H | H | OMe |
| 1580. | I | H | Cl | H | F | H | H |
| 1581. | I | H | NO₂ | H | Cl | H | H |
| 1582. | I | H | NO₂ | H | H | Me | H |
| 1583. | I | H | F | H | H | H | I |
| 1584. | I | H | F | H | H | H | Br |
| 1585. | I | H | Br | H | H | H | Br |
| 1586. | I | H | Cl | H | H | H | Me |
| 1587. | I | H | Cl | H | H | H | OCHF₂ |
| 1588. | I | H | Cl | H | H | H | OMe |
| 1589. | I | H | Me | H | H | H | OMe |
| 1590. | I | H | OEt | H | H | H | CF₃ |
| 1591. | I | H | OC(O)Me | H | H | H | H |
| 1592. | I | H | OEt | H | H | H | Me |
| 1593. | I | H | Me | Me | H | H | Me |
| 1594. | I | H | Cl | H | H | H | C(O)OMe |
| 1595. | I | H | Cl | H | H | OMe | H |
| 1596. | I | H | F | F | H | F | F |
| 1597. | I | H | Cl | H | H | F | H |
| 1598. | I | H | F | H | H | F | Cl |
| 1599. | I | H | F | H | H | Cl | H |
| 1600. | I | H | Cl | H | H | CF₃ | H |
| 1601. | I | H | Cl | Me | H | H | H |
| 1602. | I | H | OCHF₂ | H | H | H | H |
| 1603. | I | H | OCH₂CF₃ | H | H | H | H |
| 1604. | I | H | CF₃ | H | H | H | OCHF₂ |
| 1605. | I | H | CF₃ | H | H | H | OCH₂CF₃ |
| 1606. | I | H | Me | H | H | H | Me |
| 1607. | I | H | Cl | H | H | H | F |
| 1608. | I | H | F | H | F | H | H |
| 1609. | I | H | F | Me | H | H | Cl |
| 1610. | I | H | F | H | H | OMe | H |
| 1611. | I | H | Cl | H | OCH₂O | | H |
| 1612. | I | H | Me | H | H | F | H |
| 1613. | I | H | OCF₃ | H | H | H | H |
| 1614. | I | H | F | F | H | H | H |
| 1615. | I | H | OMe | H | H | Cl | H |
| 1616. | H | Cl | H | H | H | H | H |
| 1617. | H | Cl | F | H | H | H | H |
| 1618. | H | Cl | F | H | H | F | H |
| 1619. | H | Cl | F | H | H | H | F |
| 1620. | H | Cl | F | Me | H | H | F |
| 1621. | H | Cl | F | H | H | H | Cl |
| 1622. | H | Cl | CF₃ | H | H | H | H |
| 1623. | H | Cl | Me | H | H | H | H |
| 1624. | H | Cl | F | H | H | H | CF₃ |
| 1625. | H | Cl | F | CF₃ | H | H | F |
| 1626. | H | Cl | Br | H | H | H | H |
| 1627. | H | Cl | I | H | H | H | H |
| 1628. | H | Cl | Cl | H | H | H | H |
| 1629. | H | Cl | Cl | H | Cl | H | H |
| 1630. | H | Cl | Cl | H | H | H | Cl |
| 1631. | H | Cl | H | Cl | Cl | H | H |
| 1632. | H | Cl | Cl | Cl | Cl | H | H |
| 1633. | H | Cl | Cl | Cl | H | Cl | H |
| 1634. | H | Cl | Cl | Cl | Cl | H | Cl |
| 1635. | H | Cl | Cl | Cl | Cl | Cl | H |
| 1636. | H | Cl | Cl | Cl | Cl | Cl | Cl |
| 1637. | H | Cl | Cl | Cl | H | H | Cl |
| 1638. | H | Cl | Cl | H | Cl | Cl | H |
| 1639. | H | Cl | Cl | H | H | Cl | Cl |
| 1640. | H | Cl | H | Cl | Cl | Cl | H |
| 1641. | H | Cl | NO₂ | H | H | H | H |
| 1642. | H | Cl | H | Cl | H | H | H |
| 1643. | H | Cl | H | H | Cl | H | H |
| 1644. | H | Cl | Cl | H | Cl | H | Cl |
| 1645. | H | Cl | Cl | Cl | H | H | H |
| 1646. | H | Cl | Cl | H | H | Cl | H |
| 1647. | H | Cl | H | Cl | H | Cl | H |
| 1648. | H | Cl | H | OMe | H | H | H |
| 1649. | H | Cl | C(O)OMe | H | H | H | H |
| 1650. | H | Cl | F | Cl | H | H | H |
| 1651. | H | Cl | F | Me | H | H | H |
| 1652. | H | Cl | H | Me | H | H | H |
| 1653. | H | Cl | OMe | H | H | H | H |
| 1654. | H | Cl | F | F | F | H | H |
| 1655. | H | Cl | F | F | H | F | H |
| 1656. | H | Cl | H | F | F | F | H |
| 1657. | H | Cl | F | H | F | F | H |
| 1658. | H | Cl | Me | H | Me | H | H |
| 1659. | H | Cl | Me | H | H | Me | H |
| 1660. | H | Cl | F | H | H | CF₃ | H |
| 1661. | H | Cl | F | H | Br | H | H |
| 1662. | H | Cl | Me | Me | H | H | H |
| 1663. | H | Cl | F | F | F | F | F |
| 1664. | H | Cl | F | H | H | H | OMe |
| 1665. | H | Cl | Cl | H | F | H | H |
| 1666. | H | Cl | NO₂ | H | Cl | H | H |
| 1667. | H | Cl | NO₂ | H | H | Me | H |
| 1668. | H | Cl | F | H | H | H | I |
| 1669. | H | Cl | F | H | H | H | Br |
| 1670. | H | Cl | Br | H | H | H | Br |
| 1671. | H | Cl | Cl | H | H | H | Me |
| 1672. | H | Cl | Cl | H | H | H | OCHF₂ |
| 1673. | H | Cl | Cl | H | H | H | OMe |
| 1674. | H | Cl | Me | H | H | H | OMe |
| 1675. | H | Cl | OEt | H | H | H | CF₃ |
| 1676. | H | Cl | OC(O)Me | H | H | H | H |
| 1677. | H | Cl | OEt | H | H | H | Me |
| 1678. | H | Cl | Me | Me | H | H | Me |
| 1679. | H | Cl | Cl | H | H | H | C(O)OMe |
| 1680. | H | Cl | Cl | H | H | OMe | H |
| 1681. | H | Cl | F | F | H | F | F |
| 1682. | H | Cl | Cl | H | H | F | H |
| 1683. | H | Cl | F | H | H | F | Cl |
| 1684. | H | Cl | F | H | H | Cl | H |
| 1685. | H | Cl | Cl | H | H | CF₃ | H |
| 1686. | H | Cl | Cl | Me | H | H | H |
| 1687. | H | Cl | OCHF₂ | H | H | H | H |
| 1688. | H | Cl | OCH₂CF₃ | H | H | H | H |
| 1689. | H | Cl | CF₃ | H | H | H | OCHF₂ |
| 1690. | H | Cl | CF₃ | H | H | H | OCH₂CF₃ |
| 1691. | H | Cl | Me | H | H | H | Me |
| 1692. | H | Cl | Cl | H | H | H | F |
| 1693. | H | Cl | F | H | F | H | H |
| 1694. | H | Cl | F | Me | H | H | Cl |
| 1695. | H | Cl | F | H | H | OMe | H |
| 1696. | H | Cl | Cl | H | OCH₂O | | H |
| 1697. | H | Cl | Me | H | H | F | H |
| 1698. | H | Cl | OCF₃ | H | H | H | H |
| 1699. | H | Cl | F | F | H | H | H |
| 1700. | H | Cl | OMe | H | H | Cl | H |
| 1701. | H | Br | H | H | H | H | H |
| 1702. | H | Br | F | H | H | H | H |
| 1703. | H | Br | F | H | H | F | H |
| 1704. | H | Br | F | H | H | H | F |
| 1705. | H | Br | F | Me | H | H | F |
| 1706. | H | Br | F | H | H | H | Cl |
| 1707. | H | Br | CF₃ | H | H | H | H |
| 1708. | H | Br | Me | H | H | H | H |
| 1709. | H | Br | F | H | H | H | CF₃ |
| 1710. | H | Br | F | CF₃ | H | H | F |
| 1711. | H | Br | Br | H | H | H | H |
| 1712. | H | Br | I | H | H | H | H |
| 1713. | H | Br | Cl | H | H | H | H |
| 1714. | H | Br | Cl | H | Cl | H | H |
| 1715. | H | Br | Cl | H | H | H | Cl |
| 1716. | H | Br | H | Cl | Cl | H | H |
| 1717. | H | Br | Cl | Cl | Cl | H | H |
| 1718. | H | Br | Cl | Cl | H | Cl | H |
| 1719. | H | Br | Cl | Cl | Cl | H | Cl |
| 1720. | H | Br | Cl | Cl | Cl | Cl | H |
| 1721. | H | Br | Cl | Cl | Cl | Cl | Cl |
| 1722. | H | Br | Cl | Cl | H | H | Cl |
| 1723. | H | Br | Cl | H | Cl | Cl | H |
| 1724. | H | Br | Cl | H | H | Cl | Cl |
| 1725. | H | Br | H | Cl | Cl | Cl | H |
| 1726. | H | Br | NO₂ | H | H | H | H |
| 1727. | H | Br | H | Cl | H | H | H |
| 1728. | H | Br | H | H | Cl | H | H |
| 1729. | H | Br | Cl | H | Cl | H | Cl |
| 1730. | H | Br | Cl | Cl | H | H | H |
| 1731. | H | Br | Cl | H | H | Cl | H |
| 1732. | H | Br | H | Cl | H | Cl | H |
| 1733. | H | Br | H | OMe | H | H | H |
| 1734. | H | Br | C(O)OMe | H | H | H | H |
| 1735. | H | Br | F | Cl | H | H | H |
| 1736. | H | Br | F | Me | H | H | H |
| 1737. | H | Br | H | Me | H | H | H |
| 1738. | H | Br | OMe | H | H | H | H |
| 1739. | H | Br | F | F | F | H | H |
| 1740. | H | Br | F | F | H | F | H |
| 1741. | H | Br | H | F | F | F | H |
| 1742. | H | Br | F | H | F | F | H |
| 1743. | H | Br | Me | H | Me | H | H |
| 1744. | H | Br | Me | H | H | Me | H |
| 1745. | H | Br | F | H | H | CF₃ | H |
| 1746. | H | Br | F | H | Br | H | H |
| 1747. | H | Br | Me | Me | H | H | H |
| 1748. | H | Br | F | F | F | F | F |
| 1749. | H | Br | F | H | H | H | OMe |
| 1750. | H | Br | Cl | H | F | H | H |
| 1751. | H | Br | NO₂ | H | Cl | H | H |
| 1752. | H | Br | NO₂ | H | H | Me | H |
| 1753. | H | Br | F | H | H | H | I |
| 1754. | H | Br | F | H | H | H | Br |
| 1755. | H | Br | Br | H | H | H | Br |
| 1756. | H | Br | Cl | H | H | H | Me |
| 1757. | H | Br | Cl | H | H | H | OCHF₂ |
| 1758. | H | Br | Cl | H | H | H | OMe |
| 1759. | H | Br | Me | H | H | H | OMe |
| 1760. | H | Br | OEt | H | H | H | CF₃ |
| 1761. | H | Br | OC(O)Me | H | H | H | H |
| 1762. | H | Br | OEt | H | H | H | Me |
| 1763. | H | Br | Me | Me | H | H | Me |
| 1764. | H | Br | Cl | H | H | H | C(O)OMe |
| 1765. | H | Br | Cl | H | H | OMe | H |
| 1766. | H | Br | F | F | H | F | F |
| 1767. | H | Br | Cl | H | H | F | H |
| 1768. | H | Br | F | H | H | F | Cl |
| 1769. | H | Br | F | H | H | Cl | H |
| 1770. | H | Br | Cl | H | H | CF₃ | H |
| 1771. | H | Br | Cl | Me | H | H | H |
| 1772. | H | Br | OCHF₂ | H | H | H | H |
| 1773. | H | Br | OCH₂CF₃ | H | H | H | H |
| 1774. | H | Br | CF₃ | H | H | H | OCHF₂ |
| 1775. | H | Br | CF₃ | H | H | H | OCH₂CF₃ |
| 1776. | H | Br | Me | H | H | H | Me |
| 1777. | H | Br | Cl | H | H | H | F |
| 1778. | H | Br | F | H | F | H | H |
| 1779. | H | Br | F | Me | H | H | Cl |
| 1780. | H | Br | F | H | H | OMe | H |
| 1781. | H | Br | Cl | H | OCH₂O | | H |
| 1782. | H | Br | Me | H | H | F | H |
| 1783. | H | Br | OCF₃ | H | H | H | H |
| 1784. | H | Br | F | F | H | H | H |
| 1785. | H | Br | OMe | H | H | Cl | H |
| 1786. | Me | H | H | H | H | H | H |
| 1787. | Me | H | F | H | H | H | H |
| 1788. | Me | H | F | H | H | F | H |
| 1789. | Me | H | F | H | H | H | F |
| 1790. | Me | H | F | Me | H | H | F |
| 1791. | Me | H | F | H | H | H | Cl |
| 1792. | Me | H | CF₃ | H | H | H | H |
| 1793. | Me | H | Me | H | H | H | H |
| 1794. | Me | H | F | H | H | H | CF₃ |
| 1795. | Me | H | F | CF₃ | H | H | F |
| 1796. | Me | H | Br | H | H | H | H |
| 1797. | Me | H | I | H | H | H | H |
| 1798. | Me | H | Cl | H | H | H | H |
| 1799. | Me | H | Cl | H | Cl | H | H |
| 1800. | Me | H | Cl | H | H | H | Cl |
| 1801. | Me | H | H | Cl | Cl | H | H |
| 1802. | Me | H | Cl | Cl | Cl | H | H |
| 1803. | Me | H | Cl | Cl | H | Cl | H |
| 1804. | Me | H | Cl | Cl | Cl | H | Cl |
| 1805. | Me | H | Cl | Cl | Cl | Cl | H |
| 1806. | Me | H | Cl | Cl | Cl | Cl | Cl |
| 1807. | Me | H | Cl | Cl | H | H | Cl |
| 1808. | Me | H | Cl | H | Cl | Cl | H |
| 1809. | Me | H | Cl | H | H | Cl | Cl |
| 1810. | Me | H | H | Cl | Cl | Cl | H |
| 1811. | Me | H | NO₂ | H | H | H | H |
| 1812. | Me | H | H | Cl | H | H | H |
| 1813. | Me | H | H | H | Cl | H | H |
| 1814. | Me | H | Cl | H | Cl | H | Cl |
| 1815. | Me | H | Cl | Cl | H | H | H |
| 1816. | Me | H | Cl | H | H | Cl | H |
| 1817. | Me | H | H | Cl | H | Cl | H |
| 1818. | Me | H | H | OMe | H | H | H |
| 1819. | Me | H | C(O)OMe | H | H | H | H |
| 1820. | Me | H | F | Cl | H | H | H |
| 1821. | Me | H | F | Me | H | H | H |
| 1822. | Me | H | H | Me | H | H | H |
| 1823. | Me | H | OMe | H | H | H | H |
| 1824. | Me | H | F | F | F | H | H |
| 1825. | Me | H | F | F | H | F | H |
| 1826. | Me | H | H | F | F | F | H |
| 1827. | Me | H | F | H | F | F | H |
| 1828. | Me | H | Me | H | Me | H | H |
| 1829. | Me | H | Me | H | H | Me | H |
| 1830. | Me | H | F | H | H | CF₃ | H |
| 1831. | Me | H | F | H | Br | H | H |
| 1832. | Me | H | Me | Me | H | H | H |
| 1833. | Me | H | F | F | F | F | F |
| 1834. | Me | H | F | H | H | H | OMe |
| 1835. | Me | H | Cl | H | F | H | H |
| 1836. | Me | H | NO₂ | H | Cl | H | H |
| 1837. | Me | H | NO₂ | H | H | Me | H |
| 1838. | Me | H | F | H | H | H | I |
| 1839. | Me | H | F | H | H | H | Br |
| 1840. | Me | H | Br | H | H | H | Br |
| 1841. | Me | H | Cl | H | H | H | Me |
| 1842. | Me | H | Cl | H | H | H | OCHF₂ |
| 1843. | Me | H | Cl | H | H | H | OMe |
| 1844. | Me | H | Me | H | H | H | OMe |
| 1845. | Me | H | OEt | H | H | H | CF₃ |
| 1846. | Me | H | OC(O)Me | H | H | H | H |
| 1847. | Me | H | OEt | H | H | H | Me |
| 1848. | Me | H | Me | Me | H | H | Me |
| 1849. | Me | H | Cl | H | H | H | C(O)OMe |
| 1850. | Me | H | Cl | H | H | OMe | H |
| 1851. | Me | H | F | F | H | F | F |
| 1852. | Me | H | Cl | H | H | F | H |
| 1853. | Me | H | F | H | H | F | Cl |
| 1854. | Me | H | F | H | H | Cl | H |
| 1855. | Me | H | Cl | H | H | CF₃ | H |
| 1856. | Me | H | Cl | Me | H | H | H |
| 1857. | Me | H | OCHF₂ | H | H | H | H |
| 1858. | Me | H | OCH₂CF₃ | H | H | H | H |
| 1859. | Me | H | CF₃ | H | H | H | OCHF₂ |
| 1860. | Me | H | CF₃ | H | H | H | OCH₂CF₃ |
| 1861. | Me | H | Me | H | H | H | Me |
| 1862. | Me | H | Cl | H | H | H | F |
| 1863. | Me | H | F | H | F | H | H |
| 1864. | Me | H | F | Me | H | H | Cl |
| 1865. | Me | H | F | H | H | OMe | H |
| 1866. | Me | H | Cl | H | OCH₂O | | H |
| 1867. | Me | H | Me | H | H | F | H |
| 1868. | Me | H | OCF₃ | H | H | H | H |
| 1869. | Me | H | F | F | H | H | H |
| 1870. | Me | H | OMe | H | H | Cl | H |
| 1871. | H | Me | H | H | H | H | H |
| 1872. | H | Me | F | H | H | H | H |
| 1873. | H | Me | F | H | H | F | H |
| 1874. | H | Me | F | H | H | H | F |
| 1875. | H | Me | F | Me | H | H | F |
| 1876. | H | Me | F | H | H | H | Cl |
| 1877. | H | Me | CF₃ | H | H | H | H |
| 1878. | H | Me | Me | H | H | H | H |
| 1879. | H | Me | F | H | H | H | CF₃ |
| 1880. | H | Me | F | CF₃ | H | H | F |
| 1881. | H | Me | Br | H | H | H | H |
| 1882. | H | Me | I | H | H | H | H |
| 1883. | H | Me | Cl | H | H | H | H |
| 1884. | H | Me | Cl | H | Cl | H | H |
| 1885. | H | Me | Cl | H | H | H | Cl |
| 1886. | H | Me | H | Cl | Cl | H | H |
| 1887. | H | Me | Cl | Cl | Cl | H | H |
| 1888. | H | Me | Cl | Cl | H | Cl | H |
| 1889. | H | Me | Cl | Cl | Cl | H | Cl |
| 1890. | H | Me | Cl | Cl | Cl | Cl | H |
| 1891. | H | Me | Cl | Cl | Cl | Cl | Cl |
| 1892. | H | Me | Cl | Cl | H | H | Cl |
| 1893. | H | Me | Cl | H | Cl | Cl | H |
| 1894. | H | Me | Cl | H | H | Cl | Cl |
| 1895. | H | Me | H | Cl | Cl | Cl | H |
| 1896. | H | Me | NO₂ | H | H | H | H |
| 1897. | H | Me | H | Cl | H | H | H |
| 1898. | H | Me | H | H | Cl | H | H |
| 1899. | H | Me | Cl | H | Cl | H | Cl |
| 1900. | H | Me | Cl | Cl | H | H | H |
| 1901. | H | Me | Cl | H | H | Cl | H |
| 1902. | H | Me | H | Cl | H | Cl | H |
| 1903. | H | Me | H | OMe | H | H | H |
| 1904. | H | Me | C(O)OMe | H | H | H | H |
| 1905. | H | Me | F | Cl | H | H | H |
| 1906. | H | Me | F | Me | H | H | H |
| 1907. | H | Me | H | Me | H | H | H |
| 1908. | H | Me | OMe | H | H | H | H |
| 1909. | H | Me | F | F | F | H | H |
| 1910. | H | Me | F | F | H | F | H |
| 1911. | H | Me | H | F | F | F | H |
| 1912. | H | Me | F | H | F | F | H |
| 1913. | H | Me | Me | H | Me | H | H |
| 1914. | H | Me | Me | H | H | Me | H |
| 1915. | H | Me | F | H | H | CF₃ | H |
| 1916. | H | Me | F | H | Br | H | H |
| 1917. | H | Me | Me | Me | H | H | H |
| 1918. | H | Me | F | F | F | F | F |
| 1919. | H | Me | F | H | H | H | OMe |
| 1920. | H | Me | Cl | H | F | H | H |
| 1921. | H | Me | NO₂ | H | Cl | H | H |
| 1922. | H | Me | NO₂ | H | H | Me | H |
| 1923. | H | Me | F | H | H | H | I |
| 1924. | H | Me | F | H | H | H | Br |
| 1925. | H | Me | Br | H | H | H | Br |
| 1926. | H | Me | Cl | H | H | H | Me |
| 1927. | H | Me | Cl | H | H | H | OCHF₂ |
| 1928. | H | Me | Cl | H | H | H | OMe |
| 1929. | H | Me | Me | H | H | H | OMe |
| 1930. | H | Me | OEt | H | H | H | CF₃ |
| 1931. | H | Me | OC(O)Me | H | H | H | H |
| 1932. | H | Me | OEt | H | H | H | Me |
| 1933. | H | Me | Me | Me | H | H | Me |
| 1934. | H | Me | Cl | H | H | H | C(O)OMe |
| 1935. | H | Me | Cl | H | H | OMe | H |
| 1936. | H | Me | F | F | H | F | F |
| 1937. | H | Me | Cl | H | H | F | H |
| 1938. | H | Me | F | H | H | F | Cl |
| 1939. | H | Me | F | H | H | Cl | H |
| 1940. | H | Me | Cl | H | H | CF₃ | H |
| 1941. | H | Me | Cl | Me | H | H | H |
| 1942. | H | Me | OCHF₂ | H | H | H | H |
| 1943. | H | Me | OCH₂CF₃ | H | H | H | H |
| 1944. | H | Me | CF₃ | H | H | H | OCHF₂ |
| 1945. | H | Me | CF₃ | H | H | H | OCH₂CF₃ |
| 1946. | H | Me | Me | H | H | H | Me |
| 1947. | H | Me | Cl | H | H | H | F |
| 1948. | H | Me | F | H | F | H | H |
| 1949. | H | Me | F | Me | H | H | Cl |
| 1950. | H | Me | F | H | H | OMe | H |
| 1951. | H | Me | Cl | H | OCH₂O | | H |
| 1952. | H | Me | Me | H | H | F | H |
| 1953. | H | Me | OCF₃ | H | H | H | H |
| 1954. | H | Me | F | F | H | H | H |
| 1955. | H | Me | OMe | H | H | Cl | H |
| 1956. | NO₂ | H | H | H | H | H | H |
| 1957. | NO₂ | H | F | H | H | H | H |
| 1958. | NO₂ | H | F | H | H | F | H |
| 1959. | NO₂ | H | F | H | H | H | F |
| 1960. | NO₂ | H | F | Me | H | H | F |
| 1961. | NO₂ | H | F | H | H | H | Cl |
| 1962. | NO₂ | H | CF₃ | H | H | H | H |
| 1963. | NO₂ | H | Me | H | H | H | H |
| 1964. | NO₂ | H | F | H | H | H | CF₃ |
| 1965. | NO₂ | H | F | CF₃ | H | H | F |
| 1966. | NO₂ | H | Br | H | H | H | H |
| 1967. | NO₂ | H | I | H | H | H | H |
| 1968. | NO₂ | H | Cl | H | H | H | H |
| 1969. | NO₂ | H | Cl | H | Cl | H | H |
| 1970. | NO₂ | H | Cl | H | H | H | Cl |
| 1971. | NO₂ | H | H | Cl | Cl | H | H |
| 1972. | NO₂ | H | Cl | Cl | Cl | H | H |
| 1973. | NO₂ | H | Cl | Cl | H | Cl | H |
| 1974. | NO₂ | H | Cl | Cl | Cl | H | Cl |
| 1975. | NO₂ | H | Cl | Cl | Cl | Cl | H |
| 1976. | NO₂ | H | Cl | Cl | Cl | Cl | Cl |
| 1977. | NO₂ | H | Cl | Cl | H | H | Cl |
| 1978. | NO₂ | H | Cl | H | Cl | Cl | H |
| 1979. | NO₂ | H | Cl | H | H | Cl | Cl |
| 1980. | NO₂ | H | H | Cl | Cl | Cl | H |
| 1981. | NO₂ | H | NO₂ | H | H | H | H |
| 1982. | NO₂ | H | H | Cl | H | H | H |
| 1983. | NO₂ | H | H | H | Cl | H | H |
| 1984. | NO₂ | H | Cl | H | Cl | H | Cl |
| 1985. | NO₂ | H | Cl | Cl | H | H | H |
| 1986. | NO₂ | H | Cl | H | H | Cl | H |
| 1987. | NO₂ | H | H | Cl | H | Cl | H |
| 1988. | NO₂ | H | H | OMe | H | H | H |
| 1989. | NO₂ | H | C(O)OMe | H | H | H | H |
| 1990. | NO₂ | H | F | Cl | H | H | H |
| 1991. | NO₂ | H | F | Me | H | H | H |
| 1992. | NO₂ | H | H | Me | H | H | H |
| 1993. | NO₂ | H | OMe | H | H | H | H |
| 1994. | NO₂ | H | F | F | F | H | H |
| 1995. | NO₂ | H | F | F | H | F | H |
| 1996. | NO₂ | H | H | F | F | F | H |
| 1997. | NO₂ | H | F | H | F | F | H |
| 1998. | NO₂ | H | Me | H | Me | H | H |
| 1999. | NO₂ | H | Me | H | H | Me | H |
| 2000. | NO₂ | H | F | H | H | CF₃ | H |
| 2001. | NO₂ | H | F | H | Br | H | H |
| 2002. | NO₂ | H | Me | Me | H | H | H |
| 2003. | NO₂ | H | F | F | F | F | F |
| 2004. | NO₂ | H | F | H | H | H | OMe |
| 2005. | NO₂ | H | Cl | H | F | H | H |
| 2006. | NO₂ | H | NO₂ | H | Cl | H | H |
| 2007. | NO₂ | H | NO₂ | H | H | Me | H |
| 2008. | NO₂ | H | F | H | H | H | I |
| 2009. | NO₂ | H | F | H | H | H | Br |
| 2010. | NO₂ | H | Br | H | H | H | Br |
| 2011. | NO₂ | H | Cl | H | H | H | Me |
| 2012. | NO₂ | H | Cl | H | H | H | OCHF₂ |
| 2013. | NO₂ | H | Cl | H | H | H | OMe |
| 2014. | NO₂ | H | Me | H | H | H | OMe |
| 2015. | NO₂ | H | OEt | H | H | H | CF₃ |
| 2016. | NO₂ | H | OC(O)Me | H | H | H | H |
| 2017. | NO₂ | H | OEt | H | H | H | Me |
| 2018. | NO₂ | H | Me | Me | H | H | Me |
| 2019. | NO₂ | H | Cl | H | H | H | C(O)OMe |
| 2020. | NO₂ | H | Cl | H | H | OMe | H |
| 2021. | NO₂ | H | F | F | H | F | F |
| 2022. | NO₂ | H | Cl | H | H | F | H |
| 2023. | NO₂ | H | F | H | H | F | Cl |
| 2024. | NO₂ | H | F | H | H | Cl | H |
| 2025. | NO₂ | H | Cl | H | H | CF₃ | H |
| 2026. | NO₂ | H | Cl | Me | H | H | H |
| 2027. | NO₂ | H | OCHF₂ | H | H | H | H |
| 2028. | NO₂ | H | OCH₂CF₃ | H | H | H | H |
| 2029. | NO₂ | H | CF₃ | H | H | H | OCHF₂ |
| 2030. | NO₂ | H | CF₃ | H | H | H | OCH₂CF₃ |
| 2031. | NO₂ | H | Me | H | H | H | Me |
| 2032. | NO₂ | H | Cl | H | H | H | F |
| 2033. | NO₂ | H | F | H | F | H | H |
| 2034. | NO₂ | H | F | Me | H | H | Cl |
| 2035. | NO₂ | H | F | H | H | OMe | H |
| 2036. | NO₂ | H | Cl | H | OCH₂O | | H |
| 2037. | NO₂ | H | Me | H | H | F | H |
| 2038. | NO₂ | H | OCF₃ | H | H | H | H |
| 2039. | NO₂ | H | F | F | H | H | H |
| 2040. | NO₂ | H | OMe | H | H | Cl | H |
| 2041. | CHF₂ | H | H | H | H | H | H |
| 2042. | CHF₂ | H | F | H | H | H | H |
| 2043. | CHF₂ | H | F | H | H | F | H |
| 2044. | CHF₂ | H | F | H | H | H | F |
| 2045. | CHF₂ | H | F | Me | H | H | F |
| 2046. | CHF₂ | H | F | H | H | H | Cl |
| 2047. | CHF₂ | H | CF₃ | H | H | H | H |
| 2048. | CHF₂ | H | Me | H | H | H | H |
| 2049. | CHF₂ | H | F | H | H | H | CF₃ |
| 2050. | CHF₂ | H | F | CF₃ | H | H | F |
| 2051. | CHF₂ | H | Br | H | H | H | H |
| 2052. | CHF₂ | H | I | H | H | H | H |
| 2053. | CHF₂ | H | Cl | H | H | H | H |
| 2054. | CHF₂ | H | Cl | H | Cl | H | H |
| 2055. | CHF₂ | H | Cl | H | H | H | Cl |
| 2056. | CHF₂ | H | H | Cl | Cl | H | H |
| 2057. | CHF₂ | H | Cl | Cl | Cl | H | H |
| 2058. | CHF₂ | H | Cl | Cl | H | Cl | H |
| 2059. | CHF₂ | H | Cl | Cl | Cl | H | Cl |
| 2060. | CHF₂ | H | Cl | Cl | Cl | Cl | H |
| 2061. | CHF₂ | H | Cl | Cl | Cl | Cl | Cl |
| 2062. | CHF₂ | H | Cl | Cl | H | H | Cl |
| 2063. | CHF₂ | H | Cl | H | Cl | Cl | H |
| 2064. | CHF₂ | H | Cl | H | H | Cl | Cl |
| 2065. | CHF₂ | H | H | Cl | Cl | Cl | H |
| 2066. | CHF₂ | H | NO₂ | H | H | H | H |
| 2067. | CHF₂ | H | H | Cl | H | H | H |
| 2068. | CHF₂ | H | H | H | Cl | H | H |
| 2069. | CHF₂ | H | Cl | H | Cl | H | Cl |
| 2070. | CHF₂ | H | Cl | Cl | H | H | H |
| 2071. | CHF₂ | H | Cl | H | H | Cl | H |
| 2072. | CHF₂ | H | H | Cl | H | Cl | H |
| 2073. | CHF₂ | H | H | OMe | H | H | H |
| 2074. | CHF₂ | H | C(O)OMe | H | H | H | H |
| 2075. | CHF₂ | H | F | Cl | H | H | H |
| 2076. | CHF₂ | H | F | Me | H | H | H |
| 2077. | CHF₂ | H | H | Me | H | H | H |
| 2078. | CHF₂ | H | OMe | H | H | H | H |
| 2079. | CHF₂ | H | F | F | F | H | H |
| 2080. | CHF₂ | H | F | F | H | F | H |
| 2081. | CHF₂ | H | H | F | F | F | H |
| 2082. | CHF₂ | H | F | H | F | F | H |
| 2083. | CHF₂ | H | Me | H | Me | H | H |
| 2084. | CHF₂ | H | Me | H | H | Me | H |
| 2085. | CHF₂ | H | F | H | H | CF₃ | H |
| 2086. | CHF₂ | H | F | H | Br | H | H |
| 2087. | CHF₂ | H | Me | Me | H | H | H |
| 2088. | CHF₂ | H | F | F | F | F | F |
| 2089. | CHF₂ | H | F | H | H | H | OMe |
| 2090. | CHF₂ | H | Cl | H | F | H | H |
| 2091. | CHF₂ | H | NO₂ | H | Cl | H | H |
| 2092. | CHF₂ | H | NO₂ | H | H | Me | H |
| 2093. | CHF₂ | H | F | H | H | H | I |
| 2094. | CHF₂ | H | F | H | H | H | Br |
| 2095. | CHF₂ | H | Br | H | H | H | Br |
| 2096. | CHF₂ | H | Cl | H | H | H | Me |
| 2097. | CHF₂ | H | Cl | H | H | H | OCHF₂ |
| 2098. | CHF₂ | H | Cl | H | H | H | OMe |
| 2099. | CHF₂ | H | Me | H | H | H | OMe |
| 2100. | CHF₂ | H | OEt | H | H | H | CF₃ |
| 2101. | CHF₂ | H | OC(O)Me | H | H | H | H |
| 2102. | CHF₂ | H | OEt | H | H | H | Me |
| 2103. | CHF₂ | H | Me | Me | H | H | Me |
| 2104. | CHF₂ | H | Cl | H | H | H | C(O)OMe |
| 2105. | CHF₂ | H | Cl | H | H | OMe | H |
| 2106. | CHF₂ | H | F | F | H | F | F |
| 2107. | CHF₂ | H | Cl | H | H | F | H |
| 2108. | CHF₂ | H | F | H | H | F | Cl |
| 2109. | CHF₂ | H | F | H | H | Cl | H |
| 2110. | CHF₂ | H | Cl | H | H | CF₃ | H |
| 2111. | CHF₂ | H | Cl | Me | H | H | H |
| 2112. | CHF₂ | H | OCHF₂ | H | H | H | H |
| 2113. | CHF₂ | H | OCH₂CF₃ | H | H | H | H |
| 2114. | CHF₂ | H | CF₃ | H | H | H | OCHF₂ |
| 2115. | CHF₂ | H | CF₃ | H | H | H | OCH₂CF₃ |
| 2116. | CHF₂ | H | Me | H | H | H | Me |
| 2117. | CHF₂ | H | Cl | H | H | H | F |
| 2118. | CHF₂ | H | F | H | F | H | H |
| 2119. | CHF₂ | H | F | Me | H | H | Cl |
| 2120. | CHF₂ | H | F | H | H | OMe | H |
| 2121. | CHF₂ | H | Cl | H | OCH₂O | | H |
| 2122. | CHF₂ | H | Me | H | H | F | H |
| 2123. | CHF₂ | H | OCF₃ | H | H | H | H |
| 2124. | CHF₂ | H | F | F | H | H | H |
| 2125. | CHF₂ | H | OMe | H | H | Cl | H |
| 2126. | Cl | Cl | H | H | H | H | H |
| 2127. | Cl | Cl | F | H | H | H | H |
| 2128. | Cl | Cl | F | H | H | F | H |
| 2129. | Cl | Cl | F | H | H | H | F |
| 2130. | Cl | Cl | F | Me | H | H | F |
| 2131. | Cl | Cl | F | H | H | H | Cl |
| 2132. | Cl | Cl | CF₃ | H | H | H | H |
| 2133. | Cl | Cl | Me | H | H | H | H |
| 2134. | Cl | Cl | F | H | H | H | CF₃ |
| 2135. | Cl | Cl | F | CF₃ | H | H | F |
| 2136. | Cl | Cl | Br | H | H | H | H |
| 2137. | Cl | Cl | I | H | H | H | H |
| 2138. | Cl | Cl | Cl | H | H | H | H |
| 2139. | Cl | Cl | Cl | H | Cl | H | H |
| 2140. | Cl | Cl | Cl | H | H | H | Cl |
| 2141. | Cl | Cl | H | Cl | Cl | H | H |
| 2142. | Cl | Cl | Cl | Cl | Cl | H | H |
| 2143. | Cl | Cl | Cl | Cl | H | Cl | H |
| 2144. | Cl | Cl | Cl | Cl | Cl | H | Cl |
| 2145. | Cl | Cl | Cl | Cl | Cl | Cl | H |
| 2146. | Cl | Cl | Cl | Cl | Cl | Cl | Cl |
| 2147. | Cl | Cl | Cl | Cl | H | H | Cl |
| 2148. | Cl | Cl | Cl | H | Cl | Cl | H |
| 2149. | Cl | Cl | Cl | H | H | Cl | Cl |
| 2150. | Cl | Cl | H | Cl | Cl | Cl | H |
| 2151. | Cl | Cl | NO₂ | H | H | H | H |
| 2152. | Cl | Cl | H | Cl | H | H | H |
| 2153. | Cl | Cl | H | H | Cl | H | H |
| 2154. | Cl | Cl | Cl | H | Cl | H | Cl |
| 2155. | Cl | Cl | Cl | Cl | H | H | H |
| 2156. | Cl | Cl | Cl | H | H | Cl | H |
| 2157. | Cl | Cl | H | Cl | H | Cl | H |
| 2158. | Cl | Cl | H | OMe | H | H | H |
| 2159. | Cl | Cl | C(O)OMe | H | H | H | H |
| 2160. | Cl | Cl | F | Cl | H | H | H |
| 2161. | Cl | Cl | F | Me | H | H | H |
| 2162. | Cl | Cl | H | Me | H | H | H |
| 2163. | Cl | Cl | OMe | H | H | H | H |
| 2164. | Cl | Cl | F | F | F | H | H |
| 2165. | Cl | Cl | F | F | H | F | H |
| 2166. | Cl | Cl | H | F | F | F | H |
| 2167. | Cl | Cl | F | H | F | F | H |
| 2168. | Cl | Cl | Me | H | Me | H | H |
| 2169. | Cl | Cl | Me | H | H | Me | H |
| 2170. | Cl | Cl | F | H | H | CF₃ | H |
| 2171. | Cl | Cl | F | H | Br | H | H |
| 2172. | Cl | Cl | Me | Me | H | H | H |
| 2173. | Cl | Cl | F | F | F | F | F |
| 2174. | Cl | Cl | F | H | H | H | OMe |
| 2175. | Cl | Cl | Cl | H | F | H | H |
| 2176. | Cl | Cl | NO₂ | H | Cl | H | H |
| 2177. | Cl | Cl | NO₂ | H | H | Me | H |
| 2178. | Cl | Cl | F | H | H | H | I |
| 2179. | Cl | Cl | F | H | H | H | Br |
| 2180. | Cl | Cl | Br | H | H | H | Br |
| 2181. | Cl | Cl | Cl | H | H | H | Me |
| 2182. | Cl | Cl | Cl | H | H | H | OCHF₂ |
| 2183. | Cl | Cl | Cl | H | H | H | OMe |
| 2184. | Cl | Cl | Me | H | H | H | OMe |
| 2185. | Cl | Cl | OEt | H | H | H | CF₃ |
| 2186. | Cl | Cl | OC(O)Me | H | H | H | H |
| 2187. | Cl | Cl | OEt | H | H | H | Me |
| 2188. | Cl | Cl | Me | Me | H | H | Me |
| 2189. | Cl | Cl | Cl | H | H | H | C(O)OMe |
| 2190. | Cl | Cl | Cl | H | H | OMe | H |
| 2191. | Cl | Cl | F | F | H | F | F |
| 2192. | Cl | Cl | Cl | H | H | F | H |
| 2193. | Cl | Cl | F | H | H | F | Cl |
| 2194. | Cl | Cl | F | H | H | Cl | H |
| 2195. | Cl | Cl | Cl | H | H | CF₃ | H |
| 2196. | Cl | Cl | Cl | Me | H | H | H |
| 2197. | Cl | Cl | OCHF₂ | H | H | H | H |
| 2198. | Cl | Cl | OCH₂CF₃ | H | H | H | H |
| 2199. | Cl | Cl | CF₃ | H | H | H | OCHF₂ |
| 2200. | Cl | Cl | CF₃ | H | H | H | OCH₂CF₃ |
| 2201. | Cl | Cl | Me | H | H | H | Me |
| 2202. | Cl | Cl | Cl | H | H | H | F |
| 2203. | Cl | Cl | F | H | F | H | H |
| 2204. | Cl | Cl | F | Me | H | H | Cl |
| 2205. | Cl | Cl | F | H | H | OMe | H |
| 2206. | Cl | Cl | Cl | H | OCH₂O | | H |
| 2207. | Cl | Cl | Me | H | H | F | H |
| 2208. | Cl | Cl | OCF₃ | H | H | H | H |
| 2209. | Cl | Cl | F | F | H | H | H |
| 2210. | Cl | Cl | OMe | H | H | Cl | H |
| 2211. | Me | Cl | H | H | H | H | H |
| 2212. | Me | Cl | F | H | H | H | H |
| 2213. | Me | Cl | F | H | H | F | H |
| 2214. | Me | Cl | F | H | H | H | F |
| 2215. | Me | Cl | F | Me | H | H | F |
| 2216. | Me | Cl | F | H | H | H | Cl |
| 2217. | Me | Cl | CF₃ | H | H | H | H |
| 2218. | Me | Cl | Me | H | H | H | H |
| 2219. | Me | Cl | F | H | H | H | CF₃ |
| 2220. | Me | Cl | F | CF₃ | H | H | F |
| 2221. | Me | Cl | Br | H | H | H | H |
| 2222. | Me | Cl | I | H | H | H | H |
| 2223. | Me | Cl | Cl | H | H | H | H |
| 2224. | Me | Cl | Cl | H | Cl | H | H |
| 2225. | Me | Cl | Cl | H | H | H | Cl |
| 2226. | Me | Cl | H | Cl | Cl | H | H |
| 2227. | Me | Cl | Cl | Cl | Cl | H | H |
| 2228. | Me | Cl | Cl | Cl | H | Cl | H |
| 2229. | Me | Cl | Cl | Cl | Cl | H | Cl |
| 2230. | Me | Cl | Cl | Cl | Cl | Cl | H |
| 2231. | Me | Cl | Cl | Cl | Cl | Cl | Cl |
| 2232. | Me | Cl | Cl | Cl | H | H | Cl |
| 2233. | Me | Cl | Cl | H | Cl | Cl | H |
| 2234. | Me | Cl | Cl | H | H | Cl | Cl |
| 2235. | Me | Cl | H | Cl | Cl | Cl | H |
| 2236. | Me | Cl | NO₂ | H | H | H | H |
| 2237. | Me | Cl | H | Cl | H | H | H |
| 2238. | Me | Cl | H | H | Cl | H | H |
| 2239. | Me | Cl | Cl | H | Cl | H | Cl |
| 2240. | Me | Cl | Cl | Cl | H | H | H |
| 2241. | Me | Cl | Cl | H | H | Cl | H |
| 2242. | Me | Cl | H | Cl | H | Cl | H |
| 2243. | Me | Cl | H | OMe | H | H | H |
| 2244. | Me | Cl | C(O)OMe | H | H | H | H |
| 2245. | Me | Cl | F | Cl | H | H | H |
| 2246. | Me | Cl | F | Me | H | H | H |
| 2247. | Me | Cl | H | Me | H | H | H |
| 2248. | Me | Cl | OMe | H | H | H | H |
| 2249. | Me | Cl | F | F | F | H | H |
| 2250. | Me | Cl | F | F | H | F | H |
| 2251. | Me | Cl | H | F | F | F | H |
| 2252. | Me | Cl | F | H | F | F | H |
| 2253. | Me | Cl | Me | H | Me | H | H |
| 2254. | Me | Cl | Me | H | H | Me | H |
| 2255. | Me | Cl | F | H | H | CF₃ | H |
| 2256. | Me | Cl | F | H | Br | H | H |
| 2257. | Me | Cl | Me | Me | H | H | H |
| 2258. | Me | Cl | F | F | F | F | F |
| 2259. | Me | Cl | F | H | H | H | OMe |
| 2260. | Me | Cl | Cl | H | F | H | H |
| 2261. | Me | Cl | NO₂ | H | Cl | H | H |
| 2262. | Me | Cl | NO₂ | H | H | Me | H |
| 2263. | Me | Cl | F | H | H | H | I |
| 2264. | Me | Cl | F | H | H | H | Br |
| 2265. | Me | Cl | Br | H | H | H | Br |
| 2266. | Me | Cl | Cl | H | H | H | Me |
| 2267. | Me | Cl | Cl | H | H | H | OCHF₂ |
| 2268. | Me | Cl | Cl | H | H | H | OMe |
| 2269. | Me | Cl | Me | H | H | H | OMe |
| 2270. | Me | Cl | OEt | H | H | H | CF₃ |
| 2271. | Me | Cl | OC(O)Me | H | H | H | H |
| 2272. | Me | Cl | OEt | H | H | H | Me |
| 2273. | Me | Cl | Me | Me | H | H | Me |
| 2274. | Me | Cl | Cl | H | H | H | C(O)OMe |
| 2275. | Me | Cl | Cl | H | H | OMe | H |
| 2276. | Me | Cl | F | F | H | F | F |
| 2277. | Me | Cl | Cl | H | H | F | H |
| 2278. | Me | Cl | F | H | H | F | Cl |
| 2279. | Me | Cl | F | H | H | Cl | H |
| 2280. | Me | Cl | Cl | H | H | CF₃ | H |
| 2281. | Me | Cl | Cl | Me | H | H | H |
| 2282. | Me | Cl | OCHF₂ | H | H | H | H |
| 2283. | Me | Cl | OCH₂CF₃ | H | H | H | H |
| 2284. | Me | Cl | CF₃ | H | H | H | OCHF₂ |
| 2285. | Me | Cl | CF₃ | H | H | H | OCH₂CF₃ |
| 2286. | Me | Cl | Me | H | H | H | Me |
| 2287. | Me | Cl | Cl | H | H | H | F |
| 2288. | Me | Cl | F | H | F | H | H |
| 2289. | Me | Cl | F | Me | H | H | Cl |
| 2290. | Me | Cl | F | H | H | OMe | H |
| 2291. | Me | Cl | Cl | H | OCH₂O | | H |
| 2292. | Me | Cl | Me | H | H | F | H |
| 2293. | Me | Cl | OCF₃ | H | H | H | H |
| 2294. | Me | Cl | F | F | H | H | H |
| 2295. | Me | Cl | OMe | H | H | Cl | H |
| 2296. | Cl | Me | H | H | H | H | H |
| 2297. | Cl | Me | F | H | H | H | H |
| 2298. | Cl | Me | F | H | H | F | H |
| 2299. | Cl | Me | F | H | H | H | F |
| 2300. | Cl | Me | F | Me | H | H | F |
| 2301. | Cl | Me | F | H | H | H | Cl |
| 2302. | Cl | Me | CF₃ | H | H | H | H |
| 2303. | Cl | Me | Me | H | H | H | H |
| 2304. | Cl | Me | F | H | H | H | CF₃ |
| 2305. | Cl | Me | F | CF₃ | H | H | F |
| 2306. | Cl | Me | Br | H | H | H | H |
| 2307. | Cl | Me | I | H | H | H | H |
| 2308. | Cl | Me | Cl | H | H | H | H |
| 2309. | Cl | Me | Cl | H | Cl | H | H |
| 2310. | Cl | Me | Cl | H | H | H | Cl |
| 2311. | Cl | Me | H | Cl | Cl | H | H |
| 2312. | Cl | Me | Cl | Cl | Cl | H | H |
| 2313. | Cl | Me | Cl | Cl | H | Cl | H |
| 2314. | Cl | Me | Cl | Cl | Cl | H | Cl |
| 2315. | Cl | Me | Cl | Cl | Cl | Cl | H |
| 2316. | Cl | Me | Cl | Cl | Cl | Cl | Cl |
| 2317. | Cl | Me | Cl | Cl | H | H | Cl |
| 2318. | Cl | Me | Cl | H | Cl | Cl | H |
| 2319. | Cl | Me | Cl | H | H | Cl | Cl |
| 2320. | Cl | Me | H | Cl | Cl | Cl | H |
| 2321. | Cl | Me | NO₂ | H | H | H | H |
| 2322. | Cl | Me | H | Cl | H | H | H |
| 2323. | Cl | Me | H | H | Cl | H | H |
| 2324. | Cl | Me | Cl | H | Cl | H | Cl |
| 2325. | Cl | Me | Cl | Cl | H | H | H |
| 2326. | Cl | Me | Cl | H | H | Cl | H |
| 2327. | Cl | Me | H | Cl | H | Cl | H |
| 2328. | Cl | Me | H | OMe | H | H | H |
| 2329. | Cl | Me | C(O)OMe | H | H | H | H |
| 2330. | Cl | Me | F | Cl | H | H | H |
| 2331. | Cl | Me | F | Me | H | H | H |
| 2332. | Cl | Me | H | Me | H | H | H |
| 2333. | Cl | Me | OMe | H | H | H | H |
| 2334. | Cl | Me | F | F | F | H | H |
| 2335. | Cl | Me | F | F | H | F | H |
| 2336. | Cl | Me | H | F | F | F | H |
| 2337. | Cl | Me | F | H | F | F | H |
| 2338. | Cl | Me | Me | H | Me | H | H |
| 2339. | Cl | Me | Me | H | H | Me | H |
| 2340. | Cl | Me | F | H | H | CF₃ | H |
| 2341. | Cl | Me | F | H | Br | H | H |
| 2342. | Cl | Me | Me | Me | H | H | H |
| 2343. | Cl | Me | F | F | F | F | F |
| 2344. | Cl | Me | F | H | H | H | OMe |
| 2345. | Cl | Me | Cl | H | F | H | H |
| 2346. | Cl | Me | NO₂ | H | Cl | H | H |
| 2347. | Cl | Me | NO₂ | H | H | Me | H |
| 2348. | Cl | Me | F | H | H | H | I |
| 2349. | Cl | Me | F | H | H | H | Br |
| 2350. | Cl | Me | Br | H | H | H | Br |
| 2351. | Cl | Me | Cl | H | H | H | Me |
| 2352. | Cl | Me | Cl | H | H | H | OCHF₂ |
| 2353. | Cl | Me | Cl | H | H | H | OMe |
| 2354. | Cl | Me | Me | H | H | H | OMe |
| 2355. | Cl | Me | OEt | H | H | H | CF₃ |
| 2356. | Cl | Me | OC(O)Me | H | H | H | H |
| 2357. | Cl | Me | OEt | H | H | H | Me |
| 2358. | Cl | Me | Me | Me | H | H | Me |
| 2359. | Cl | Me | Cl | H | H | H | C(O)OMe |
| 2360. | Cl | Me | Cl | H | H | OMe | H |
| 2361. | Cl | Me | F | F | H | F | F |
| 2362. | Cl | Me | Cl | H | H | F | H |
| 2363. | Cl | Me | F | H | H | F | Cl |
| 2364. | Cl | Me | F | H | H | Cl | H |
| 2365. | Cl | Me | Cl | H | H | CF₃ | H |
| 2366. | Cl | Me | Cl | Me | H | H | H |
| 2367. | Cl | Me | OCHF₂ | H | H | H | H |
| 2368. | Cl | Me | OCH₂CF₃ | H | H | H | H |
| 2369. | Cl | Me | CF₃ | H | H | H | OCHF₂ |
| 2370. | Cl | Me | CF₃ | H | H | H | OCH₂CF₃ |
| 2371. | Cl | Me | Me | H | H | H | Me |
| 2372. | Cl | Me | Cl | H | H | H | F |
| 2373. | Cl | Me | F | H | F | H | H |
| 2374. | Cl | Me | F | Me | H | H | Cl |
| 2375. | Cl | Me | F | H | H | OMe | H |
| 2376. | Cl | Me | Cl | H | OCH₂O | | H |
| 2377. | Cl | Me | Me | H | H | F | H |
| 2378. | Cl | Me | OCF₃ | H | H | H | H |
| 2379. | Cl | Me | F | F | H | H | H |
| 2380. | Cl | Me | OMe | H | H | Cl | H |

**Tabelle 3: Verbindungen der Formel Ib-S**

| 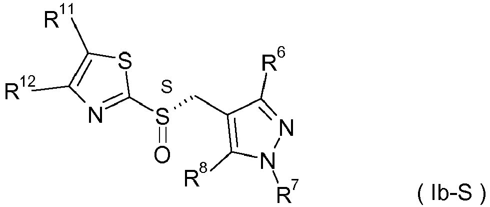 | | | | | |
|---|---|---|---|---|---|
| Bsp. Nr. | R¹¹ | R¹² | R⁶ | R⁷ | R⁸ |
| 2381. | H | H | CF₃ | Ph | Cl |
| 2382. | H | H | CF₃ | tBu | Cl |
| 2383. | H | H | CF₃ | CHF₂ | Cl |

| Bsp. Nr. | R¹¹ | R¹² | R⁶ | R⁷ | R⁸ |
|---|---|---|---|---|---|
| 2384. | H | H | Cl | CHF₂ | CF₃ |
| 2385. | H | H | CF₃ | Me | OMe |
| 2386. | H | H | CF₃ | Me | CN |
| 2387. | H | H | Cl | Et | Cl |
| 2388. | H | H | CHF₂ | Me | Cl |
| 2389. | H | H | Me | Me | Me |
| 2390. | H | H | Me | Me | Cl |
| 2391. | H | H | Cl | Me | Cl |
| 2392. | H | H | CF₃ | Me | Cl |
| 2393. | H | H | Cl | Me | CF₃ |
| 2394. | H | H | CF₃ | Me | F |
| 2395. | H | H | OMe | Me | CF₃ |
| 2396. | H | H | CF₃ | Me | OEt |
| 2397. | H | H | CF₃ | Me | OCHF₂ |
| 2398. | H | H | OCHF₂ | Me | CF₃ |
| 2399. | H | H | CF₃ | Me | OCH₂CHF₂ |
| 2400. | H | H | CF₃ | Me | OCH₂CF₃ |
| 2401. | H | H | CF₃ | Me | OCH₂CN |
| 2402. | H | H | CF₃ | Me | SO₂Me |
| 2403. | H | H | CF₃ | Me | SEt |
| 2404. | H | H | CF₃ | Me | Me |
| 2405. | H | H | CF₃ | Me | Et |
| 2406. | H | H | CF₃ | Et | Cl |
| 2407. | H | H | Cl | Et | CF₃ |
| 2408. | H | H | CF₃ | iPr | Cl |
| 2409. | H | H | Cl | iPr | CF₃ |
| 2410. | H | H | CF₃ | tBu | Cl |
| 2411. | H | H | Cl | tBu | CF₃ |
| 2412. | H | H | CF₃ | cPen | Cl |
| 2413. | H | H | Cl | cPen | CF₃ |
| 2414. | H | H | CF₃ | CH₂cPr | Cl |
| 2415. | H | H | Cl | CH₂cPr | CF₃ |
| 2416. | H | H | CF₃ | CH₂CH=CH₂ | Cl |
| 2417. | H | H | Cl | CH₂CH=CH₂ | CF₃ |
| 2418. | H | H | CF₃ | CHF₂ | OMe |
| 2419. | H | H | OMe | CHF₂ | CF₃ |
| 2420. | H | H | CF₃ | CH₂CF₃ | Cl |
| 2421. | H | H | Cl | CH₂CF₃ | CF₃ |
| 2422. | H | H | CF₃ | CH₂OMe | Cl |
| 2423. | H | H | Cl | CH₂OMe | CF₃ |
| 2424. | H | H | CF₃ | CH₂CN | Cl |
| 2425. | H | H | Me | Ph | Me |
| 2426. | H | H | Me | Ph | Cl |
| 2427. | H | H | Et | Ph | Cl |
| 2428. | H | H | Pr | Ph | Cl |
| 2429. | H | H | iPr | Ph | Cl |
| 2430. | H | H | CF₃ | Ph | Cl |
| 2431. | H | H | CF₃ | Ph | Me |
| 2432. | H | H | CF₃ | Ph | CF₃ |
| 2433. | H | H | CF₃ | Ph | F |
| 2434. | H | H | CF₃ | Ph | OMe |
| 2435. | H | H | CF₃ | Ph | OEt |
| 2436. | H | H | CF₃ | Ph | OCHF₂ |
| 2437. | H | H | CF₃ | Ph | CN |
| 2438. | H | H | CF₃ | Ph(4-Cl) | Cl |
| 2439. | H | H | Me | Me | OCH₂CF₃ |
| 2440. | H | H | CF₃ | Me | 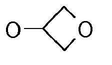 |
| 2441. | H | H | CF₃ | Me | H |
| 2442. | H | H | CF₃ | Me | OCH₂CH₂OMe |
| 2443. | H | H | CF₃ | Me | SMe |
| 2444. | H | H | CF₃ | Me | OCH₂CH₂CH₂F |
| 2445. | H | H | CF₃ | Me | OCH(CH₂F)₂ |
| 2446. | H | H | CF₃ | Me | OCH₂CF₂CHF₂ |
| 2447. | H | H | CF₃ | Me | OCH₂CF=CH₂ |
| 2448. | H | H | CF₃ | Me | OCH(Me)CF₃ |
| 2449. | H | H | CF₃ | Me | OCH(Me)CH₂F |
| 2450. | H | H | OCH₂CF₃ | Me | CF₃ |
| 2451. | H | H | OCH₂CF₃ | Me | CHF₂ |
| 2452. | H | H | CHF₂ | Me | CHF₂ |
| 2453. | H | H | CF₃ | Me | CHF₂ |
| 2454. | H | H | Cl | Me | OCHF₂ |
| 2455. | H | H | Br | Me | OCHF₂ |
| 2456. | H | H | Br | Me | CF₃ |
| 2457. | F | H | CF₃ | Ph | Cl |
| 2458. | F | H | CF₃ | tBu | Cl |
| 2459. | F | H | CF₃ | CHF₂ | Cl |
| 2460. | F | H | Cl | CHF₂ | CF₃ |
| 2461. | F | H | CF₃ | Me | OMe |
| 2462. | F | H | CF₃ | Me | CN |
| 2463. | F | H | Cl | Et | Cl |
| 2464. | F | H | CHF₂ | Me | Cl |
| 2465. | F | H | Me | Me | Me |
| 2466. | F | H | Me | Me | Cl |
| 2467. | F | H | Cl | Me | Cl |
| 2468. | F | H | CF₃ | Me | Cl |
| 2469. | F | H | Cl | Me | CF₃ |
| 2470. | F | H | CF₃ | Me | F |
| 2471. | F | H | OMe | Me | CF₃ |
| 2472. | F | H | CF₃ | Me | OEt |
| 2473. | F | H | CF₃ | Me | OCHF₂ |
| 2474. | F | H | OCHF₂ | Me | CF₃ |
| 2475. | F | H | CF₃ | Me | OCH₂CHF₂ |
| 2476. | F | H | CF₃ | Me | OCH₂CF₃ |
| 2477. | F | H | CF₃ | Me | OCH₂CN |
| 2478. | F | H | CF₃ | Me | SO₂Me |
| 2479. | F | H | CF₃ | Me | SEt |
| 2480. | F | H | CF₃ | Me | Me |
| 2481. | F | H | CF₃ | Me | Et |
| 2482. | F | H | CF₃ | Et | Cl |
| 2483. | F | H | Cl | Et | CF₃ |
| 2484. | F | H | CF₃ | iPr | Cl |
| 2485. | F | H | Cl | iPr | CF₃ |
| 2486. | F | H | CF₃ | tBu | Cl |
| 2487. | F | H | Cl | tBu | CF₃ |
| 2488. | F | H | CF₃ | cPen | Cl |
| 2489. | F | H | Cl | cPen | CF₃ |
| 2490. | F | H | CF₃ | CH₂cPr | Cl |
| 2491. | F | H | Cl | CH₂cPr | CF₃ |
| 2492. | F | H | CF₃ | CH₂CH=CH₂ | Cl |
| 2493. | F | H | Cl | CH₂CH=CH₂ | CF₃ |
| 2494. | F | H | CF₃ | CHF₂ | OMe |
| 2495. | F | H | OMe | CHF₂ | CF₃ |
| 2496. | F | H | CF₃ | CH₂CF₃ | Cl |
| 2497. | F | H | Cl | CH₂CF₃ | CF₃ |
| 2498. | F | H | CF₃ | CH₂OMe | Cl |
| 2499. | F | H | Cl | CH₂OMe | CF₃ |
| 2500. | F | H | CF₃ | CH₂CN | Cl |
| 2501. | F | H | Me | Ph | Me |
| 2502. | F | H | Me | Ph | Cl |
| 2503. | F | H | Et | Ph | Cl |
| 2504. | F | H | Pr | Ph | Cl |
| 2505. | F | H | iPr | Ph | Cl |
| 2506. | F | H | CF₃ | Ph | Cl |
| 2507. | F | H | CF₃ | Ph | Me |
| 2508. | F | H | CF₃ | Ph | CF₃ |
| 2509. | F | H | CF₃ | Ph | F |
| 2510. | F | H | CF₃ | Ph | OMe |
| 2511. | F | H | CF₃ | Ph | OEt |
| 2512. | F | H | CF₃ | Ph | OCHF₂ |
| 2513. | F | H | CF₃ | Ph | CN |
| 2514. | F | H | CF₃ | Ph(4-Cl) | Cl |
| 2515. | F | H | Me | Me | OCH₂CF₃ |
| 2516. | F | H | CF₃ | Me | 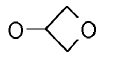 |
| 2517. | F | H | CF₃ | Me | H |
| 2518. | F | H | CF₃ | Me | OCH₂CH₂OMe |
| 2519. | F | H | CF₃ | Me | SMe |
| 2520. | F | H | CF₃ | Me | OCH₂CH₂CH₂F |
| 2521. | F | H | CF₃ | Me | OCH(CH₂F)₂ |
| 2522. | F | H | CF₃ | Me | OCH₂CF₂CHF₂ |
| 2523. | F | H | CF₃ | Me | OCH₂CF=CH₂ |
| 2524. | F | H | CF₃ | Me | OCH(Me)CF₃ |
| 2525. | F | H | CF₃ | Me | OCH(Me)CH₂F |
| 2526. | F | H | OCH₂CF₃ | Me | CF₃ |
| 2527. | F | H | OCH₂CF₃ | Me | CHF₂ |
| 2528. | F | H | CHF₂ | Me | CHF₂ |
| 2529. | F | H | CF₃ | Me | CHF₂ |
| 2530. | F | H | Cl | Me | OCHF₂ |
| 2531. | F | H | Br | Me | OCHF₂ |
| 2532. | F | H | Br | Me | CF₃ |
| 2533. | F | H | CF₃ | Me | CF₃ |
| 2534. | F | H | CHF₂ | Me | OCHF₂ |
| 2535. | F | H | CHF₂ | Me | CF₃ |
| 2536. | F | H | CF₂CF₃ | Me | CF₃ |
| 2537. | F | H | CF₃ | Me | CF₂CF₃ |
| 2538. | F | H | CHF₂ | Me | OCH₂CF₃ |
| 2539. | Cl | H | CF₃ | Ph | Cl |
| 2540. | Cl | H | CF₃ | tBu | Cl |
| 2541. | Cl | H | CF₃ | CHF₂ | Cl |
| 2542. | Cl | H | Cl | CHF₂ | CF₃ |
| 2543. | Cl | H | CF₃ | Me | OMe |
| 2544. | Cl | H | CF₃ | Me | CN |
| 2545. | Cl | H | Cl | Et | Cl |
| 2546. | Cl | H | CHF₂ | Me | Cl |
| 2547. | Cl | H | Me | Me | Me |
| 2548. | Cl | H | Me | Me | Cl |
| 2549. | Cl | H | Cl | Me | Cl |
| 2550. | Cl | H | CF₃ | Me | Cl |
| 2551. | Cl | H | Cl | Me | CF₃ |
| 2552. | Cl | H | CF₃ | Me | F |
| 2553. | Cl | H | OMe | Me | CF₃ |
| 2554. | Cl | H | CF₃ | Me | OEt |
| 2555. | Cl | H | CF₃ | Me | OCHF₂ |
| 2556. | Cl | H | OCHF₂ | Me | CF₃ |
| 2557. | Cl | H | CF₃ | Me | OCH₂CHF₂ |
| 2558. | Cl | H | CF₃ | Me | OCH₂CF₃ |
| 2559. | Cl | H | CF₃ | Me | OCH₂CN |
| 2560. | Cl | H | CF₃ | Me | SO₂Me |
| 2561. | Cl | H | CF₃ | Me | SEt |
| 2562. | Cl | H | CF₃ | Me | Me |
| 2563. | Cl | H | CF₃ | Me | Et |
| 2564. | Cl | H | CF₃ | Et | Cl |
| 2565. | Cl | H | Cl | Et | CF₃ |
| 2566. | Cl | H | CF₃ | iPr | Cl |
| 2567. | Cl | H | Cl | iPr | CF₃ |
| 2568. | Cl | H | CF₃ | tBu | Cl |
| 2569. | Cl | H | Cl | tBu | CF₃ |
| 2570. | Cl | H | CF₃ | cPen | Cl |
| 2571. | Cl | H | Cl | cPen | CF₃ |
| 2572. | Cl | H | CF₃ | CH₂cPr | Cl |
| 2573. | Cl | H | Cl | CH₂cPr | CF₃ |
| 2574. | Cl | H | CF₃ | CH₂CH=CH₂ | Cl |
| 2575. | Cl | H | Cl | CH₂CH=CH₂ | CF₃ |
| 2576. | Cl | H | CF₃ | CHF₂ | OMe |
| 2577. | Cl | H | OMe | CHF₂ | CF₃ |
| 2578. | Cl | H | CF₃ | CH₂CF₃ | Cl |
| 2579. | Cl | H | Cl | CH₂CF₃ | CF₃ |
| 2580. | Cl | H | CF₃ | CH₂OMe | Cl |
| 2581. | Cl | H | Cl | CH₂OMe | CF₃ |
| 2582. | Cl | H | CF₃ | CH₂CN | Cl |
| 2583. | Cl | H | Me | Ph | Me |
| 2584. | Cl | H | Me | Ph | Cl |
| 2585. | Cl | H | Et | Ph | Cl |
| 2586. | Cl | H | Pr | Ph | Cl |
| 2587. | Cl | H | iPr | Ph | Cl |
| 2588. | Cl | H | CF₃ | Ph | Cl |
| 2589. | Cl | H | CF₃ | Ph | Me |
| 2590. | Cl | H | CF₃ | Ph | CF₃ |
| 2591. | Cl | H | CF₃ | Ph | F |
| 2592. | Cl | H | CF₃ | Ph | OMe |
| 2593. | Cl | H | CF₃ | Ph | OEt |
| 2594. | Cl | H | CF₃ | Ph | OCHF₂ |
| 2595. | Cl | H | CF₃ | Ph | CN |
| 2596. | Cl | H | CF₃ | Ph(4-Cl) | Cl |
| 2597. | Cl | H | Me | Me | OCH₂CF₃ |
| 2598. | Cl | H | CF₃ | Me | 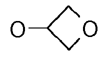 |
| 2599. | Cl | H | CF₃ | Me | H |
| 2600. | Cl | H | CF₃ | Me | OCH₂CH₂OMe |
| 2601. | Cl | H | CF₃ | Me | SMe |
| 2602. | Cl | H | CF₃ | Me | OCH₂CH₂CH₂F |
| 2603. | Cl | H | CF₃ | Me | OCH(CH₂F)₂ |
| 2604. | Cl | H | CF₃ | Me | OCH₂CF₂CHF₂ |
| 2605. | Cl | H | CF₃ | Me | OCH₂CF=CH₂ |
| 2606. | Cl | H | CF₃ | Me | OCH(Me)CF₃ |
| 2607. | Cl | H | CF₃ | Me | OCH(Me)CH₂F |
| 2608. | Cl | H | OCH₂CF₃ | Me | CF₃ |
| 2609. | Cl | H | OCH₂CF₃ | Me | CHF₂ |
| 2610. | Cl | H | CHF₂ | Me | CHF₂ |
| 2611. | Cl | H | CF₃ | Me | CHF₂ |
| 2612. | Cl | H | Cl | Me | OCHF₂ |
| 2613. | Cl | H | Br | Me | OCHF₂ |
| 2614. | Cl | H | Br | Me | CF₃ |
| 2615. | Cl | H | CF₃ | Me | CF₃ |
| 2616. | Cl | H | CHF₂ | Me | OCHF₂ |
| 2617. | Cl | H | CHF₂ | Me | CF₃ |
| 2618. | Cl | H | CF₂CF₃ | Me | CF₃ |
| 2619. | Cl | H | CF₃ | Me | CF₂CF₃ |
| 2620. | Cl | H | CHF₂ | Me | OCH₂CF₃ |
| 2621. | Br | H | CF₃ | Ph | Cl |
| 2622. | Br | H | CF₃ | tBu | Cl |
| 2623. | Br | H | CF₃ | CHF₂ | Cl |
| 2624. | Br | H | Cl | CHF₂ | CF₃ |
| 2625. | Br | H | CF₃ | Me | OMe |
| 2626. | Br | H | CF₃ | Me | CN |
| 2627. | Br | H | Cl | Et | Cl |
| 2628. | Br | H | CHF₂ | Me | Cl |
| 2629. | Br | H | Me | Me | Me |
| 2630. | Br | H | Me | Me | Cl |
| 2631. | Br | H | Cl | Me | Cl |
| 2632. | Br | H | CF₃ | Me | Cl |
| 2633. | Br | H | Cl | Me | CF₃ |
| 2634. | Br | H | CF₃ | Me | F |
| 2635. | Br | H | OMe | Me | CF₃ |
| 2636. | Br | H | CF₃ | Me | OEt |
| 2637. | Br | H | CF₃ | Me | OCHF₂ |
| 2638. | Br | H | OCHF₂ | Me | CF₃ |
| 2639. | Br | H | CF₃ | Me | OCH₂CHF₂ |
| 2640. | Br | H | CF₃ | Me | OCH₂CF₃ |
| 2641. | Br | H | CF₃ | Me | OCH₂CN |
| 2642. | Br | H | CF₃ | Me | SO₂Me |
| 2643. | Br | H | CF₃ | Me | SEt |
| 2644. | Br | H | CF₃ | Me | Me |
| 2645. | Br | H | CF₃ | Me | Et |
| 2646. | Br | H | CF₃ | Et | Cl |
| 2647. | Br | H | Cl | Et | CF₃ |
| 2648. | Br | H | CF₃ | iPr | Cl |
| 2649. | Br | H | Cl | iPr | CF₃ |
| 2650. | Br | H | CF₃ | tBu | Cl |
| 2651. | Br | H | Cl | tBu | CF₃ |
| 2652. | Br | H | CF₃ | cPen | Cl |
| 2653. | Br | H | Cl | cPen | CF₃ |
| 2654. | Br | H | CF₃ | CH₂cPr | Cl |
| 2655. | Br | H | Cl | CH₂cPr | CF₃ |
| 2656. | Br | H | CF₃ | CH₂CH=CH₂ | Cl |
| 2657. | Br | H | Cl | CH₂CH=CH₂ | CF₃ |
| 2658. | Br | H | CF₃ | CHF₂ | OMe |
| 2659. | Br | H | OMe | CHF₂ | CF₃ |
| 2660. | Br | H | CF₃ | CH₂CF₃ | Cl |
| 2661. | Br | H | Cl | CH₂CF₃ | CF₃ |
| 2662. | Br | H | CF₃ | CH₂OMe | Cl |
| 2663. | Br | H | Cl | CH₂OMe | CF₃ |
| 2664. | Br | H | CF₃ | CH₂CN | Cl |
| 2665. | Br | H | Me | Ph | Me |
| 2666. | Br | H | Me | Ph | Cl |
| 2667. | Br | H | Et | Ph | Cl |
| 2668. | Br | H | Pr | Ph | Cl |
| 2669. | Br | H | iPr | Ph | Cl |
| 2670. | Br | H | CF₃ | Ph | Cl |
| 2671. | Br | H | CF₃ | Ph | Me |
| 2672. | Br | H | CF₃ | Ph | CF₃ |
| 2673. | Br | H | CF₃ | Ph | F |
| 2674. | Br | H | CF₃ | Ph | OMe |
| 2675. | Br | H | CF₃ | Ph | OEt |
| 2676. | Br | H | CF₃ | Ph | OCHF₂ |
| 2677. | Br | H | CF₃ | Ph | CN |
| 2678. | Br | H | CF₃ | Ph(4-Cl) | Cl |
| 2679. | Br | H | Me | Me | OCH₂CF₃ |
| 2680. | Br | H | CF₃ | Me | 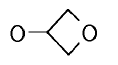 |
| 2681. | Br | H | CF₃ | Me | H |
| 2682. | Br | H | CF₃ | Me | OCH₂CH₂OMe |
| 2683. | Br | H | CF₃ | Me | SMe |
| 2684. | Br | H | CF₃ | Me | OCH₂CH₂CH₂F |
| 2685. | Br | H | CF₃ | Me | OCH(CH₂F)₂ |
| 2686. | Br | H | CF₃ | Me | OCH₂CF₂CHF₂ |
| 2687. | Br | H | CF₃ | Me | OCH₂CF=CH₂ |
| 2688. | Br | H | CF₃ | Me | OCH(Me)CF₃ |
| 2689. | Br | H | CF₃ | Me | OCH(Me)CH₂F |
| 2690. | Br | H | OCH₂CF₃ | Me | CF₃ |
| 2691. | Br | H | OCH₂CF₃ | Me | CHF₂ |
| 2692. | Br | H | CHF₂ | Me | CHF₂ |
| 2693. | Br | H | CF₃ | Me | CHF₂ |
| 2694. | Br | H | Cl | Me | OCHF₂ |
| 2695. | Br | H | Br | Me | OCHF₂ |
| 2696. | Br | H | Br | Me | CF₃ |
| 2697. | Br | H | CF₃ | Me | CF₃ |
| 2698. | Br | H | CHF₂ | Me | OCHF₂ |
| 2699. | Br | H | CHF₂ | Me | CF₃ |
| 2700. | Br | H | CF₂CF₃ | Me | CF₃ |
| 2701. | Br | H | CF₃ | Me | CF₂CF₃ |
| 2702. | Br | H | CHF₂ | Me | OCH₂CF₃ |
| 2703. | I | H | CF₃ | Ph | Cl |
| 2704. | I | H | CF₃ | tBu | Cl |
| 2705. | I | H | CF₃ | CHF₂ | Cl |
| 2706. | I | H | Cl | CHF₂ | CF₃ |
| 2707. | I | H | CF₃ | Me | OMe |
| 2708. | I | H | CF₃ | Me | CN |
| 2709. | I | H | Cl | Et | Cl |
| 2710. | I | H | CHF₂ | Me | Cl |
| 2711. | I | H | Me | Me | Me |
| 2712. | I | H | Me | Me | Cl |
| 2713. | I | H | Cl | Me | Cl |
| 2714. | I | H | CF₃ | Me | Cl |
| 2715. | I | H | Cl | Me | CF₃ |
| 2716. | I | H | CF₃ | Me | F |
| 2717. | I | H | OMe | Me | CF₃ |
| 2718. | I | H | CF₃ | Me | OEt |
| 2719. | I | H | CF₃ | Me | OCHF₂ |
| 2720. | I | H | OCHF₂ | Me | CF₃ |
| 2721. | I | H | CF₃ | Me | OCH₂CHF₂ |
| 2722. | I | H | CF₃ | Me | OCH₂CF₃ |
| 2723. | I | H | CF₃ | Me | OCH₂CN |
| 2724. | I | H | CF₃ | Me | SO₂Me |
| 2725. | I | H | CF₃ | Me | SEt |
| 2726. | I | H | CF₃ | Me | Me |
| 2727. | I | H | CF₃ | Me | Et |
| 2728. | I | H | CF₃ | Et | Cl |
| 2729. | I | H | Cl | Et | CF₃ |
| 2730. | I | H | CF₃ | iPr | Cl |
| 2731. | I | H | Cl | iPr | CF₃ |
| 2732. | I | H | CF₃ | tBu | Cl |
| 2733. | I | H | Cl | tBu | CF₃ |
| 2734. | I | H | CF₃ | cPen | Cl |
| 2735. | I | H | Cl | cPen | CF₃ |
| 2736. | I | H | CF₃ | CH₂cPr | Cl |
| 2737. | I | H | Cl | CH₂cPr | CF₃ |
| 2738. | I | H | CF₃ | CH₂CH=CH₂ | Cl |
| 2739. | I | H | Cl | CH₂CH=CH₂ | CF₃ |
| 2740. | I | H | CF₃ | CHF₂ | OMe |
| 2741. | I | H | OMe | CHF₂ | CF₃ |
| 2742. | I | H | CF₃ | CH₂CF₃ | Cl |
| 2743. | I | H | Cl | CH₂CF₃ | CF₃ |
| 2744. | I | H | CF₃ | CH₂OMe | Cl |
| 2745. | I | H | Cl | CH₂OMe | CF₃ |
| 2746. | I | H | CF₃ | CH₂CN | Cl |
| 2747. | I | H | Me | Ph | Me |
| 2748. | I | H | Me | Ph | Cl |
| 2749. | I | H | Et | Ph | Cl |
| 2750. | I | H | Pr | Ph | Cl |
| 2751. | I | H | iPr | Ph | Cl |
| 2752. | I | H | CF₃ | Ph | Cl |
| 2753. | I | H | CF₃ | Ph | Me |
| 2754. | I | H | CF₃ | Ph | CF₃ |
| 2755. | I | H | CF₃ | Ph | F |
| 2756. | I | H | CF₃ | Ph | OMe |
| 2757. | I | H | CF₃ | Ph | OEt |
| 2758. | I | H | CF₃ | Ph | OCHF₂ |
| 2759. | I | H | CF₃ | Ph | CN |
| 2760. | I | H | CF₃ | Ph(4-Cl) | Cl |
| 2761. | I | H | Me | Me | OCH₂CF₃ |
| 2762. | I | H | CF₃ | Me | 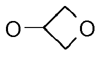 |
| 2763. | I | H | CF₃ | Me | H |
| 2764. | I | H | CF₃ | Me | OCH₂CH₂OMe |
| 2765. | I | H | CF₃ | Me | SMe |
| 2766. | I | H | CF₃ | Me | OCH₂CH₂CH₂F |
| 2767. | I | H | CF₃ | Me | OCH(CH₂F)₂ |
| 2768. | I | H | CF₃ | Me | OCH₂CF₂CHF₂ |
| 2769. | I | H | CF₃ | Me | OCH₂CF=CH₂ |
| 2770. | I | H | CF₃ | Me | OCH(Me)CF₃ |
| 2771. | I | H | CF₃ | Me | OCH(Me)CH₂F |
| 2772. | I | H | OCH₂CF₃ | Me | CF₃ |
| 2773. | I | H | OCH₂CF₃ | Me | CHF₂ |
| 2774. | I | H | CHF₂ | Me | CHF₂ |
| 2775. | I | H | CF₃ | Me | CHF₂ |
| 2776. | I | H | Cl | Me | OCHF₂ |
| 2777. | I | H | Br | Me | OCHF₂ |
| 2778. | I | H | Br | Me | CF₃ |
| 2779. | I | H | CF₃ | Me | CF₃ |
| 2780. | I | H | CHF₂ | Me | OCHF₂ |
| 2781. | I | H | CHF₂ | Me | CF₃ |
| 2782. | I | H | CF₂CF₃ | Me | CF₃ |
| 2783. | I | H | CF₃ | Me | CF₂CF₃ |
| 2784. | I | H | CHF₂ | Me | OCH₂CF₃ |
| 2785. | H | Cl | CF₃ | Ph | Cl |
| 2786. | H | Cl | CF₃ | tBu | Cl |
| 2787. | H | Cl | CF₃ | CHF₂ | Cl |
| 2788. | H | Cl | Cl | CHF₂ | CF₃ |
| 2789. | H | Cl | CF₃ | Me | OMe |
| 2790. | H | Cl | CF₃ | Me | CN |
| 2791. | H | Cl | Cl | Et | Cl |
| 2792. | H | Cl | CHF₂ | Me | Cl |
| 2793. | H | Cl | Me | Me | Me |
| 2794. | H | Cl | Me | Me | Cl |
| 2795. | H | Cl | Cl | Me | Cl |
| 2796. | H | Cl | CF₃ | Me | Cl |
| 2797. | H | Cl | Cl | Me | CF₃ |
| 2798. | H | Cl | CF₃ | Me | F |
| 2799. | H | Cl | OMe | Me | CF₃ |
| 2800. | H | Cl | CF₃ | Me | OEt |
| 2801. | H | Cl | CF₃ | Me | OCHF₂ |
| 2802. | H | Cl | OCHF₂ | Me | CF₃ |
| 2803. | H | Cl | CF₃ | Me | OCH₂CHF₂ |
| 2804. | H | Cl | CF₃ | Me | OCH₂CF₃ |
| 2805. | H | Cl | CF₃ | Me | OCH₂CN |
| 2806. | H | Cl | CF₃ | Me | SO₂Me |
| 2807. | H | Cl | CF₃ | Me | SEt |
| 2808. | H | Cl | CF₃ | Me | Me |
| 2809. | H | Cl | CF₃ | Me | Et |
| 2810. | H | Cl | CF₃ | Et | Cl |
| 2811. | H | Cl | Cl | Et | CF₃ |
| 2812. | H | Cl | CF₃ | iPr | Cl |
| 2813. | H | Cl | Cl | iPr | CF₃ |
| 2814. | H | Cl | CF₃ | tBu | Cl |
| 2815. | H | Cl | Cl | tBu | CF3 |
| 2816. | H | Cl | CF₃ | cPen | Cl |
| 2817. | H | Cl | Cl | cPen | CF₃ |
| 2818. | H | Cl | CF₃ | CH₂cPr | Cl |
| 2819. | H | Cl | Cl | CH₂cPr | CF₃ |
| 2820. | H | Cl | CF₃ | CH₂CH=CH₂ | Cl |
| 2821. | H | Cl | Cl | CH₂CH=CH₂ | CF₃ |
| 2822. | H | Cl | CF₃ | CHF₂ | OMe |
| 2823. | H | Cl | OMe | CHF₂ | CF₃ |
| 2824. | H | Cl | CF₃ | CH₂CF₃ | Cl |
| 2825. | H | Cl | Cl | CH₂CF₃ | CF₃ |
| 2826. | H | Cl | CF₃ | CH₂OMe | Cl |
| 2827. | H | Cl | Cl | CH₂OMe | CF₃ |
| 2828. | H | Cl | CF₃ | CH₂CN | Cl |
| 2829. | H | Cl | Me | Ph | Me |
| 2830. | H | Cl | Me | Ph | Cl |
| 2831. | H | Cl | Et | Ph | Cl |
| 2832. | H | Cl | Pr | Ph | Cl |
| 2833. | H | Cl | iPr | Ph | Cl |
| 2834. | H | Cl | CF₃ | Ph | Cl |
| 2835. | H | Cl | CF₃ | Ph | Me |
| 2836. | H | Cl | CF₃ | Ph | CF₃ |
| 2837. | H | Cl | CF₃ | Ph | F |
| 2838. | H | Cl | CF₃ | Ph | OMe |
| 2839. | H | Cl | CF₃ | Ph | OEt |
| 2840. | H | Cl | CF₃ | Ph | OCHF₂ |
| 2841. | H | Cl | CF₃ | Ph | CN |
| 2842. | H | Cl | CF₃ | Ph(4-Cl) | Cl |
| 2843. | H | Cl | Me | Me | OCH₂CF₃ |
| 2844. | H | Cl | CF₃ | Me | 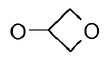 |
| 2845. | H | Cl | CF₃ | Me | H |
| 2846. | H | Cl | CF₃ | Me | OCH₂CH₂OMe |
| 2847. | H | Cl | CF₃ | Me | SMe |
| 2848. | H | Cl | CF₃ | Me | OCH₂CH₂CH₂F |
| 2849. | H | Cl | CF₃ | Me | OCH(CH₂F)₂ |
| 2850. | H | Cl | CF₃ | Me | OCH₂CF₂CHF₂ |
| 2851. | H | Cl | CF₃ | Me | OCH₂CF=CH₂ |
| 2852. | H | Cl | CF₃ | Me | OCH(Me)CF₃ |
| 2853. | H | Cl | CF₃ | Me | OCH(Me)CH₂F |
| 2854. | H | Cl | OCH₂CF₃ | Me | CF₃ |
| 2855. | H | Cl | OCH₂CF₃ | Me | CHF₂ |
| 2856. | H | Cl | CHF₂ | Me | CHF₂ |
| 2857. | H | Cl | CF₃ | Me | CHF₂ |
| 2858. | H | Cl | Cl | Me | OCHF₂ |
| 2859. | H | Cl | Br | Me | OCHF₂ |
| 2860. | H | Cl | Br | Me | CF₃ |
| 2861. | H | Br | CF₃ | Ph | Cl |
| 2862. | H | Br | CF₃ | tBu | Cl |
| 2863. | H | Br | CF₃ | CHF₂ | Cl |
| 2864. | H | Br | Cl | CHF₂ | CF₃ |
| 2865. | H | Br | CF₃ | Me | OMe |
| 2866. | H | Br | CF₃ | Me | CN |
| 2867. | H | Br | Cl | Et | Cl |
| 2868. | H | Br | CHF₂ | Me | Cl |
| 2869. | H | Br | Me | Me | Me |
| 2870. | H | Br | Me | Me | Cl |
| 2871. | H | Br | Cl | Me | Cl |
| 2872. | H | Br | CF₃ | Me | Cl |
| 2873. | H | Br | Cl | Me | CF₃ |
| 2874. | H | Br | CF₃ | Me | F |
| 2875. | H | Br | OMe | Me | CF₃ |
| 2876. | H | Br | CF₃ | Me | OEt |
| 2877. | H | Br | CF₃ | Me | OCHF₂ |
| 2878. | H | Br | OCHF₂ | Me | CF₃ |
| 2879. | H | Br | CF₃ | Me | OCH₂CHF₂ |
| 2880. | H | Br | CF₃ | Me | OCH₂CF₃ |
| 2881. | H | Br | CF₃ | Me | OCH₂CN |
| 2882. | H | Br | CF₃ | Me | SO₂Me |
| 2883. | H | Br | CF₃ | Me | SEt |
| 2884. | H | Br | CF₃ | Me | Me |
| 2885. | H | Br | CF₃ | Me | Et |
| 2886. | H | Br | CF₃ | Et | Cl |
| 2887. | H | Br | Cl | Et | CF₃ |
| 2888. | H | Br | CF₃ | iPr | Cl |
| 2889. | H | Br | Cl | iPr | CF₃ |
| 2890. | H | Br | CF₃ | tBu | Cl |
| 2891. | H | Br | Cl | tBu | CF₃ |
| 2892. | H | Br | CF₃ | cPen | Cl |
| 2893. | H | Br | Cl | cPen | CF₃ |
| 2894. | H | Br | CF₃ | CH₂cPr | Cl |
| 2895. | H | Br | Cl | CH₂cPr | CF₃ |
| 2896. | H | Br | CF₃ | CH₂CH=CH₂ | Cl |
| 2897. | H | Br | Cl | CH₂CH=CH₂ | CF₃ |
| 2898. | H | Br | CF₃ | CHF₂ | OMe |
| 2899. | H | Br | OMe | CHF₂ | CF₃ |
| 2900. | H | Br | CF₃ | CH₂CF₃ | Cl |
| 2901. | H | Br | Cl | CH₂CF₃ | CF₃ |
| 2902. | H | Br | CF₃ | CH₂OMe | Cl |
| 2903. | H | Br | Cl | CH₂OMe | CF₃ |
| 2904. | H | Br | CF₃ | CH₂CN | Cl |
| 2905. | H | Br | Me | Ph | Me |
| 2906. | H | Br | Me | Ph | Cl |
| 2907. | H | Br | Et | Ph | Cl |
| 2908. | H | Br | Pr | Ph | Cl |
| 2909. | H | Br | iPr | Ph | Cl |
| 2910. | H | Br | CF₃ | Ph | Cl |
| 2911. | H | Br | CF₃ | Ph | Me |
| 2912. | H | Br | CF₃ | Ph | CF₃ |
| 2913. | H | Br | CF₃ | Ph | F |
| 2914. | H | Br | CF₃ | Ph | OMe |
| 2915. | H | Br | CF₃ | Ph | OEt |
| 2916. | H | Br | CF₃ | Ph | OCHF₂ |
| 2917. | H | Br | CF₃ | Ph | CN |
| 2918. | H | Br | CF₃ | Ph(4-Cl) | Cl |
| 2919. | H | Br | Me | Me | OCH₂CF₃ |
| 2920. | H | Br | CF₃ | Me | 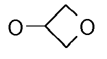 |
| 2921. | H | Br | CF₃ | Me | H |
| 2922. | H | Br | CF₃ | Me | OCH₂CH₂OMe |
| 2923. | H | Br | CF₃ | Me | SMe |
| 2924. | H | Br | CF₃ | Me | OCH₂CH₂CH₂F |
| 2925. | H | Br | CF₃ | Me | OCH(CH₂F)₂ |
| 2926. | H | Br | CF₃ | Me | OCH₂CF₂CHF₂ |
| 2927. | H | Br | CF₃ | Me | OCH₂CF=CH₂ |
| 2928. | H | Br | CF₃ | Me | OCH(Me)CF₃ |
| 2929. | H | Br | CF₃ | Me | OCH(Me)CH₂F |
| 2930. | H | Br | OCH₂CF₃ | Me | CF₃ |
| 2931. | H | Br | OCH₂CF₃ | Me | CHF₂ |
| 2932. | H | Br | CHF₂ | Me | CHF₂ |
| 2933. | H | Br | CF₃ | Me | CHF₂ |
| 2934. | H | Br | Cl | Me | OCHF₂ |
| 2935. | H | Br | Br | Me | OCHF₂ |
| 2936. | H | Br | Br | Me | CF₃ |
| 2937. | Me | H | CF₃ | Ph | Cl |
| 2938. | Me | H | CF₃ | tBu | Cl |
| 2939. | Me | H | CF₃ | CHF₂ | Cl |
| 2940. | Me | H | Cl | CHF₂ | CF₃ |
| 2941. | Me | H | CF₃ | Me | OMe |
| 2942. | Me | H | CF₃ | Me | CN |
| 2943. | Me | H | Cl | Et | Cl |
| 2944. | Me | H | CHF₂ | Me | Cl |
| 2945. | Me | H | Me | Me | Me |
| 2946. | Me | H | Me | Me | Cl |
| 2947. | Me | H | Cl | Me | Cl |
| 2948. | Me | H | CF₃ | Me | Cl |
| 2949. | Me | H | Cl | Me | CF₃ |
| 2950. | Me | H | CF₃ | Me | F |
| 2951. | Me | H | OMe | Me | CF₃ |
| 2952. | Me | H | CF₃ | Me | OEt |
| 2953. | Me | H | CF₃ | Me | OCHF₂ |
| 2954. | Me | H | OCHF₂ | Me | CF₃ |
| 2955. | Me | H | CF₃ | Me | OCH₂CHF₂ |
| 2956. | Me | H | CF₃ | Me | OCH₂CF₃ |
| 2957. | Me | H | CF₃ | Me | OCH₂CN |
| 2958. | Me | H | CF₃ | Me | SO₂Me |
| 2959. | Me | H | CF₃ | Me | SEt |
| 2960. | Me | H | CF₃ | Me | Me |
| 2961. | Me | H | CF₃ | Me | Et |
| 2962. | Me | H | CF₃ | Et | Cl |
| 2963. | Me | H | Cl | Et | CF₃ |
| 2964. | Me | H | CF₃ | iPr | Cl |
| 2965. | Me | H | Cl | iPr | CF₃ |
| 2966. | Me | H | CF₃ | tBu | Cl |
| 2967. | Me | H | Cl | tBu | CF₃ |
| 2968. | Me | H | CF₃ | cPen | Cl |
| 2969. | Me | H | Cl | cPen | CF₃ |
| 2970. | Me | H | CF₃ | CH₂cPr | Cl |
| 2971. | Me | H | Cl | CH₂cPr | CF₃ |
| 2972. | Me | H | CF₃ | CH₂CH=CH₂ | Cl |
| 2973. | Me | H | Cl | CH₂CH=CH₂ | CF₃ |
| 2974. | Me | H | CF₃ | CHF₂ | OMe |
| 2975. | Me | H | OMe | CHF₂ | CF₃ |
| 2976. | Me | H | CF₃ | CH₂CF₃ | Cl |
| 2977. | Me | H | Cl | CH₂CF₃ | CF₃ |
| 2978. | Me | H | CF₃ | CH₂OMe | Cl |
| 2979. | Me | H | Cl | CH₂OMe | CF₃ |
| 2980. | Me | H | CF₃ | CH₂CN | Cl |
| 2981. | Me | H | Me | Ph | Me |
| 2982. | Me | H | Me | Ph | Cl |
| 2983. | Me | H | Et | Ph | Cl |
| 2984. | Me | H | Pr | Ph | Cl |
| 2985. | Me | H | iPr | Ph | Cl |
| 2986. | Me | H | CF₃ | Ph | Cl |
| 2987. | Me | H | CF₃ | Ph | Me |
| 2988. | Me | H | CF₃ | Ph | CF₃ |
| 2989. | Me | H | CF₃ | Ph | F |
| 2990. | Me | H | CF₃ | Ph | OMe |
| 2991. | Me | H | CF₃ | Ph | OEt |
| 2992. | Me | H | CF₃ | Ph | OCHF₂ |
| 2993. | Me | H | CF₃ | Ph | CN |
| 2994. | Me | H | CF₃ | Ph(4-Cl) | Cl |
| 2995. | Me | H | Me | Me | OCH₂CF₃ |
| 2996. | Me | H | CF₃ | Me | 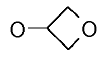 |
| 2997. | Me | H | CF₃ | Me | H |
| 2998. | Me | H | CF₃ | Me | OCH₂CH₂OMe |
| 2999. | Me | H | CF₃ | Me | SMe |
| 3000. | Me | H | CF₃ | Me | OCH₂CH₂CH₂F |
| 3001. | Me | H | CF₃ | Me | OCH(CH₂F)₂ |
| 3002. | Me | H | CF₃ | Me | OCH₂CF₂CHF₂ |
| 3003. | Me | H | CF₃ | Me | OCH₂CF=CH₂ |
| 3004. | Me | H | CF₃ | Me | OCH(Me)CF₃ |
| 3005. | Me | H | CF₃ | Me | OCH(Me)CH₂F |
| 3006. | Me | H | OCH₂CF₃ | Me | CF₃ |
| 3007. | Me | H | OCH₂CF₃ | Me | CHF₂ |
| 3008. | Me | H | CHF₂ | Me | CHF₂ |
| 3009. | Me | H | CF₃ | Me | CHF₂ |
| 3010. | Me | H | Cl | Me | OCHF₂ |
| 3011. | Me | H | Br | Me | OCHF₂ |
| 3012. | Me | H | Br | Me | CF₃ |
| 3013. | H | Me | CF₃ | Ph | Cl |
| 3014. | H | Me | CF₃ | tBu | Cl |
| 3015. | H | Me | CF₃ | CHF₂ | Cl |
| 3016. | H | Me | Cl | CHF₂ | CF₃ |
| 3017. | H | Me | CF₃ | Me | OMe |
| 3018. | H | Me | CF₃ | Me | CN |
| 3019. | H | Me | Cl | Et | Cl |
| 3020. | H | Me | CHF₂ | Me | Cl |
| 3021. | H | Me | Me | Me | Me |
| 3022. | H | Me | Me | Me | Cl |
| 3023. | H | Me | Cl | Me | Cl |
| 3024. | H | Me | CF₃ | Me | Cl |
| 3025. | H | Me | Cl | Me | CF₃ |
| 3026. | H | Me | CF₃ | Me | F |
| 3027. | H | Me | OMe | Me | CF₃ |
| 3028. | H | Me | CF₃ | Me | OEt |
| 3029. | H | Me | CF₃ | Me | OCHF₂ |
| 3030. | H | Me | OCHF₂ | Me | CF₃ |
| 3031. | H | Me | CF₃ | Me | OCH₂CHF₂ |
| 3032. | H | Me | CF₃ | Me | OCH₂CF₃ |
| 3033. | H | Me | CF₃ | Me | OCH₂CN |
| 3034. | H | Me | CF₃ | Me | SO₂Me |
| 3035. | H | Me | CF₃ | Me | SEt |
| 3036. | H | Me | CF₃ | Me | Me |
| 3037. | H | Me | CF₃ | Me | Et |
| 3038. | H | Me | CF₃ | Et | Cl |
| 3039. | H | Me | Cl | Et | CF₃ |
| 3040. | H | Me | CF₃ | iPr | Cl |
| 3041. | H | Me | Cl | iPr | CF₃ |
| 3042. | H | Me | CF₃ | tBu | Cl |
| 3043. | H | Me | Cl | tBu | CF₃ |
| 3044. | H | Me | CF₃ | cPen | Cl |
| 3045. | H | Me | Cl | cPen | CF₃ |
| 3046. | H | Me | CF₃ | CH₂cPr | Cl |
| 3047. | H | Me | Cl | CH₂cPr | CF₃ |
| 3048. | H | Me | CF₃ | CH₂CH=CH₂ | Cl |
| 3049. | H | Me | Cl | CH₂CH=CH₂ | CF₃ |
| 3050. | H | Me | CF₃ | CHF₂ | OMe |
| 3051. | H | Me | OMe | CHF₂ | CF₃ |
| 3052. | H | Me | CF₃ | CH₂CF₃ | Cl |
| 3053. | H | Me | Cl | CH₂CF₃ | CF₃ |
| 3054. | H | Me | CF₃ | CH₂OMe | Cl |
| 3055. | H | Me | Cl | CH₂OMe | CF₃ |
| 3056. | H | Me | CF₃ | CH₂CN | Cl |
| 3057. | H | Me | Me | Ph | Me |
| 3058. | H | Me | Me | Ph | Cl |
| 3059. | H | Me | Et | Ph | Cl |
| 3060. | H | Me | Pr | Ph | Cl |
| 3061. | H | Me | iPr | Ph | Cl |
| 3062. | H | Me | CF₃ | Ph | Cl |
| 3063. | H | Me | CF₃ | Ph | Me |
| 3064. | H | Me | CF₃ | Ph | CF₃ |
| 3065. | H | Me | CF₃ | Ph | F |
| 3066. | H | Me | CF₃ | Ph | OMe |
| 3067. | H | Me | CF₃ | Ph | OEt |
| 3068. | H | Me | CF₃ | Ph | OCHF₂ |
| 3069. | H | Me | CF₃ | Ph | CN |
| 3070. | H | Me | CF₃ | Ph(4-Cl) | Cl |
| 3071. | H | Me | Me | Me | OCH₂CF₃ |
| 3072. | H | Me | CF₃ | Me | 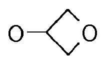 |
| 3073. | H | Me | CF₃ | Me | H |
| 3074. | H | Me | CF₃ | Me | OCH₂CH₂OMe |
| 3075. | H | Me | CF₃ | Me | SMe |
| 3076. | H | Me | CF₃ | Me | OCH₂CH₂CH₂F |
| 3077. | H | Me | CF₃ | Me | OCH(CH₂F)₂ |
| 3078. | H | Me | CF₃ | Me | OCH₂CF₂CHF₂ |
| 3079. | H | Me | CF₃ | Me | OCH₂CF=CH₂ |
| 3080. | H | Me | CF₃ | Me | OCH(Me)CF₃ |
| 3081. | H | Me | CF₃ | Me | OCH(Me)CH₂F |
| 3082. | H | Me | OCH₂CF₃ | Me | CF₃ |
| 3083. | H | Me | OCH₂CF₃ | Me | CHF₂ |
| 3084. | H | Me | CHF₂ | Me | CHF₂ |
| 3085. | H | Me | CF₃ | Me | CHF₂ |
| 3086. | H | Me | Cl | Me | OCHF₂ |
| 3087. | H | Me | Br | Me | OCHF₂ |
| 3088. | H | Me | Br | Me | CF₃ |
| 3089. | NO₂ | H | CF₃ | Ph | Cl |
| 3090. | NO₂ | H | CF₃ | tBu | Cl |
| 3091. | NO₂ | H | CF₃ | CHF₂ | Cl |
| 3092. | NO₂ | H | Cl | CHF₂ | CF₃ |
| 3093. | NO₂ | H | CF₃ | Me | OMe |
| 3094. | NO₂ | H | CF₃ | Me | CN |
| 3095. | NO₂ | H | Cl | Et | Cl |
| 3096. | NO₂ | H | CHF₂ | Me | Cl |
| 3097. | NO₂ | H | Me | Me | Me |
| 3098. | NO₂ | H | Me | Me | Cl |
| 3099. | NO₂ | H | Cl | Me | Cl |
| 3100. | NO₂ | H | CF₃ | Me | Cl |
| 3101. | NO₂ | H | Cl | Me | CF₃ |
| 3102. | NO₂ | H | CF₃ | Me | F |
| 3103. | NO₂ | H | OMe | Me | CF₃ |
| 3104. | NO₂ | H | CF₃ | Me | OEt |
| 3105. | NO₂ | H | CF₃ | Me | OCHF₂ |
| 3106. | NO₂ | H | OCHF₂ | Me | CF₃ |
| 3107. | NO₂ | H | CF₃ | Me | OCH₂CHF₂ |
| 3108. | NO₂ | H | CF₃ | Me | OCH₂CF₃ |
| 3109. | NO₂ | H | CF₃ | Me | OCH₂CN |
| 3110. | NO₂ | H | CF₃ | Me | SO₂Me |
| 3111. | NO₂ | H | CF₃ | Me | SEt |
| 3112. | NO₂ | H | CF₃ | Me | Me |
| 3113. | NO₂ | H | CF₃ | Me | Et |
| 3114. | NO₂ | H | CF₃ | Et | Cl |
| 3115. | NO₂ | H | Cl | Et | CF₃ |
| 3116. | NO₂ | H | CF₃ | iPr | Cl |
| 3117. | NO₂ | H | Cl | iPr | CF₃ |
| 3118. | NO₂ | H | CF₃ | tBu | Cl |
| 3119. | NO₂ | H | Cl | tBu | CF₃ |
| 3120. | NO₂ | H | CF₃ | cPen | Cl |
| 3121. | NO₂ | H | Cl | cPen | CF₃ |
| 3122. | NO₂ | H | CF₃ | CH₂cPr | Cl |
| 3123. | NO₂ | H | Cl | CH₂cPr | CF₃ |
| 3124. | NO₂ | H | CF₃ | CH₂CH=CH₂ | Cl |
| 3125. | NO₂ | H | Cl | CH₂CH=CH₂ | CF₃ |
| 3126. | NO₂ | H | CF₃ | CHF₂ | OMe |
| 3127. | NO₂ | H | OMe | CHF₂ | CF₃ |
| 3128. | NO₂ | H | CF₃ | CH₂CF₃ | Cl |
| 3129. | NO₂ | H | Cl | CH₂CF₃ | CF₃ |
| 3130. | NO₂ | H | CF₃ | CH₂OMe | Cl |
| 3131. | NO₂ | H | Cl | CH₂OMe | CF₃ |
| 3132. | NO₂ | H | CF₃ | CH₂CN | Cl |
| 3133. | NO₂ | H | Me | Ph | Me |
| 3134. | NO₂ | H | Me | Ph | Cl |
| 3135. | NO₂ | H | Et | Ph | Cl |
| 3136. | NO₂ | H | Pr | Ph | Cl |
| 3137. | NO₂ | H | iPr | Ph | Cl |
| 3138. | NO₂ | H | CF₃ | Ph | Cl |
| 3139. | NO₂ | H | CF₃ | Ph | Me |
| 3140. | NO₂ | H | CF₃ | Ph | CF₃ |
| 3141. | NO₂ | H | CF₃ | Ph | F |
| 3142. | NO₂ | H | CF₃ | Ph | OMe |
| 3143. | NO₂ | H | CF₃ | Ph | OEt |
| 3144. | NO₂ | H | CF₃ | Ph | OCHF₂ |
| 3145. | NO₂ | H | CF₃ | Ph | CN |
| 3146. | NO₂ | H | CF₃ | Ph(4-Cl) | Cl |
| 3147. | NO₂ | H | Me | Me | OCH₂CF₃ |
| 3148. | NO₂ | H | CF₃ | Me | 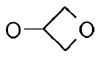 |
| 3149. | NO₂ | H | CF₃ | Me | H |
| 3150. | NO₂ | H | CF₃ | Me | OCH₂CH₂OMe |
| 3151. | NO₂ | H | CF₃ | Me | SMe |
| 3152. | NO₂ | H | CF₃ | Me | OCH₂CH₂CH₂F |
| 3153. | NO₂ | H | CF₃ | Me | OCH(CH₂F)₂ |
| 3154. | NO₂ | H | CF₃ | Me | OCH₂CF₂CHF₂ |
| 3155. | NO₂ | H | CF₃ | Me | OCH₂CF=CH₂ |
| 3156. | NO₂ | H | CF₃ | Me | OCH(Me)CF₃ |
| 3157. | NO₂ | H | CF₃ | Me | OCH(Me)CH₂F |
| 3158. | NO₂ | H | OCH₂CF₃ | Me | CF₃ |
| 3159. | NO₂ | H | OCH₂CF₃ | Me | CHF₂ |
| 3160. | NO₂ | H | CHF₂ | Me | CHF₂ |
| 3161. | NO₂ | H | CF₃ | Me | CHF₂ |
| 3162. | NO₂ | H | Cl | Me | OCHF₂ |
| 3163. | NO₂ | H | Br | Me | OCHF₂ |
| 3164. | NO₂ | H | Br | Me | CF₃ |
| 3165. | CHF₂ | H | CF₃ | Ph | Cl |
| 3166. | CHF₂ | H | CF₃ | tBu | Cl |
| 3167. | CHF₂ | H | CF₃ | CHF₂ | Cl |
| 3168. | CHF₂ | H | Cl | CHF₂ | CF₃ |
| 3169. | CHF₂ | H | CF₃ | Me | OMe |
| 3170. | CHF₂ | H | CF₃ | Me | CN |
| 3171. | CHF₂ | H | Cl | Et | Cl |
| 3172. | CHF₂ | H | CHF₂ | Me | Cl |
| 3173. | CHF₂ | H | Me | Me | Me |
| 3174. | CHF₂ | H | Me | Me | Cl |
| 3175. | CHF₂ | H | Cl | Me | Cl |
| 3176. | CHF₂ | H | CF₃ | Me | Cl |
| 3177. | CHF₂ | H | Cl | Me | CF₃ |
| 3178. | CHF₂ | H | CF₃ | Me | F |
| 3179. | CHF₂ | H | OMe | Me | CF₃ |
| 3180. | CHF₂ | H | CF₃ | Me | OEt |
| 3181. | CHF₂ | H | CF₃ | Me | OCHF₂ |
| 3182. | CHF₂ | H | OCHF₂ | Me | CF₃ |
| 3183. | CHF₂ | H | CF₃ | Me | OCH₂CHF₂ |
| 3184. | CHF₂ | H | CF₃ | Me | OCH₂CF₃ |
| 3185. | CHF₂ | H | CF₃ | Me | OCH₂CN |
| 3186. | CHF₂ | H | CF₃ | Me | SO₂Me |
| 3187. | CHF₂ | H | CF₃ | Me | SEt |
| 3188. | CHF₂ | H | CF₃ | Me | Me |
| 3189. | CHF₂ | H | CF₃ | Me | Et |
| 3190. | CHF₂ | H | CF₃ | Et | Cl |
| 3191. | CHF₂ | H | Cl | Et | CF₃ |
| 3192. | CHF₂ | H | CF₃ | iPr | Cl |
| 3193. | CHF₂ | H | Cl | iPr | CF₃ |
| 3194. | CHF₂ | H | CF₃ | tBu | Cl |
| 3195. | CHF₂ | H | Cl | tBu | CF₃ |
| 3196. | CHF₂ | H | CF₃ | cPen | Cl |
| 3197. | CHF₂ | H | Cl | cPen | CF₃ |
| 3198. | CHF₂ | H | CF₃ | CH₂cPr | Cl |
| 3199. | CHF₂ | H | Cl | CH₂cPr | CF₃ |
| 3200. | CHF₂ | H | CF₃ | CH₂CH=CH₂ | Cl |
| 3201. | CHF₂ | H | Cl | CH₂CH=CH₂ | CF₃ |
| 3202. | CHF₂ | H | CF₃ | CHF₂ | OMe |
| 3203. | CHF₂ | H | OMe | CHF₂ | CF₃ |
| 3204. | CHF₂ | H | CF₃ | CH₂CF₃ | Cl |
| 3205. | CHF₂ | H | Cl | CH₂CF₃ | CF₃ |
| 3206. | CHF₂ | H | CF₃ | CH₂OMe | Cl |
| 3207. | CHF₂ | H | Cl | CH₂OMe | CF₃ |
| 3208. | CHF₂ | H | CF₃ | CH₂CN | Cl |
| 3209. | CHF₂ | H | Me | Ph | Me |
| 3210. | CHF₂ | H | Me | Ph | Cl |
| 3211. | CHF₂ | H | Et | Ph | Cl |
| 3212. | CHF₂ | H | Pr | Ph | Cl |
| 3213. | CHF₂ | H | iPr | Ph | Cl |
| 3214. | CHF₂ | H | CF₃ | Ph | Cl |
| 3215. | CHF₂ | H | CF₃ | Ph | Me |
| 3216. | CHF₂ | H | CF₃ | Ph | CF₃ |
| 3217. | CHF₂ | H | CF₃ | Ph | F |
| 3218. | CHF₂ | H | CF₃ | Ph | OMe |
| 3219. | CHF₂ | H | CF₃ | Ph | OEt |
| 3220. | CHF₂ | H | CF₃ | Ph | OCHF₂ |
| 3221. | CHF₂ | H | CF₃ | Ph | CN |
| 3222. | CHF₂ | H | CF₃ | Ph(4-Cl) | Cl |
| 3223. | CHF₂ | H | Me | Me | OCH₂CF₃ |
| 3224. | CHF₂ | H | CF₃ | Me | 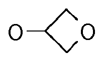 |
| 3225. | CHF₂ | H | CF₃ | Me | H |
| 3226. | CHF₂ | H | CF₃ | Me | OCH₂CH₂OMe |
| 3227. | CHF₂ | H | CF₃ | Me | SMe |
| 3228. | CHF₂ | H | CF₃ | Me | OCH₂CH₂CH₂F |
| 3229. | CHF₂ | H | CF₃ | Me | OCH(CH₂F)₂ |
| 3230. | CHF₂ | H | CF₃ | Me | OCH₂CF₂CHF₂ |
| 3231. | CHF₂ | H | CF₃ | Me | OCH₂CF=CH₂ |
| 3232. | CHF₂ | H | CF₃ | Me | OCH(Me)CF₃ |
| 3233. | CHF₂ | H | CF₃ | Me | OCH(Me)CH₂F |
| 3234. | CHF₂ | H | OCH₂CF₃ | Me | CF₃ |
| 3235. | CHF₂ | H | OCH₂CF₃ | Me | CHF₂ |
| 3236. | CHF₂ | H | CHF₂ | Me | CHF₂ |
| 3237. | CHF₂ | H | CF₃ | Me | CHF₂ |
| 3238. | CHF₂ | H | Cl | Me | OCHF₂ |
| 3239. | CHF₂ | H | Br | Me | OCHF₂ |
| 3240. | CHF₂ | H | Br | Me | CF₃ |
| 3241. | Cl | Cl | CF₃ | Ph | Cl |
| 3242. | Cl | Cl | CF₃ | tBu | Cl |
| 3243. | Cl | Cl | CF₃ | CHF₂ | Cl |
| 3244. | Cl | Cl | Cl | CHF₂ | CF₃ |
| 3245. | Cl | Cl | CF₃ | Me | OMe |
| 3246. | Cl | Cl | CF₃ | Me | CN |
| 3247. | Cl | Cl | Cl | Et | Cl |
| 3248. | Cl | Cl | CHF₂ | Me | Cl |
| 3249. | Cl | Cl | Me | Me | Me |
| 3250. | Cl | Cl | Me | Me | Cl |
| 3251. | Cl | Cl | Cl | Me | Cl |
| 3252. | Cl | Cl | CF₃ | Me | Cl |
| 3253. | Cl | Cl | Cl | Me | CF₃ |
| 3254. | Cl | Cl | CF₃ | Me | F |
| 3255. | Cl | Cl | OMe | Me | CF₃ |
| 3256. | Cl | Cl | CF₃ | Me | OEt |
| 3257. | Cl | Cl | CF₃ | Me | OCHF₂ |
| 3258. | Cl | Cl | OCHF₂ | Me | CF₃ |
| 3259. | Cl | Cl | CF₃ | Me | OCH₂CHF₂ |
| 3260. | Cl | Cl | CF₃ | Me | OCH₂CF₃ |
| 3261. | Cl | Cl | CF₃ | Me | OCH₂CN |
| 3262. | Cl | Cl | CF₃ | Me | SO₂Me |
| 3263. | Cl | Cl | CF₃ | Me | SEt |
| 3264. | Cl | Cl | CF₃ | Me | Me |
| 3265. | Cl | Cl | CF₃ | Me | Et |
| 3266. | Cl | Cl | CF₃ | Et | Cl |
| 3267. | Cl | Cl | Cl | Et | CF₃ |
| 3268. | Cl | Cl | CF₃ | iPr | Cl |
| 3269. | Cl | Cl | Cl | iPr | CF₃ |
| 3270. | Cl | Cl | CF₃ | tBu | Cl |
| 3271. | Cl | Cl | Cl | tBu | CF₃ |
| 3272. | Cl | Cl | CF₃ | cPen | Cl |
| 3273. | Cl | Cl | Cl | cPen | CF₃ |
| 3274. | Cl | Cl | CF₃ | CH₂cPr | Cl |
| 3275. | Cl | Cl | Cl | CH₂cPr | CF₃ |
| 3276. | Cl | Cl | CF₃ | CH₂CH=CH₂ | Cl |
| 3277. | Cl | Cl | Cl | CH₂CH=CH₂ | CF₃ |
| 3278. | Cl | Cl | CF₃ | CHF₂ | OMe |
| 3279. | Cl | Cl | OMe | CHF₂ | CF₃ |
| 3280. | Cl | Cl | CF₃ | CH₂CF₃ | Cl |
| 3281. | Cl | Cl | Cl | CH₂CF₃ | CF₃ |
| 3282. | Cl | Cl | CF₃ | CH₂OMe | Cl |
| 3283. | Cl | Cl | Cl | CH₂OMe | CF₃ |
| 3284. | Cl | Cl | CF₃ | CH₂CN | Cl |
| 3285. | Cl | Cl | Me | Ph | Me |
| 3286. | Cl | Cl | Me | Ph | Cl |
| 3287. | Cl | Cl | Et | Ph | Cl |
| 3288. | Cl | Cl | Pr | Ph | Cl |
| 3289. | Cl | Cl | iPr | Ph | Cl |
| 3290. | Cl | Cl | CF₃ | Ph | Cl |
| 3291. | Cl | Cl | CF₃ | Ph | Me |
| 3292. | Cl | Cl | CF₃ | Ph | CF₃ |
| 3293. | Cl | Cl | CF₃ | Ph | F |
| 3294. | Cl | Cl | CF₃ | Ph | OMe |
| 3295. | Cl | Cl | CF₃ | Ph | OEt |
| 3296. | Cl | Cl | CF₃ | Ph | OCHF₂ |
| 3297. | Cl | Cl | CF₃ | Ph | CN |
| 3298. | Cl | Cl | CF₃ | Ph(4-Cl) | Cl |
| 3299. | Cl | Cl | Me | Me | OCH₂CF₃ |
| 3300. | Cl | Cl | CF₃ | Me | 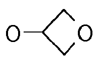 |
| 3301. | Cl | Cl | CF₃ | Me | H |
| 3302. | Cl | Cl | CF₃ | Me | OCH₂CH₂OMe |
| 3303. | Cl | Cl | CF₃ | Me | SMe |
| 3304. | Cl | Cl | CF₃ | Me | OCH₂CH₂CH₂F |
| 3305. | Cl | Cl | CF₃ | Me | OCH(CH₂F)₂ |
| 3306. | Cl | Cl | CF₃ | Me | OCH₂CF₂CHF₂ |
| 3307. | Cl | Cl | CF₃ | Me | OCH₂CF=CH₂ |
| 3308. | Cl | Cl | CF₃ | Me | OCH(Me)CF₃ |
| 3309. | Cl | Cl | CF₃ | Me | OCH(Me)CH₂F |
| 3310. | Cl | Cl | OCH₂CF₃ | Me | CF₃ |
| 3311. | Cl | Cl | OCH₂CF₃ | Me | CHF₂ |
| 3312. | Cl | Cl | CHF₂ | Me | CHF₂ |
| 3313. | Cl | Cl | CF₃ | Me | CHF₂ |
| 3314. | Cl | Cl | Cl | Me | OCHF₂ |
| 3315. | Cl | Cl | Br | Me | OCHF₂ |
| 3316. | Cl | Cl | Br | Me | CF₃ |
| 3317. | Me | Cl | CF₃ | Ph | Cl |
| 3318. | Me | Cl | CF₃ | tBu | Cl |
| 3319. | Me | Cl | CF₃ | CHF₂ | Cl |
| 3320. | Me | Cl | Cl | CHF₂ | CF₃ |
| 3321. | Me | Cl | CF₃ | Me | OMe |
| 3322. | Me | Cl | CF₃ | Me | CN |
| 3323. | Me | Cl | Cl | Et | Cl |
| 3324. | Me | Cl | CHF₂ | Me | Cl |
| 3325. | Me | Cl | Me | Me | Me |
| 3326. | Me | Cl | Me | Me | Cl |
| 3327. | Me | Cl | Cl | Me | Cl |
| 3328. | Me | Cl | CF₃ | Me | Cl |
| 3329. | Me | Cl | Cl | Me | CF₃ |
| 3330. | Me | Cl | CF₃ | Me | F |
| 3331. | Me | Cl | OMe | Me | CF₃ |
| 3332. | Me | Cl | CF₃ | Me | OEt |
| 3333. | Me | Cl | CF₃ | Me | OCHF₂ |
| 3334. | Me | Cl | OCHF₂ | Me | CF₃ |
| 3335. | Me | Cl | CF₃ | Me | OCH₂CHF₂ |
| 3336. | Me | Cl | CF₃ | Me | OCH₂CF₃ |
| 3337. | Me | Cl | CF₃ | Me | OCH₂CN |
| 3338. | Me | Cl | CF₃ | Me | SO₂Me |
| 3339. | Me | Cl | CF₃ | Me | SEt |
| 3340. | Me | Cl | CF₃ | Me | Me |
| 3341. | Me | Cl | CF₃ | Me | Et |
| 3342. | Me | Cl | CF₃ | Et | Cl |
| 3343. | Me | Cl | Cl | Et | CF₃ |
| 3344. | Me | Cl | CF₃ | iPr | Cl |
| 3345. | Me | Cl | Cl | iPr | CF₃ |
| 3346. | Me | Cl | CF₃ | tBu | Cl |
| 3347. | Me | Cl | Cl | tBu | CF₃ |
| 3348. | Me | Cl | CF₃ | cPen | Cl |
| 3349. | Me | Cl | Cl | cPen | CF₃ |
| 3350. | Me | Cl | CF₃ | CH₂cPr | Cl |
| 3351. | Me | Cl | Cl | CH₂cPr | CF₃ |
| 3352. | Me | Cl | CF₃ | CH₂CH=CH₂ | Cl |
| 3353. | Me | Cl | Cl | CH₂CH=CH₂ | CF₃ |
| 3354. | Me | Cl | CF₃ | CHF₂ | OMe |
| 3355. | Me | Cl | OMe | CHF₂ | CF₃ |
| 3356. | Me | Cl | CF₃ | CH₂CF₃ | Cl |
| 3357. | Me | Cl | Cl | CH₂CF₃ | CF₃ |
| 3358. | Me | Cl | CF₃ | CH₂OMe | Cl |
| 3359. | Me | Cl | Cl | CH₂OMe | CF₃ |
| 3360. | Me | Cl | CF₃ | CH₂CN | Cl |
| 3361. | Me | Cl | Me | Ph | Me |
| 3362. | Me | Cl | Me | Ph | Cl |
| 3363. | Me | Cl | Et | Ph | Cl |
| 3364. | Me | Cl | Pr | Ph | Cl |
| 3365. | Me | Cl | iPr | Ph | Cl |
| 3366. | Me | Cl | CF₃ | Ph | Cl |
| 3367. | Me | Cl | CF₃ | Ph | Me |
| 3368. | Me | Cl | CF₃ | Ph | CF₃ |
| 3369. | Me | Cl | CF₃ | Ph | F |
| 3370. | Me | Cl | CF₃ | Ph | OMe |
| 3371. | Me | Cl | CF₃ | Ph | OEt |
| 3372. | Me | Cl | CF₃ | Ph | OCHF₂ |
| 3373. | Me | Cl | CF₃ | Ph | CN |
| 3374. | Me | Cl | CF₃ | Ph(4-Cl) | Cl |
| 3375. | Me | Cl | Me | Me | OCH₂CF₃ |
| 3376. | Me | Cl | CF₃ | Me | 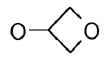 |
| 3377. | Me | Cl | CF₃ | Me | H |
| 3378. | Me | Cl | CF₃ | Me | OCH₂CH₂OMe |
| 3379. | Me | Cl | CF₃ | Me | SMe |
| 3380. | Me | Cl | CF₃ | Me | OCH₂CH₂CH₂F |
| 3381. | Me | Cl | CF₃ | Me | OCH(CH₂F)₂ |
| 3382. | Me | Cl | CF₃ | Me | OCH₂CF₂CHF₂ |
| 3383. | Me | Cl | CF₃ | Me | OCH₂CF=CH₂ |
| 3384. | Me | Cl | CF₃ | Me | OCH(Me)CF₃ |
| 3385. | Me | Cl | CF₃ | Me | OCH(Me)CH₂F |
| 3386. | Me | Cl | OCH₂CF₃ | Me | CF₃ |
| 3387. | Me | Cl | OCH₂CF₃ | Me | CHF₂ |
| 3388. | Me | Cl | CHF₂ | Me | CHF₂ |
| 3389. | Me | Cl | CF₃ | Me | CHF₂ |
| 3390. | Me | Cl | Cl | Me | OCHF₂ |
| 3391. | Me | Cl | Br | Me | OCHF₂ |
| 3392. | Me | Cl | Br | Me | CF₃ |
| 3393. | Cl | Me | CF₃ | Ph | Cl |
| 3394. | Cl | Me | CF₃ | tBu | Cl |
| 3395. | Cl | Me | CF₃ | CHF₂ | Cl |
| 3396. | Cl | Me | Cl | CHF₂ | CF₃ |
| 3397. | Cl | Me | CF₃ | Me | OMe |
| 3398. | Cl | Me | CF₃ | Me | CN |
| 3399. | Cl | Me | Cl | Et | Cl |
| 3400. | Cl | Me | CHF₂ | Me | Cl |
| 3401. | Cl | Me | Me | Me | Me |
| 3402. | Cl | Me | Me | Me | Cl |
| 3403. | Cl | Me | Cl | Me | Cl |
| 3404. | Cl | Me | CF₃ | Me | Cl |
| 3405. | Cl | Me | Cl | Me | CF₃ |
| 3406. | Cl | Me | CF₃ | Me | F |
| 3407. | Cl | Me | OMe | Me | CF₃ |
| 3408. | Cl | Me | CF₃ | Me | OEt |
| 3409. | Cl | Me | CF₃ | Me | OCHF₂ |
| 3410. | Cl | Me | OCHF₂ | Me | CF₃ |
| 3411. | Cl | Me | CF₃ | Me | OCH₂CHF₂ |
| 3412. | Cl | Me | CF₃ | Me | OCH₂CF₃ |
| 3413. | Cl | Me | CF₃ | Me | OCH₂CN |
| 3414. | Cl | Me | CF₃ | Me | SO₂Me |
| 3415. | Cl | Me | CF₃ | Me | SEt |
| 3416. | Cl | Me | CF₃ | Me | Me |
| 3417. | Cl | Me | CF₃ | Me | Et |
| 3418. | Cl | Me | CF₃ | Et | Cl |
| 3419. | Cl | Me | Cl | Et | CF₃ |
| 3420. | Cl | Me | CF₃ | iPr | Cl |
| 3421. | Cl | Me | Cl | iPr | CF₃ |
| 3422. | Cl | Me | CF₃ | tBu | Cl |
| 3423. | Cl | Me | Cl | tBu | CF₃ |
| 3424. | Cl | Me | CF₃ | cPen | Cl |
| 3425. | Cl | Me | Cl | cPen | CF₃ |
| 3426. | Cl | Me | CF₃ | CH₂cPr | Cl |
| 3427. | Cl | Me | Cl | CH₂cPr | CF₃ |
| 3428. | Cl | Me | CF₃ | CH₂CH=CH₂ | Cl |
| 3429. | Cl | Me | Cl | CH₂CH=CH₂ | CF₃ |
| 3430. | Cl | Me | CF₃ | CHF₂ | OMe |
| 3431. | Cl | Me | OMe | CHF₂ | CF₃ |
| 3432. | Cl | Me | CF₃ | CH₂CF₃ | Cl |
| 3433. | Cl | Me | Cl | CH₂CF₃ | CF₃ |
| 3434. | Cl | Me | CF₃ | CH₂OMe | Cl |
| 3435. | Cl | Me | Cl | CH₂OMe | CF₃ |
| 3436. | Cl | Me | CF₃ | CH₂CN | Cl |
| 3437. | Cl | Me | Me | Ph | Me |
| 3438. | Cl | Me | Me | Ph | Cl |
| 3439. | Cl | Me | Et | Ph | Cl |
| 3440. | Cl | Me | Pr | Ph | Cl |
| 3441. | Cl | Me | iPr | Ph | Cl |
| 3442. | Cl | Me | CF₃ | Ph | Cl |
| 3443. | Cl | Me | CF₃ | Ph | Me |
| 3444. | Cl | Me | CF₃ | Ph | CF₃ |
| 3445. | Cl | Me | CF₃ | Ph | F |
| 3446. | Cl | Me | CF₃ | Ph | OMe |
| 3447. | Cl | Me | CF₃ | Ph | OEt |
| 3448. | Cl | Me | CF₃ | Ph | OCHF₂ |
| 3449. | Cl | Me | CF₃ | Ph | CN |
| 3450. | Cl | Me | CF₃ | Ph(4-Cl) | Cl |
| 3451. | Cl | Me | Me | Me | OCH₂CF₃ |
| 3452. | Cl | Me | CF₃ | Me | 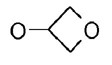 |
| 3453. | Cl | Me | CF₃ | Me | H |
| 3454. | Cl | Me | CF₃ | Me | OCH₂CH₂OMe |
| 3455. | Cl | Me | CF₃ | Me | SMe |
| 3456. | Cl | Me | CF₃ | Me | OCH₂CH₂CH₂F |
| 3457. | Cl | Me | CF₃ | Me | OCH(CH₂F)₂ |
| 3458. | Cl | Me | CF₃ | Me | OCH₂CF₂CHF₂ |
| 3459. | Cl | Me | CF₃ | Me | OCH₂CF=CH₂ |
| 3460. | Cl | Me | CF₃ | Me | OCH(Me)CF₃ |
| 3461. | Cl | Me | CF₃ | Me | OCH(Me)CH₂F |
| 3462. | Cl | Me | OCH₂CF₃ | Me | CF₃ |
| 3463. | Cl | Me | OCH₂CF₃ | Me | CHF₂ |
| 3464. | Cl | Me | CHF₂ | Me | CHF₂ |
| 3465. | Cl | Me | CF₃ | Me | CHF₂ |
| 3466. | Cl | Me | Cl | Me | OCHF₂ |
| 3467. | Cl | Me | Br | Me | OCHF₂ |
| 3468. | Cl | Me | Br | Me | CF₃ |

**Tabelle 4: Verbindungen der Formel Ib-R**

| | | | | | |
|---|---|---|---|---|---|
| 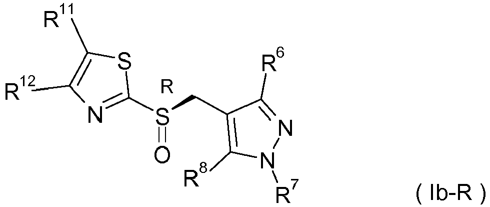 | | | | | |

| Bsp. Nr. | R¹¹ | R¹² | R⁶ | R⁷ | R⁸ |
|---|---|---|---|---|---|
| 3469. | H | H | CF₃ | Ph | Cl |
| 3470. | H | H | CF₃ | tBu | Cl |
| 3471. | H | H | CF₃ | CHF₂ | Cl |
| 3472. | H | H | Cl | CHF₂ | CF₃ |
| 3473. | H | H | CF₃ | Me | OMe |
| 3474. | H | H | CF₃ | Me | CN |
| 3475. | H | H | Cl | Et | Cl |
| 3476. | H | H | CHF₂ | Me | Cl |
| 3477. | H | H | Me | Me | Me |
| 3478. | H | H | Me | Me | Cl |
| 3479. | H | H | Cl | Me | Cl |
| 3480. | H | H | CF₃ | Me | Cl |
| 3481. | H | H | Cl | Me | CF₃ |
| 3482. | H | H | CF₃ | Me | F |
| 3483. | H | H | OMe | Me | CF₃ |
| 3484. | H | H | CF₃ | Me | OEt |
| 3485. | H | H | CF₃ | Me | OCHF₂ |
| 3486. | H | H | OCHF₂ | Me | CF₃ |
| 3487. | H | H | CF₃ | Me | OCH₂CHF₂ |
| 3488. | H | H | CF₃ | Me | OCH₂CF₃ |
| 3489. | H | H | CF₃ | Me | OCH₂CN |
| 3490. | H | H | CF₃ | Me | SO₂Me |
| 3491. | H | H | CF₃ | Me | SEt |
| 3492. | H | H | CF₃ | Me | Me |
| 3493. | H | H | CF₃ | Me | Et |
| 3494. | H | H | CF₃ | Et | Cl |
| 3495. | H | H | Cl | Et | CF₃ |
| 3496. | H | H | CF₃ | iPr | Cl |
| 3497. | H | H | Cl | iPr | CF₃ |
| 3498. | H | H | CF₃ | tBu | Cl |
| 3499. | H | H | Cl | tBu | CF₃ |
| 3500. | H | H | CF₃ | cPen | Cl |
| 3501. | H | H | Cl | cPen | CF₃ |
| 3502. | H | H | CF₃ | CH₂cPr | Cl |
| 3503. | H | H | Cl | CH₂cPr | CF₃ |
| 3504. | H | H | CF₃ | CH₂CH=CH₂ | Cl |
| 3505. | H | H | Cl | CH₂CH=CH₂ | CF₃ |
| 3506. | H | H | CF₃ | CHF₂ | OMe |
| 3507. | H | H | OMe | CHF₂ | CF₃ |
| 3508. | H | H | CF₃ | CH₂CF₃ | Cl |
| 3509. | H | H | Cl | CH₂CF₃ | CF₃ |
| 3510. | H | H | CF₃ | CH₂OMe | Cl |
| 3511. | H | H | Cl | CH₂OMe | CF₃ |
| 3512. | H | H | CF₃ | CH₂CN | Cl |
| 3513. | H | H | Me | Ph | Me |
| 3514. | H | H | Me | Ph | Cl |
| 3515. | H | H | Et | Ph | Cl |
| 3516. | H | H | Pr | Ph | Cl |
| 3517. | H | H | iPr | Ph | Cl |
| 3518. | H | H | CF₃ | Ph | Cl |
| 3519. | H | H | CF₃ | Ph | Me |
| 3520. | H | H | CF₃ | Ph | CF₃ |
| 3521. | H | H | CF₃ | Ph | F |
| 3522. | H | H | CF₃ | Ph | OMe |
| 3523. | H | H | CF₃ | Ph | OEt |
| 3524. | H | H | CF₃ | Ph | OCHF₂ |
| 3525. | H | H | CF₃ | Ph | CN |
| 3526. | H | H | CF₃ | Ph(4-Cl) | Cl |
| 3527. | H | H | Me | Me | OCH₂CF₃ |
| 3528. | H | H | CF₃ | Me | 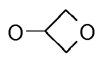 |
| 3529. | H | H | CF₃ | Me | H |
| 3530. | H | H | CF₃ | Me | OCH₂CH₂OMe |
| 3531. | H | H | CF₃ | Me | SMe |
| 3532. | H | H | CF₃ | Me | OCH₂CH₂CH₂F |
| 3533. | H | H | CF₃ | Me | OCH(CH₂F)₂ |
| 3534. | H | H | CF₃ | Me | OCH₂CF₂CHF₂ |
| 3535. | H | H | CF₃ | Me | OCH₂CF=CH₂ |
| 3536. | H | H | CF₃ | Me | OCH(Me)CF₃ |
| 3537. | H | H | CF₃ | Me | OCH(Me)CH₂F |
| 3538. | H | H | OCH₂CF₃ | Me | CF₃ |
| 3539. | H | H | OCH₂CF₃ | Me | CHF₂ |
| 3540. | H | H | CHF₂ | Me | CHF₂ |
| 3541. | H | H | CF₃ | Me | CHF₂ |
| 3542. | H | H | Cl | Me | OCHF₂ |
| 3543. | H | H | Br | Me | OCHF₂ |
| 3544. | H | H | Br | Me | CF₃ |
| 3545. | F | H | CF₃ | Ph | Cl |
| 3546. | F | H | CF₃ | tBu | Cl |
| 3547. | F | H | CF₃ | CHF₂ | Cl |
| 3548. | F | H | Cl | CHF₂ | CF₃ |
| 3549. | F | H | CF₃ | Me | OMe |
| 3550. | F | H | CF₃ | Me | CN |
| 3551. | F | H | Cl | Et | Cl |
| 3552. | F | H | CHF₂ | Me | Cl |
| 3553. | F | H | Me | Me | Me |
| 3554. | F | H | Me | Me | Cl |
| 3555. | F | H | Cl | Me | Cl |
| 3556. | F | H | CF₃ | Me | Cl |
| 3557. | F | H | Cl | Me | CF₃ |
| 3558. | F | H | CF₃ | Me | F |
| 3559. | F | H | OMe | Me | CF₃ |
| 3560. | F | H | CF₃ | Me | OEt |
| 3561. | F | H | CF₃ | Me | OCHF₂ |
| 3562. | F | H | OCHF₂ | Me | CF₃ |
| 3563. | F | H | CF₃ | Me | OCH₂CHF₂ |
| 3564. | F | H | CF₃ | Me | OCH₂CF₃ |
| 3565. | F | H | CF₃ | Me | OCH₂CN |
| 3566. | F | H | CF₃ | Me | SO₂Me |
| 3567. | F | H | CF₃ | Me | SEt |
| 3568. | F | H | CF₃ | Me | Me |
| 3569. | F | H | CF₃ | Me | Et |
| 3570. | F | H | CF₃ | Et | Cl |
| 3571. | F | H | Cl | Et | CF₃ |
| 3572. | F | H | CF₃ | iPr | Cl |
| 3573. | F | H | Cl | iPr | CF₃ |
| 3574. | F | H | CF₃ | tBu | Cl |
| 3575. | F | H | Cl | tBu | CF₃ |
| 3576. | F | H | CF₃ | cPen | Cl |
| 3577. | F | H | Cl | cPen | CF₃ |
| 3578. | F | H | CF₃ | CH₂cPr | Cl |
| 3579. | F | H | Cl | CH₂cPr | CF₃ |
| 3580. | F | H | CF₃ | CH₂CH=CH₂ | Cl |
| 3581. | F | H | Cl | CH₂CH=CH₂ | CF₃ |
| 3582. | F | H | CF₃ | CHF₂ | OMe |
| 3583. | F | H | OMe | CHF₂ | CF₃ |
| 3584. | F | H | CF₃ | CH₂CF₃ | Cl |
| 3585. | F | H | Cl | CH₂CF₃ | CF₃ |
| 3586. | F | H | CF₃ | CH₂OMe | Cl |
| 3587. | F | H | Cl | CH₂OMe | CF₃ |
| 3588. | F | H | CF₃ | CH₂CN | Cl |
| 3589. | F | H | Me | Ph | Me |
| 3590. | F | H | Me | Ph | Cl |
| 3591. | F | H | Et | Ph | Cl |
| 3592. | F | H | Pr | Ph | Cl |
| 3593. | F | H | iPr | Ph | Cl |
| 3594. | F | H | CF₃ | Ph | Cl |
| 3595. | F | H | CF₃ | Ph | Me |
| 3596. | F | H | CF₃ | Ph | CF₃ |
| 3597. | F | H | CF₃ | Ph | F |
| 3598. | F | H | CF₃ | Ph | OMe |
| 3599. | F | H | CF₃ | Ph | OEt |
| 3600. | F | H | CF₃ | Ph | OCHF₂ |
| 3601. | F | H | CF₃ | Ph | CN |
| 3602. | F | H | CF₃ | Ph(4-Cl) | Cl |
| 3603. | F | H | Me | Me | OCH₂CF₃ |
| 3604. | F | H | CF₃ | Me | 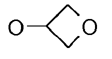 |
| 3605. | F | H | CF₃ | Me | H |
| 3606. | F | H | CF₃ | Me | OCH₂CH₂OMe |
| 3607. | F | H | CF₃ | Me | SMe |
| 3608. | F | H | CF₃ | Me | OCH₂CH₂CH₂F |
| 3609. | F | H | CF₃ | Me | OCH(CH₂F)₂ |
| 3610. | F | H | CF₃ | Me | OCH₂CF₂CHF₂ |
| 3611. | F | H | CF₃ | Me | OCH₂CF=CH₂ |
| 3612. | F | H | CF₃ | Me | OCH(Me)CF₃ |
| 3613. | F | H | CF₃ | Me | OCH(Me)CH₂F |
| 3614. | F | H | OCH₂CF₃ | Me | CF₃ |
| 3615. | F | H | OCH₂CF₃ | Me | CHF₂ |
| 3616. | F | H | CHF₂ | Me | CHF₂ |
| 3617. | F | H | CF₃ | Me | CHF₂ |
| 3618. | F | H | Cl | Me | OCHF₂ |
| 3619. | F | H | Br | Me | OCHF₂ |
| 3620. | F | H | Br | Me | CF₃ |
| 3621. | F | H | CF₃ | Me | CF₃ |
| 3622. | F | H | CHF₂ | Me | OCHF₂ |
| 3623. | F | H | CHF₂ | Me | CF₃ |
| 3624. | F | H | CF₂CF₃ | Me | CF₃ |
| 3625. | F | H | CF₃ | Me | CF₂CF₃ |
| 3626. | F | H | CHF₂ | Me | OCH₂CF₃ |
| 3627. | Cl | H | CF₃ | Ph | Cl |
| 3628. | Cl | H | CF₃ | tBu | Cl |
| 3629. | Cl | H | CF₃ | CHF₂ | Cl |
| 3630. | Cl | H | Cl | CHF₂ | CF₃ |
| 3631. | Cl | H | CF₃ | Me | OMe |
| 3632. | Cl | H | CF₃ | Me | CN |
| 3633. | Cl | H | Cl | Et | Cl |
| 3634. | Cl | H | CHF₂ | Me | Cl |
| 3635. | Cl | H | Me | Me | Me |
| 3636. | Cl | H | Me | Me | Cl |
| 3637. | Cl | H | Cl | Me | Cl |
| 3638. | Cl | H | CF₃ | Me | Cl |
| 3639. | Cl | H | Cl | Me | CF₃ |
| 3640. | Cl | H | CF₃ | Me | F |
| 3641. | Cl | H | OMe | Me | CF₃ |
| 3642. | Cl | H | CF₃ | Me | OEt |
| 3643. | Cl | H | CF₃ | Me | OCHF₂ |
| 3644. | Cl | H | OCHF₂ | Me | CF₃ |
| 3645. | Cl | H | CF₃ | Me | OCH₂CHF₂ |
| 3646. | Cl | H | CF₃ | Me | OCH₂CF₃ |
| 3647. | Cl | H | CF₃ | Me | OCH₂CN |
| 3648. | Cl | H | CF₃ | Me | SO₂Me |
| 3649. | Cl | H | CF₃ | Me | SEt |
| 3650. | Cl | H | CF₃ | Me | Me |
| 3651. | Cl | H | CF₃ | Me | Et |
| 3652. | Cl | H | CF₃ | Et | Cl |
| 3653. | Cl | H | Cl | Et | CF₃ |
| 3654. | Cl | H | CF₃ | iPr | Cl |
| 3655. | Cl | H | Cl | iPr | CF₃ |
| 3656. | Cl | H | CF₃ | tBu | Cl |
| 3657. | Cl | H | Cl | tBu | CF₃ |
| 3658. | Cl | H | CF₃ | cPen | Cl |
| 3659. | Cl | H | Cl | cPen | CF₃ |
| 3660. | Cl | H | CF₃ | CH₂cPr | Cl |
| 3661. | Cl | H | Cl | CH₂cPr | CF₃ |
| 3662. | Cl | H | CF₃ | CH₂CH=CH₂ | Cl |
| 3663. | Cl | H | Cl | CH₂CH=CH₂ | CF₃ |
| 3664. | Cl | H | CF₃ | CHF₂ | OMe |
| 3665. | Cl | H | OMe | CHF₂ | CF₃ |
| 3666. | Cl | H | CF₃ | CH₂CF₃ | Cl |
| 3667. | Cl | H | Cl | CH₂CF₃ | CF₃ |
| 3668. | Cl | H | CF₃ | CH₂OMe | Cl |
| 3669. | Cl | H | Cl | CH₂OMe | CF₃ |
| 3670. | Cl | H | CF₃ | CH₂CN | Cl |
| 3671. | Cl | H | Me | Ph | Me |
| 3672. | Cl | H | Me | Ph | Cl |
| 3673. | Cl | H | Et | Ph | Cl |
| 3674. | Cl | H | Pr | Ph | Cl |
| 3675. | Cl | H | iPr | Ph | Cl |
| 3676. | Cl | H | CF₃ | Ph | Cl |
| 3677. | Cl | H | CF₃ | Ph | Me |
| 3678. | Cl | H | CF₃ | Ph | CF₃ |
| 3679. | Cl | H | CF₃ | Ph | F |
| 3680. | Cl | H | CF₃ | Ph | OMe |
| 3681. | Cl | H | CF₃ | Ph | OEt |
| 3682. | Cl | H | CF₃ | Ph | OCHF₂ |
| 3683. | Cl | H | CF₃ | Ph | CN |
| 3684. | Cl | H | CF₃ | Ph(4-Cl) | Cl |
| 3685. | Cl | H | Me | Me | OCH₂CF₃ |
| 3686. | Cl | H | CF₃ | Me | 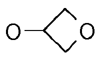 |
| 3687. | Cl | H | CF₃ | Me | H |
| 3688. | Cl | H | CF₃ | Me | OCH₂CH₂OMe |
| 3689. | Cl | H | CF₃ | Me | SMe |
| 3690. | Cl | H | CF₃ | Me | OCH₂CH₂CH₂F |
| 3691. | Cl | H | CF₃ | Me | OCH(CH₂F)₂ |
| 3692. | Cl | H | CF₃ | Me | OCH₂CF₂CHF₂ |
| 3693. | Cl | H | CF₃ | Me | OCH₂CF=CH₂ |
| 3694. | Cl | H | CF₃ | Me | OCH(Me)CF₃ |
| 3695. | Cl | H | CF₃ | Me | OCH(Me)CH₂F |
| 3696. | Cl | H | OCH₂CF₃ | Me | CF₃ |
| 3697. | Cl | H | OCH₂CF₃ | Me | CHF₂ |
| 3698. | Cl | H | CHF₂ | Me | CHF₂ |
| 3699. | Cl | H | CF₃ | Me | CHF₂ |
| 3700. | Cl | H | Cl | Me | OCHF₂ |
| 3701. | Cl | H | Br | Me | OCHF₂ |
| 3702. | Cl | H | Br | Me | CF₃ |
| 3703. | Cl | H | CF₃ | Me | CF₃ |
| 3704. | Cl | H | CHF₂ | Me | OCHF₂ |
| 3705. | Cl | H | CHF₂ | Me | CF₃ |
| 3706. | Cl | H | CF₂CF₃ | Me | CF₃ |
| 3707. | Cl | H | CF₃ | Me | CF₂CF₃ |
| 3708. | Cl | H | CHF₂ | Me | OCH₂CF₃ |
| 3709. | Br | H | CF₃ | Ph | Cl |
| 3710. | Br | H | CF₃ | tBu | Cl |
| 3711. | Br | H | CF₃ | CHF₂ | Cl |
| 3712. | Br | H | Cl | CHF₂ | CF₃ |
| 3713. | Br | H | CF₃ | Me | OMe |
| 3714. | Br | H | CF₃ | Me | CN |
| 3715. | Br | H | Cl | Et | Cl |
| 3716. | Br | H | CHF₂ | Me | Cl |
| 3717. | Br | H | Me | Me | Me |
| 3718. | Br | H | Me | Me | Cl |
| 3719. | Br | H | Cl | Me | Cl |
| 3720. | Br | H | CF₃ | Me | Cl |
| 3721. | Br | H | Cl | Me | CF₃ |
| 3722. | Br | H | CF₃ | Me | F |
| 3723. | Br | H | OMe | Me | CF₃ |
| 3724. | Br | H | CF₃ | Me | OEt |
| 3725. | Br | H | CF₃ | Me | OCHF₂ |
| 3726. | Br | H | OCHF₂ | Me | CF₃ |
| 3727. | Br | H | CF₃ | Me | OCH₂CHF₂ |
| 3728. | Br | H | CF₃ | Me | OCH₂CF₃ |
| 3729. | Br | H | CF₃ | Me | OCH₂CN |
| 3730. | Br | H | CF₃ | Me | SO₂Me |
| 3731. | Br | H | CF₃ | Me | SEt |
| 3732. | Br | H | CF₃ | Me | Me |
| 3733. | Br | H | CF₃ | Me | Et |
| 3734. | Br | H | CF₃ | Et | Cl |
| 3735. | Br | H | Cl | Et | CF₃ |
| 3736. | Br | H | CF₃ | iPr | Cl |
| 3737. | Br | H | Cl | iPr | CF₃ |
| 3738. | Br | H | CF₃ | tBu | Cl |
| 3739. | Br | H | Cl | tBu | CF₃ |
| 3740. | Br | H | CF₃ | cPen | Cl |
| 3741. | Br | H | Cl | cPen | CF₃ |
| 3742. | Br | H | CF₃ | CH₂cPr | Cl |
| 3743. | Br | H | Cl | CH₂cPr | CF₃ |
| 3744. | Br | H | CF₃ | CH₂CH=CH₂ | Cl |
| 3745. | Br | H | Cl | CH₂CH=CH₂ | CF₃ |
| 3746. | Br | H | CF₃ | CHF₂ | OMe |
| 3747. | Br | H | OMe | CHF₂ | CF₃ |
| 3748. | Br | H | CF₃ | CH₂CF₃ | Cl |
| 3749. | Br | H | Cl | CH₂CF₃ | CF₃ |
| 3750. | Br | H | CF₃ | CH₂OMe | Cl |
| 3751. | Br | H | Cl | CH₂OMe | CF₃ |
| 3752. | Br | H | CF₃ | CH₂CN | Cl |
| 3753. | Br | H | Me | Ph | Me |
| 3754. | Br | H | Me | Ph | Cl |
| 3755. | Br | H | Et | Ph | Cl |
| 3756. | Br | H | Pr | Ph | Cl |
| 3757. | Br | H | iPr | Ph | Cl |
| 3758. | Br | H | CF₃ | Ph | Cl |
| 3759. | Br | H | CF₃ | Ph | Me |
| 3760. | Br | H | CF₃ | Ph | CF₃ |
| 3761. | Br | H | CF₃ | Ph | F |
| 3762. | Br | H | CF₃ | Ph | OMe |
| 3763. | Br | H | CF₃ | Ph | OEt |
| 3764. | Br | H | CF₃ | Ph | OCHF₂ |
| 3765. | Br | H | CF₃ | Ph | CN |
| 3766. | Br | H | CF₃ | Ph(4-Cl) | Cl |
| 3767. | Br | H | Me | Me | OCH₂CF₃ |
| 3768. | Br | H | CF₃ | Me | 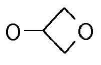 |
| 3769. | Br | H | CF₃ | Me | H |
| 3770. | Br | H | CF₃ | Me | OCH₂CH₂OMe |
| 3771. | Br | H | CF₃ | Me | SMe |
| 3772. | Br | H | CF₃ | Me | OCH₂CH₂CH₂F |
| 3773. | Br | H | CF₃ | Me | OCH(CH₂F)₂ |
| 3774. | Br | H | CF₃ | Me | OCH₂CF₂CHF₂ |
| 3775. | Br | H | CF₃ | Me | OCH₂CF=CH₂ |
| 3776. | Br | H | CF₃ | Me | OCH(Me)CF₃ |
| 3777. | Br | H | CF₃ | Me | OCH(Me)CH₂F |
| 3778. | Br | H | OCH₂CF₃ | Me | CF₃ |
| 3779. | Br | H | OCH₂CF₃ | Me | CHF₂ |
| 3780. | Br | H | CHF₂ | Me | CHF₂ |
| 3781. | Br | H | CF₃ | Me | CHF₂ |
| 3782. | Br | H | Cl | Me | OCHF₂ |
| 3783. | Br | H | Br | Me | OCHF₂ |
| 3784. | Br | H | Br | Me | CF₃ |
| 3785. | Br | H | CF₃ | Me | CF₃ |
| 3786. | Br | H | CHF₂ | Me | OCHF₂ |
| 3787. | Br | H | CHF₂ | Me | CF₃ |
| 3788. | Br | H | CF₂CF₃ | Me | CF₃ |
| 3789. | Br | H | CF₃ | Me | CF₂CF₃ |
| 3790. | Br | H | CHF₂ | Me | OCH₂CF₃ |
| 3791. | I | H | CF₃ | Ph | Cl |
| 3792. | I | H | CF₃ | tBu | Cl |
| 3793. | I | H | CF₃ | CHF₂ | Cl |
| 3794. | I | H | Cl | CHF₂ | CF₃ |
| 3795. | I | H | CF₃ | Me | OMe |
| 3796. | I | H | CF₃ | Me | CN |
| 3797. | I | H | Cl | Et | Cl |
| 3798. | I | H | CHF₂ | Me | Cl |
| 3799. | I | H | Me | Me | Me |
| 3800. | I | H | Me | Me | Cl |
| 3801. | I | H | Cl | Me | Cl |
| 3802. | I | H | CF₃ | Me | Cl |
| 3803. | I | H | Cl | Me | CF₃ |
| 3804. | I | H | CF₃ | Me | F |
| 3805. | I | H | OMe | Me | CF₃ |
| 3806. | I | H | CF₃ | Me | OEt |
| 3807. | I | H | CF₃ | Me | OCHF₂ |
| 3808. | I | H | OCHF₂ | Me | CF₃ |
| 3809. | I | H | CF₃ | Me | OCH₂CHF₂ |
| 3810. | I | H | CF₃ | Me | OCH₂CF₃ |
| 3811. | I | H | CF₃ | Me | OCH₂CN |
| 3812. | I | H | CF₃ | Me | SO₂Me |
| 3813. | I | H | CF₃ | Me | SEt |
| 3814. | I | H | CF₃ | Me | Me |
| 3815. | I | H | CF₃ | Me | Et |
| 3816. | I | H | CF₃ | Et | Cl |
| 3817. | I | H | Cl | Et | CF₃ |
| 3818. | I | H | CF₃ | iPr | Cl |
| 3819. | I | H | Cl | iPr | CF₃ |
| 3820. | I | H | CF₃ | tBu | Cl |
| 3821. | I | H | Cl | tBu | CF₃ |
| 3822. | I | H | CF₃ | cPen | Cl |
| 3823. | I | H | Cl | cPen | CF₃ |
| 3824. | I | H | CF₃ | CH₂cPr | Cl |
| 3825. | I | H | Cl | CH₂cPr | CF₃ |
| 3826. | I | H | CF₃ | CH₂CH=CH₂ | Cl |
| 3827. | I | H | Cl | CH₂CH=CH₂ | CF₃ |
| 3828. | I | H | CF₃ | CHF₂ | OMe |
| 3829. | I | H | OMe | CHF₂ | CF₃ |
| 3830. | I | H | CF₃ | CH₂CF₃ | Cl |
| 3831. | I | H | Cl | CH₂CF₃ | CF₃ |
| 3832. | I | H | CF₃ | CH₂OMe | Cl |
| 3833. | I | H | Cl | CH₂OMe | CF₃ |
| 3834. | I | H | CF₃ | CH₂CN | Cl |
| 3835. | I | H | Me | Ph | Me |
| 3836. | I | H | Me | Ph | Cl |
| 3837. | I | H | Et | Ph | Cl |
| 3838. | I | H | Pr | Ph | Cl |
| 3839. | I | H | iPr | Ph | Cl |
| 3840. | I | H | CF₃ | Ph | Cl |
| 3841. | I | H | CF₃ | Ph | Me |
| 3842. | I | H | CF₃ | Ph | CF₃ |
| 3843. | I | H | CF₃ | Ph | F |
| 3844. | I | H | CF₃ | Ph | OMe |
| 3845. | I | H | CF₃ | Ph | OEt |
| 3846. | I | H | CF₃ | Ph | OCHF₂ |
| 3847. | I | H | CF₃ | Ph | CN |
| 3848. | I | H | CF₃ | Ph(4-Cl) | Cl |
| 3849. | I | H | Me | Me | OCH₂CF₃ |
| 3850. | I | H | CF₃ | Me | 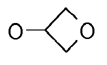 |
| 3851. | I | H | CF₃ | Me | H |
| 3852. | I | H | CF₃ | Me | OCH₂CH₂OMe |
| 3853. | I | H | CF₃ | Me | SMe |
| 3854. | I | H | CF₃ | Me | OCH₂CH₂CH₂F |
| 3855. | I | H | CF₃ | Me | OCH(CH₂F)₂ |
| 3856. | I | H | CF₃ | Me | OCH₂CF₂CHF₂ |
| 3857. | I | H | CF₃ | Me | OCH₂CF=CH₂ |
| 3858. | I | H | CF₃ | Me | OCH(Me)CF₃ |
| 3859. | I | H | CF₃ | Me | OCH(Me)CH₂F |
| 3860. | I | H | OCH₂CF₃ | Me | CF₃ |
| 3861. | I | H | OCH₂CF₃ | Me | CHF₂ |
| 3862. | I | H | CHF₂ | Me | CHF₂ |
| 3863. | I | H | CF₃ | Me | CHF₂ |
| 3864. | I | H | Cl | Me | OCHF₂ |
| 3865. | I | H | Br | Me | OCHF₂ |
| 3866. | I | H | Br | Me | CF₃ |
| 3867. | I | H | CF₃ | Me | CF₃ |
| 3868. | I | H | CHF₂ | Me | OCHF₂ |
| 3869. | I | H | CHF₂ | Me | CF₃ |
| 3870. | I | H | CF₂CF₃ | Me | CF₃ |
| 3871. | I | H | CF₃ | Me | CF₂CF₃ |
| 3872. | I | H | CHF₂ | Me | OCH₂CF₃ |
| 3873. | H | Cl | CF₃ | Ph | Cl |
| 3874. | H | Cl | CF₃ | tBu | Cl |
| 3875. | H | Cl | CF₃ | CHF₂ | Cl |
| 3876. | H | Cl | Cl | CHF₂ | CF₃ |
| 3877. | H | Cl | CF₃ | Me | OMe |
| 3878. | H | Cl | CF₃ | Me | CN |
| 3879. | H | Cl | Cl | Et | Cl |
| 3880. | H | Cl | CHF₂ | Me | Cl |
| 3881. | H | Cl | Me | Me | Me |
| 3882. | H | Cl | Me | Me | Cl |
| 3883. | H | Cl | Cl | Me | Cl |
| 3884. | H | Cl | CF₃ | Me | Cl |
| 3885. | H | Cl | Cl | Me | CF₃ |
| 3886. | H | Cl | CF₃ | Me | F |
| 3887. | H | Cl | OMe | Me | CF₃ |
| 3888. | H | Cl | CF₃ | Me | OEt |
| 3889. | H | Cl | CF₃ | Me | OCHF₂ |
| 3890. | H | Cl | OCHF₂ | Me | CF₃ |
| 3891. | H | Cl | CF₃ | Me | OCH₂CHF₂ |
| 3892. | H | Cl | CF₃ | Me | OCH₂CF₃ |
| 3893. | H | Cl | CF₃ | Me | OCH₂CN |
| 3894. | H | Cl | CF₃ | Me | SO₂Me |
| 3895. | H | Cl | CF₃ | Me | SEt |
| 3896. | H | Cl | CF₃ | Me | Me |
| 3897. | H | Cl | CF₃ | Me | Et |
| 3898. | H | Cl | CF₃ | Et | Cl |
| 3899. | H | Cl | Cl | Et | CF₃ |
| 3900. | H | Cl | CF₃ | iPr | Cl |
| 3901. | H | Cl | Cl | iPr | CF₃ |
| 3902. | H | Cl | CF₃ | tBu | Cl |
| 3903. | H | Cl | Cl | tBu | CF₃ |
| 3904. | H | Cl | CF₃ | cPen | Cl |
| 3905. | H | Cl | Cl | cPen | CF₃ |
| 3906. | H | Cl | CF₃ | CH₂cPr | Cl |
| 3907. | H | Cl | Cl | CH₂cPr | CF₃ |
| 3908. | H | Cl | CF₃ | CH₂CH=CH₂ | Cl |
| 3909. | H | Cl | Cl | CH₂CH=CH₂ | CF₃ |
| 3910. | H | Cl | CF₃ | CHF₂ | OMe |
| 3911. | H | Cl | OMe | CHF₂ | CF₃ |
| 3912. | H | Cl | CF₃ | CH₂CF₃ | Cl |
| 3913. | H | Cl | Cl | CH₂CF₃ | CF₃ |
| 3914. | H | Cl | CF₃ | CH₂OMe | Cl |
| 3915. | H | Cl | Cl | CH₂OMe | CF₃ |
| 3916. | H | Cl | CF₃ | CH₂CN | Cl |
| 3917. | H | Cl | Me | Ph | Me |
| 3918. | H | Cl | Me | Ph | Cl |
| 3919. | H | Cl | Et | Ph | Cl |
| 3920. | H | Cl | Pr | Ph | Cl |
| 3921. | H | Cl | iPr | Ph | Cl |
| 3922. | H | Cl | CF₃ | Ph | Cl |
| 3923. | H | Cl | CF₃ | Ph | Me |
| 3924. | H | Cl | CF₃ | Ph | CF₃ |
| 3925. | H | Cl | CF₃ | Ph | F |
| 3926. | H | Cl | CF₃ | Ph | OMe |
| 3927. | H | Cl | CF₃ | Ph | OEt |
| 3928. | H | Cl | CF₃ | Ph | OCHF₂ |
| 3929. | H | Cl | CF₃ | Ph | CN |
| 3930. | H | Cl | CF₃ | Ph(4-Cl) | Cl |
| 3931. | H | Cl | Me | Me | OCH₂CF₃ |
| 3932. | H | Cl | CF₃ | Me | 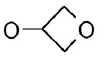 |
| 3933. | H | Cl | CF₃ | Me | H |
| 3934. | H | Cl | CF₃ | Me | OCH₂CH₂OMe |
| 3935. | H | Cl | CF₃ | Me | SMe |
| 3936. | H | Cl | CF₃ | Me | OCH₂CH₂CH₂F |
| 3937. | H | Cl | CF₃ | Me | OCH(CH₂F)₂ |
| 3938. | H | Cl | CF₃ | Me | OCH₂CF₂CHF₂ |
| 3939. | H | Cl | CF₃ | Me | OCH₂CF=CH₂ |
| 3940. | H | Cl | CF₃ | Me | OCH(Me)CF₃ |
| 3941. | H | Cl | CF₃ | Me | OCH(Me)CH₂F |
| 3942. | H | Cl | OCH₂CF₃ | Me | CF₃ |
| 3943. | H | Cl | OCH₂CF₃ | Me | CHF₂ |
| 3944. | H | Cl | CHF₂ | Me | CHF₂ |
| 3945. | H | Cl | CF₃ | Me | CHF₂ |
| 3946. | H | Cl | Cl | Me | OCHF₂ |
| 3947. | H | Cl | Br | Me | OCHF₂ |
| 3948. | H | Cl | Br | Me | CF₃ |
| 3949. | H | Br | CF₃ | Ph | Cl |
| 3950. | H | Br | CF₃ | tBu | Cl |
| 3951. | H | Br | CF₃ | CHF₂ | Cl |
| 3952. | H | Br | Cl | CHF₂ | CF₃ |
| 3953. | H | Br | CF₃ | Me | OMe |
| 3954. | H | Br | CF₃ | Me | CN |
| 3955. | H | Br | Cl | Et | Cl |
| 3956. | H | Br | CHF₂ | Me | Cl |
| 3957. | H | Br | Me | Me | Me |
| 3958. | H | Br | Me | Me | Cl |
| 3959. | H | Br | Cl | Me | Cl |
| 3960. | H | Br | CF₃ | Me | Cl |
| 3961. | H | Br | Cl | Me | CF₃ |
| 3962. | H | Br | CF₃ | Me | F |
| 3963. | H | Br | OMe | Me | CF₃ |
| 3964. | H | Br | CF₃ | Me | OEt |
| 3965. | H | Br | CF₃ | Me | OCHF₂ |
| 3966. | H | Br | OCHF₂ | Me | CF₃ |
| 3967. | H | Br | CF₃ | Me | OCH₂CHF₂ |
| 3968. | H | Br | CF₃ | Me | OCH₂CF₃ |
| 3969. | H | Br | CF₃ | Me | OCH₂CN |
| 3970. | H | Br | CF₃ | Me | SO₂Me |
| 3971. | H | Br | CF₃ | Me | SEt |
| 3972. | H | Br | CF₃ | Me | Me |
| 3973. | H | Br | CF₃ | Me | Et |
| 3974. | H | Br | CF₃ | Et | Cl |
| 3975. | H | Br | Cl | Et | CF₃ |
| 3976. | H | Br | CF₃ | iPr | Cl |
| 3977. | H | Br | Cl | iPr | CF₃ |
| 3978. | H | Br | CF₃ | tBu | Cl |
| 3979. | H | Br | Cl | tBu | CF₃ |
| 3980. | H | Br | CF₃ | cPen | Cl |
| 3981. | H | Br | Cl | cPen | CF₃ |
| 3982. | H | Br | CF₃ | CH₂cPr | Cl |
| 3983. | H | Br | Cl | CH₂cPr | CF₃ |
| 3984. | H | Br | CF₃ | CH₂CH=CH₂ | Cl |
| 3985. | H | Br | Cl | CH₂CH=CH₂ | CF₃ |
| 3986. | H | Br | CF₃ | CHF₂ | OMe |
| 3987. | H | Br | OMe | CHF₂ | CF₃ |
| 3988. | H | Br | CF₃ | CH₂CF₃ | Cl |
| 3989. | H | Br | Cl | CH₂CF₃ | CF₃ |
| 3990. | H | Br | CF₃ | CH₂OMe | Cl |
| 3991. | H | Br | Cl | CH₂OMe | CF₃ |
| 3992. | H | Br | CF₃ | CH₂CN | Cl |
| 3993. | H | Br | Me | Ph | Me |
| 3994. | H | Br | Me | Ph | Cl |
| 3995. | H | Br | Et | Ph | Cl |
| 3996. | H | Br | Pr | Ph | Cl |
| 3997. | H | Br | iPr | Ph | Cl |
| 3998. | H | Br | CF₃ | Ph | Cl |
| 3999. | H | Br | CF₃ | Ph | Me |
| 4000. | H | Br | CF₃ | Ph | CF₃ |
| 4001. | H | Br | CF₃ | Ph | F |
| 4002. | H | Br | CF₃ | Ph | OMe |
| 4003. | H | Br | CF₃ | Ph | OEt |
| 4004. | H | Br | CF₃ | Ph | OCHF₂ |
| 4005. | H | Br | CF₃ | Ph | CN |
| 4006. | H | Br | CF₃ | Ph(4-Cl) | Cl |
| 4007. | H | Br | Me | Me | OCH₂CF₃ |
| 4008. | H | Br | CF₃ | Me | 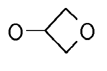 |
| 4009. | H | Br | CF₃ | Me | H |
| 4010. | H | Br | CF₃ | Me | OCH₂CH₂OMe |
| 4011. | H | Br | CF₃ | Me | SMe |
| 4012. | H | Br | CF₃ | Me | OCH₂CH₂CH₂F |
| 4013. | H | Br | CF₃ | Me | OCH(CH₂F)₂ |
| 4014. | H | Br | CF₃ | Me | OCH₂CF₂CHF₂ |
| 4015. | H | Br | CF₃ | Me | OCH₂CF=CH₂ |
| 4016. | H | Br | CF₃ | Me | OCH(Me)CF₃ |
| 4017. | H | Br | CF₃ | Me | OCH(Me)CH₂F |
| 4018. | H | Br | OCH₂CF₃ | Me | CF₃ |
| 4019. | H | Br | OCH₂CF₃ | Me | CHF₂ |
| 4020. | H | Br | CHF₂ | Me | CHF₂ |
| 4021. | H | Br | CF₃ | Me | CHF₂ |
| 4022. | H | Br | Cl | Me | OCHF₂ |
| 4023. | H | Br | Br | Me | OCHF₂ |
| 4024. | H | Br | Br | Me | CF₃ |
| 4025. | Me | H | CF₃ | Ph | Cl |
| 4026. | Me | H | CF₃ | tBu | Cl |
| 4027. | Me | H | CF₃ | CHF₂ | Cl |
| 4028. | Me | H | Cl | CHF₂ | CF₃ |
| 4029. | Me | H | CF₃ | Me | OMe |
| 4030. | Me | H | CF₃ | Me | CN |
| 4031. | Me | H | Cl | Et | Cl |
| 4032. | Me | H | CHF₂ | Me | Cl |
| 4033. | Me | H | Me | Me | Me |
| 4034. | Me | H | Me | Me | Cl |
| 4035. | Me | H | Cl | Me | Cl |
| 4036. | Me | H | CF₃ | Me | Cl |
| 4037. | Me | H | Cl | Me | CF₃ |
| 4038. | Me | H | CF₃ | Me | F |
| 4039. | Me | H | OMe | Me | CF₃ |
| 4040. | Me | H | CF₃ | Me | OEt |
| 4041. | Me | H | CF₃ | Me | OCHF₂ |
| 4042. | Me | H | OCHF₂ | Me | CF₃ |
| 4043. | Me | H | CF₃ | Me | OCH₂CHF₂ |
| 4044. | Me | H | CF₃ | Me | OCH₂CF₃ |
| 4045. | Me | H | CF₃ | Me | OCH₂CN |
| 4046. | Me | H | CF₃ | Me | SO₂Me |
| 4047. | Me | H | CF₃ | Me | SEt |
| 4048. | Me | H | CF₃ | Me | Me |
| 4049. | Me | H | CF₃ | Me | Et |
| 4050. | Me | H | CF₃ | Et | Cl |
| 4051. | Me | H | Cl | Et | CF₃ |
| 4052. | Me | H | CF₃ | iPr | Cl |
| 4053. | Me | H | Cl | iPr | CF₃ |
| 4054. | Me | H | CF₃ | tBu | Cl |
| 4055. | Me | H | Cl | tBu | CF₃ |
| 4056. | Me | H | CF₃ | cPen | Cl |
| 4057. | Me | H | Cl | cPen | CF₃ |
| 4058. | Me | H | CF₃ | CH₂cPr | Cl |
| 4059. | Me | H | Cl | CH₂cPr | CF₃ |
| 4060. | Me | H | CF₃ | CH₂CH=CH₂ | Cl |
| 4061. | Me | H | Cl | CH₂CH=CH₂ | CF₃ |
| 4062. | Me | H | CF₃ | CHF₂ | OMe |
| 4063. | Me | H | OMe | CHF₂ | CF₃ |
| 4064. | Me | H | CF₃ | CH₂CF₃ | Cl |
| 4065. | Me | H | Cl | CH₂CF₃ | CF₃ |
| 4066. | Me | H | CF₃ | CH₂OMe | Cl |
| 4067. | Me | H | Cl | CH₂OMe | CF₃ |
| 4068. | Me | H | CF₃ | CH₂CN | Cl |
| 4069. | Me | H | Me | Ph | Me |
| 4070. | Me | H | Me | Ph | Cl |
| 4071. | Me | H | Et | Ph | Cl |
| 4072. | Me | H | Pr | Ph | Cl |
| 4073. | Me | H | iPr | Ph | Cl |
| 4074. | Me | H | CF₃ | Ph | Cl |
| 4075. | Me | H | CF₃ | Ph | Me |
| 4076. | Me | H | CF₃ | Ph | CF₃ |
| 4077. | Me | H | CF₃ | Ph | F |
| 4078. | Me | H | CF₃ | Ph | OMe |
| 4079. | Me | H | CF₃ | Ph | OEt |
| 4080. | Me | H | CF₃ | Ph | OCHF₂ |
| 4081. | Me | H | CF₃ | Ph | CN |
| 4082. | Me | H | CF₃ | Ph(4-Cl) | Cl |
| 4083. | Me | H | Me | Me | OCH₂CF₃ |
| 4084. | Me | H | CF₃ | Me | 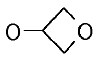 |
| 4085. | Me | H | CF₃ | Me | H |
| 4086. | Me | H | CF₃ | Me | OCH₂CH₂OMe |
| 4087. | Me | H | CF₃ | Me | SMe |
| 4088. | Me | H | CF₃ | Me | OCH₂CH₂CH₂F |
| 4089. | Me | H | CF₃ | Me | OCH(CH₂F)₂ |
| 4090. | Me | H | CF₃ | Me | OCH₂CF₂CHF₂ |
| 4091. | Me | H | CF₃ | Me | OCH₂CF=CH₂ |
| 4092. | Me | H | CF₃ | Me | OCH(Me)CF₃ |
| 4093. | Me | H | CF₃ | Me | OCH(Me)CH₂F |
| 4094. | Me | H | OCH₂CF₃ | Me | CF₃ |
| 4095. | Me | H | OCH₂CF₃ | Me | CHF₂ |
| 4096. | Me | H | CHF₂ | Me | CHF₂ |
| 4097. | Me | H | CF₃ | Me | CHF₂ |
| 4098. | Me | H | Cl | Me | OCHF₂ |
| 4099. | Me | H | Br | Me | OCHF₂ |
| 4100. | Me | H | Br | Me | CF₃ |
| 4101. | H | Me | CF₃ | Ph | Cl |
| 4102. | H | Me | CF₃ | tBu | Cl |
| 4103. | H | Me | CF₃ | CHF₂ | Cl |
| 4104. | H | Me | Cl | CHF₂ | CF₃ |
| 4105. | H | Me | CF₃ | Me | OMe |
| 4106. | H | Me | CF₃ | Me | CN |
| 4107. | H | Me | Cl | Et | Cl |
| 4108. | H | Me | CHF₂ | Me | Cl |
| 4109. | H | Me | Me | Me | Me |
| 4110. | H | Me | Me | Me | Cl |
| 4111. | H | Me | Cl | Me | Cl |
| 4112. | H | Me | CF₃ | Me | Cl |
| 4113. | H | Me | Cl | Me | CF₃ |
| 4114. | H | Me | CF₃ | Me | F |
| 4115. | H | Me | OMe | Me | CF₃ |
| 4116. | H | Me | CF₃ | Me | OEt |
| 4117. | H | Me | CF₃ | Me | OCHF₂ |
| 4118. | H | Me | OCHF₂ | Me | CF₃ |
| 4119. | H | Me | CF₃ | Me | OCH₂CHF₂ |
| 4120. | H | Me | CF₃ | Me | OCH₂CF₃ |
| 4121. | H | Me | CF₃ | Me | OCH₂CN |
| 4122. | H | Me | CF₃ | Me | SO₂Me |
| 4123. | H | Me | CF₃ | Me | SEt |
| 4124. | H | Me | CF₃ | Me | Me |
| 4125. | H | Me | CF₃ | Me | Et |
| 4126. | H | Me | CF₃ | Et | Cl |
| 4127. | H | Me | Cl | Et | CF₃ |
| 4128. | H | Me | CF₃ | iPr | Cl |
| 4129. | H | Me | Cl | iPr | CF₃ |
| 4130. | H | Me | CF₃ | tBu | Cl |
| 4131. | H | Me | Cl | tBu | CF₃ |
| 4132. | H | Me | CF₃ | cPen | Cl |
| 4133. | H | Me | Cl | cPen | CF₃ |
| 4134. | H | Me | CF₃ | CH₂cPr | Cl |
| 4135. | H | Me | Cl | CH₂cPr | CF₃ |
| 4136. | H | Me | CF₃ | CH₂CH=CH₂ | Cl |
| 4137. | H | Me | Cl | CH₂CH=CH₂ | CF₃ |
| 4138. | H | Me | CF₃ | CHF₂ | OMe |
| 4139. | H | Me | OMe | CHF₂ | CF₃ |
| 4140. | H | Me | CF₃ | CH₂CF₃ | Cl |
| 4141. | H | Me | Cl | CH₂CF₃ | CF₃ |
| 4142. | H | Me | CF₃ | CH₂OMe | Cl |
| 4143. | H | Me | Cl | CH₂OMe | CF₃ |
| 4144. | H | Me | CF₃ | CH₂CN | Cl |
| 4145. | H | Me | Me | Ph | Me |
| 4146. | H | Me | Me | Ph | Cl |
| 4147. | H | Me | Et | Ph | Cl |
| 4148. | H | Me | Pr | Ph | Cl |
| 4149. | H | Me | iPr | Ph | Cl |
| 4150. | H | Me | CF₃ | Ph | Cl |
| 4151. | H | Me | CF₃ | Ph | Me |
| 4152. | H | Me | CF₃ | Ph | CF₃ |
| 4153. | H | Me | CF₃ | Ph | F |
| 4154. | H | Me | CF₃ | Ph | OMe |
| 4155. | H | Me | CF₃ | Ph | OEt |
| 4156. | H | Me | CF₃ | Ph | OCHF₂ |
| 4157. | H | Me | CF₃ | Ph | CN |
| 4158. | H | Me | CF₃ | Ph(4-Cl) | Cl |
| 4159. | H | Me | Me | Me | OCH₂CF₃ |
| 4160. | H | Me | CF₃ | Me | 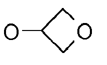 |
| 4161. | H | Me | CF₃ | Me | H |
| 4162. | H | Me | CF₃ | Me | OCH₂CH₂OMe |
| 4163. | H | Me | CF₃ | Me | SMe |
| 4164. | H | Me | CF₃ | Me | OCH₂CH₂CH₂F |
| 4165. | H | Me | CF₃ | Me | OCH(CH₂F)₂ |
| 4166. | H | Me | CF₃ | Me | OCH₂CF₂CHF₂ |
| 4167. | H | Me | CF₃ | Me | OCH₂CF=CH₂ |
| 4168. | H | Me | CF₃ | Me | OCH(Me)CF₃ |
| 4169. | H | Me | CF₃ | Me | OCH(Me)CH₂F |
| 4170. | H | Me | OCH₂CF₃ | Me | CF₃ |
| 4171. | H | Me | OCH₂CF₃ | Me | CHF₂ |
| 4172. | H | Me | CHF₂ | Me | CHF₂ |
| 4173. | H | Me | CF₃ | Me | CHF₂ |
| 4174. | H | Me | Cl | Me | OCHF₂ |
| 4175. | H | Me | Br | Me | OCHF₂ |
| 4176. | H | Me | Br | Me | CF₃ |
| 4177. | NO₂ | H | CF₃ | Ph | Cl |
| 4178. | NO₂ | H | CF₃ | tBu | Cl |
| 4179. | NO₂ | H | CF₃ | CHF₂ | Cl |
| 4180. | NO₂ | H | Cl | CHF₂ | CF₃ |
| 4181. | NO₂ | H | CF₃ | Me | OMe |
| 4182. | NO₂ | H | CF₃ | Me | CN |
| 4183. | NO₂ | H | Cl | Et | Cl |
| 4184. | NO₂ | H | CHF₂ | Me | Cl |
| 4185. | NO₂ | H | Me | Me | Me |
| 4186. | NO₂ | H | Me | Me | Cl |
| 4187. | NO₂ | H | Cl | Me | Cl |
| 4188. | NO₂ | H | CF₃ | Me | Cl |
| 4189. | NO₂ | H | Cl | Me | CF₃ |
| 4190. | NO₂ | H | CF₃ | Me | F |
| 4191. | NO₂ | H | OMe | Me | CF₃ |
| 4192. | NO₂ | H | CF₃ | Me | OEt |
| 4193. | NO₂ | H | CF₃ | Me | OCHF₂ |
| 4194. | NO₂ | H | OCHF₂ | Me | CF₃ |
| 4195. | NO₂ | H | CF₃ | Me | OCH₂CHF₂ |
| 4196. | NO₂ | H | CF₃ | Me | OCH₂CF₃ |
| 4197. | NO₂ | H | CF₃ | Me | OCH₂CN |
| 4198. | NO₂ | H | CF₃ | Me | SO₂Me |
| 4199. | NO₂ | H | CF₃ | Me | SEt |
| 4200. | NO₂ | H | CF₃ | Me | Me |
| 4201. | NO₂ | H | CF₃ | Me | Et |
| 4202. | NO₂ | H | CF₃ | Et | Cl |
| 4203. | NO₂ | H | Cl | Et | CF₃ |
| 4204. | NO₂ | H | CF₃ | iPr | Cl |
| 4205. | NO₂ | H | Cl | iPr | CF₃ |
| 4206. | NO₂ | H | CF₃ | tBu | Cl |
| 4207. | NO₂ | H | Cl | tBu | CF₃ |
| 4208. | NO₂ | H | CF₃ | cPen | Cl |
| 4209. | NO₂ | H | Cl | cPen | CF₃ |
| 4210. | NO₂ | H | CF₃ | CH₂cPr | Cl |
| 4211. | NO₂ | H | Cl | CH₂cPr | CF₃ |
| 4212. | NO₂ | H | CF₃ | CH₂CH=CH₂ | Cl |
| 4213. | NO₂ | H | Cl | CH₂CH=CH₂ | CF₃ |
| 4214. | NO₂ | H | CF₃ | CHF₂ | OMe |
| 4215. | NO₂ | H | OMe | CHF₂ | CF₃ |
| 4216. | NO₂ | H | CF₃ | CH₂CF₃ | Cl |
| 4217. | NO₂ | H | Cl | CH₂CF₃ | CF₃ |
| 4218. | NO₂ | H | CF₃ | CH₂OMe | Cl |
| 4219. | NO₂ | H | Cl | CH₂OMe | CF₃ |
| 4220. | NO₂ | H | CF₃ | CH₂CN | Cl |
| 4221. | NO₂ | H | Me | Ph | Me |
| 4222. | NO₂ | H | Me | Ph | Cl |
| 4223. | NO₂ | H | Et | Ph | Cl |
| 4224. | NO₂ | H | Pr | Ph | Cl |
| 4225. | NO₂ | H | iPr | Ph | Cl |
| 4226. | NO₂ | H | CF₃ | Ph | Cl |
| 4227. | NO₂ | H | CF₃ | Ph | Me |
| 4228. | NO₂ | H | CF₃ | Ph | CF₃ |
| 4229. | NO₂ | H | CF₃ | Ph | F |
| 4230. | NO₂ | H | CF₃ | Ph | OMe |
| 4231. | NO₂ | H | CF₃ | Ph | OEt |
| 4232. | NO₂ | H | CF₃ | Ph | OCHF₂ |
| 4233. | NO₂ | H | CF₃ | Ph | CN |
| 4234. | NO₂ | H | CF₃ | Ph(4-Cl) | Cl |
| 4235. | NO₂ | H | Me | Me | OCH₂CF₃ |
| 4236. | NO₂ | H | CF₃ | Me | 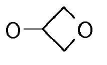 |
| 4237. | NO₂ | H | CF₃ | Me | H |
| 4238. | NO₂ | H | CF₃ | Me | OCH₂CH₂OMe |
| 4239. | NO₂ | H | CF₃ | Me | SMe |
| 4240. | NO₂ | H | CF₃ | Me | OCH₂CH₂CH₂F |
| 4241. | NO₂ | H | CF₃ | Me | OCH(CH₂F)₂ |
| 4242. | NO₂ | H | CF₃ | Me | OCH₂CF₂CHF₂ |
| 4243. | NO₂ | H | CF₃ | Me | OCH₂CF=CH₂ |
| 4244. | NO₂ | H | CF₃ | Me | OCH(Me)CF₃ |
| 4245. | NO₂ | H | CF₃ | Me | OCH(Me)CH₂F |
| 4246. | NO₂ | H | OCH₂CF₃ | Me | CF₃ |
| 4247. | NO₂ | H | OCH₂CF₃ | Me | CHF₂ |
| 4248. | NO₂ | H | CHF₂ | Me | CHF₂ |
| 4249. | NO₂ | H | CF₃ | Me | CHF₂ |
| 4250. | NO₂ | H | Cl | Me | OCHF₂ |
| 4251. | NO₂ | H | Br | Me | OCHF₂ |
| 4252. | NO₂ | H | Br | Me | CF₃ |
| 4253. | CHF₂ | H | CF₃ | Ph | Cl |
| 4254. | CHF₂ | H | CF₃ | tBu | Cl |
| 4255. | CHF₂ | H | CF₃ | CHF₂ | Cl |
| 4256. | CHF₂ | H | Cl | CHF₂ | CF₃ |
| 4257. | CHF₂ | H | CF₃ | Me | OMe |
| 4258. | CHF₂ | H | CF₃ | Me | CN |
| 4259. | CHF₂ | H | Cl | Et | Cl |
| 4260. | CHF₂ | H | CHF₂ | Me | Cl |
| 4261. | CHF₂ | H | Me | Me | Me |
| 4262. | CHF₂ | H | Me | Me | Cl |
| 4263. | CHF₂ | H | Cl | Me | Cl |
| 4264. | CHF₂ | H | CF₃ | Me | Cl |
| 4265. | CHF₂ | H | Cl | Me | CF₃ |
| 4266. | CHF₂ | H | CF₃ | Me | F |
| 4267. | CHF₂ | H | OMe | Me | CF₃ |
| 4268. | CHF₂ | H | CF₃ | Me | OEt |
| 4269. | CHF₂ | H | CF₃ | Me | OCHF₂ |
| 4270. | CHF₂ | H | OCHF₂ | Me | CF₃ |
| 4271. | CHF₂ | H | CF₃ | Me | OCH₂CHF₂ |
| 4272. | CHF₂ | H | CF₃ | Me | OCH₂CF₃ |
| 4273. | CHF₂ | H | CF₃ | Me | OCH₂CN |
| 4274. | CHF₂ | H | CF₃ | Me | SO₂Me |
| 4275. | CHF₂ | H | CF₃ | Me | SEt |
| 4276. | CHF₂ | H | CF₃ | Me | Me |
| 4277. | CHF₂ | H | CF₃ | Me | Et |
| 4278. | CHF₂ | H | CF₃ | Et | Cl |
| 4279. | CHF₂ | H | Cl | Et | CF₃ |
| 4280. | CHF₂ | H | CF₃ | iPr | Cl |
| 4281. | CHF₂ | H | Cl | iPr | CF₃ |
| 4282. | CHF₂ | H | CF₃ | tBu | Cl |
| 4283. | CHF₂ | H | Cl | tBu | CF₃ |
| 4284. | CHF₂ | H | CF₃ | cPen | Cl |
| 4285. | CHF₂ | H | Cl | cPen | CF₃ |
| 4286. | CHF₂ | H | CF₃ | CH₂cPr | Cl |
| 4287. | CHF₂ | H | Cl | CH₂cPr | CF₃ |
| 4288. | CHF₂ | H | CF₃ | CH₂CH=CH₂ | Cl |
| 4289. | CHF₂ | H | Cl | CH₂CH=CH₂ | CF₃ |
| 4290. | CHF₂ | H | CF₃ | CHF₂ | OMe |
| 4291. | CHF₂ | H | OMe | CHF₂ | CF₃ |
| 4292. | CHF₂ | H | CF₃ | CH₂CF₃ | Cl |
| 4293. | CHF₂ | H | Cl | CH₂CF₃ | CF₃ |
| 4294. | CHF₂ | H | CF₃ | CH₂OMe | Cl |
| 4295. | CHF₂ | H | Cl | CH₂OMe | CF₃ |
| 4296. | CHF₂ | H | CF₃ | CH₂CN | Cl |
| 4297. | CHF₂ | H | Me | Ph | Me |
| 4298. | CHF₂ | H | Me | Ph | Cl |
| 4299. | CHF₂ | H | Et | Ph | Cl |
| 4300. | CHF₂ | H | Pr | Ph | Cl |
| 4301. | CHF₂ | H | iPr | Ph | Cl |
| 4302. | CHF₂ | H | CF₃ | Ph | Cl |
| 4303. | CHF₂ | H | CF₃ | Ph | Me |
| 4304. | CHF₂ | H | CF₃ | Ph | CF₃ |
| 4305. | CHF₂ | H | CF₃ | Ph | F |
| 4306. | CHF₂ | H | CF₃ | Ph | OMe |
| 4307. | CHF₂ | H | CF₃ | Ph | OEt |
| 4308. | CHF₂ | H | CF₃ | Ph | OCHF₂ |
| 4309. | CHF₂ | H | CF₃ | Ph | CN |
| 4310. | CHF₂ | H | CF₃ | Ph(4-Cl) | Cl |
| 4311. | CHF₂ | H | Me | Me | OCH₂CF₃ |
| 4312. | CHF₂ | H | CF₃ | Me | 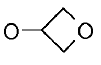 |
| 4313. | CHF₂ | H | CF₃ | Me | H |
| 4314. | CHF₂ | H | CF₃ | Me | OCH₂CH₂OMe |
| 4315. | CHF₂ | H | CF₃ | Me | SMe |
| 4316. | CHF₂ | H | CF₃ | Me | OCH₂CH₂CH₂F |
| 4317. | CHF₂ | H | CF₃ | Me | OCH(CH₂F)₂ |
| 4318. | CHF₂ | H | CF₃ | Me | OCH₂CF₂CHF₂ |
| 4319. | CHF₂ | H | CF₃ | Me | OCH₂CF=CH₂ |
| 4320. | CHF₂ | H | CF₃ | Me | OCH(Me)CF₃ |
| 4321. | CHF₂ | H | CF₃ | Me | OCH(Me)CH₂F |
| 4322. | CHF₂ | H | OCH₂CF₃ | Me | CF₃ |
| 4323. | CHF₂ | H | OCH₂CF₃ | Me | CHF₂ |
| 4324. | CHF₂ | H | CHF₂ | Me | CHF₂ |
| 4325. | CHF₂ | H | CF₃ | Me | CHF₂ |
| 4326. | CHF₂ | H | Cl | Me | OCHF₂ |
| 4327. | CHF₂ | H | Br | Me | OCHF₂ |
| 4328. | CHF₂ | H | Br | Me | CF₃ |
| 4329. | Cl | Cl | CF₃ | Ph | Cl |
| 4330. | Cl | Cl | CF₃ | tBu | Cl |
| 4331. | Cl | Cl | CF₃ | CHF₂ | Cl |
| 4332. | Cl | Cl | Cl | CHF₂ | CF₃ |
| 4333. | Cl | Cl | CF₃ | Me | OMe |
| 4334. | Cl | Cl | CF₃ | Me | CN |
| 4335. | Cl | Cl | Cl | Et | Cl |
| 4336. | Cl | Cl | CHF₂ | Me | Cl |
| 4337. | Cl | Cl | Me | Me | Me |
| 4338. | Cl | Cl | Me | Me | Cl |
| 4339. | Cl | Cl | Cl | Me | Cl |
| 4340. | Cl | Cl | CF₃ | Me | Cl |
| 4341. | Cl | Cl | Cl | Me | CF₃ |
| 4342. | Cl | Cl | CF₃ | Me | F |
| 4343. | Cl | Cl | OMe | Me | CF₃ |
| 4344. | Cl | Cl | CF₃ | Me | OEt |
| 4345. | Cl | Cl | CF₃ | Me | OCHF₂ |
| 4346. | Cl | Cl | OCHF₂ | Me | CF₃ |
| 4347. | Cl | Cl | CF₃ | Me | OCH₂CHF₂ |
| 4348. | Cl | Cl | CF₃ | Me | OCH₂CF₃ |
| 4349. | Cl | Cl | CF₃ | Me | OCH₂CN |
| 4350. | Cl | Cl | CF₃ | Me | SO₂Me |
| 4351. | Cl | Cl | CF₃ | Me | SEt |
| 4352. | Cl | Cl | CF₃ | Me | Me |
| 4353. | Cl | Cl | CF₃ | Me | Et |
| 4354. | Cl | Cl | CF₃ | Et | Cl |
| 4355. | Cl | Cl | Cl | Et | CF₃ |
| 4356. | Cl | Cl | CF₃ | iPr | Cl |
| 4357. | Cl | Cl | Cl | iPr | CF₃ |
| 4358. | Cl | Cl | CF₃ | tBu | Cl |
| 4359. | Cl | Cl | Cl | tBu | CF₃ |
| 4360. | Cl | Cl | CF₃ | cPen | Cl |
| 4361. | Cl | Cl | Cl | cPen | CF₃ |
| 4362. | Cl | Cl | CF₃ | CH₂cPr | Cl |
| 4363. | Cl | Cl | Cl | CH₂cPr | CF₃ |
| 4364. | Cl | Cl | CF₃ | CH₂CH=CH₂ | Cl |
| 4365. | Cl | Cl | Cl | CH₂CH=CH₂ | CF₃ |
| 4366. | Cl | Cl | CF₃ | CHF₂ | OMe |
| 4367. | Cl | Cl | OMe | CHF₂ | CF₃ |
| 4368. | Cl | Cl | CF₃ | CH₂CF₃ | Cl |
| 4369. | Cl | Cl | Cl | CH₂CF₃ | CF₃ |
| 4370. | Cl | Cl | CF₃ | CH₂OMe | Cl |
| 4371. | Cl | Cl | Cl | CH₂OMe | CF₃ |
| 4372. | Cl | Cl | CF₃ | CH₂CN | Cl |
| 4373. | Cl | Cl | Me | Ph | Me |
| 4374. | Cl | Cl | Me | Ph | Cl |
| 4375. | Cl | Cl | Et | Ph | Cl |
| 4376. | Cl | Cl | Pr | Ph | Cl |
| 4377. | Cl | Cl | iPr | Ph | Cl |
| 4378. | Cl | Cl | CF₃ | Ph | Cl |
| 4379. | Cl | Cl | CF₃ | Ph | Me |
| 4380. | Cl | Cl | CF₃ | Ph | CF₃ |
| 4381. | Cl | Cl | CF₃ | Ph | F |
| 4382. | Cl | Cl | CF₃ | Ph | OMe |
| 4383. | Cl | Cl | CF₃ | Ph | OEt |
| 4384. | Cl | Cl | CF₃ | Ph | OCHF₂ |
| 4385. | Cl | Cl | CF₃ | Ph | CN |
| 4386. | Cl | Cl | CF₃ | Ph(4-Cl) | Cl |
| 4387. | Cl | Cl | Me | Me | OCH₂CF₃ |
| 4388. | Cl | Cl | CF₃ | Me | 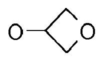 |
| 4389. | Cl | Cl | CF₃ | Me | H |
| 4390. | Cl | Cl | CF₃ | Me | OCH₂CH₂OMe |
| 4391. | Cl | Cl | CF₃ | Me | SMe |
| 4392. | Cl | Cl | CF₃ | Me | OCH₂CH₂CH₂F |
| 4393. | Cl | Cl | CF₃ | Me | OCH(CH₂F)₂ |
| 4394. | Cl | Cl | CF₃ | Me | OCH₂CF₂CHF₂ |
| 4395. | Cl | Cl | CF₃ | Me | OCH₂CF=CH₂ |
| 4396. | Cl | Cl | CF₃ | Me | OCH(Me)CF₃ |
| 4397. | Cl | Cl | CF₃ | Me | OCH(Me)CH₂F |
| 4398. | Cl | Cl | OCH₂CF₃ | Me | CF₃ |
| 4399. | Cl | Cl | OCH₂CF₃ | Me | CHF₂ |
| 4400. | Cl | Cl | CHF₂ | Me | CHF₂ |
| 4401. | Cl | Cl | CF₃ | Me | CHF₂ |
| 4402. | Cl | Cl | Cl | Me | OCHF₂ |
| 4403. | Cl | Cl | Br | Me | OCHF₂ |
| 4404. | Cl | Cl | Br | Me | CF₃ |
| 4405. | Me | Cl | CF₃ | Ph | Cl |
| 4406. | Me | Cl | CF₃ | tBu | Cl |
| 4407. | Me | Cl | CF₃ | CHF₂ | Cl |
| 4408. | Me | Cl | Cl | CHF₂ | CF₃ |
| 4409. | Me | Cl | CF₃ | Me | OMe |
| 4410. | Me | Cl | CF₃ | Me | CN |
| 4411. | Me | Cl | Cl | Et | Cl |
| 4412. | Me | Cl | CHF₂ | Me | Cl |
| 4413. | Me | Cl | Me | Me | Me |
| 4414. | Me | Cl | Me | Me | Cl |
| 4415. | Me | Cl | Cl | Me | Cl |
| 4416. | Me | Cl | CF₃ | Me | Cl |
| 4417. | Me | Cl | Cl | Me | CF₃ |
| 4418. | Me | Cl | CF₃ | Me | F |
| 4419. | Me | Cl | OMe | Me | CF₃ |
| 4420. | Me | Cl | CF₃ | Me | OEt |
| 4421. | Me | Cl | CF₃ | Me | OCHF₂ |
| 4422. | Me | Cl | OCHF₂ | Me | CF₃ |
| 4423. | Me | Cl | CF₃ | Me | OCH₂CHF₂ |
| 4424. | Me | Cl | CF₃ | Me | OCH₂CF₃ |
| 4425. | Me | Cl | CF₃ | Me | OCH₂CN |
| 4426. | Me | Cl | CF₃ | Me | SO₂Me |
| 4427. | Me | Cl | CF₃ | Me | SEt |
| 4428. | Me | Cl | CF₃ | Me | Me |
| 4429. | Me | Cl | CF₃ | Me | Et |
| 4430. | Me | Cl | CF₃ | Et | Cl |
| 4431. | Me | Cl | Cl | Et | CF₃ |
| 4432. | Me | Cl | CF₃ | iPr | Cl |
| 4433. | Me | Cl | Cl | iPr | CF₃ |
| 4434. | Me | Cl | CF₃ | tBu | Cl |
| 4435. | Me | Cl | Cl | tBu | CF₃ |
| 4436. | Me | Cl | CF₃ | cPen | Cl |
| 4437. | Me | Cl | Cl | cPen | CF₃ |
| 4438. | Me | Cl | CF₃ | CH₂cPr | Cl |
| 4439. | Me | Cl | Cl | CH₂cPr | CF₃ |
| 4440. | Me | Cl | CF₃ | CH₂CH=CH₂ | Cl |
| 4441. | Me | Cl | Cl | CH₂CH=CH₂ | CF₃ |
| 4442. | Me | Cl | CF₃ | CHF₂ | OMe |
| 4443. | Me | Cl | OMe | CHF₂ | CF₃ |
| 4444. | Me | Cl | CF₃ | CH₂CF₃ | Cl |
| 4445. | Me | Cl | Cl | CH₂CF₃ | CF₃ |
| 4446. | Me | Cl | CF₃ | CH₂OMe | Cl |
| 4447. | Me | Cl | Cl | CH₂OMe | CF₃ |
| 4448. | Me | Cl | CF₃ | CH₂CN | Cl |
| 4449. | Me | Cl | Me | Ph | Me |
| 4450. | Me | Cl | Me | Ph | Cl |
| 4451. | Me | Cl | Et | Ph | Cl |
| 4452. | Me | Cl | Pr | Ph | Cl |
| 4453. | Me | Cl | iPr | Ph | Cl |
| 4454. | Me | Cl | CF₃ | Ph | Cl |
| 4455. | Me | Cl | CF₃ | Ph | Me |
| 4456. | Me | Cl | CF₃ | Ph | CF₃ |
| 4457. | Me | Cl | CF₃ | Ph | F |
| 4458. | Me | Cl | CF₃ | Ph | OMe |
| 4459. | Me | Cl | CF₃ | Ph | OEt |
| 4460. | Me | Cl | CF₃ | Ph | OCHF₂ |
| 4461. | Me | Cl | CF₃ | Ph | CN |
| 4462. | Me | Cl | CF₃ | Ph(4-Cl) | Cl |
| 4463. | Me | Cl | Me | Me | OCH₂CF₃ |
| 4464. | Me | Cl | CF₃ | Me | 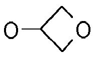 |
| 4465. | Me | Cl | CF₃ | Me | H |
| 4466. | Me | Cl | CF₃ | Me | OCH₂CH₂OMe |
| 4467. | Me | Cl | CF₃ | Me | SMe |
| 4468. | Me | Cl | CF₃ | Me | OCH₂CH₂CH₂F |
| 4469. | Me | Cl | CF₃ | Me | OCH(CH₂F)₂ |
| 4470. | Me | Cl | CF₃ | Me | OCH₂CF₂CHF₂ |
| 4471. | Me | Cl | CF₃ | Me | OCH₂CF=CH₂ |
| 4472. | Me | Cl | CF₃ | Me | OCH(Me)CF₃ |
| 4473. | Me | Cl | CF₃ | Me | OCH(Me)CH₂F |
| 4474. | Me | Cl | OCH₂CF₃ | Me | CF₃ |
| 4475. | Me | Cl | OCH₂CF₃ | Me | CHF₂ |
| 4476. | Me | Cl | CHF₂ | Me | CHF₂ |
| 4477. | Me | Cl | CF₃ | Me | CHF₂ |
| 4478. | Me | Cl | Cl | Me | OCHF₂ |
| 4479. | Me | Cl | Br | Me | OCHF₂ |
| 4480. | Me | Cl | Br | Me | CF₃ |
| 4481. | Cl | Me | CF₃ | Ph | Cl |
| 4482. | Cl | Me | CF₃ | tBu | Cl |
| 4483. | Cl | Me | CF₃ | CHF₂ | Cl |
| 4484. | Cl | Me | Cl | CHF₂ | CF₃ |
| 4485. | Cl | Me | CF₃ | Me | OMe |
| 4486. | Cl | Me | CF₃ | Me | CN |
| 4487. | Cl | Me | Cl | Et | Cl |
| 4488. | Cl | Me | CHF₂ | Me | Cl |
| 4489. | Cl | Me | Me | Me | Me |
| 4490. | Cl | Me | Me | Me | Cl |
| 4491. | Cl | Me | Cl | Me | Cl |
| 4492. | Cl | Me | CF₃ | Me | Cl |
| 4493. | Cl | Me | Cl | Me | CF₃ |
| 4494. | Cl | Me | CF₃ | Me | F |
| 4495. | Cl | Me | OMe | Me | CF₃ |
| 4496. | Cl | Me | CF₃ | Me | OEt |
| 4497. | Cl | Me | CF₃ | Me | OCHF₂ |
| 4498. | Cl | Me | OCHF₂ | Me | CF₃ |
| 4499. | Cl | Me | CF₃ | Me | OCH₂CHF₂ |
| 4500. | Cl | Me | CF₃ | Me | OCH₂CF₃ |
| 4501. | Cl | Me | CF₃ | Me | OCH₂CN |
| 4502. | Cl | Me | CF₃ | Me | SO₂Me |
| 4503. | Cl | Me | CF₃ | Me | SEt |
| 4504. | Cl | Me | CF₃ | Me | Me |
| 4505. | Cl | Me | CF₃ | Me | Et |
| 4506. | Cl | Me | CF₃ | Et | Cl |
| 4507. | Cl | Me | Cl | Et | CF₃ |
| 4508. | Cl | Me | CF₃ | iPr | Cl |
| 4509. | Cl | Me | Cl | iPr | CF₃ |
| 4510. | Cl | Me | CF₃ | tBu | Cl |
| 4511. | Cl | Me | Cl | tBu | CF₃ |
| 4512. | Cl | Me | CF₃ | cPen | Cl |
| 4513. | Cl | Me | Cl | cPen | CF₃ |
| 4514. | Cl | Me | CF₃ | CH₂cPr | Cl |
| 4515. | Cl | Me | Cl | CH₂cPr | CF₃ |
| 4516. | Cl | Me | CF₃ | CH₂CH=CH₂ | Cl |
| 4517. | Cl | Me | Cl | CH₂CH=CH₂ | CF₃ |
| 4518. | Cl | Me | CF₃ | CHF₂ | OMe |
| 4519. | Cl | Me | OMe | CHF₂ | CF₃ |
| 4520. | Cl | Me | CF₃ | CH₂CF₃ | Cl |
| 4521. | Cl | Me | Cl | CH₂CF₃ | CF₃ |
| 4522. | Cl | Me | CF₃ | CH₂OMe | Cl |
| 4523. | Cl | Me | Cl | CH₂OMe | CF₃ |
| 4524. | Cl | Me | CF₃ | CH₂CN | Cl |
| 4525. | Cl | Me | Me | Ph | Me |
| 4526. | Cl | Me | Me | Ph | Cl |
| 4527. | Cl | Me | Et | Ph | Cl |
| 4528. | Cl | Me | Pr | Ph | Cl |
| 4529. | Cl | Me | iPr | Ph | Cl |
| 4530. | Cl | Me | CF₃ | Ph | Cl |
| 4531. | Cl | Me | CF₃ | Ph | Me |
| 4532. | Cl | Me | CF₃ | Ph | CF₃ |
| 4533. | Cl | Me | CF₃ | Ph | F |
| 4534. | Cl | Me | CF₃ | Ph | OMe |
| 4535. | Cl | Me | CF₃ | Ph | OEt |
| 4536. | Cl | Me | CF₃ | Ph | OCHF₂ |
| 4537. | Cl | Me | CF₃ | Ph | CN |
| 4538. | Cl | Me | CF₃ | Ph(4-Cl) | Cl |
| 4539. | Cl | Me | Me | Me | OCH₂CF₃ |
| 4540. | Cl | Me | CF₃ | Me | 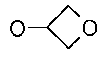 |
| 4541. | Cl | Me | CF₃ | Me | H |
| 4542. | Cl | Me | CF₃ | Me | OCH₂CH₂OMe |
| 4543. | Cl | Me | CF₃ | Me | SMe |
| 4544. | Cl | Me | CF₃ | Me | OCH₂CH₂CH₂F |
| 4545. | Cl | Me | CF₃ | Me | OCH(CH₂F)₂ |
| 4546. | Cl | Me | CF₃ | Me | OCH₂CF₂CHF₂ |
| 4547. | Cl | Me | CF₃ | Me | OCH₂CF=CH₂ |
| 4548. | Cl | Me | CF₃ | Me | OCH(Me)CF₃ |
| 4549. | Cl | Me | CF₃ | Me | OCH(Me)CH₂F |
| 4550. | Cl | Me | OCH₂CF₃ | Me | CF₃ |
| 4551. | Cl | Me | OCH₂CF₃ | Me | CHF₂ |
| 4552. | Cl | Me | CHF₂ | Me | CHF₂ |
| 4553. | Cl | Me | CF₃ | Me | CHF₂ |
| 4554. | Cl | Me | Cl | Me | OCHF₂ |
| 4555. | Cl | Me | Br | Me | OCHF₂ |
| 4556. | Cl | Me | Br | Me | CF₃ |

In den nachfolgenden Tabellen wurden Retentionszeiten (Rt, in Minuten) ausgewählter Verbindungen der Tabellen 1 - 4 von chiralen Verbindungen an Chiralen HPLC gemessen [Chiralcel^{®} OD Säule (250 x 4.6 mm), Temperatur 25 °C, Fluss 0.6 ml/min, Eluent Hexan / 2-Propanol 90:10].

**Tabelle 5: Verbindungen der Formel la-S**

| | | | | | | | | | |
|---|---|---|---|---|---|---|---|---|---|
| 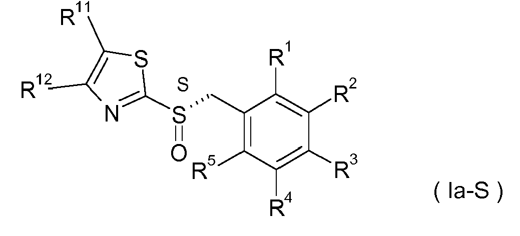 | | | | | | | | | |

| Bsp. Nr. | R¹¹ | R¹² | R¹ | R² | R³ | R⁴ | R⁵ | Optische Drehung | Physikalische Daten |
|---|---|---|---|---|---|---|---|---|---|
| 185 | Cl | H | Cl | H | H | H | Cl | | *Rₜ = 20.123 min |
| 259 | Br | H | F | H | H | H | F | | Rₜ = 17.053 min |
| 270 | Br | H | Cl | H | H | H | Cl | [α]_{D}=+160° | **Rₜ = 26.474 min |

| | | | | | | | | | |
|---|---|---|---|---|---|---|---|---|---|
| *Hexan / 2-Propanol 95:5 v/v. **Hexan / 2-Propanol 97:3 v/v. | | | | | | | | | |

**Tabelle 6: Verbindungen der Formel la-R**

| | | | | | | | | | |
|---|---|---|---|---|---|---|---|---|---|
| 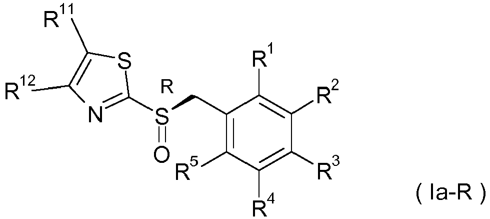 | | | | | | | | | |

| Bsp. Nr. | R¹¹ | R¹² | R¹ | R² | R³ | R⁴ | R⁵ | Optische Drehung | Physikalische Daten |
|---|---|---|---|---|---|---|---|---|---|
| 1375 | Cl | H | Cl | H | H | H | Cl | | *Rₜ = 22.213 min |
| 1449 | Br | H | F | H | H | H | F | | Rₜ = 19.430 min |
| 1460 | Br | H | Cl | H | H | H | Cl | [α]_{D} = -123° | **Rₜ = 29.369 min |

| | | | | | | | | | |
|---|---|---|---|---|---|---|---|---|---|
| *Hexan / 2-Propanol 95:5 v/v. **Hexan / 2-Propanol 97:3 v/v. | | | | | | | | | |

**Tabelle 7: Verbindungen aus den Tabellen 5 und 6 der allgemeinen Formel (Ia) (Racemate)**

| | | | | | | | |
|---|---|---|---|---|---|---|---|
| 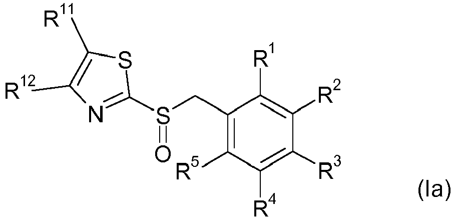 | | | | | | | |

| Bsp. Nr. | R¹¹ | R¹² | R¹ | R² | R³ | R⁴ | R⁵ |
|---|---|---|---|---|---|---|---|
| 185/1375 | Cl | H | Cl | H | H | H | Cl |
| 259/1449 | Br | H | F | H | H | H | F |
| 270/1460 | Br | H | Cl | H | H | H | Cl |

NMR Daten von den Verbindungen der Tabellen 5+6 (CDCl₃, 400 MHz, δ in ppm):

NMR Verbindung 185/1375 (CDCl₃, 400 MHz):
4.74 (d, 1 H, S(O)CH₂); 4.83 (d, 1 H, S(O)CH₂); 7.23 *(m,* 1 H, Ar); 7.33 *(m,* 2H, Ar); 7.67 (s, 1 H, Thiazolyl-H).

NMR Verbindung 259/1449 (CDCl₃, 400 MHz):
4.43 (d, 1 H, S(O)CH₂); 4.52 (d, 1 H, S(O)CH₂); 6.91 *(m,* 2H, Ar); 7.32 *(m,* 1 H, Ar); 7.79 (s, 1 H, Thiazolyl-H).

NMR Verbindung 270/1460 (CDCl₃, 400 MHz):
4.74 (d, 1 H, S(O)CH₂); 4.83 (d, 1 H, S(O)CH₂); 7.24 *(m,* 1 H, Ar); 7.34 *(m,* 2H, Ar); 7.77 (s, 1 H, Thiazolyl-H).

**Tabelle 8: Verbindungen der Formel Ib-S**

| | | | | | | | |
|---|---|---|---|---|---|---|---|
| 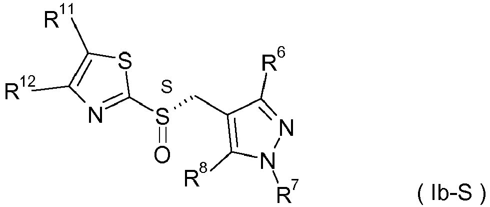 | | | | | | | |

| Bsp. Nr. | R¹¹ | R¹² | R⁶ | R⁷ | R⁸ | Optische Drehung | Physikalische Daten |
|---|---|---|---|---|---|---|---|
| 2555 | Cl | H | CF₃ | Me | OCHF₂ | [α]_{D}= -69.6° | Rₜ = 10.081 min |
| 2632 | Br | H | CF₃ | Me | Cl | [α]_{D} = -38.4° | Rₜ = 18.600 min |
| 2637 | Br | H | CF₃ | Me | OCHF₂ | [α]_{D} = -66.6° | [α]_{D} =-66.6° Rₜ = 10.258 min |
| 2719 | I | H | CF₃ | Me | OCHF₂ | [α]_{D} = -59.8° | Rₜ = 12.775 min |
| 2640 | Br | H | CF₃ | Me | OCH₂CF₃ | [α]_{D} = -36.2° | ***Rₜ = 17.973 min |

| | | | | | | | |
|---|---|---|---|---|---|---|---|
| ***Column Chiralpak IC, Heptane 2-Propanol 90:10, 0.6 ml/min, 25°C. | | | | | | | |

**Tabelle 9: Verbindungen der Formel Ib-R**

| | | | | | | | |
|---|---|---|---|---|---|---|---|
| 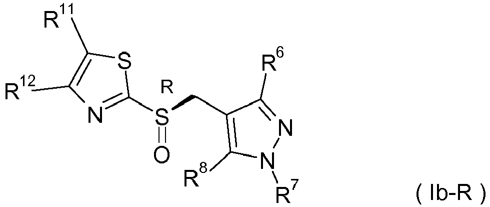 | | | | | | | |

| Bsp. Nr. | R¹¹ | R¹² | R⁶ | R⁷ | R⁸ | Optische Drehung | Pysikalische Daten |
|---|---|---|---|---|---|---|---|
| 3643 | Cl | H | CF₃ | Me | OCHF₂ | [α]_{D} = +71.0° | Rₜ = 12.219 min |
| 3720 | Br | H | CF₃ | Me | Cl | [α]_{D} = +40.3° | Rt = 22.652 min |
| 3725 | Br | H | CF₃ | Me | OCHF₂ | [α]_{D} = +78.3° | [α]_{D} = +78.3° Rₜ = 12.557 min |
| 3807 | I | H | CF₃ | Me | OCHF₂ | [α]_{D} = +56.1° | Rₜ = 15.893 min |
| 3728 | Br | H | CF₃ | Me | OCH₂CF₃ | [α]_{D} = +68.4° | ***Rₜ = 16.446 min |

| | | | | | | | |
|---|---|---|---|---|---|---|---|
| ***Column Chiralpak IC, Heptane 2-Propanol 90:10, 0.6 ml/min, 25°C. | | | | | | | |

**Tabelle 10: Verbindungen der Formel (Ib) (Racemate)**

| | | | | | |
|---|---|---|---|---|---|
| 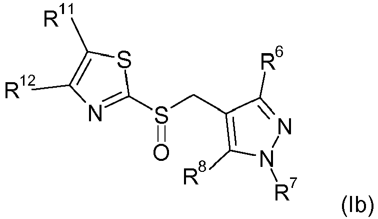 | | | | | |

| Bsp. Nr. | R¹¹ | R¹² | R⁶ | R⁷ | R⁸ |
|---|---|---|---|---|---|
| 2555/3643 | Cl | H | CF₃ | Me | OCHF₂ |
| 2632/3720 | Br | H | CF₃ | Me | Cl |
| 2637/3725 | Br | H | CF₃ | Me | OCHF₂ |
| 2719/3807 | I | H | CF₃ | Me | OCHF₂ |
| 2640/3728 | Br | H | CF3 | Me | OCH2CF3 |

NMR Daten von den Verbindungen der Tabelle 8+9 (CDCl₃, 400 MHz, δ in ppm):

NMR Verbindung 2555/3643 (CDCl₃, 400 MHz):
3.86 (s, 3H, NCH₃); 4.11 (*d*, 1 H, S(O)CH₂); 4.37 (*d*, 1 H, S(O)CH₂); 6.93 (*dd,* 1 H, OCHF₂); 7.78 (s, 1 H, Thiazolyl-H).

NMR Verbindung 2632/3720 (CDCl₃, 400 MHz):
3.90 (s, 3H, NCH₃); 4.26 (d, 1 H, S(O)CH₂); 4.35 (d, 1 H, S(O)CH₂); 7.81 (s, 1 H, Thiazolyl-H).

NMR Verbindung 2637/3725 (CDCl₃, 400 MHz):
3.85 (s, 3H, NCH₃); 4.11 (*d*, 1 H, S(O)CH₂); 4.37 (*d*, 1 H, S(O)CH₂); 6.94 (*dd,* 1 H, OCHF₂); 7.87 (s, 1 H, Thiazolyl-H).

NMR Verbindung 2719/3807 (CDCl₃, 400 MHz):
3.86 (s, 3H, NCH₃); 4.10 *(d,* 1 H, S(O)CH₂); 4.36 (d, 1 H, S(O)CH₂); 6.96 (*dd*, 1 H, OCHF₂); 7.99 (s, 1 H, Thiazolyl-H).

NMR Verbindung 2640/3728 (CDCl₃, 400 MHz):
3.81 (s, 3H, NCH₃); 4.10 (*d*, 1 H, S(O)CH₂); 4.35 (*d*, 1 H, S(O)CH₂); 4.72 (*qd*, 1 H, CH₂CF₃); 4.76 (*qd*, 1 H, CH₂CF₃); 7.88 (s, 1 H, Thiazolyl-H).

Die Retentionszeiten (Rt, in Minuten) ausgewählter Verbindungen der Tabellen 1 - 4 von chiralen Verbindungen wurden mittels analytischer chiraler HPLC ermittelt [Chiralcel OD Säule (250 x 4.6 mm, Korngröße 5 µm), Temperatur 25 °C, Fluss 0.6 ml/min, Hexan / 2-Propanol 90:10 v/v oder Chiralpak IC Säule, (250 x 4.6 mm, Korngröße 5 µm), Temperatur 25 °C, Fluss 0.6 ml/min, Hexan / 2-Propanol 90:10 v/v; falls das Lösemittelverhältnis anders ist, wird im Text vermerkt].

NMR Daten wurden bei 400 MHz und in CDCl₃ als Lösemittel gemessen. Die chemische Verschiebung δ ist in ppm angegeben (TMS Standard).

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z. B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösemittel und 10 Gew.-Teilen oxethyliertes Nonylphenol als Emulgator.
e) Ein in Wasser dispergierbares Granulat wird erhalten indem man
   - 75 Gewichtsteile: einer V erbindung der Formel (I),
   - 10 ": ligninsulfonsaures Calcium,
   - 5 ": Natriumlaurylsulfat,
   - 3 ": Polyvinylalkohol und
   - 7 ": Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),
   - 5 ": 2,2'-dinaphthylmethan-6,6'-disulfonsaures Natrium,
   - 2 ": oleoylmethyltaurinsaures Natrium,
   - 1 Gewichtsteil: Polyvinylalkohol,
   - 17 Gewichtsteile: Calciumcarbonat und
   - 50 ": Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen werden dann als wäßrige Suspension mit einer Wasseraufwandmenge von umgerechnet 600 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen Nr. 185, 259, 270, 2555, 2632, 2637, 2719, 1375, 1449, 1460, 3643, 3720, 3725, 2640, 3728 und andere Verbindungen aus Tabelle 1 - 4 sehr gute herbizide Wirkung gegen Schadpflanzen wie beispielsweise Avena fatua, Stellaria media, Echinochloa crus galli, Lolium multiflorum, Setaria viridis, Abutilon theophrasti, Amaranthus retroflexus, Viola tricolor, Veronica persica und Alopecurus myosuroides im Vorauflaufverfahren bei einer Aufwandmenge von 0.32 kg und weniger Aktivsubstanz pro Hektar.
Einige Substanzen schonen darüber hinaus auch einkeimblättrige und zweikeimblättrige Kulturen wie Weizen und Raps. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen.

Folgende Ergebnisse wurden mit den Verbindungen der allgemeinen Formel (Ia) im Vorauflauf erreicht:

| Verbindung | Aufwandmenge | TRZAS | ECHCG | LOLMU | SETVI | VIOTR |
|---|---|---|---|---|---|---|
| 185 (S-Konfig.) | 80 g Wirkstoff/ha | 10 | 100 | 90 | 100 | 70 |
| | 20 g Wirkstoff/ha | 0 | 90 | 70 | 70 | 60 |
| Racemat aus 185 (S-Konfig.) und 1375 (R-Konfig.) | 80 g Wirkstoff/ha | 50 | 90 | 40 | 80 | 0 |
| | 20 g Wirkstoff/ha | 50 | 50 | 40 | 30 | 0 |
| 1375 (R-Konfig.) | 80 g Wirkstoff/ha | 0 | 80 | 60 | 20 | 80 |
| | 20 g Wirkstoff/ha | 0 | 0 | 0 | 0 | 60 |

Aus vorstehender Tabelle lässt sich ableiten, dass die (S)- und (R)-Stereoisomeren der erfindungsgemäßen Verbindugen der allgemeinen Formel (Ia) bei den untersuchten Ungräsern eine verbesserte herbizide Wirkung als das racemische Gemisch zeigen. Gleichzeitig findet man eine überraschend hohe Kulturverträglichkeit in Weizen bei den (S)- und (R)-Stereoisomeren. Insbesondere der gleichzeitig auftretende Effekt einer gesteigerten Wirksamkeit gegenüber Ungräsern und eine bessere Verträglichkeit gegenüber spezifischen Kulturen ist ausgehend von dem Stand der Technik für die einzelnen Stereoisomeren nicht zu erwarten gewesen.

Folgende Ergebnisse wurden mit den Verbindungen der allgemeinen Formel (Ib) im Vorauflauf erreicht:

| Verbindung | Aufwandmenge | BRSNW | ALOMY | LOLMU | SETVI |
|---|---|---|---|---|---|
| 2637 (S-Konfig.) | 80 g Wirkstoff/ha | 0 | 90 | 100 | 90 |
| | 20 g Wirkstoff/ha | 0 | 60 | 10 | 80 |
| Racemat aus 2637 (S-Konfig.) und 3725 (R-Konfig.) | 80 g Wirkstoff/ha | 0 | 90 | 80 | 80 |
| | 20 g Wirkstoff/ha | 0 | 30 | 0 | 0 |
| 3725 (R-Konfig.) | 80 g Wirkstoff/ha | 0 | 20 | 0 | 0 |
| | 20 g Wirkstoff/ha | 0 | 0 | 0 | 0 |

Aus vorstehender Tabelle lässt sich ableiten, dass die (S)-Stereoisomeren der erfindungsgemäßen Verbindungen der allgemeinen Formel (I) bei den untersuchten Ungräsern eine verbesserte herbizide Wirkung als das racemische Gemisch zeigen. Gleichzeitig findet man eine überraschend hohe Kulturverträglichkeit in Raps bei den (S)-Stereoisomeren. D.h. bei höherer Aktivität wird zugleich eine höhere Selektivität erreicht.

Folgende Ergebnisse wurden mit den Verbindungen der allgemeinen Formel (Ib) im Nachauflauf erreicht.

| Verbindung | Aufwandmenge | ZEAMX | AVEFA | LOLMU | SETVI | POLCO | STEME |
|---|---|---|---|---|---|---|---|
| 2640 (S-Konfig.) | 80 g Wirkstoff/ha | 0 | 90 | 100 | 100 | 100 | 90 |
| | 20 g Wirkstoff/ha | 0 | 20 | 60 | 100 | 20 | 10 |
| Racemat aus 2640 (S-Konfig.) und 3728 (R-Konfig.) | 80 g Wirkstoff/ha | 0 | 70 | 80 | 100 | 80 | 20 |
| | 20 g Wirkstoff/ha | 0 | 20 | 0 | 20 | 0 | 10 |
| 3728 (R-Konfig.) | 80 g Wirkstoff/ha | 0 | 10 | 0 | 10 | 10 | 10 |
| | 20 g Wirkstoff/ha | 0 | 0 | 0 | 10 | - | 0 |

Aus vorstehender Tabelle lässt sich ableiten, dass die (S)-Stereoisomeren der erfindungsgemäßen Verbindungen der allgemeinen Formel (I) bei den untersuchten Ungräsern eine verbesserte herbizide Wirkung als das racemische Gemisch zeigen. Gleichzeitig findet man eine überraschend hohe Kulturverträglichkeit in Raps bei den (S)-Stereoisomeren. D.h. bei höherer Aktivität wird zugleich eine höhere Selektivität erreicht.

### Abkürzungen:

- ZEAMX:: Zea mays (Mais)
- TRZAS:: Triticum aestivum (Sommerweizen)
- ALOMY:: Alopecurus myosuroides (Ackerfuchsschwanz)
- LOLMU:: Lolium multiflorum (Welsches Weidelgras)
- SETVI:: Setaria viridis (Grüne Borstenhirse)
- BRSNW:: Brassica napus (Winterraps)
- VIOTR:: Viola tricolor (Wildes Stiefmütterchen)
- ECHCG:: Echinochloa crus-galli (Gemeine Hühnerhirse)
- AVEFA:: Avena fatua
- POLCO:: Polygunum convolvulus
- STEME:: Stellaria media

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten erfindungsgemäßen Verbindungen werden dann als wäßrige Suspension mit einer Wasseraufwandmenge von umgerechnet 600 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Wie die Ergebnisse zeigen, weisen erfindungsgemäße Verbindungen eine gute herbizide Nachauflaufwirksamkeit gegen mehrere Ungräser bzw. Unkräuter auf. Beispielsweise haben die Verbindungen Nr. 185, 259, 270, 2555, 2632, 2637, 2719, 1375, 1449, 3643, 3720, 3725, 2640, 3728 und andere Verbindungen aus Tabelle 1-4 sehr gute herbizide Wirkung gegen Schadpflanzen wie beispielweise Avena fatua, Echinochloa crus galli, Lolium multiflorum, Setaria viridis und Alopecurus myosuroides im Nachauflaufverfahren bei einer Aufwandmenge von 0.32 kg und weniger Aktivsubstanz pro Hektar.
Einige Substanzen schonen darüber hinaus auch Gramineen und zweikeimblättrige Kulturen wie Mais und Raps. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen.

## Patentansprüche

1. Optisch aktive Verbindungen der allgemeinen Formel (I) mit S-Konfiguration, deren agrochemisch verträglichen Salze und deren agrochemisch verträgliche quaternierte Stickstoff-Derivate 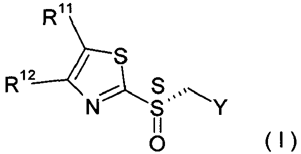 worin
Y entweder 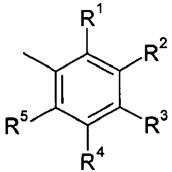 oder 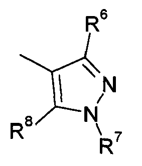 ist und
- die Substituenten R¹ bis R⁸, jeweils unabhängig voneinander, ausgewählt sind aus der Gruppe, bestehend aus
- Wasserstoff, Halogen, Hydroxy, Cyano, Nitro, Amino, C(O)OH, Formyl,
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl, (C₁-C₆)-Haloalkylcarbonyl-(C₁-C₄)-alkyl, (C₁-C₆)-Alkylcarbonyl-(C₁-C₄)-haloalkyl, (C₁-C₆)-Haloalkylcarbonyl-(C₁-C₄)-haloalkyl,
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl,
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl,
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl,
- (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy,
- (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy.
- (C₆-C₁₄)-Aryl, (C₆-C₁₄)-Aryloxy, (C₆-C₁₄)-Arylcarbonyl, (C₆-C₁₄)-Aryloxycarbonyl,
- (C₆-C₁₄)-Aryl-(C₁-C₆)-alkyl, (C₆-C₁₄)-Aryl-(C₁-C₆)-alkoxy, (C₆-C₁₄)-Aryloxy-(C₁-C₆)-alkyl, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy,
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy,
- (C₄-C₁₄)-Arylsulfonyl, (C₆-C₁₄)-Arylthio, (C₆-C₁₄)-Arylsulfinyl,
- Mono-((C₁-C₆)-alkyl)-amino, Mono-((C₁-C₆)-haloalkyl)-amino, Di-((C₁-C₆)-alkyl)-amino, Di-((C₁-C₆)-haloalkyl)-amino, ((C₁-C₆)-Alkyl-(C₁-C₆)-haloalkyl)-amino, N-((C₁-C₆)-Alkanoyl)-amino, N-((C₁-C₆)-Haloalkanoyl)-amino, Aminocarbonyl-C₁-C₆)-alkyl, Mono-(C₁-C₆)-Alkylaminocarbonyl-(C₁-C₆)-alkyl, Di-(C₁-C₆)-Alkylaminocarbonyl-(C₁-C₆)-alkyl, Mono-((C₁-C₆)-alkyl)-aminocarbonyl,
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy,
- (C₃-C₈)-Cycloalkyl, (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy,
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- (C₃-C₈)-Cycloalkylthio, (C₃-C₈)-Alkenylthio, (C₃-C₈)-Cycloalkenylthio, (C₃-C₆)-Alkinylthio,
- Hydroxy-(C₁-C₆)-alkyl, Hydroxy-(C₁-C₆)-alkoxy Cyano-(C₁-C₆)-alkoxy, Cyano-(C₁-C₆)-alkyl,
- 3-Oxetanyloxy-,
- C(O)NR⁹R¹⁰ wobei R⁹ und R¹⁰ unabhängig voneinander Wasserstoff, (C₁-C₆)-Alkyl, (C₃-C₆)-Cycloalkyl, (C₁-C₆)-Haloalkyl, oder wo R⁹ und R¹⁰ zusammen eine (C₁-C₆)-Alkylen-Gruppe bilden, die ein Sauerstoff-, oder Schwefel-Atom oder ein oder zwei Amino oder (C₁-C₆)-Alkylamino-Gruppen enthalten kann,
wobei die genannten Reste gegebenenfalls untereinander zyklisch verknüpft sein können, unter der Voraussetzung, dass sie *ortho*-ständig sind
und/oder
zwei ortho-ständige Substituenten zusammen eine (C₁-C₆)-Alkylengruppe bilden, die ein oder mehrere Sauerstoff- und/oder Schwefelatome enthalten können, wobei die (C₁-C₆)-Alkylengruppe durch Halogen einfach oder mehrfach substituiert sein kann und die jeweilgen Halogensubstituenten gleich oder verschieden sein können;
wobei die zuvor genannten Reste R¹ bis R⁸ einfach oder mehrfach und unabhängig voneinander durch Reste substituiert sein können, welche ausgewählt sind , aus der Gruppe, bestehend aus (C₁-C₆)-Alkyl, (C₁-C₆)-Alkoxy, Nitro, Cyano, (C₁-C₃)-Cycloalkyl, (C₁-C₆)-Haloalkoxy, (C₁-C₆)-Alkylthio oder Halogen, wobei die genannten Reste gegebenenfalls untereinander zyklisch verknüpft sein können, unter der Voraussetzung, dass sie ortho-ständig sind; und
die Substituenten R¹¹ und R¹², jeweils unabhängig voneinander, ausgewählt sind aus der Gruppe, bestehend aus H, F, Cl, Br, I, Me, CHF₂ und CF₃.

2. Verbindung der allgemeinen Formel (I) mit S-Konfiguration nach Anspruch 1, **dadurch gekennzeichnet, dass** Y 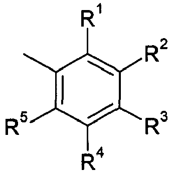 ist und die Substitenten R¹ bis R⁵, unabhängig voneinander, ausgewählt sind aus der Gruppe, bestehend aus Wasserstoff, Hydroxy, Halogen, Cyano, Nitro, Amino, C(O)OH, (C₁-C₄)-Alkyl, (C₃-C₆)-Cycloalkyl, (C₁-C₄)-Haloalkyl, (C₁-C₄)-Alkoxy, (C₁-C₄)-Haloalkoxy, (C₁-C₄)-Alkoxy-(C₁-C₂)-alkyl, (C₁-C₃)-Alkylcarbonyl, (C₁-C₃)-Alkylcarbonyloxy, (C₁-C₄)-Alkoxycarbonyl, (C₃-C₆)-Cycloalkoxycarbonyl, (C₃-C₆)-Cycloalkyl-(C₁-C₂)-alkoxy, (C₃-C₆)-Cycloalkoxy, (C₁-C₄)-Alkoxycarbonyl-(C₁-C₂)-alkoxy, (C₃-C₄)-Alkenyloxy, (C₃-C₄)-Alkinyloxy, (C₁-C₄)-Alkylthio, (C₁-C₄)-Haloalkylthio, (C₁-C₄)-Alkylsulfinyl, (C₁-C₄)-Haloalkylsulfinyl, (C₁-C₄)-Alkylsulfonyl, (C₁-C₄)-Haloalkylsulfonyl, (C₁-C₄)-Alkylsulfonyloxy, Di-(C₁-C₄)-Alkylamino, C₆-Aryl-(C₁-C₄)-alkyl, (C₃-C₄)-Alkenyloxycarbonyl, (C₂-C₄)-Alkinyloxycarbonyl, C₆-Aryl-(C₁-C₄)-alkoxycarbonyl, C₆-Aryl-(C₁-C₄)-alkoxy, Formyl, (C₂-C₄)-Alkenyl, (C₂-C₄)-Alkinyl, Phenyl, -C(O)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann.

3. Verbindung der allgemeinen Formel (I) mit S-Konfiguration nach Anspruch 1, **dadurch gekennzeichnet, dass** Y 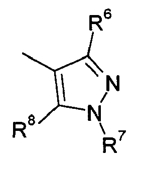 ist und R⁶ und R⁸, unabhängig voneinander, ausgewählt ist aus der Gruppe, bestehend aus Wasserstoff, Halogen, Hydroxy, Cyano, Nitro, Amino, (C₁-C₄)-Alkyl, (C₃-C₆)-Cycloalkyl, (C₁-C₄)-Haloalkyl, (C₁-C₄)-Alkoxy, (C₁-C₄)-Alkoxy-(C₁-C₂)-alkyl, (C₃-C₆)-Cycloalkoxy, (C₁-C₄)-Haloalkoxy, (C₁-C₄)-Alkylthio, (C₁-C₄)-Alkylthio-(C₁-C₂)-alkyl, (C₁-C₄)-Alkylsulfinyl, (C₁-C₄)-Alkylsulfinyl-(C₁-C₂)-alkyl, (C₁-C₄)-Alkylsulfonyl, (C₁-C₄)-Alkylsulfonyl-(C₁-C₂)-alkyl, Di-(C₁-C₄)-Alkylamino, (C₂-C₄)-Alkenyl, (C₂-C₄)-Alkinyl, (C₃-C₄)-Alkenyloxy, (C₃-C₄)-Alkinyloxy, (C₃-C₆)-Cycloalkyl-(C₁-C₂)-alkoxy, Hydroxy-(C₁-C₂)-alkyl, Hydroxy-(C₁-C₂)-alkoxy, Cyano-(C₁-C₂)-alkoxy, Cyano-(C₁-C₂)-alkyl, Phenyl, Phenyl-(C₁-C₂)-alkyl, Phenyl-(C₁-C₂)-alkoxy, Phenoxy, (C₁-C₄)-Alkylcarbonyloxy, (C₃-C₆)-Cycloalkyl-(C₁-C₂)-alkyl, (C₁-C₄)-Alkylcarbonyl-(C₁-C₂)-alkyl, (C₁-C₄)-Alkoxycarbonyl-(C₁-C₂)-alkyl, Aminocarbonyl-(C₁-C₂)-alkyl und 3-Oxetanyloxy-,C(0)NR⁹R¹⁰, wobei R⁹ und R¹⁰, unabhängig voneinander, ausgewählt sind aus der Gruppe, bestehend aus Wasserstoff, (C₁-C₆)-Alkyl, (C₃-C₆)-Cycloalkyl, (C₁-C₆)-Haloalkyl, oder worin R⁹ und R¹⁰ zusammen eine (C₁-C₆)-Alkylen-Gruppe bilden, die ein Sauerstoff- oder Schwefel-Atom oder ein oder zwei Amino- oder (C₁-C₆)-Alkylamino-Gruppen enthalten kann.

4. Verbindung der allgemeinen Formel (I) mit S-Konfiguration nach Anspruch 1 oder 3, **dadurch gekennzeichnet, dass** Y 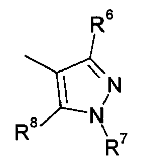 ist und R⁷ ausgewählt ist aus der Gruppe, bestehend aus Wasserstoff, (C₁-C₄)-Alkyl, (C₁-C₄)-Haloalkyl, Phenyl, Phenyl-(C₁-C₂)-alkyl, (C₃-C₆)-Cycloalkyl; (C₃-C₆)-Cycloalkyl-(C₁-C₂)-alkyl, wobei der Cycloalkylrest gegebenenfalls mit (C₁-C₄)-Alkyl substituiert ist; (C₂-C₄)-Alkenyl, (C₂-C₄)-Alkinyl, (C₁-C₄)-Alkoxy-(C₁-C₂)-alkyl, (C₁-C₄)-Alkylthio-(C₁-C₂)-alkyl, (C₁-C₄)-Alkylsulfinyl-(C₁-C₂)-alkyl, Cyano-(C₁-C₂)-alkyl, (C₁-C₄)-Alkylsulfonyl-(C₁-C₂)-alkyl, (C₁-C₄)-Alkoxycarbonyl-(C₁-C₂)-alkyl, Aminocarbonyl-(C₁-C₂)-alkyl, Mono-(C₁-C₆)-Alkylaminocarbonyl-(C₁-C₆)-alkyl, Di-(C₁-C₄)-Alkylaminocarbonyl-(C₁-C₂)-alkyl, Hydroxy-(C₁-C₄)-alkyl, (C₁-C₄)-Alkylcarbonyl-(C₁-C₄)-alkyl, (C₁-C₄)-Alkoxycarbonyl-(C₁-C₂)-alkyl, (C₁-C₄)-Alkylsulfonyl; Phenylsulfonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; (C₁-C₄)-Alkylcarbonyl; Phenylcarbonyl, das gegebenenfalls durch einen oder mehrere, gleiche oder verschiedene Reste aus der Gruppe Halogen, Nitro, Cyano, (C₁-C₆)-Alkyl, (C₁-C₆)-Haloalkyl, (C₃-C₆)-Cycloalkyl, (C₁-C₆)-Alkoxy, (C₁-C₆)-Haloalkoxy oder (C₁-C₆)-Alkylthio substituiert ist; und (C₁-C₄)-Alkoxycarbonyl.

5. Verbindungen der allgemeinen Formel (I) mit S-Konfiguration nach einem der Ansprüche 1 bis 4, **dadurch gekennzeichnet, dass** R¹¹ und R¹², unabhängig voneinander, ausgewählt sind aus der Gruppe bestehend aus F, Cl, Br, und I.

6. Verfahren zur Herstellung von einer Verbindung der allgemeinen Formel (I) mit S-Konfiguration gemäß einem der Ansprüche 1 bis 5, **dadurch gekennzeichnet, dass** ein Thioether der allgemeinen Formel (II), 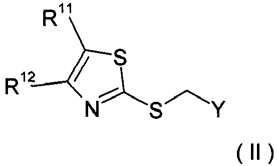 worin Y die gemäß der allgemeinen Formel (I) mit S-Konfiguration in einem der Ansprüche 1 bis 5 definierte Bedeutung aufweist, mit einem Äquivalent eines Oxidationsmittels zu den Sulfoxiden der allgemeinen Formel (I) oxidiert wird.

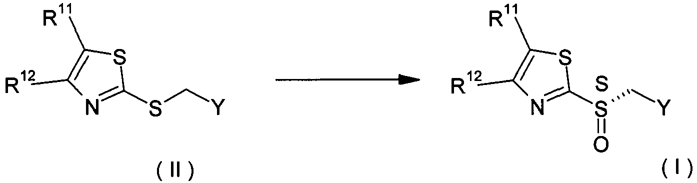

7. Verfahren nach Anspruch 6, **dadurch gekennzeichnet, dass** das Oxidationsmittel ausgewählt ist aus der Gruppe, bestehend aus Wasserstoffperoxid, Natriummetaperjodat, organische Peroxiden und organischen Persäuren.

8. Zusammensetzungen, enthaltend mindestens eine Verbindung der allgemeinen Formel (I) mit S-Konfiguration gemäß einem der Ansprüche 1 bis 5.

9. Zusammensetzung nach Anspruch 8, **dadurch gekennzeichnet, dass** die Zusammensetzung mindestens einen weiteren Wirkstoff umfasst, welcher ausgewählt ist aus der Gruppe, bestehend aus mindestens einem weiteren Herbizid und mindestens einem Safener.

10. Verwendung der Verbindungen der allgemeinen Formel (I) mit S-Konfiguration gemäß einem der Ansprüche 1 bis 5 als Pflanzenwachstumsregulatoren.

11. Verwendung der Zusammensetzungen gemäß einem Ansprüche 8 oder 9 als Pflanzenwachstumsregulatoren.

12. Verwendung nach Anspruch 10 oder 11 zur Pflanzenbekämpfung in speziellen Pflanzenkulturen oder als Pflanzenschutzregulator.

## Claims

1. An optically active compound of the formula (I) having S configuration or an agrochemically acceptable salt or an agrochemically acceptable quaternized nitrogen derivative thereof 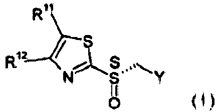 in which
Y is either 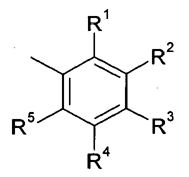 or 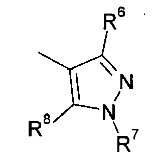 and
- the substituents R¹ to R⁸ are each independently of one another selected from the group consisting of
- hydrogen, halogen, hydroxyl, cyano, nitro, amino, C(O)OH, formyl,
- (C₁-C₆)-alkyl, (C₁-C₆)-haloalkyl, (C₁-C₆)-alkylcarbonyl, (C₁-C₆)-haloalkylcarbonyl, (C₁-C₆) -alkylcarbonyloxy, (C₁-C₆)-haloalkylcarbonyloxy, (C₁-C₆)-alkylcarbonyl-(C₁-C₄)-alkyl, (C₁-C₆) -haloalkylcarbonyl-(C₁-C₄) -alkyl, (C₁-C₆) -alkylcarbonyl- (C₁-C₄)-haloalkyl, (C₁-C₆) -haloalkylcarbonyl- (C₁-C₄)-haloalkyl,
- (C₁-C₆) -alkoxy, (C₁-C₆) -haloalkoxy, (C₁-C₆) alkoxycarbonyl, (C₁-C₆)-haloalkoxycarbonyl, (C₁-C₆)-alkoxycarbonyl-(C₁-C₆)-alkyl, (C₁-C₆)-haloalkoxycarbonyl-(C₁-C₆) -alkyl, (C₁-C₆)-alkoxycarbonyl-(C₁-C₆)-haloalkyl, (C₁-C₆)-haloalkoxycarbonyl-(C₁-C₆)-haloalkyl,
- (C₂-C₆)-alkenyl, (C₂-C₆)-haloalkenyl, (C₂-C₆)-alkenylcarbonyl, (C₂-C₆)-haloalkenylcarbonyl, (C₂-C₆)-alkenyloxy, (C₂-C₆)-haloalkenyloxy, (C₂-C₆)-alkenyloxycarbonyl, (C₂-C₆)-haloalkenyloxycarbonyl,
- (C₂-C₆) -alkynyl, (C₂-C₆) -haloalkynyl, (C₂-C₆)-alkynylcarbonyl, (C₂-C₆)-haloalkynylcarbonyl, (C₂-C₆) -alkynyloxy, (C₂-C₆) -haloalkynyloxy, (C₂-C₆)-alkynyloxycarbonyl, (C₂-C₆)-haloalkynyloxycarbonyl,
- (C₁-C₆) -alkylthiocarbonyl, (C₁-C₆)-haloalkylthiocarbonyl, (C₁-C₆)-alkylthiocarbonyloxy, (C₁-C₆)-haloalkylthio-carbonyloxy,
- (C₁-C₆)-alkylthio-(C₁-C₆)-alkoxy, (C₁-C₆)-alkylthio- (C₁-C₆) -alkylcarbonyl, (C₁-C₆) -alkylthio-(C₁-C₆)-alkylcarbonyloxy,
- (C₆-C₁₄)-aryl, (C₆-C₁₄) -aryloxy, (C₆-C₁₄)-arylcarbonyl, (C₆-C₁₄) -aryloxycarbonyl,
- (C₆-C₁₄)-aryl-(C₁-C₆)-alkyl, (C₆-C₁₄) -aryl- (C₁-C₆) -alkoxy, (C₆-C₁₄) -aryloxy- (C₁-C₆) -alkyl, (C₆-C₁₄) -aryl- (C₁-C₆) -alkyl-carbonyl, (C₆-C₁₄) -aryl-(C₁-C₆) -alkyl-carbonyloxy, (C₆-C₁₄) -aryl- (C₁-C₆) -alkoxycarbonyl, (C₆-C₁₄) -aryl- (C₁-C₆)-alkoxycarbonyloxy,
- (C₁-C₆) -alkylsulfonyl, (C₁-C₆) -alkylthio, (C₁-C₆)-alkylsulfinyl, (C₁-C₆)-haloalkylsulfonyl, (C₁-C₆) -haloalkylthio, (C₁-C₆)-haloalkylsulfinyl, (C₁-C₆) -alkylsulfonyl- (C₁-C₆) -alkyl, (C₁-C₆) -alkylthio- (C₁-C₆) -alkyl, (C₁-C₆) -alkylsulfinyl- (C₁-C₆) -alkyl, (C₁-C₆)-haloalkylsulfonyl- (C₁-C₆) -alkyl, (C₁-C₆)-haloalkylthio- (C₁-C₆) -alkyl, (C₁-C₆)-haloalkylsulfinyl- (C₁-C₆) -alkyl, (C₁-C₆)-alkylsulfonyl- (C₁-C₆) -haloalkyl, (C₁-C₆)-alkylthio- (C₁-C₆) -haloalkyl, (C₁-C₆)-alkylsulfinyl- (C₁-C₆) -haloalkyl, (C₁-C₆)-haloalkylsulfonyl- (C₁-C₆) -haloalkyl, (C₁-C₆)-haloalkylthio- (C₁-C₆) -haloalkyl, (C₁-C₆)-haloalkylsulfinyl- (C₁-C₆) -haloalkyl, (C₁-C₆)-alkylsulfonyloxy, (C₁-C₆)-haloalkylsulfonyloxy,
- (C₄-C₁₄) -arylsulfonyl, (C₆-C₁₄)-arylthio, (C₆-C₁₄) -arylsulfinyl,
- mono- ((C₁-C₆) -alkyl) -amino, mono- ((C₁-C₆)-haloalkyl)-amino, di- ((C₁-C₆) -alkyl) -amino, di-((C₁-C₆)-haloalkyl)-amino, ((C₁-C₆)-alkyl-(C₁-C₆)-haloalkyl)-amino, N-((C₁-C₆)-alkanoyl)-amino, N-((C₁-C₆)-haloalkanoyl)-amino, aminocarbonyl-(C₁-C₆)-alkyl, mono-(C₁-C₆)-alkylaminocarbonyl-(C₁-C₆)-alkyl, di-(C₁-C₆)-alkylaminocarbonyl-(C₁-C₆)-alkyl, mono-((C₁-C₆)-alkyl)-aminocarbonyl,
- (C₁-C₆)-alkoxy-(C₁-C₆)-alkyl, (C₁-C₆)-alkoxy-(C₁-C₆)-alkoxy, (C₁-C₆)-alkoxycarbonyl-(C₁-C₆)-alkoxy,
- (C₃-C₈)-cycloalkyl, (C₃-C₈)-cycloalkoxy, (C₃-C₈)-cycloalkyl-(C₁-C₆) -alkyl, (C₃-C₈)-cycloalkyl-(C₁-C₆)-haloalkyl, (C₃-C₈)-cycloalkyl-(C₁-C₆)-alkoxy, (C₃-C₈)-cycloalkyl-(C₁-C₆)-haloalkoxy, (C₃-C₈)-cycloalkylcarbonyl, (C₃-C₈)-cycloalkoxycarbonyl, (C₃-C₈)-cycloalkyl-(C₁-C₆)-alkylcarbonyl, (C₃-C₈)-cycloalkyl-(C₁-C₆)-haloalkylcarbonyl, (C₃-C₈)-cycloalkyl-(C₁-C₆)-alkoxycarbonyl, (C₃-C₈)- cycloalkyl-(C₁-C₆)-haloalkoxycarbonyl, (C₃-C₈)- cycloalkylcarbonyloxy, (C₃-C₈)- cycloalkoxycarbonyloxy, (C₃-C₈)-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl-(C₁-C₆)-alkoxycarbonyloxy, (C₃-C₈)-cycloalkyl-(C₁-C₆)-haloalkoxycarbonyloxy,
- (C₃-C₈)-cycloalkenyl, (C₃-C₈)-cycloalkenyloxy, (C₃-C₈)-cycloalkenyl- (C₁-C₆)-alkyl, (C₃-C₈)-cycloalkenyl-(C₁-C₆)-haloalkyl, (C₃-C₈)-cycloalkenyl-(C₁-C₆)-alkoxy, (C₃-C₈)-cycloalkenyl-(C₁-C₆)-haloalkoxy, (C₃-C₈)-cyclo-alkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl- (C₁-C₆)-haloalkylcarbonyl, (C₃-C₈)-cycloalkenyl-(C₁-C₆)-alkoxycarbonyl, (C₃-C₈)-cycloalkenyl-(C₁-C₆)-haloalkoxycarbonyl, (C₃-C₈)-cycloalkenylcarbonyloxy, (C₃-C₈)-cycloalkenyl-oxycarbonyloxy, (C₃-C₈) -cycloalkenyl-(C₁-C₆) - alkylcarbonyloxy, (C₃-C₈)-cycloalkenyl-(C₁-C₆)-haloalkylcarbonyloxy, (C₃-C₈)-cycloalkenyl-(C₁-C₆) -alkoxycarbonyloxy, (C₃-C₈)-cycloalkenyl-(C₁-C₆)-haloalkoxycarbonyloxy,
- (C₃-C₈) -cycloalkylthio, (C₃-C₈) -alkenylthio, (C₃-C₈) -cycloalkenylthio, (C₃-C₆) -alkynylthio,
- hydroxy-(C₁-C₆)-alkyl, hydroxy- (C₁-C₆) -alkoxy, cyano-(C₁-C₆)-alkoxy, cyano-(C₁-C₆)-alkyl,
- 3-oxetanyloxy,
- C(O)NR⁹R¹⁰ where R⁹ and R¹⁰ independently of one another are hydrogen, (C₁-C₆)-alkyl, (C₃-C₆)-cycloalkyl, (C₁-C₆)-haloalkyl, or where R⁹ and R¹⁰ together form a (C₁-C₆) -alkylene group which may contain one oxygen or sulfur atom or one or two amino or (C₁-C₆)-alkylamino groups, where the radicals mentioned may, if appropriate, be attached cyclically to one another, provided they are *ortho* to one another
and/or
two substituents ortho to one another together form a (C₁-C₆)-alkylene group which may contain one or more oxygen and/or sulfur atoms, where the (C₁-C₆)-alkylene group may be mono- or polysubstituted by halogen and the halogen substituents in question may be identical or different;
where the radicals R¹ to R⁸ mentioned above may be mono- or polysubstituted independently of one another by radicals selected from the group consisting of (C₁-C₆) -alkyl, (C₁-C₆) -alkoxy, nitro, cyano, (C₁-C₃) -cycloalkyl, (C₁-C₆)-haloalkoxy, (C₁-C₆)-alkylthio and halogen, where the radicals mentioned may optionally be cyclically attached to one another, provided they are ortho to each other; and
the substituents R¹¹ and R¹², in each case independently of one another, are selected from the group consisting of H, F, Cl, Br, I, Me, CHF₂ and CF₃.

2. The compound of the formula (I) having S configuration as claimed in claim 1 wherein Y is 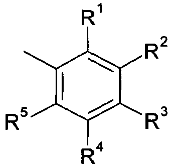 and the substituents R¹ to R⁵ independently of one another are selected from the group consisting of hydrogen, hydroxyl, halogen, cyano, nitro, amino, C(O)OH, (C₁-C₄)-alkyl, (C₃-C₆)-cycloalkyl, (C₁-C₄)-haloalkyl, (C₁-C₄)-alkoxy, (C₁-C₄)-haloalkoxy, (C₁-C₄) -alkoxy- (C₁-C₂)-alkyl, (C₁-C₃)-alkylcarbonyl, (C₁-C₃)-alkylcarbonyloxy, (C₁-C₄)-alkoxycarbonyl, (C₃-C₆) -cycloalkoxycarbonyl, (C₃-C₆) -cycloalkyl- (C₁-C₂)-alkoxy, (C₃-C₆) -cycloalkoxy, (C₁-C₄)- alkoxycarbonyl- (C₁-C₂) -alkoxy, (C₃-C₄) -alkenyloxy, (C₃-C₄)-alkynyloxy, (C₁-C₄) -alkylthio, (C₁-C₄) -halo-alkylthio, (C₁-C₄) -alkylsulfinyl, (C₁-C₄)-haloalkylsulfinyl, (C₁-C₄) -alkylsulfonyl, (C₁-C₄)-haloalkylsulfonyl, (C₁-C₄) -alkylsulfonyloxy, di-(C₁-C₄)-alkylamino, C₆-aryl-(C₁-C₄) -alkyl, (C₃-C₄)-alkenyloxycarbonyl, (C₂-C₄)-alkynyloxycarbonyl, C₆-aryl- (C₁-C₄) -alkoxycarbonyl, C₆-aryl-(C₁-C₄)-alkoxy, formyl, (C₂-C₄) -alkenyl, (C₂-C₄) -alkynyl, phenyl, - C(O)NR⁹R¹⁰, where R⁹ and R¹⁰ independently of one another are selected from the group consisting of hydrogen, (C₁-C₆) -alkyl, (C₃-C₆) -cycloalkyl, (C₁-C₆)-haloalkyl, or where R⁹ and R¹⁰ together form a (C₁-C₆)-alkylene group which may contain one oxygen or sulfur atom or one or two amino or (C₁-C₆)-alkylamino groups.

3. The compound of the formula (I) having S configuration as claimed in claim 1 wherein Y is 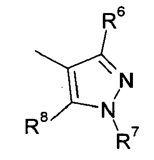 and R⁶ and R⁸ independently of one another are selected from the group consisting of hydrogen, halogen, hydroxyl, cyano, nitro, amino, (C₁-C₄) - alkyl, (C₃-C₆)-cycloalkyl, (C₁-C₄) -haloalkyl, (C₁-C₄)-alkoxy, (C₁-C₄) -alkoxy- (C₁-C₂) -alkyl, (C₃-C₆)-cycloalkoxy, (C₁-C₄) -haloalkoxy, (C₁-C₄) -alkylthio, (C₁-C₄) -alkylthio- (C₁-C₂) -alkyl, (C₁-C₄) -alkylsulfinyl, (C₁-C₄) -alkylsulfinyl- (C₁-C₂) -alkyl, (C₁-C₄) -alkylsulfonyl, (C₁-C₄)-alkylsulfonyl- (C₁-C₂)-alkyl, di-(C₁-C₄)-alkylamino, (C₂-C₄)-alkenyl, (C₂-C₄)-alkynyl, (C₃-C₄)-alkenyloxy, (C₃-C₄)-alkynyloxy, (C₃-C₆) -cycloalkyl- (C₁-C₂) -alkoxy, hydroxy- (C₁-C₂) -alkyl, hydroxy-(C₁-C₂)-alkoxy, cyano-(C₁-C₂)-alkoxy, cyano-(C₁-C₂)-alkyl, phenyl, phenyl- (C₁-C₂) -alkyl, phenyl- (C₁-C₂) -alkoxy, phenoxy, (C₁-C₄)-alkylcarbonyloxy, (C₃-C₆)-cycloalkyl- (C₁-C₂) -alkyl, (C₁-C₄) -alkylcarbonyl- (C₁-C₂)-alkyl, (C₁-C₄) -alkoxycarbonyl- (C₁-C₂)-alkyl, aminocarbonyl-(C₁-C₂)-alkyl and 3-oxetanyloxy, - C(O)NR⁹R¹⁰, where R⁹ and R¹⁰ independently of one another are selected from the group consisting of hydrogen, (C₁-C₆) -alkyl, (C₃-C₆)-cycloalkyl, (C₁-C₆)-haloalkyl, or where R⁹ and R¹⁰ together form a (C₁-C₆)-alkylene group which may contain one oxygen or sulfur atom or one or two amino or (C₁-C₆)-alkylamino groups.

4. The compound of the formula (I) having S configuration as claimed in claim 1 or 3 wherein Y is 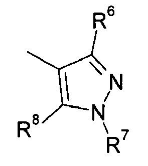 and R⁷ is selected from the group consisting of hydrogen, (C₁-C₄) -alkyl, (C₁-C₄)-haloalkyl, phenyl, phenyl-(C₁-C₂)-alkyl, (C₃-C₆) -cycloalkyl; (C₃-C₆)-cycloalkyl-(C₁-C₂)-alkyl, where the cycloalkyl radical is optionally substituted by (C₁-C₄)-alkyl; (C₂-C₄)-alkenyl, (C₂-C₄)-alkynyl, (C₁-C₄) -alkoxy- (C₁-C₂)-alkyl, (C₁-C₄)-alkylthio-(C₁-C₂)-alkyl, (C₁-C₄)-alkylsulfinyl- (C₁-C₂) -alkyl, cyano-(C₁-C₂)-alkyl, (C₁-C₄)-alkylsulfonyl-(C₁-C₂)-alkyl, (C₁-C₄)-alkoxy-carbonyl- (C₁-C₂) -alkyl, aminocarbonyl-(C₁-C₂)-alkyl, mono- (C₁-C₆) -alkylaminocarbonyl- (C₁-C₆) -alkyl, di-(C₁-C₄) -alkylaminocarbonyl- (C₁-C₂) -alkyl, hydroxy-(C₁-C₄) -alkyl, (C₁-C₄) -alkylcarbonyl- (C₁-C₄) -alkyl, (C₁-C₄) -alkoxycarbonyl- (C₁-C₂)-alkyl, (C₁-C₄) -alkylsulfonyl; phenylsulfonyl which is optionally substituted by one or more identical or different radicals from the group consisting of halogen, nitro, cyano, (C₁-C₆) -alkyl, (C₁-C₆) -haloalkyl, (C₃-C₆) -cycloalkyl, (C₁-C₆) -alkoxy, (C₁-C₆) -haloalkoxy and (C₁-C₆) -alkylthio; (C₁-C₄)-alkylcarbonyl; phenylcarbonyl which is optionally substituted by one or more identical or different radicals from the group consisting of halogen, nitro, cyano, (C₁-C₆)-alkyl, (C₁-C₆)-haloalkyl, (C₃-C₆)-cycloalkyl, (C₁-C₆) -alkoxy, (C₁-C₆) -haloalkoxy and (C₁-C₆)-alkylthio; and (C₁-C₄) -alkoxycarbonyl.

5. The compound of the formula (I) having S configuration as claimed in any of claims 1 to 4 wherein R¹¹ and R¹² independently of one another are selected from the group consisting of F, Cl, Br and I.

6. A process for preparing a compound of the formula (I) having S configuration as claimed in any of claims 1 to 5, which comprises oxidizing a thioether of the formula (II) 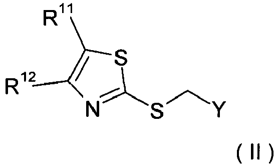 in which Y has the meaning defined according to formula (I) having S configuration in any of claims 1 to 5 with one equivalent of an oxidizing agent to give a sulfoxide of the formula (I).

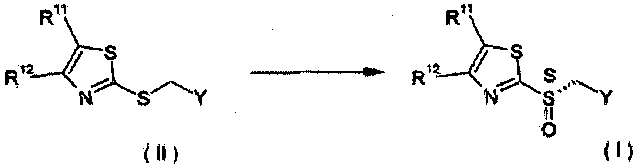

7. The process as claimed in claim 6 wherein the oxidizing agent is selected from the group consisting of hydrogen peroxide, sodium metaperiodate, organic peroxides and organic peracids.

8. A composition comprising at least one compound of the formula (I) having S configuration as claimed in any of claims 1 to 5.

9. The composition as claimed in claim 8 wherein the composition comprises at least one further active compound selected from the group consisting of at least one further herbicide and at least one safener.

10. The use of a compound of the formula (I) having S configuration as claimed in any of claims 1 to 5 as plant growth regulator.

11. The use of a composition as claimed in claim 8 or 9 as plant growth regulator.

12. The use as claimed in claim 10 or 11 for controlling plants in specific plant crops or as plant protection regulator.

## Revendications

1. Composés optiquement actifs de formule générale (I) à configuration S, leurs sels agrochimiquement acceptables et leurs dérivés sur l'atome d'azote quaternisé, agrochimiquement acceptables 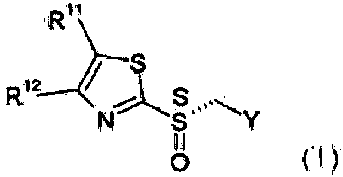 formule dans laquelle
Y est soit 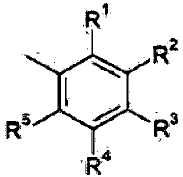 soit 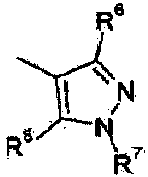 et
- les substituants R¹ à R⁸ sont choisis chacun, indépendamment les uns des autres, dans le groupe constitué par
- un atome d'hydrogène, un atome d'halogène, un groupe hydroxy, cyano, nitro, amino, C(O)OH, formyle,
- alkyle en C₁-C₆, halogénoalkyle(C₁-C₆), alkyl (C₁-C₆) carbonyle, halogénoalkyl (C₁-C₆)-carbonyle, alkyl (C₁-C₆) carbonyloxy, halogéno-alkyl (C₁-C₆) carbonyloxy, alkyl (C₁-C₆) carbonyl-alkyle(C₁-C₄), halogénoalkyl(C₁-C₆)carbonyl-alkyle(C₁-C₄), alkyl(C₁-C₆)carbonyl-halogéno-alkyle (C₁-C₄), halogénoalkyl (C₁-C₆) carbonyl-halogénoalkyle (C₁-C₄),
- alcoxy(C₁-C₆), halogénoalcoxy(C₁-C₆), alcoxy(C₁-C₆)-carbonyle, halogénoalcoxy (C₁-C₆) carbonyle, alcoxy(C₁-C₆)carbonyl-alkyle(C₁-C₆), halogéno-alcoxy (C₁-C₆) carbonyl-alkyle (C₁-C₆) , alcoxy (C₁-C₆)-carbonyl-halogénoalkyle(C₁-C₆), halogénoalcoxy (C₁-C₆) carbonyl-halogénoalkyle (C₁-C₆),
- alcényle en C₂-C₆, halogénoalcényle(C₂-C₆), alcényl(C₂-C₆)carbonyle, halogénoalcényl(C₂-C₆)-carbonyle, alcényloxy en C₂-C₆, halogéno-alcényloxy(C₂-C₆), alcényloxy(C₂-C₆)carbonyle, halogénoalcényloxy(C₂-C₆)carbonyle,
- alcynyle en C₂-C₆, halogénoalcynyle(C₂-C₆), alcynyl(C₂-C₆)carbonyle, halogénoalcynyl(C₂-C₆)-carbonyle, alcynyloxy en C₂-C₆, halogéno-alcynyloxy(C₂-C₆), alcynyloxy(C₂-C₆)carbonyle, halogénoalcynyloxy (C₂-C₆) carbonyle,
- alkyl (C₁-C₆) thiocarbonyle, halogénoalkyl(C₁-C₆)-thiocarbonyle, alkyl(C₁-C₆)thiocarbonyloxy, halogénoalkyl (C₁-C₆) thiocarbonyloxy,
- alkyl (C₁-C₆) thio-alcoxy (C₁-C₆), alkyl (C₁-C₆) thio-alkyl (C₁-C₆) carbonyle, alkyl(C₁-C₆)thio-alkyl(C₁-C₆)carbonyloxy,
- aryle en C₆-C₁₄, aryloxy en C₆-C₁₄, aryl(C₆-C₁₄)-carbonyle, aryloxy (C₆-C₁₄) carbonyle,
- aryl (C₆-C₁₄)-alkyle (C₁-C₆), aryl (C₆-C₁₄)-alcoxy (C₁-C₆), aryloxy(C₆-C₁₄)-alkyle(C₁-C₆), aryl (C₆-C₁₄) -alkyl (C₁-C₆) -carbonyle, aryl (C₆-C₁₄)-alkyl (C₁-C₆) -carbonyloxy, aryl(C₆-C₁₄)- alcoxy (C₁-C₆)carbonyle, aryl(C₆-C₁₄)-alcoxy(C₁-C₆) - carbonyloxy,
- alkyl(C₁-C₆)sulfonyle, alkyl(C₁-C₆)thio, alkyl(C₁-C₆)sulfinyle, halogénoalkyl (C₁-C₆)-sulfonyle, halogénoalkyl(C₁-C₆)thio, halogénoalkyl(C₁-C₆)sulfinyle, alkyl(C₁-C₆)sulfonyl-alkyle(C₁-C₆), alkyl(C₁-C₆) thio-alkyle (C₁-C₆), alkyl(C₁-C₆)sulfinyl-alkyle(C₁-C₆), halogéno-alkyl(C₁-C₆)sulfonyl-alkyle(C₁-C₆), halogéno-alkyl(C₁-C₆)thio-alkyle(C₁-C₆), halogéno-alkyl(C₁-C₆)sulfinyl-alkyle(C₁-C₆), alkyl (C₁-C₆)-sulfonyl-halogénoalkyle(C₁-C₆), alkyl(C₁-C₆)thio-halogénoalkyle(C₁-C₆), alkyl (C₁-C₆) sulfinyl-halogénoalkyle(C₁-C₆), halogénoalkyl(C₁-C₆)-sulfonyl-halogénoalkyle(C₁-C₆), halogéno-alkyl(C₁-C₆)thio-halogénoalkyle(C₁-C₆), halogéno-alkyl(C₁-C₆)sulfinyl-halogénoalkyle(C₁-C₆), alkyl(C₁-C₆)sulfonyloxy, halogénoalkyl (C₁-C₆)-sulfonyloxy,
- aryl (C₄-C₁₄) sulfonyle, aryl (C₆-C₁₄) thio, aryl (C₆-C₁₄) sulfinyle,
- mono- (alkyl (C₁-C₆)) -amino, mono-(halogénoalkyl (C₁-C₆))-amino,di-(alkyl(C₁-C₆))-amino, di-(halogénoalkyl(C₁-C₆))-amino, (alkyl(C₁-C₆))-halogénoalkyl (C₁-C₆))-amino, N-(alcanoyl(C₁-C₆))-amino, N-(halogénoalcanoyl(C₁-C₆))-amino, aminocarbonyl-alkyle(C₁-C₆), mono-alkyl(C₁-C₆)-aminocarbonyl-alkyle(C₁-C₆), di-alkyl(C₁-C₆)amino-carbonyl-alkyle(C₁-C₆), mono-(alkyl(C₁-C₆))-aminocarbonyle,
- alcoxy(C₁-C₆)-alkyle(C₁-C₆), alcoxy(C₁-C₆)-alcoxy(C₁-C₆), alcoxy(C₁-C₆)carbonyl-alcoxy(C₁-C₆),
- cycloalkyle en C₃-C₈, cycloalcoxy en C₃-C₈, cycloalkyl(C₃-C₈)-alkyle(C₁-C₆), cycloalkyl (C₃-C₈)-halogénoalkyle (C₁-C₆), cycloalkyl(C₃-C₈)-alcoxy(C₁-C₆), cycloalkyl(C₃-C₈)-halogéno-alcoxy(C₁-C₆), cycloalkyl(C₃-C₈)carbonyle, cycloalcoxy(C₃-C₈)carbonyle, cycloalkyl(C₃-C₈)-alkyl (C₁-C₆) carbonyle, cycloalkyl (C₃-C₈) -halogéno-alkyl (C₁-C₆) carbonyle, cycloalkyl(C₃-C₈)-alcoxy (C₁-C₆) carbonyle, cycloalkyl(C₃-C₈)-halogéno-alcoxy (C₁-C₆) carbonyle, cycloalkyl(C₃-C₈)-carbonyloxy, cycloalcoxy(C₃-C₈)carbonyloxy, cycloalkyl (C₃-C₈)-alkyl(C₁-C₆) carbonyloxy, cycloalkyl (C₃-C₈) -halogénoalkyl (C₁-C₆) carbonyloxy, cycloalkyl(C₃-C₈)-alcoxy(C₁-C₆) carbonyloxy, cycloalkyl(C₃-C₈)-halogénoalcoxy(C₁-C₆) carbonyloxy,
- cycloalcényle en C₃-C₈, cycloalcényloxy en C₃-C₈, cycloalcényl(C₃-C₈)-alkyle(C₁-C₆), cycloalcényl(C₃-C₈)-halogénoalkyle(C₁-C₆), cycloalcényl(C₃-C₈)-alcoxy (C₁-C₆), cycloalcényl(C₃-C₈)-halogénoalcoxy(C₁-C₆), cycloalcényl(C₃-C₈)carbonyle, cycloalcényloxy (C₃-C₈)carbonyle, cycloalcényl (C₃-C₈) -alkyl (C₁-C₆) carbonyle, cycloalcényl(C₃-C₈)-halogénoalkyl (C₁-C₆) carbonyle, cycloalcényl(C₃-C₈)-alcoxy(C₁-C₆)carbonyle, cycloalcényl(C₃-C₈)-halogénoalcoxy(C₁-C₆)carbonyle, cycloalcényl(C₃-C₈)carbonyloxy, cycloalcényloxy(C₃-C₈)carbonyloxy, cycloalcényl(C₃-C₈)-alkyl(C₁-C₆)carbonyloxy, cycloalcényl(C₃-C₈)-halogénoalkyl(C₁-C₆) - carbonyloxy, cycloalcényl(C₃-C₈)-alcoxy(C₁-C₆)-carbonyloxy, cycloalcényl(C₃-C₈)halogéno-alcoxy(C₁-C₆)carbonyloxy ;
- cycloalkyl(C₃-C₈)thio, alcényl(C₃-C₈)thio, cycloalcényl (C₃-C₈) thio, alcynyl(C₃-C₈)thio,
- hydroxy-alkyle(C₁-C₆), hydroxy-alcoxy(C₁-C₆), cyano-alcoxy(C₁-C₆), cyano-alkyle(C₁-C₆),
- 3-oxétanyloxy-,
- C(O)NR⁹R¹⁰, R⁹ et R¹⁰ représentant indépendamment l'un de l'autre un atome d'hydrogène, un groupe alkyle en C₁-C₆, cycloalkyle en C₃-C₆, halogénoalkyle(C₁-C₆), ou R⁹ et R¹⁰ formant ensemble un groupe alkylène en C₁-C₆, qui peut contenir un atome d'oxygène ou de soufre ou un ou deux groupes amino ou alkyl(C₁-C₆)amino,
les radicaux nommés pouvant éventuellement être liés entre eux en un cycle, à la condition qu'ils soient en position *ortho*
et/ou
deux substituants en position ortho formant ensemble un groupe alkylène en C₁-C₆, pouvant contenir un ou plusieurs atomes d'oxygène et/ou de soufre, le groupe alkylène en C₁-C₆ pouvant être une ou plusieurs fois substitué par halogéno et les substituants halogéno respectifs pouvant être identiques ou différents ;
les radicaux R¹ à R⁸ nommés précédemment pouvant être substitués une ou plusieurs fois et indépendamment les uns des autres par des radicaux qui sont choisis dans le groupe constitué par alkyle en C₁-C₆, alcoxy en C₁-C₆, nitro, cyano, cycloalkyle en C₁-C₃, halogénoalcoxy(C₁-C₆), alkyl(C₁-C₆)thio ou halogéno, les radicaux nommés pouvant être liés entre eux en un cycle, à la condition qu'ils soient en position ortho ; et
les substituants R¹¹ et R¹² étant choisis chacun, indépendamment l'un de l'autre, dans le groupe constitué par H, F, Cl, Br, I, Me, CHF₂ et CF₃.

2. Composé de formule générale (I) à configuration S selon la revendication 1, **caractérisé en ce que** Y est 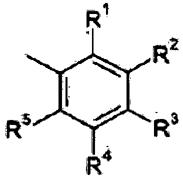 et les substituants R¹ à R⁵, indépendamment les uns des autres, sont choisis dans le groupe constitué par un atome d'hydrogène, un groupe hydroxy, un atome d'halogène, un groupe cyano, nitro, amino, C(O)OH, alkyle en C₁-C₄, cycloalkyle en C₃-C₆, halogéno-alkyle(C₁-C₄), alcoxy en C₁-C₄, halogénoalcoxy(C₁-C₄), alcoxy(C₁-C₄)-alkyle(C₁-C₂), alkyl(C₁-C₃)carbonyle, alkyl(C₁-C₃)-carbonyloxy, alcoxy(C₁-C₄)carbonyle, cycloalcoxy(C₃-C₆)carbonyle, cycloalkyl(C₃-C₆)-alcoxy(C₁-C₂), cycloalcoxy en C₃-C₆, alcoxy(C₁-C₄)-carbonyl-alcoxy(C₁-C₂), alcényloxy en C₃-C₄, alcynyloxy en C₃-C₄, alkyl(C₁-C₄)thio, halogénoalkyl(C₁-C₄)thio, alkyl (C₁-C₄) sulfinyle, halogénoalkyl (C₁-C₄) sulfinyle, alkyl(C₁-C₄)sulfonyle, halogénoalkyl (C₁-C₄) sulfonyle, alkyl(C₁-C₄)sulfonyloxy, di-alkyl (C₁-C₄) -amino, aryl(C₆)-alkyle(C₁-C₄), alcényloxy(C₃-C₄)carbonyle, alcynyloxy(C₂-C₄) carbonyle, aryl(C₆)-alcoxy(C₁-C₄)-carbonyle, aryl(C₆)-alcoxy(C₁-C₄), formyle, alcényle en C₂-C₄, alcynyle en C₂-C₄, phényle, C(O)NR⁹R¹⁰, R⁹ et R¹⁰ étant choisis, indépendamment l'un de l'autre, dans le groupe constitué par un atome d'hydrogène, un groupe alkyle en C₁-C₆, cycloalkyle en C₃-C₆, halogénoalkyle(C₁-C₆), ou R⁹ et R¹⁰ formant ensemble un groupe alkylène en C₁-C₆, qui peut contenir un atome d'oxygène ou de soufre ou un ou deux groupes amino ou alkyl(C₁-C₆)amino.

3. Composé de formule générale (I) à configuration S selon la revendication 1, **caractérisé en ce que** Y est 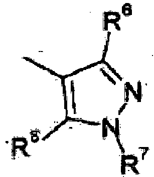 et R⁶ et R⁸, indépendamment l'un de l'autre, sont choisis dans le groupe constitué par un atome d'hydrogène, un atome d'halogène, un groupe hydroxy, cyano, nitro, amino, alkyle en C₁-C₄, cycloalkyle en C₃-C₆, halogénoalkyle(C₁-C₄), alcoxy en C₁-C₄, alcoxy(C₁-C₄)-alkyle(C₁-C₂), cycloalcoxy en C₃-C₆, halogénoalcoxy(C₁-C₄), alkyl(C₁-C₄)thio, alkyl(C₁-C₄)-thio-alkyle(C₁-C₂), alkyl (C₁-C₄) sulfinyle, alkyl(C₁-C₄)-sulfinyl-alkyle(C₁-C₂), alkyl(C₁-C₄)sulfonyle, alkyl(C₁-C₄)sulfonyl-alkyle(C₁-C₂), di-alkyl(C₁-C₄)-amino, alcényle en C₂-C₄, alcynyle en C₂-C₄, alcényloxy en C₃-C₄, alcynyloxy en C₃-C₄, cycloalkyl(C₃-C₆)-alcoxy(C₁-C₂), hydroxy-alkyle(C₁-C₂), hydroxy-alcoxy(C₁-C₂), cyano-alcoxy(C₁-C₂), cyano-alkyle (C₁-C₂), phényle, phényl-alkyle(C₁-C₂), phényl-alcoxy(C₁-C₂), phénoxy, alkyl(C₁-C₄)carbonyloxy, cycloalkyl(C₃-C₆)-alkyle(C₁-C₂), alkyl(C₁-C₄)carbonyl-alkyle (C₁-C₂), alcoxy(C₁-C₄)carbonyl-alkyle(C₁-C₂), aminocarbonyl-alkyle (C₁-C₂) et 3-oxétanyloxy-, -C (O) NR⁹R¹⁰, R⁹ et R¹⁰ étant choisis indépendamment l'un de l'autre dans le groupe constitué par un atome d'hydrogène, un groupe alkyle en C₁-C₆, cycloalkyle en C₃-C₆, halogénoalkyle (C₁-C₆), ou R⁹ et R¹⁰ formant ensemble un groupe alkylène en C₁-C₆, qui peut contenir un atome d'oxygène ou de soufre ou un ou deux groupes amino ou alkyl (C₁-C₆) amino,

4. Composé de formule générale (I) à configuration S selon la revendication 1 ou 3, **caractérisé en ce que** Y est 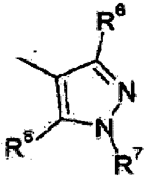 et R⁷ est choisi dans le groupe constitué par un atome d'hydrogène, un groupe alkyle en C₁-C₄, halogéno-alkyle(C₁-C₄), phényle, phényl-alkyle(C₁-C₂), cycloalkyle en C₃-C₆, cycloalkyl (C₃-C₆) -alkyle (C₁-C₂), le radical cycloalkyle étant éventuellement substitué par alkyle en C₁-C₄ ; un groupe alcényle en C₂-C₄, alcynyle en C₂-C₄, alcoxy(C₁-C₄)-alkyle(C₁-C₂), alkyl(C₁-C₄)thio-alkyle (C₁-C₂), alkyl (C₁-C₄) sulfinyl-alkyle (C₁-C₂), cyano-alkyle (C₁-C₂), alkyl (C₁-C₄) sulfonyl-alkyle (C₁-C₂), alcoxy(C₁-C₄)carbonyl-alkyle(C₁-C₂), aminocarbonyl-alkyle(C₁-C₂), mono-alkyl(C₁-C₆)-aminocarbonyl-alkyle(C₁-C₆), di-alkyl(C₁-C₄)aminocarbonyl-alkyle(C₁-C₂), hydroxy-alkyle(C₁-C₄), alkyl(C₁-C₄)-carbonyl-alkyle(C₁-C₄), alcoxy(C₁-C₄)carbonyl-alkyle(C₁-C₂), alkyl(C₁-C₄)sulfonyle ; un groupe phénylsulfonyle, qui est éventuellement substitué par un ou plusieurs radicaux, identiques ou différents, choisis dans le groupe constitué par halogéno, nitro, cyano, alkyle en C₁-C₆, halogénoalkyle(C₁-C₆), cycloalkyle en C₃-C₆, alcoxy en C₁-C₆, halogénoalcoxy(C₁-C₆) ou alkyl(C₁-C₆)thio ; un groupe alkyl(C₁-C₄)carbonyle ; un groupe phénylcarbonyle, qui est éventuellement substitué par un ou plusieurs radicaux, identiques ou différents, choisis dans le groupe constitué par halogéno, nitro, cyano, alkyle en C₁-C₆, halogénoalkyle(C₁-C₆), cycloalkyle(C₃-C₆), alcoxy en C₁-C₆, halogénoalcoxy(C₁-C₆) ou alkyl(C₁-C₆)thio ; et un groupe alcoxy(C₁-C₄)carbonyle.

5. Composés de formule générale (I) à configuration S selon l'une quelconque des revendications 1 à 4, **caractérisés en ce que** R¹¹ et R¹² sont choisis, indépendamment l'un de l'autre, dans le groupe constitué par F, Cl, Br et I.

6. Procédé pour la préparation d'un composé de formule générale (I) à configuration S selon l'une quelconque des revendications 1 à 5, **caractérisé en ce qu'**on oxyde un thioéther de formule générale (II), 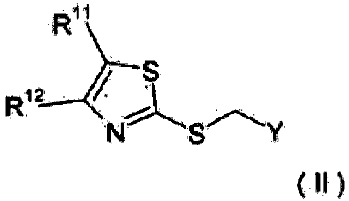 dans laquelle Y a la signification selon la formule générale (I) à configuration S, définie dans l'une quelconque des revendications 1 à 5, à l'aide d'un équivalent d'un oxydant, pour aboutir aux sulfoxydes de formule générale (I)

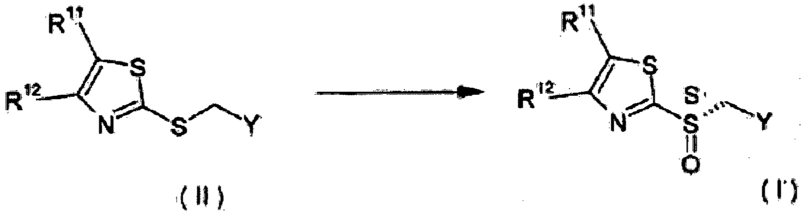

7. Procédé selon la revendication 6, **caractérisé en ce que** l'oxydant est choisi dans le groupe constitué par le peroxyde d'hydrogène, le métaperiodate de sodium, des peroxydes organiques et des peracides organiques.

8. Compositions, contenant au moins un composé de formule générale (I) à configuration S selon l'une quelconque des revendications 1 à 5.

9. Composition selon la revendication 8, **caractérisée en ce que** la composition comprend au moins une autre substance active qui est choisie dans le groupe constitué par au moins un autre herbicide et au moins un phytoprotecteur.

10. Utilisation des composés de formule générale (I) à configuration S selon l'une quelconque des revendications 1 à 5, en tant que régulateurs de croissance végétale.

11. Utilisation des compositions selon l'une quelconque des revendications 8 et 9, en tant que régulateurs de croissance végétale.

12. Utilisation selon la revendication 10 ou 11, pour la lutte contre des plantes dans des cultures de plantes spéciales ou en tant que régulateur phytosanitaire.
